# EUROPÄISCHE PATENTSCHRIFT

(11) **EP 0 842 157 B1**
(45) Veröffentlichungstag und Bekanntmachung des Hinweises auf die Patenterteilung: **27.08.2003**
(21) Anmeldenummer: 96924805.3
(22) Anmeldetag: 28.06.1996
(51) Int. Cl.: C07D 249/12, C07D 271/06, C07D 271/10, C07D 263/34, A01N 47/38, A01N 43/82, A01N 43/74, C07C 309/87, C07C 311/29, C07C 311/65

(54) **HERBIZIDE SULFONYLAMINO(THIO)CARBONYLVERBINDUNGEN**
HERBICIDAL SULPHONYLAMINO(THIO)CARBONYL COMPOUNDS
COMPOSES DE SULFONYLAMINO(THIO)CARBONYLE HERBICIDES

(30) Priorität: 11.07.1995 DE 19525162
(43) Veröffentlichungstag der Anmeldung: 20.05.1998
(60) Teilanmeldung: 03011479.7
(73) Patentinhaber: Bayer CropScience AG, 40789 Monheim (DE)
(72) Erfinder: MÜLLER, Klaus-Helmut, D-40593 Düsseldorf (DE); KIRSTEN, Rolf, D-40789 Monheim (DE); GESING, Ernst, Rudolf, D-40699 Erkrath (DE); KLUTH, Joachim, D-40764 Langenfeld (DE); DREWES, Mark, Wilhelm, D-40764 Langenfeld (DE); FINDEISEN, Kurt, D-51375 Leverkusen (DE); JANSEN, Johannes, Rudolf, D-40789 Monheim (DE); KÖNIG, Klaus, D-51519 Odenthal (DE); RIEBEL, Hans-Jochem, D-42113 Wuppertal (DE); SCHALLNER, Otto, D-40789 Monheim (DE); DOLLINGER, Markus, D-51381 Leverkusen (DE); SANTEL, Hans-Joachim, D-51371 Leverkusen (DE)
(86) Internationale Anmeldenummer: EP9602826
(87) Internationale Veröffentlichungsnummer: WO97003056

(56) Entgegenhaltungen:
- EP-A- 0 425 948
- EP-A- 0 482 349
- EP-A- 0 534 266
- EP-A- 0 708 087
- WO-A-95/27703
- WO-A-96/11188
- DE-A- 4 234 801

## Beschreibung

Die Erfindung betrifft neue Sulfonylamino(thio)carbonylverbindungen, mehrere Verfahren zu ihrer Herstellung sowie ihre Verwendung als Herbizide.

Es ist bereits bekannt, daß bestimmte Sulfonylaminocarbonylverbindungen, wie z.B. die Verbindungen 4,5-Dimethoxy-2-(2-methoxy-phenylsulfonylaminocarbonyl)-2,4-dihydro-3H-1,2,4-triazol-3-on, 4,5-Dimethoxy-2-(2,5-dimethoxy-phenylsulfonylaminocarbonyl)-2,4-dihydro-3H-1,2,4-triazol-3-on, 4,5-Diethoxy-2-(2,5-dimethoxy-phenylsulfanylaminocarbonyl)-2,4-dihydro-3H-1,2,4-triazol-3-on und N-(2,5-Dimethoxyphenylsulfonyl)-1,5-dimethyl-1H-pyrazol-3-carboxamid, herbizide Eigenschaften aufweisen (vgl. EP 341489, EP 422469, EP 425948, EP 431291, EP 507171, EP 534266, DE 4029753). Die Wirkung dieser Verbindungen ist jedoch nicht in allen Belangen zufriedenstellend.

Es wurden nun die neuen Sulfonylamino(thio)carbonylverbindungen der allgemeinen Formel (I), 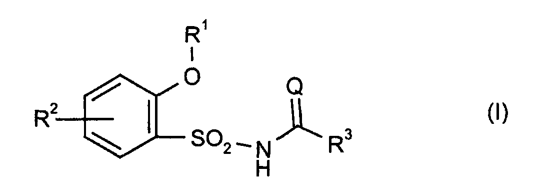 in welcher
- Q: für Sauerstoff oder Schwefel steht,
- R¹: für Wasserstoff, Formyl, für jeweils gegebenenfalls durch Fluor, Chlor, Brom, Methoxy oder Ethoxy substituiertes Methyl, Ethyl, n- oder i-Propyl, n-, i- oder s-Butyl, Propenyl, Butenyl, Propinyl, Butinyl, Acetyl, Propionyl, Butyroyl, Methoxycarbonyl, Ethoxycarbonyl, n- oder i-Propoxycarbonyl, Methylsulfonyl, Ethylsulfonyl, n- oder i-Propylsulfonyl, n-, i-, s- oder t-Butylsulfonyl, oder für jeweils gegebenenfalls durch Fluor, Chlor oder Methyl substituiertes Cyclopropyl, Cyclopropylcarbonyl oder Cyclopropylsulfonyl steht,
- R²: für Cyano, Fluor, Chlor, Brom, oder für jeweils gegebenenfalls durch Fluor, Chlor, Methoxy oder Ethoxy substituiertes Methyl, Ethyl, n- oder i- Propyl, n-, i- oder s-Butyl, Propenyl, Butenyl, Propinyl, Butinyl, Methoxy, Ethoxy, n- oder i-Propoxy, n-, i- oder s-Butoxy, Propenyloxy, Butenyloxy, Propinyloxy oder Butinyloxy steht und
- R³: für jeweils gegebenenfalls substituiertes Heterocyclyl der nachstehenden Formel steht, 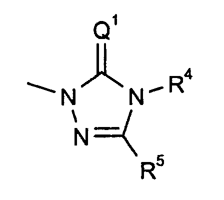
worin
- Q¹: jeweils für Sauerstoff oder Schwefel steht sowie
- R⁴: für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor, Methyl und/oder Ethyl substituiertes Cyclopropyl, Cyclobutyl, Cyclopentyl oder Cyclohexyl steht,
- R⁵: für Wasserstoff, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch
Cyano, Methoxy oder Ethoxy substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl oder Cyclohexyl steht
sowie Salze von Verbindungen der Formel (I).

Gegenstand der Erfindung sind weiter vorzugsweise Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium-, Tri-(C₁-C₄-alkyl)-sulfonium-, C₅- oder C₆-Cycloalkyl-ammonium- und Di-(C₁-C₂-alkyl)-benzyl-ammonium-Salze von Verbindungen der Formel (I), in welcher A, Q, R¹, R² und R³ die oben vorzugsweise angegebenen Bedeutungen haben.

Eine bevorzugte Gruppe erfindungsgemäßer Verbindungen sind die Verbindungen der Formel (I), in welcher
- Q: für Sauerstoff oder Schwefel steht,
- R¹: für Methyl, Ethyl, n- oder i-Propyl steht,
- R²: für Chlor oder Methyl - jeweils in 5- oder 6-Position - steht und
- R³: für gegebenenfalls substituiertes Triazolinyl der nachstehenden Formel steht, 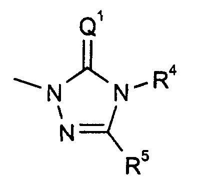
worin
- Q¹: für Sauerstoff oder Schwefel steht sowie
- R⁴: für jeweils gegebenenfalls durch Fluor, Chlor, Cyano, Methoxy oder Ethoxy substituiertes Methyl, Ethyl, n- oder i-Propyl oder für Cyclopropyl steht und
- R⁵: für Wasserstoff, Chlor, Brom, für jeweils gegebenenfalls durch Fluor, Chlor, Cyano, Methoxy oder Ethoxy substituiertes Methyl, Ethyl, n- oder i-Propyl, für jeweils gegebenenfalls durch Cyano, Methoxy oder Ethoxy substituiertes Methoxy, Ethoxy, n- oder i-Propoxy, Methylthio, Ethylthio, n- oder i-Propylthio oder Cyclopropyl steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangsstoffe bzw. Zwischenprodukte. Diese Restedefinitionen können untereinander, also auch zwischen den angegebenen bevorzugten Bereichen beliebig kombiniert werden.

Man erhält die neuen Sulfonylamino(thio)carbonylverbindungen der allgemeinen Formel (I), wenn man
(a) Aminosulfonylverbindungen der allgemeinen Formel (II) 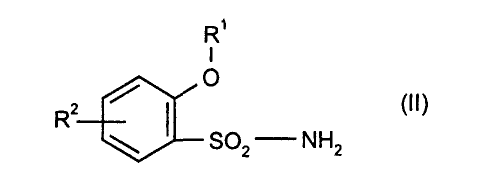 in welcher
   R¹ und R² die oben angegebenen Bedeutungen haben,
   mit (Thio)Carbonsäurederivaten der allgemeinen Formel (III) 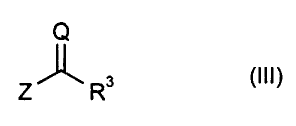 in welcher
   - Q und R³: die oben angegebenen Bedeutungen haben und
   - Z: für Halogen, Alkoxy, Aryloxy oder Arylalkoxy steht,
   gegebenenfalls in Gegenwart eines Säureakzeptors und gegebenenfalls in Gegenwart eines Verdünnungsmittels umsetzt,
   oder wenn man
(b) Sulfonyliso(thio)cyanate der allgemeinen Formel (IV) 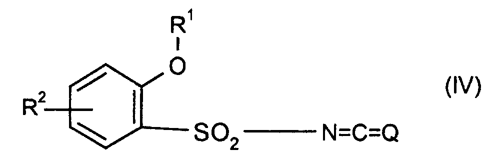 in welcher
   Q, R¹ und R² die oben angegebenen Bedeutungen haben,
   mit Heterocyclen der allgemeinen Formel (V)

   H-R³ (V)

   in welcher
   - R³: die oben angegebene Bedeutung hat,
   gegebenenfalls in Gegenwart eines Reaktionshilfsmittels und gegebenenfalls in Gegenwart eines Verdünnungsmittels umsetzt,
   oder wenn man
(c) Chlorsulfonylverbindungen der allgemeinen Formel (VI) 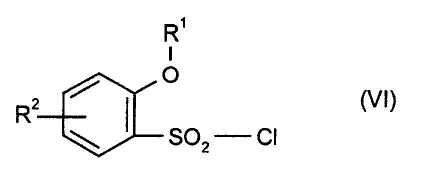 in welcher
   R¹ und R² die oben angegebenen Bedeutungen haben,
   mit Heterocyclen der allgemeinen Formel (V)

   H-R³ (V)

   in welcher
   - R³: die oben angegebene Bedeutung hat,
   und Metall(thio)cyanaten der allgemeinen Formel (VII)

   MQCN (VII)

   in welcher
   - Q: die oben angegebene Bedeutung hat und
   - M: für ein Alkali- oder Erdalkalimetall-Äquivalent steht,
   gegebenenfalls in Gegenwart eines Reaktionshilfsmittels und gegebenenfalls in Gegenwart eines Verdünnungsmittels umsetzt,
   oder wenn man
(d) Chlorsulfonylverbindungen der allgemeinen Formel (VI) 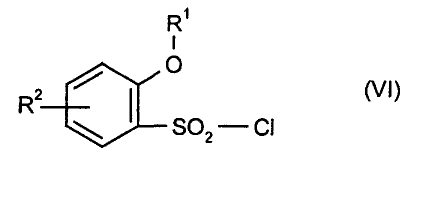 in welcher
   R¹ und R² die oben angegebenen Bedeutungen haben,
   mit (Thio)Carbonsäureamiden der allgemeinen Formel (VIII) 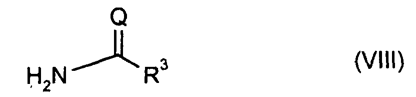 in welcher
   Q und R³ die oben angegebenen Bedeutungen haben,
   gegebenenfalls in Gegenwart eines Säureakzeptors und gegebenenfalls in Gegenwart eines Verdünnungsmittels umsetzt,
   oder wenn man
(e) Sulfonylamino(thio)carbonylverbindungen der allgemeinen Formel (IX) 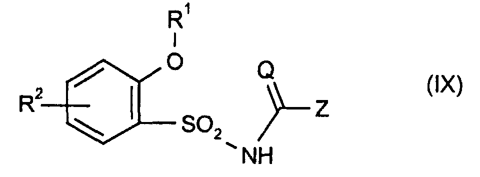 in welcher
   Q, R¹ und R² die oben angegebenen Bedeutungen haben und
   - Z: für Halogen, Alkoxy, Aryloxy oder Arylalkoxy steht,
   mit Heterocyclen der allgemeinen Formel (V)

   H-R³ (V)

   in welcher
   - R³: die oben angegebene Bedeutung hat,
   gegebenenfalls in Gegenwart eines Säureakzeptors und gegebenenfalls in Gegenwart eines Verdünnungsmittels umsetzt,
   und gegebenenfalls die nach den Verfahren (a), (b), (c), (d) oder (e) erhaltenen Verbindungen der Formel (I) nach üblichen Methoden in Salze überführt.

Die neuen Sulfonylamino(thio)carbonylverbindungen der allgemeinen Formel (I) zeichnen sich durch starke herbizide Wirksamkeit aus.

Verwendet man beispielsweise 2-Fluor-6-methoxy-benzolsulfonamid und 5-Ethoxy-4-methyl-2-phenoxycarbonyl-2,4-dihydro-3H-1,2,4-triazol-3-thion als Ausgangsstoffe, so kann der Reaktionsablauf beim erfindungsgemäßen Verfahren (a) durch das folgende Formelschema skizziert werden:

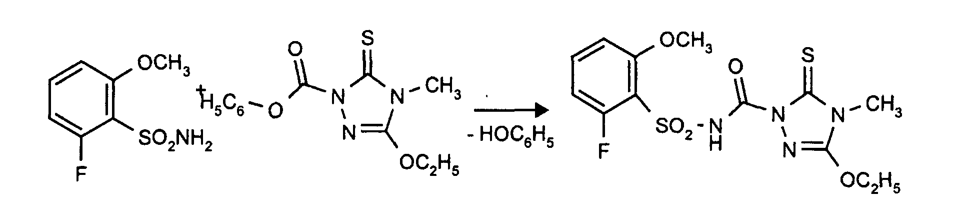

Verwendet man beispielsweise 2-Ethoxy-6-methyl-phenylsulfonyl-isothiocyanat und 5-Ethyl-4-methoxy-2,4-dihydro-3H-1,2,4-triazol-3-on als Ausgangsstoffe, so kann der Reaktionsablauf beim erfindungsgemäßen Verfahren (b) durch das folgende Formelschema skizziert werden:

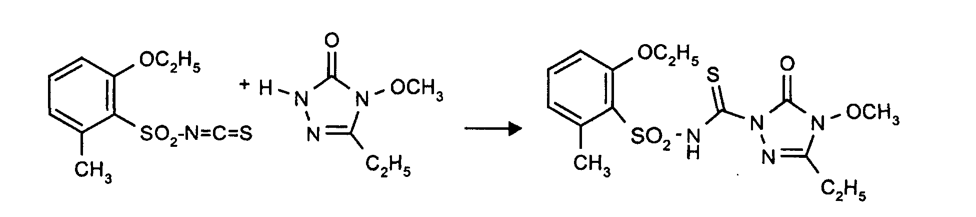

Verwendet man beispielsweise 2-Methoxy-3-methyl-benzolsulfochlorid, 5-Ethylthio-4-methoxy-2,4-dihydro-3H-1,2,4-triazol-3-on und Kaliumcyanat als Ausgangsstoffe, so kann der Reaktionsablauf beim erfindungsgemäßen Verfahren (c) durch das folgende Formelschema skizziert werden:

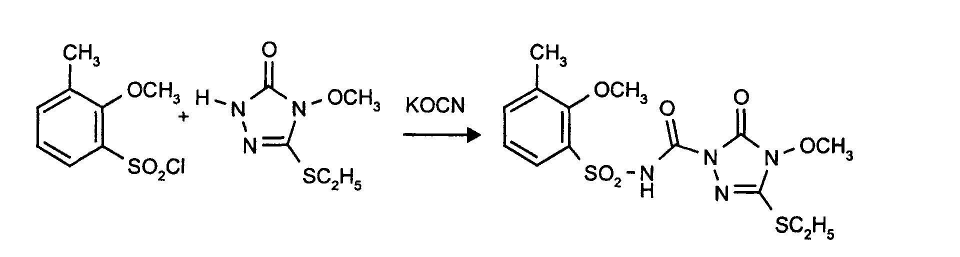

Verwendet man beispielsweise N-(2-Chlor-6-propoxy-phenylsulfonyl)-O-methylurethan und 4-Methyl-5-methylthio-2,4-dihydro-3H-1,2,4-triazol-3-on als Ausgangsstoffe, so kann der Reaktionsablauf beim erfindungsgemäßen Verfahren (e) durch das folgende Formelschema skizziert werden:

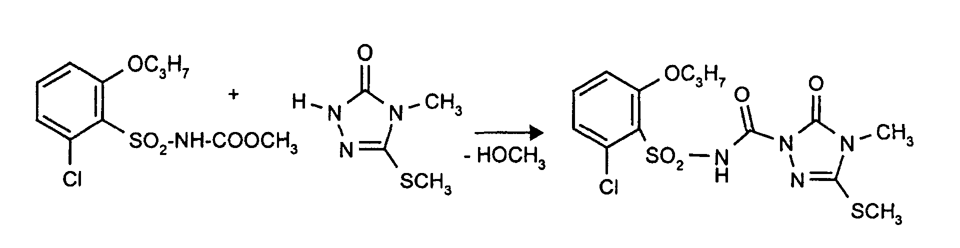

Die beim erfindungsgemäßen Verfahren (a) zur Herstellung der Verbindungen der Formel (I) als Ausgangsstoffe zu verwendenden Aminosulfonylverbindungen sind durch die Formel (II) allgemein definiert. In der Formel (II) haben A, R¹ und R² vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für A, R¹ und R² angegeben wurde.

Die Ausgangsstoffe der Formel (II) sind bekannt und/oder können nach an sich bekannten Verfahren hergestellt werden (vgl. EP 216504, DE 3208189, EP 44807, EP 23422).

Noch nicht aus der Literatur bekannt sind die Sulfonamide der allgemeinen Formel (IIa), 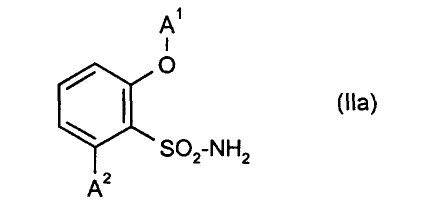 in welcher
- A¹: für Ethyl, n- oder i-Propyl, n-, i-, s- oder t-Butyl, Trifluormethyl, Fluorethyl, Chlorethyl, Difluorethyl, Trifluorethyl, Chlortrifluorethyl, Methoxyethyl, Ethoxyethyl, Allyl, Propargyl oder Benzyl steht und
- A²: für Methyl, Ethyl, n- oder i-Propyl, n-, i-, s- oder t-Butyl steht.

Man erhält die neuen Sulfonamide der Formel (IIa), wenn man Sulfonsäurechloride der Formel (VIa) 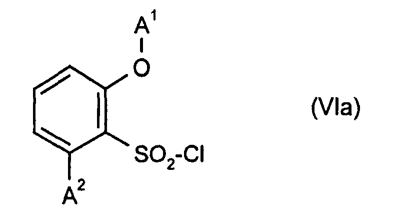 in welcher
A¹ und A² die oben angegebenen Bedeutungen haben,
mit Ammoniak gegebenenfalls in Gegenwart eines Verdünnungsmittels, wie z.B. Wasser, bei Temperaturen zwischen 0°C und 50°C umsetzt (vgl. die Herstellungsbeispiele).

Die Ausgangsstoffe der Formel (II) können im allgemeinen auch durch Umsetzung von Phenolderivaten der Formel (IIb) 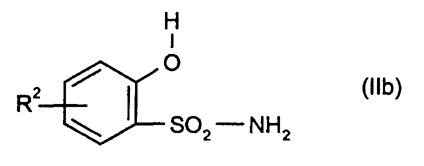 in welcher
R² die oben angegebenen Bedeutungen hat,
mit Alkylierungsmitteln der Formel (XI)

X-R¹ (XI)

in welcher
- R¹: die oben angegebene Bedeutung hat und
- X: für Halogen oder die Gruppierung R¹-O-SO₂-O- steht,
gegebenenfalls in Gegenwart eines Säureakzeptors, wie z.B. Kaliumcarbonat, und gegebenenfalls in Gegenwart eines Verdünnungsmittels, wie z.B. Toluol, bei Temperaturen zwischen 10°C und 150°C erhalten werden (vgl. die Herstellungsbeispiele).

Die als Vorprodukte benötigten Phenolderivate der Formel (IIb) sind bekannt und/oder können nach an sich bekannten Verfahren hergestellt werden (vgl. EP 44807, Metalloberfläche - Angew. Elektrochemie 27 (1973), 217-227 - zitiert in Chem. Abstracts 79:86733; Herstellungsbeispiele).

Die weiter als Vorprodukte benötigten Alkylierungsmittel der Formel (XI) sind bekannte Synthesechemikalien.

Die beim erfindungsgemäßen Verfahren (a) zur Herstellung der Verbindungen der Formel (I) weiter als Ausgangsstoffe zu verwendenden (Thio)Carbonsäurederivate sind durch die Formel (III) allgemein definiert. In der Formel (III) haben Q und R³ vorzugsweise diejenige Bedeutung, die bereits oben bei der Beschreibung der erfindungsgemäß herzustellenden Verbindungen der Formel (I) vorzugsweise für Q und R³ angegeben wurde; Z steht vorzugsweise für Fluor, Chlor, Brom, C₁-C₄-Alkoxy, Phenoxy oder Benzyloxy, insbesondere für Chlor, Methoxy, Ethoxy oder Phenoxy.

Die Ausgangsstoffe der Formel (III) sind bekannt und/oder können nach an sich bekannten Verfahren hergestellt werden (vgl. EP 459244, EP 341489, EP 422469, EP 425948, EP 431291, EP 507171, EP 534266).

Die beim erfindungsgemäßen Verfahren (b) zur Herstellung der Verbindungen der Formel (I) als Ausgangsstoffe zu verwendenden Sulfonyliso(thio)cyanate sind durch die Formel (IV) allgemein definiert. In der Formel (IV) haben Q, R¹ und R² vorzugsweise diejenige Bedeutung, die bereits oben bei der Beschreibung der erfindungsgemäß herzustellenden Verbindungen der Formel (I) vorzugsweise für Q, R¹ und R² angegeben wurde.

Die Ausgangsstoffe der Formel (IV) sind bekannt und/oder können nach an sich bekannten Verfahren hergestellt werden (vgl. EP 23422, EP 216504).

Noch nicht aus der Literatur bekannt sind die Sulfonyliso(thio)cyanate der allgemeinen Formel (IVa) 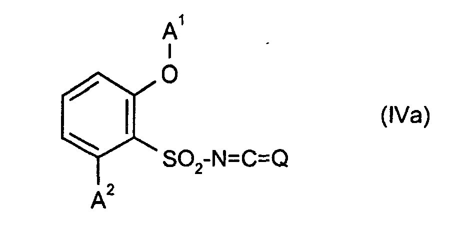 in welcher
- Q: für Sauerstoff oder Schwefel steht,
- A¹: für Ethyl, n- oder i-Propyl, n-, i-, s- oder t-Butyl, Trifluormethyl, Fluorethyl, Chlorethyl, Difluorethyl, Trifluorethyl, Chlortrifluorethyl, Methoxyethyl, Ethoxyethyl, Allyl, Propargyl oder Benzyl steht und
- A²: für Methyl, Ethyl, n- oder i-Propyl, n-, i-, s- oder t-Butyl steht.

Man erhält die neuen Sulfonyliso(thio)cyanate der Formel (IVa), wenn man Sulfonamide der Formel (IIa) - oben - mit Phosgen bzw. Thiophosgen, gegebenenfalls in Gegenwart eines Alkylisocyanats, wie z.B. Butylisocyanat, gegebenenfalls in Gegenwart eines Reaktionshilfsmittels, wie z.B. Diazabicyclo[2.2.2]octan, und in Gegenwart eines Verdünnungsmittels, wie z.B. Toluol, Xylol oder Chlorbenzol, bei Temperaturen zwischen 80°C und 150°C umsetzt und nach Ende der Umsetzung die flüchtigen Komponenten unter vermindertem Druck abdestilliert.

Die bei den erfindungsgemäßen Verfahren (b), (c), (e) und (f) zur Herstellung der Verbindungen der Formel (I) weiter als Ausgangsstoffe zu verwendenden Heterocyclen sind durch die Formel (V) allgemein definiert. In der Formel (V) hat R³ vorzugsweise diejenige Bedeutung, die bereits oben bei der Beschreibung der erfindungsgemäß herzustellenden Verbindungen der Formel (I) vorzugsweise R³ angegeben wurde.

Die Ausgangsstoffe der Formel (V) sind bekannt und/oder können nach an sich bekannten Verfahren hergestellt werden (vgl. EP 341489, EP 422469, EP 425948, EP 431291, EP 507171, EP 534266).

Die bei den erfindungsgemäßen Verfahren (c) und (d) zur Herstellung der Verbindungen der Formel (I) als Ausgangsstoffe zu verwendenden Chlorsulfonylverbindungen sind durch die Formel (VI) allgemein definiert. In der Formel (VI) haben A, R¹ und R² vorzugsweise diejenige Bedeutung, die bereits oben bei der Beschreibung der erfindungsgemäß herzustellenden Verbindungen der Formel (I) vorzugsweise für A, R¹ und R² angegeben wurde.

Die Ausgangsstoffe der Formel (VI) sind bekannt und/oder können nach an sich bekannten Verfahren hergestellt werden (vgl. EP 511826, DE 3208189, EP23422).

Noch nicht aus der Literatur bekannt sind die Sulfonsäurechloride der Formel (VIa) 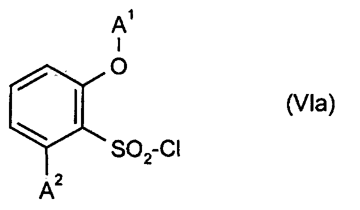 in welcher
- A¹: für Ethyl, n- oder i-Propyl, n-, i-, s- oder t-Butyl, Trifluormethyl, Fluorethyl, Chlorethyl, Difluorethyl, Trifluorethyl, Chlortrifluorethyl, Methoxyethyl, Ethoxyethyl, Allyl, Propargyl oder Benzyl steht und
- A²: für Methyl, Ethyl, n- oder i-Propyl, n-, i-, s- oder t-Butyl steht.

Man erhält die neuen Sulfonsäurechloride der Formel (VIa), wenn man Anilinderivate der Formel (XII) 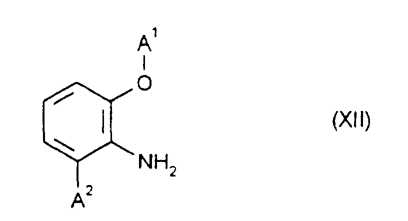 in welcher
A¹ und A² die oben angegebenen Bedeutungen haben,
mit einem Alkalimetallnitrit, wie z.B. Natriumnitrit, in Gegenwart von Salzsäure bei Temperaturen zwischen -10°C und +10°C umsetzt und die so erhaltene Diazoniumsalzlösung mit Schwefeldioxid in Gegenwart eines Verdünnungsmittels, wie z.B. Dichlormethan oder 1,2-Dichlor-ethan, und in Gegenwart eines Katalysators, wie z.B. Kupfer(1)-chlorid, gegebenenfalls in Gegenwart eines weiteren Katalysators, wie z.B. Dodecyltrimethylammoniumbromid, bei Temperaturen zwischen -10°C und +50°C umsetzt (vgl. die Herstellungsbeispiele)

Die als Vorprodukte benötigten Anilinderivate der Formel (XII) sind bekannt und/oder können nach an sich bekannten Verfahren hergestellt werden (vgl EP 511826, US 4992091, EP 185128, DE 2405479, Herstellungsbeispiele)

Die vorgenannten neuen Benzolsulfonsaurederivate der Formeln (IIa), (IVa) und (VIa) konnen zusammenfassend durch die folgende Formel (XIII) definiert werden 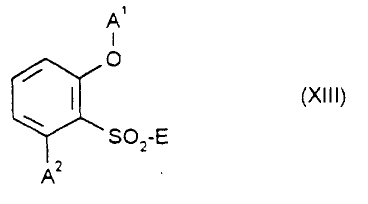 in welcher
- E: für -NH₂, -N=C=Q oder -Cl steht, wobei
- Q: für O oder S steht, und ferner
- A¹: für Ethyl, n- oder i-Propyl, n-, i-, s- oder t-Butyl, Trifluormethyl, Fluorethyl, Chlorethyl, Difluorethyl, Trifluorethyl, Chlortrifluorethyl, Methoxyethyl, Ethoxyethyl, Allyl, Propargyl oder Benzyl steht und
- A²: für Methyl, Ethyl, n- oder i-Propyl, n-, i-, s- oder t-Butyl steht.

Die beim erfindungsgemäßen Verfahren (d) zur Herstellung der Verbindungen der Formel (I) als Ausgangsstoffe zu verwendenden (Thio)Carbonsäureamide sind durch die Formel (VIII) allgemein definiert. In der Formel (VIII) haben Q und R³ vorzugsweise diejenige Bedeutung, die bereits oben bei der Beschreibung der erfindungsgemäß herzustellenden Verbindungen der Formel (I) vorzugsweise bevorzugt für Q und R³ angegeben wurde.

Die Ausgangsstoffe der Formel (VIII) sind bekannt und/oder können nach an sich bekannten Verfahren hergestellt werden (vgl. EP 459244).

Die beim erfindungsgemäßen Verfahren (e) zur Herstellung der Verbindungen der Formel (I) als Ausgangsstoffe zu verwendenden Sulfonylamino(thio)carbonylverbindungen sind durch die Formel (IX) allgemein definiert. In der Formel (IX) haben A, Q, R¹ und R² vorzugsweise diejenige Bedeutung, die bereits oben bei der Beschreibung der erfindungsgemäß herzustellenden Verbindungen der Formel (I) vorzugsweise bevorzugt für A, Q, R¹ und R² angegeben wurde; Z steht vorzugsweise für Fluor, Chlor, Brom, C₁-C₄-Alkoxy, Phenoxy oder Benzyloxy, insbesondere für Chlor, Methoxy, Ethoxy oder Phenoxy.

Die beim erfindungsgemäßen Verfahren (f) zur Herstellung der Verbindungen der Formel (I) als Ausgangsstoffe zu verwendenden Benzolderivate sind durch die Formel (X) allgemein definiert. In der Formel (X) haben A, R¹ und R² vorzugsweise diejenige Bedeutung, die bereits oben bei der Beschreibung der erfindungsgemäß herzustellenden Verbindungen der Formel (I) vorzugsweise für A, R¹ und R² angegeben wurde.

Die Ausgangsstoffe der Formel (X) sind bekannt und/oder können nach an sich bekannten Verfahren hergestellt werden (vgl. EP 511826, US 4992091, EP 185128, DE 2405479, Herstellungsbeispiele).

Die erfindungsgemäßen Verfahren (a), (b), (c), (d), (e) und (f) zur Herstellung der neuen Verbindungen der Formel (I) werden vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol; Ether wie Diethyl- und Dibutylether, Glykoldimethylether und Diglykoldimethylether, Tetrahydrofuran und Dioxan; Ketone wie Aceton, Methyl-ethyl-, Methyl-isopropyl- und Methyl-isobutyl-keton; Ester wie Essigssauremethylester und -ethylester; Nitrile wie z.B Acetonitril und Propionitril, Amide wie z.B. Dimethylformamid, Dimethylacetamid und N-Methylpyrrolidon, sowie Dimethylsulfoxid, Tetramethylensulfon und Hexamethylphosphorsauretriamid

Als Reaktionshilfsmittel bzw. als Säureakzeptoren konnen bei den erfindungsgemäßen Verfahren (a), (b), (c), (d), (e) und (f) alle üblicherweise für derartige Umsetzungen verwendbaren Säurebindemittel eingesetzt werden Vorzugsweise in Frage kommen Alkalimetallhydroxide wie z.B. Natrium- und Kaliumhydroxid, Erdalkalihydroxide wie z.B. Calciumhydroxid. Alkalicarbonate und -alkoholate wie Natrium- und Kaliumcarbonat, Natrium- und Kalium-tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexyiamin, N,N-Dimethylbenzylamin, N,N-Dimethyl-anilin, Pyridin, 2-Methyl- 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO)

Die Reaktionstemperaturen können bei den erfindungsgemäßen Verfahren (a), (b), (c), (d), (e) und (f) in einem größeren Bereich variiert werden. Im allgemeinen arbeitet man bei Temperaturen zwischen -20°C und +150°C, vorzugsweise bei Temperaturen zwischen 0°C und +100°C.

Die erfindungsgemäßen Verfahren (a), (b), (c), (d), (e) und (f) werden im allgemeinen unter Normaldruck durchgeführt. Es ist jedoch auch möglich, unter erhohtem oder vermindertem Druck zu arbeiten.

Zur Durchführung der erfindungsgemäßen Verfahren (a), (b), (c), (d), (e) und (f) werden die jeweils benötigten Ausgangsstoffe im allgemeinen in angenähert äquimolaren Mengen eingesetzt. Es ist jedoch auch möglich, eine der jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b), (c), (d), (e) und (f) jeweils nach üblichen Methoden (vgl. die Herstellungsbeispiele)

Aus den erfindungsgemäßen Verbindungen der allgemeinen Formel (I) können gegebenenfalls Salze hergestellt werden Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden

Die erfindungsgemäßen Wirkstoffe können als Defoliants, Desiccants, Krautabtötungsmittel und insbesondere als Unkrautvernichtungsmittel verwendet werden Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab

Die erfindungsgemaßen Wirkstoffe können z.B. bei den folgenden Pflanzen verwendet werden:
Dikotyle Unkrauter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekampfung z.B. auf Industrie- und Gleisanlagen und auf Wegen und Plätzen mit und ohne Baumbewuchs. Ebenso konnen die Verbindungen zur Unkrautbekampfung in Dauerkulturen, z.B Forst, Ziergeholz-, Obst-, Wein-, Citrus-, Nuß-, Bananen-, Kaffee-, Tee-, Gummi-, Olpalm-, Kakao-, Beerenfrucht- und Hopfenanlagen, auf Zier- und Sportrasen und Weideflachen und zur selektiven Unkrautbekampfung in einjahrigen Kulturen eingesetzt werden

Die erfindungsgemaßen Verbindungen der Formel (1) eignen sich vorzugsweise zur Bekampfung von monokotylen und dikotylen Unkräutern sowohl im Voraufiaufals auch im Nachauflauf-Verfahren Sie zeigen starke herbizide Aktivitat und ein breites Wirkungsspektrum bei Anwendung auf den Boden und auf oberirdische Pflanzenteile

Die Wirkstoffe konnen in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Tragerstoffe fur Granulate kommen in Frage z.B. gebrochene und fraktionierte natürliche Gesteine wie Caicit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln, als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es konnen in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Fur die Mischungen kommen bekannte Herbizide infrage, beispielsweise Anilide, wie z.B Diflufenican und Propanil; Arylcarbonsäuren, wie z.B Dichlorpicolin-saure, Dicamba und Picloram; Aryloxyalkansäuren, wie z.B 2,4-D, 2,4-DB, 2,4-DP, Fluroxypyr, MCPA, MCPP und Triclopyr; Aryloxy-phenoxyalkansaureester, wie z.B. Diclofop-methyl, Fenoxaprop-ethyl, Fluazifop-butyl, Haloxyfop-methyl und Quizalofop-ethyl, Azinone, wie z.B Chloridazon und Norflurazon, Carbamate, wie z.B Chlorpropham, Desmedipham, Phenmedipham und Propham, Chloracetanilide, wie z.B Alachlor, Acetochlor, Butachlor, Metazachlor, Metolachlor, Pretilachlor und Propachlor, Dinitroaniline, wie z.B. Oryzalin, Pendimethalin und Trifluralin, Diphenylether, wie z.B Acifluorfen, Bifenox, Fluoroglycofen, Fomesafen, Halosafen, Lactofen und Oxyfluorfen; Harnstoffe, wie z.B Chlortoluron, Diuron, Fluometuron, Isoproturon, Linuron und Methabenzthiazuron, Hydroxylamine, wie z.B. Alloxydim, Clethodim, Cycloxydim, Sethoxydim und Tralkoxydim, Imidazolinone, wie z B Imazethapyr, Imazamethabenz, Imazapyr und Imazaquin; Nitrile, wie z.B. Bromoxynil, Dichlobenil und loxynil, Oxyacetamide, wie z.B. Mefenacet, Sulfonylharnstoffe, wie z B Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können ais solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken Sie hängt im wesentlichen von der Art des gewünschten Effektes ab. Im allgemeinen liegen die Aufwandmengen zwischen 1 g und 10 kg Wirkstoff pro Hektar Bodenflache, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

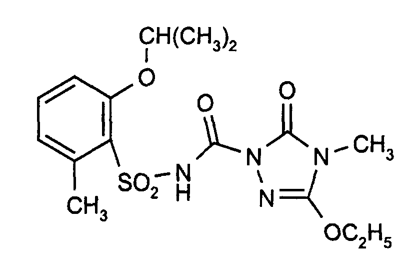

### (Verfahren (a))

Eine Mischung aus 2,5 g (10 mMol) 5-Ethoxy-4-methyl-2-phenoxycarbonyl-2,4-dihydro-3H-1,2,4-triazol-3-on, 2,3 g (10 mMol) 2-Isopropoxy-6-methyl-benzolsulfonamid, 1,5 g (10 mMol) Diazabicyclo[5.4.0]undec-7-en (DBU) und 50 ml Acetonitril wird 5 Stunden bei 20°C gerührt. Dann wird im Wasserstrahlvakuum eingeengt und der Rückstand mit 50 ml 1N-Salzsäure verrührt, abgesaugt, mit Diethylether verrührt und erneut abgesaugt.

Man erhält 2,4 g (60% der Theorie) 5-Ethoxy-4-methyl-2-(2-isopropoxy-6-methylphenylsulfonyl-aminocarbonyl)-2,4-dihydro-3H-1,2,4-triazol-3-on vom Schmelzpunkt 155°C.

### Beispiel 2 (nicht erfindungsgemäß)

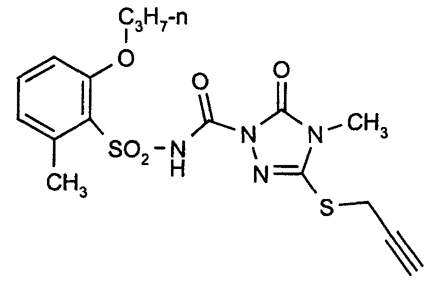

### (Verfahren (c))

Eine Mischung aus 1,7 g (10 mMol) 4-Methyl-5-propargylthio-2,4-dihydro-3H-1,2,4-triazol-3-on, 1,3 g (20 mMol) Natriumcyanat, 2,5 g (10 mMol) 2-Methyl-6-n-propoxy-benzolsulfochlorid und 50 ml Acetonitril wird 3 Stunden unter Rückfluß erhitzt. Dann wird im Wasserstrahlvakuum eingeengt, der Rückstand mit IN-Salzsäure verrührt und dreimal mit je 50 ml Methylenchlorid extrahiert. Die vereinigten organischen Extraktionslösungen werden eingeengt, der Rückstand mit Isopropanol digeriert und das kristallin angefallene Produkt durch Absaugen isoliert.

Man erhält 2,2 g (52% der Theorie) 4-Methyl-5-propargylthio-2-(2-methyl-6-npropoxy-phenylsulfonyl-aminocarbonyl)-2,4-dihydro-3H-1,2,4-triazol-3-on vom Schmelzpunkt 151°C.

### Beispiel 3 (nicht erfindungsgemäß)

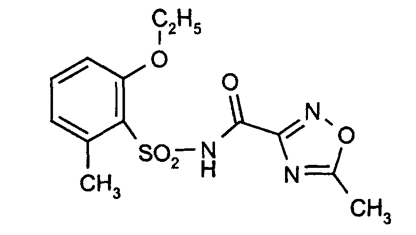

### (Verfahren (d))

Eine Mischung aus 3,2 g (25 mMol) 5-Methyl-1,2,4-oxadiazol-3-carboxamid, 4,2 g (75 mMol) Kaliumhydroxid (Pulver) und 200 ml Dioxan wird 30 Minuten bei 60°C gerührt. Dann wird im Wasserstrahlvakuum auf etwa das halbe Volumen eingeengt und bei ca. 20°C wird eine Lösung von 7 g (30 mMol) 2-Ethoxy-6-methyl-benzolsulfochlorid in 10 ml Dioxan tropfenweise dazu gegeben. Die Reaktionsmischung wird dann noch ca. 15 Stunden bei 20°C gerührt. Anschließend wird im Wasserstrahlvakuum eingeengt, der Rückstand mit 50 ml 1N-Salzsäure verrührt und das kristallin angefallene Produkt durch Absaugen isoliert.
Man erhält 4,0 g (49% der Theorie) N-(2-Ethoxy-6-methyl-phenylsulfonyl)-5-methyl-1,2,4-oxadiazol-3-carboxamid vom Schmelzpunkt 168°C.

### Beispiel 4

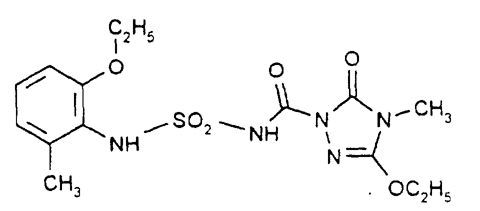

### (Verfahren (f))

1,7 g (12 mMol) Chlorsulfonylisocyanat werden zu einer auf 5°C abgekühlten Lösung von 1,4 g (10 mMol) 5-Ethoxy-4-methyl-2,4-dihydro-3H-1,2,4-triazol-3-on in 50 ml Methylenchlorid hinzugegeben und dann wird eine Lösung von 1,5 g (10 mMol) 2-Ethoxy-6-methyl-anilin und 1,0 g (10 mMol) Triethylamin in 10 ml Methylenchlorid ebenfalls bei 5°C zugetropft. Die Reaktionsmischung wird dann 15 Stunden bei ca 20°C gerührt. Anschließend werden 100 ml IN-Salzsaure dazugegeben. Nach gutem Durchrühren wird die organische Phase abgetrennt, mit Natriumsulfat getrocknet und filtriert. Das Filtrat wird im Wasserstrahlvakuum eingeengt, der Rückstand mit Isopropanol digeriert und das kristallin angefallene Produkt durch Absaugen isoliert.

Man erhalt 1,8 g (45% der Theorie) 5-Ethoxy-4-methyl-2-(2-ethoxy-6-methylphenyl-aminosulfonyl-aminocarbonyl)-2,4-dihydro-3H 1,2,4-triazol-3-on vom Schmelzpunkt 147°C.

Analog Beispiel 1 bis 4 sowie entsprechend der allgemeinen Beschreibung der erfindungsgemäßen Herstellungsverfahren konnen beispielsweise auch die in der nachstehenden Tabelle 1 aufgeführten Verbindungen der Formel (I) hergestellt werden

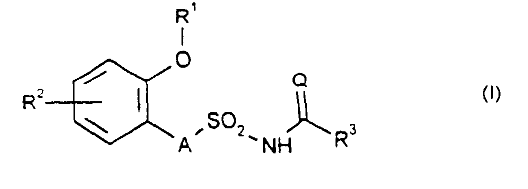

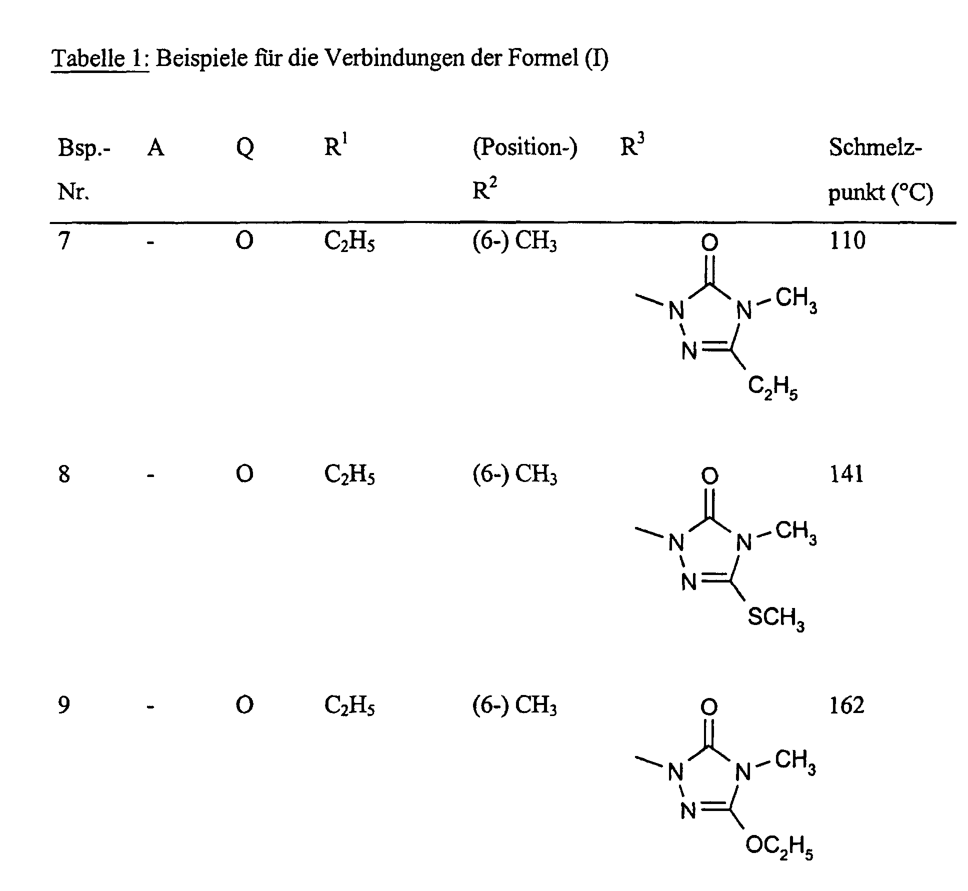

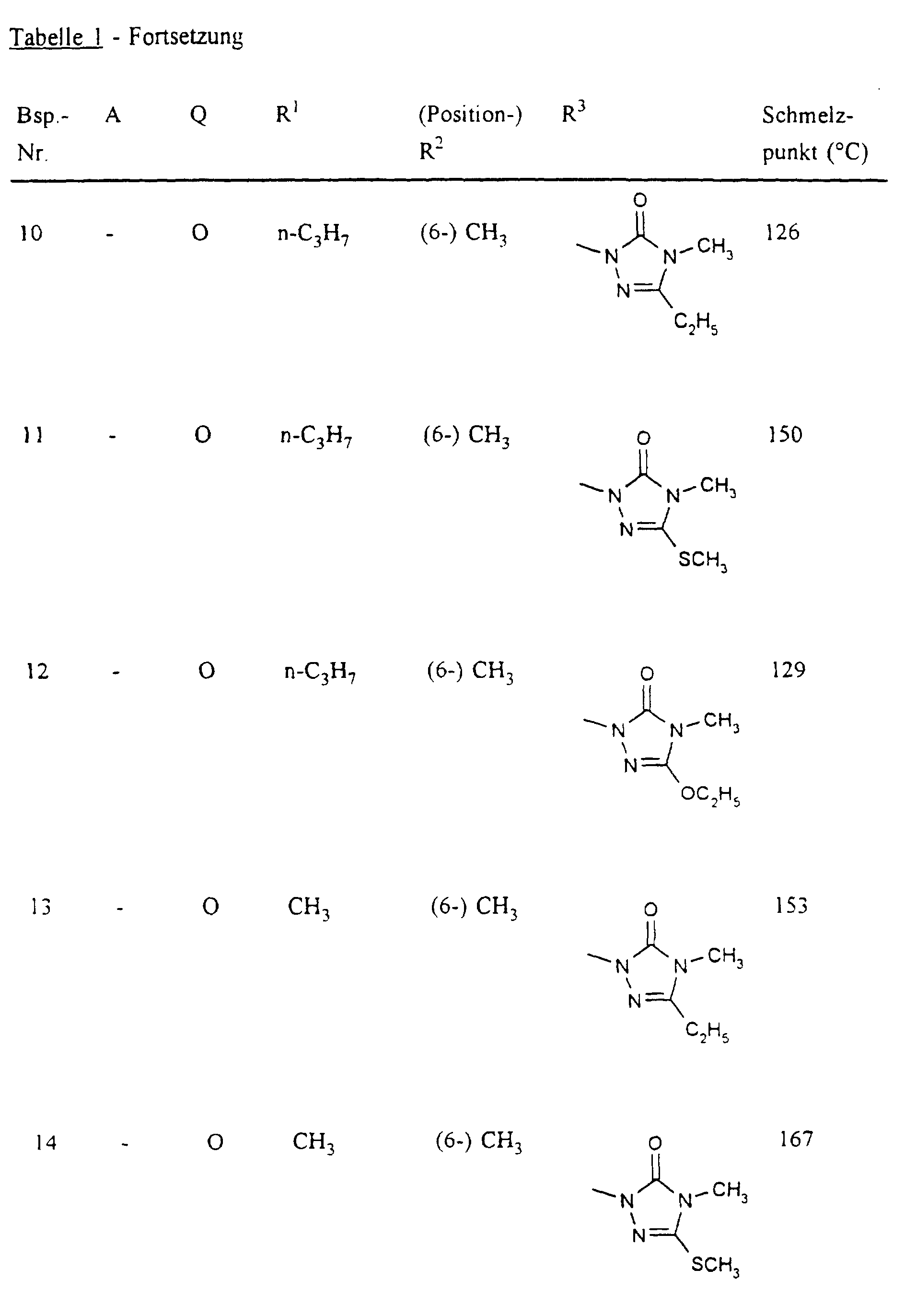

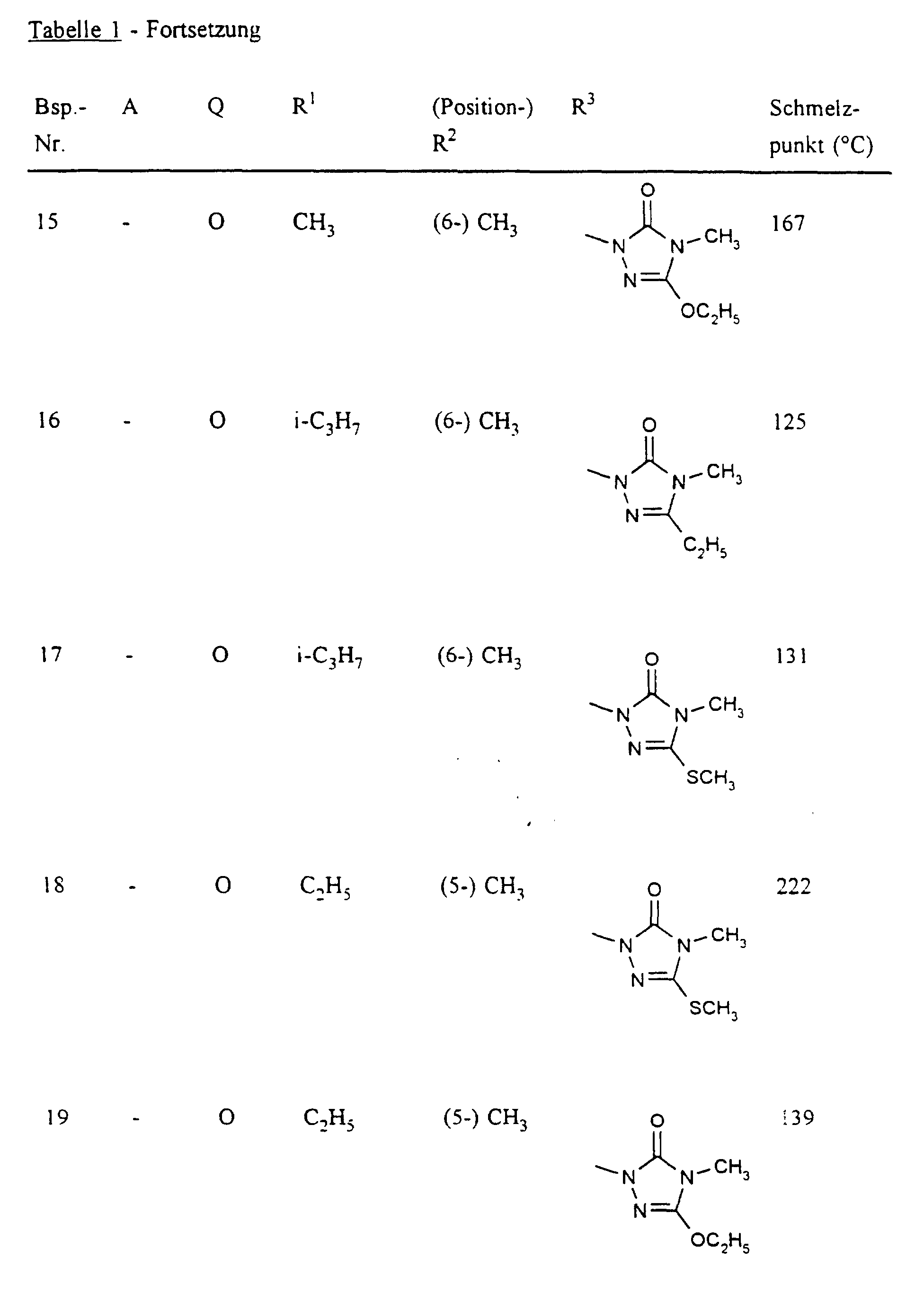

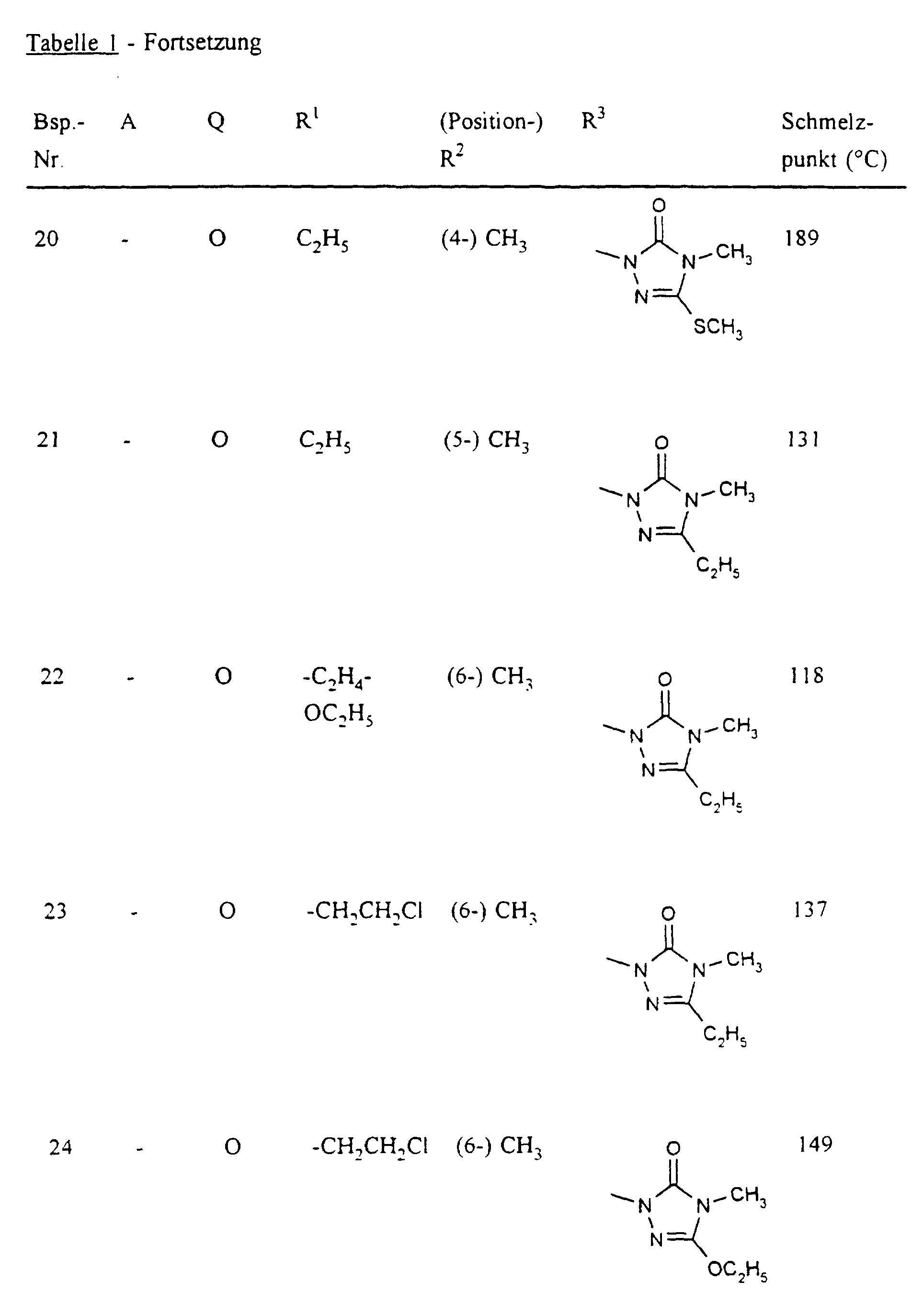

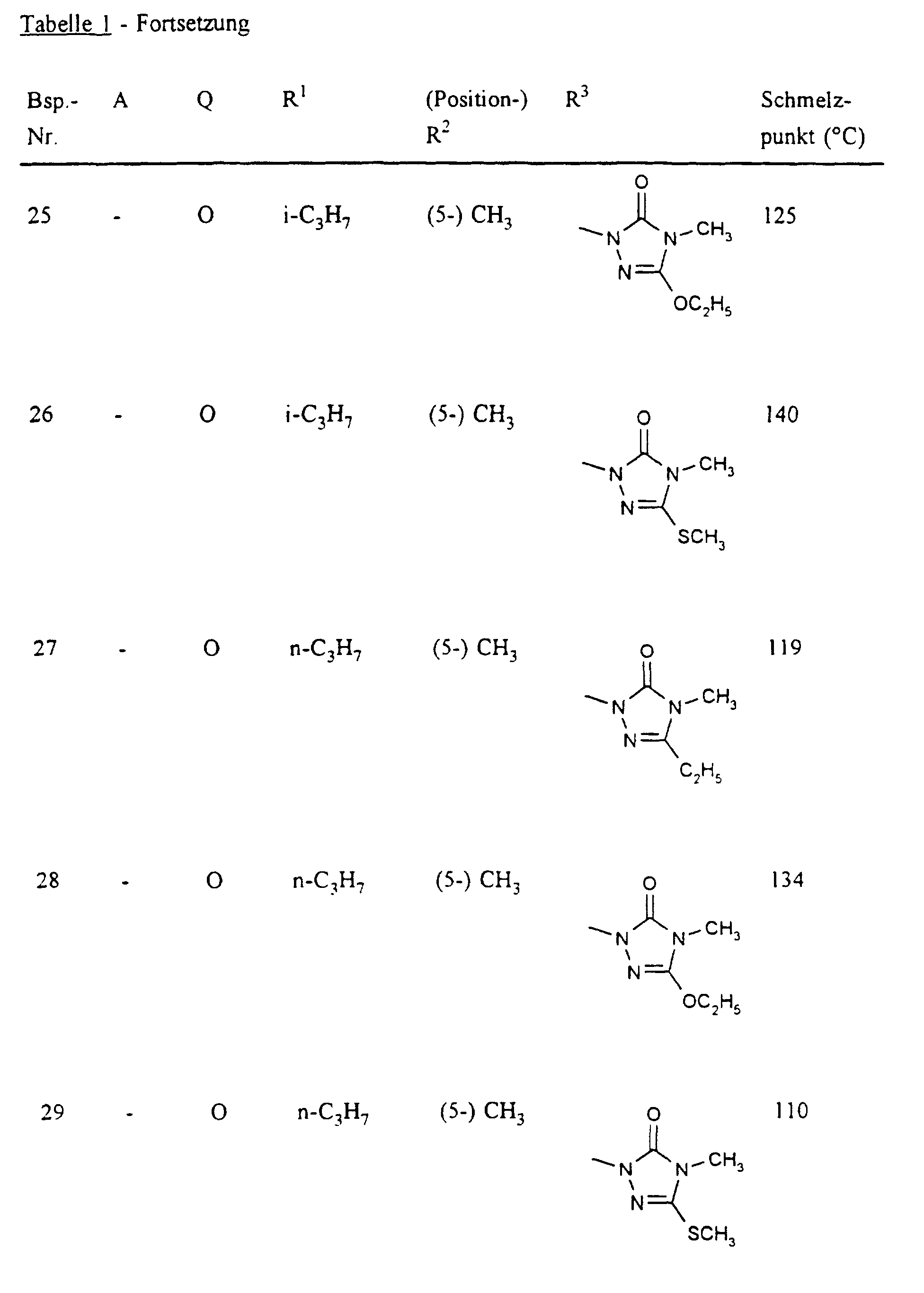

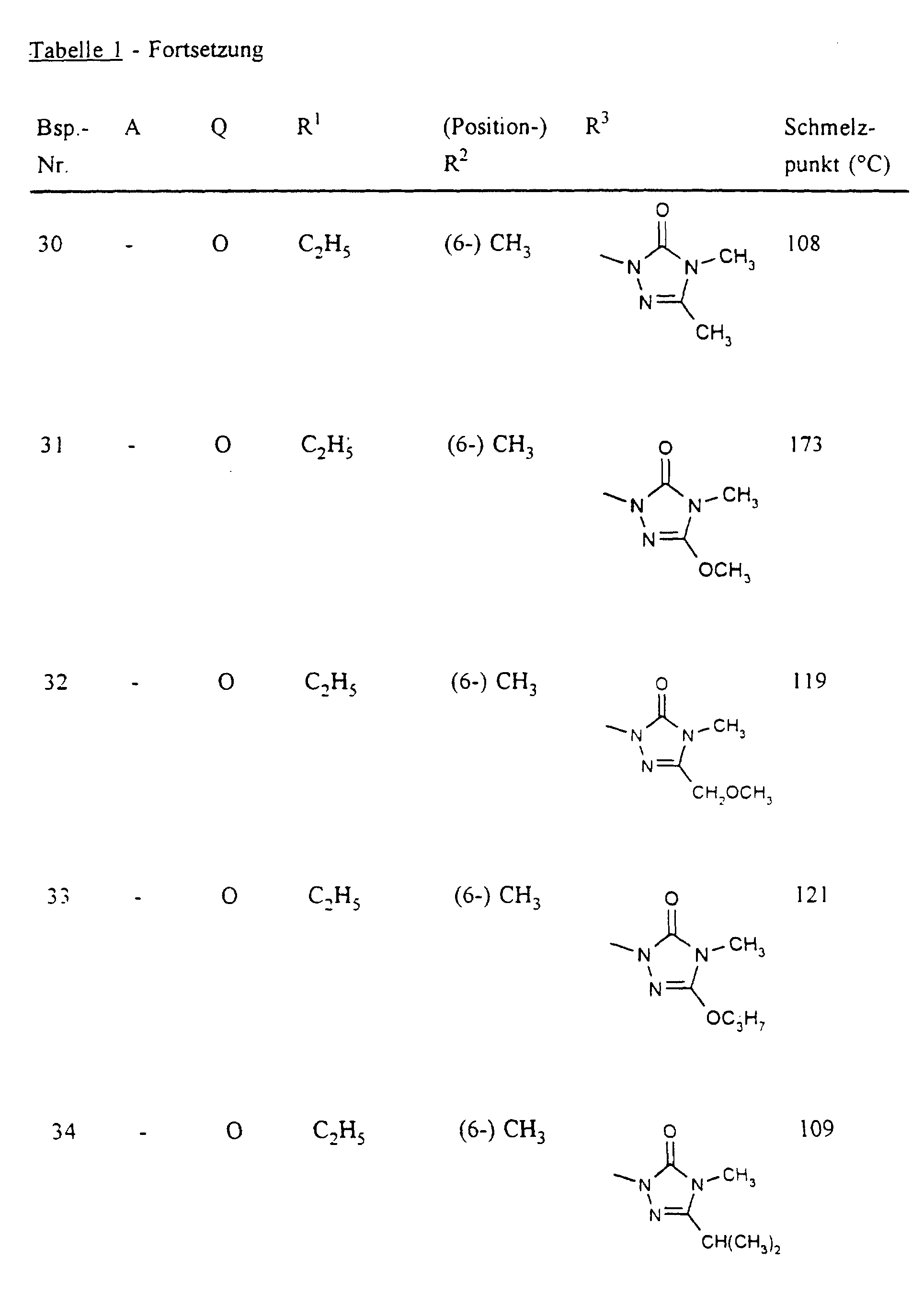

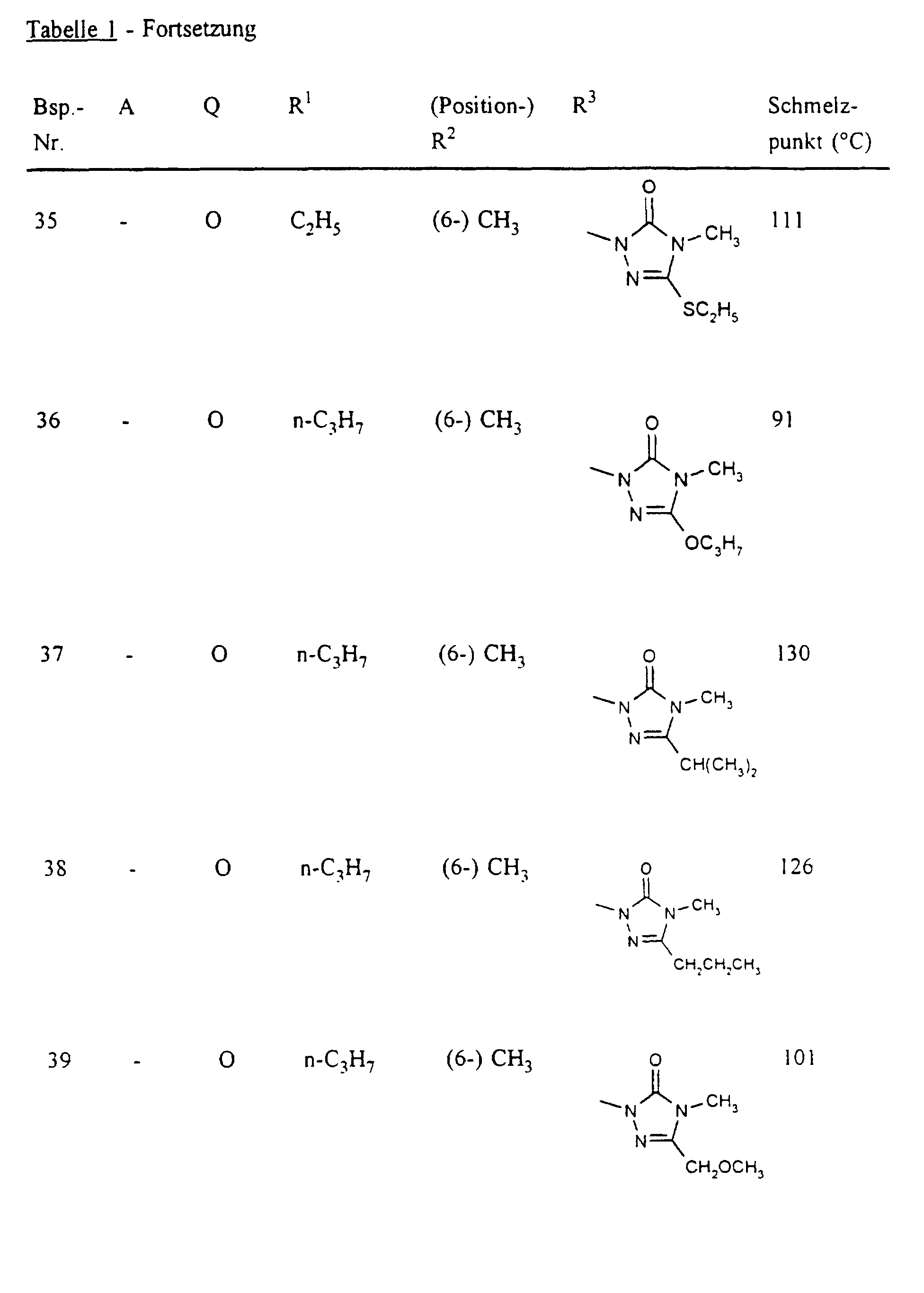

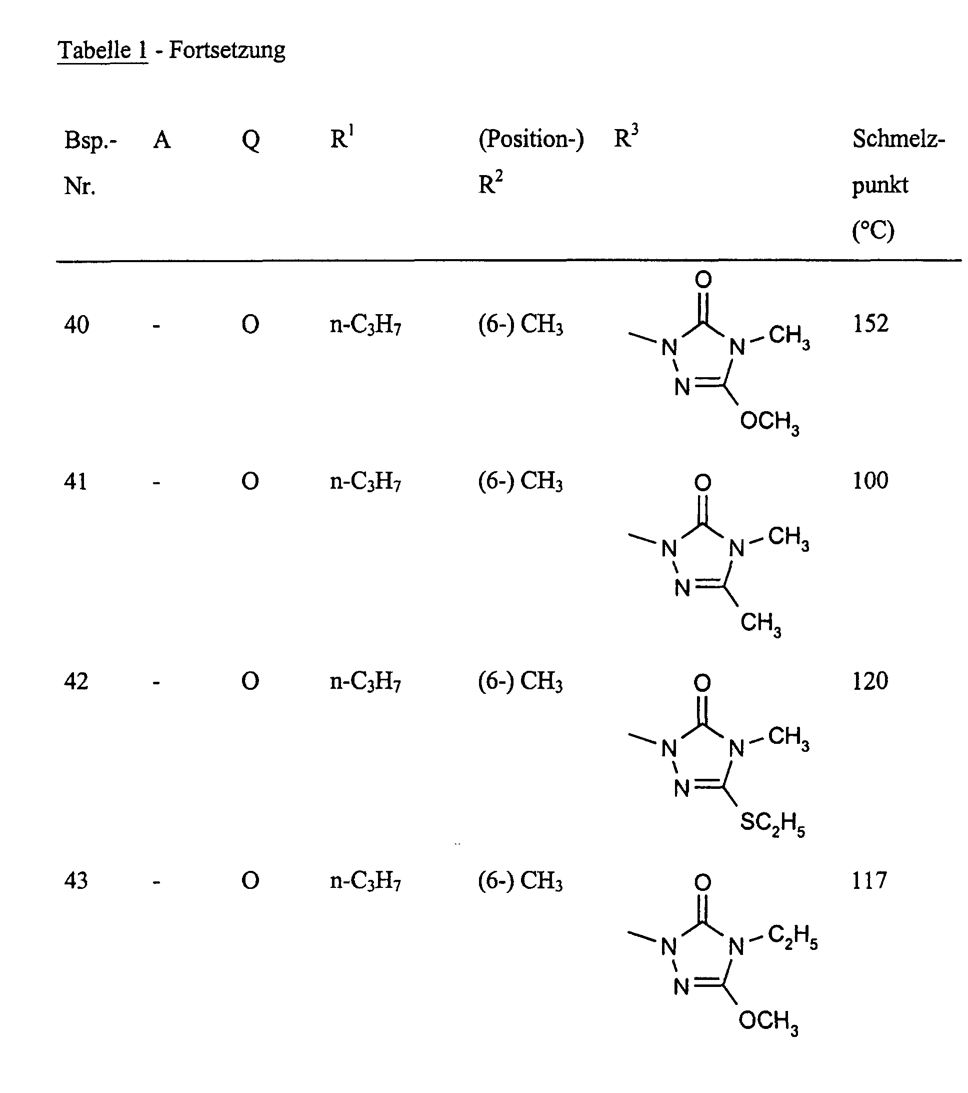

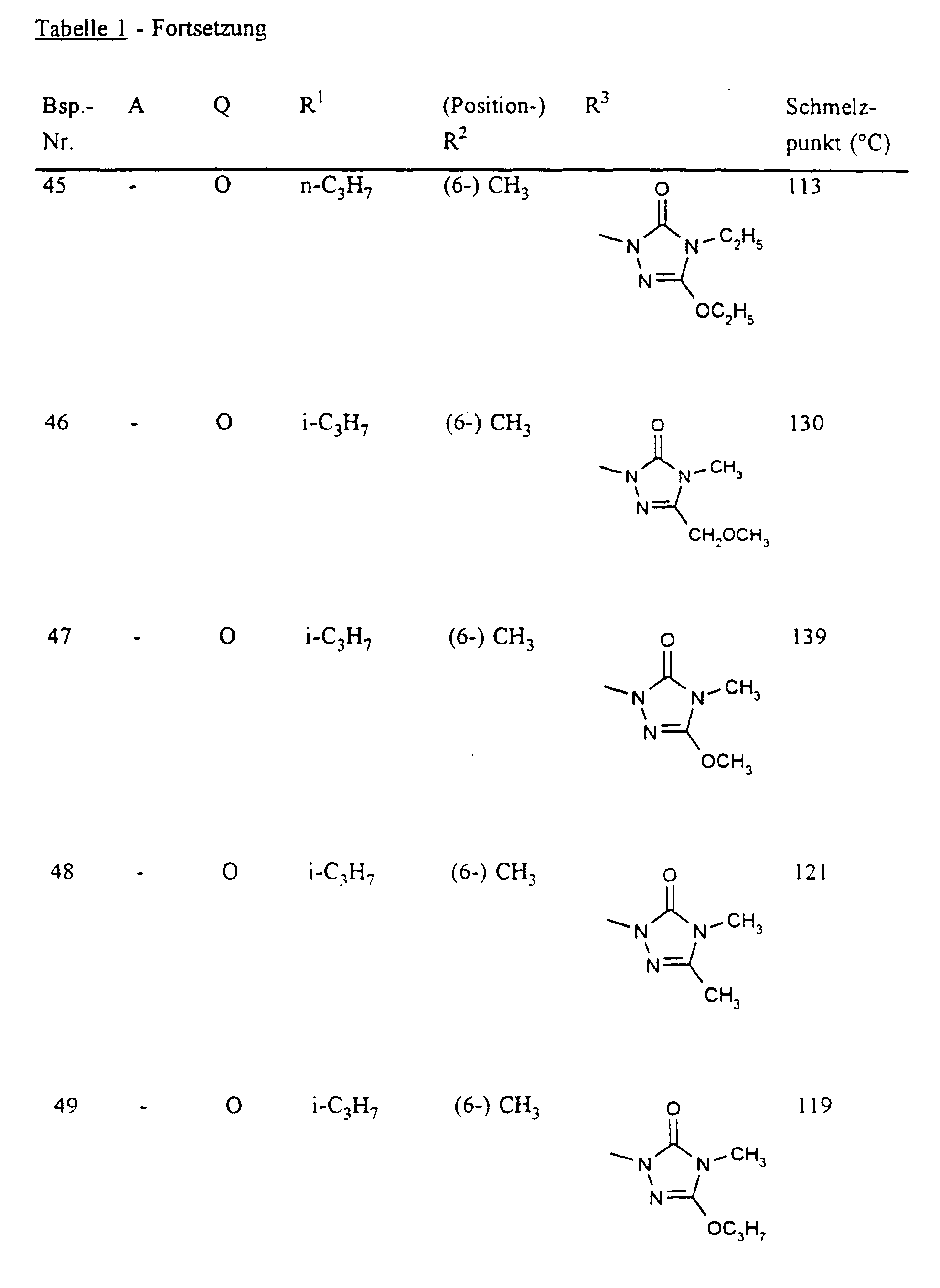

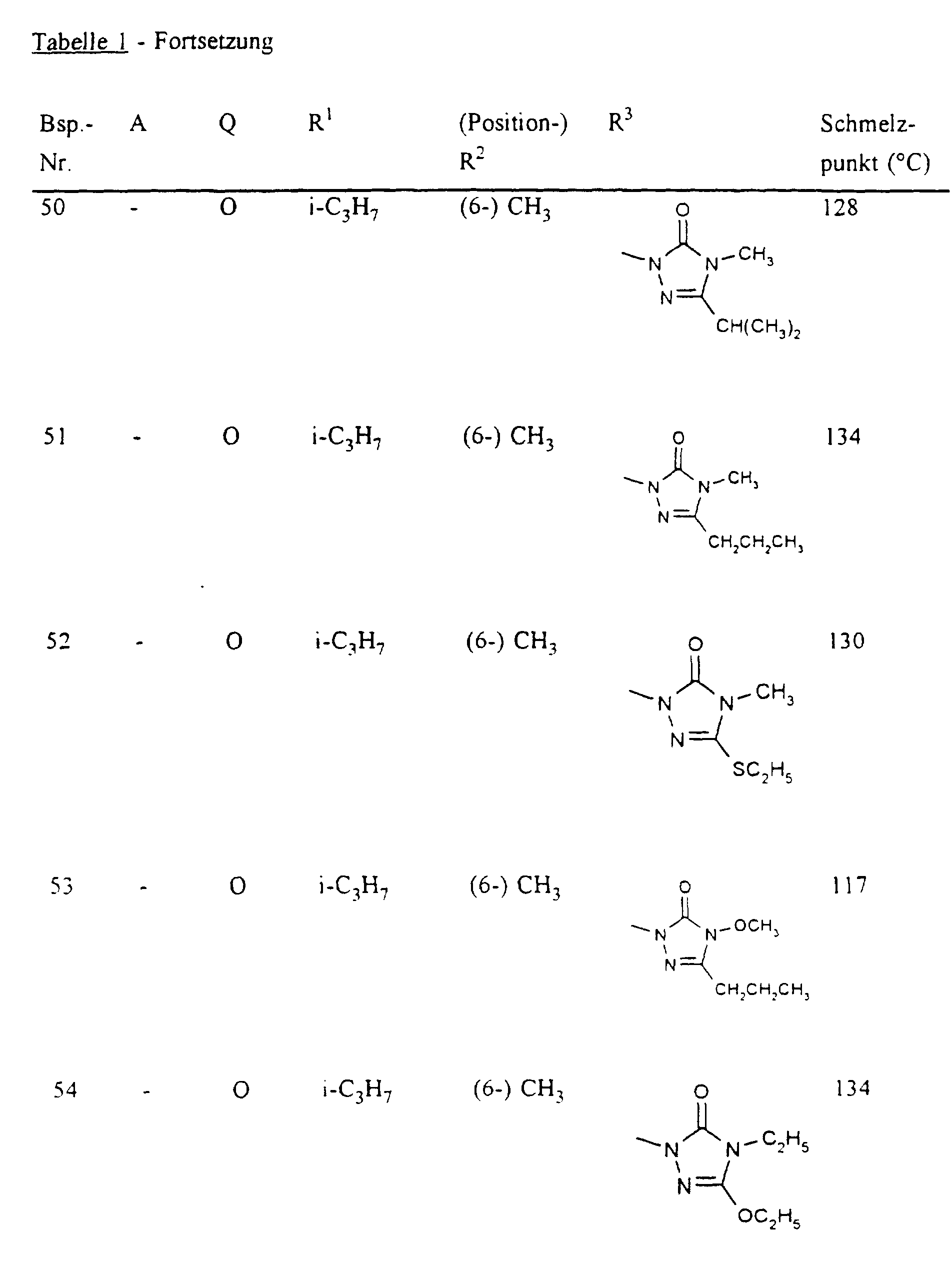

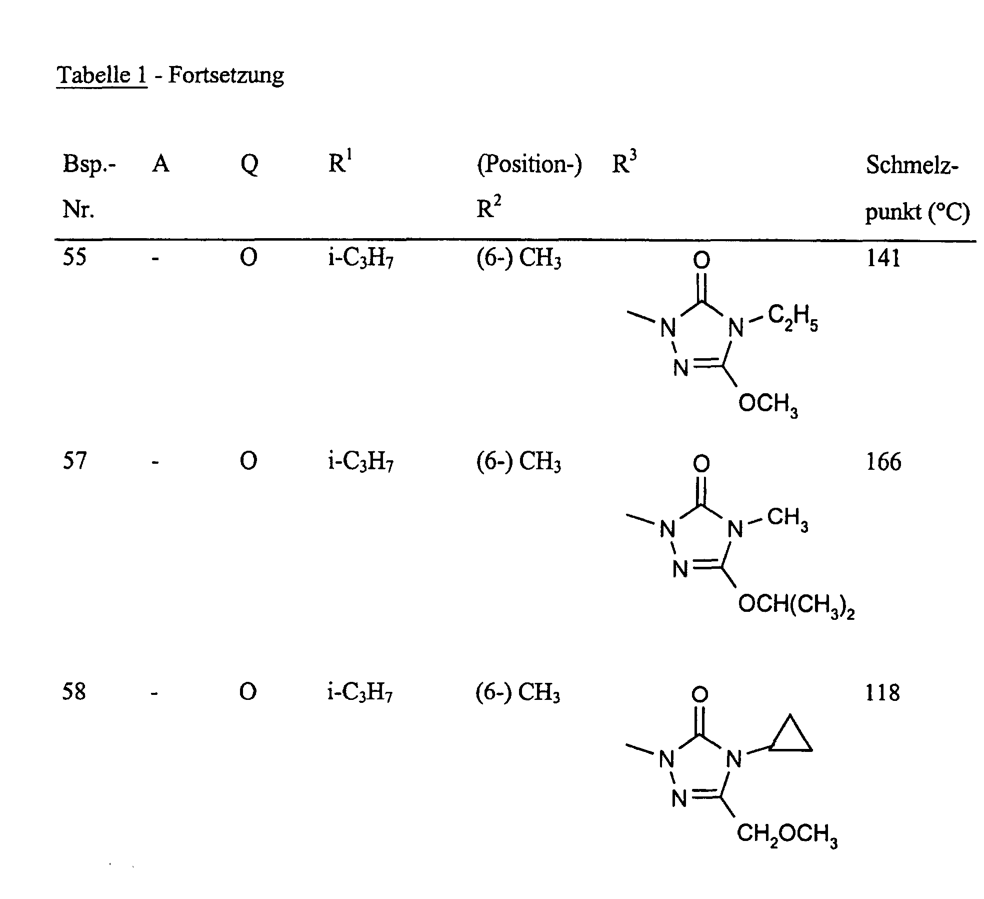

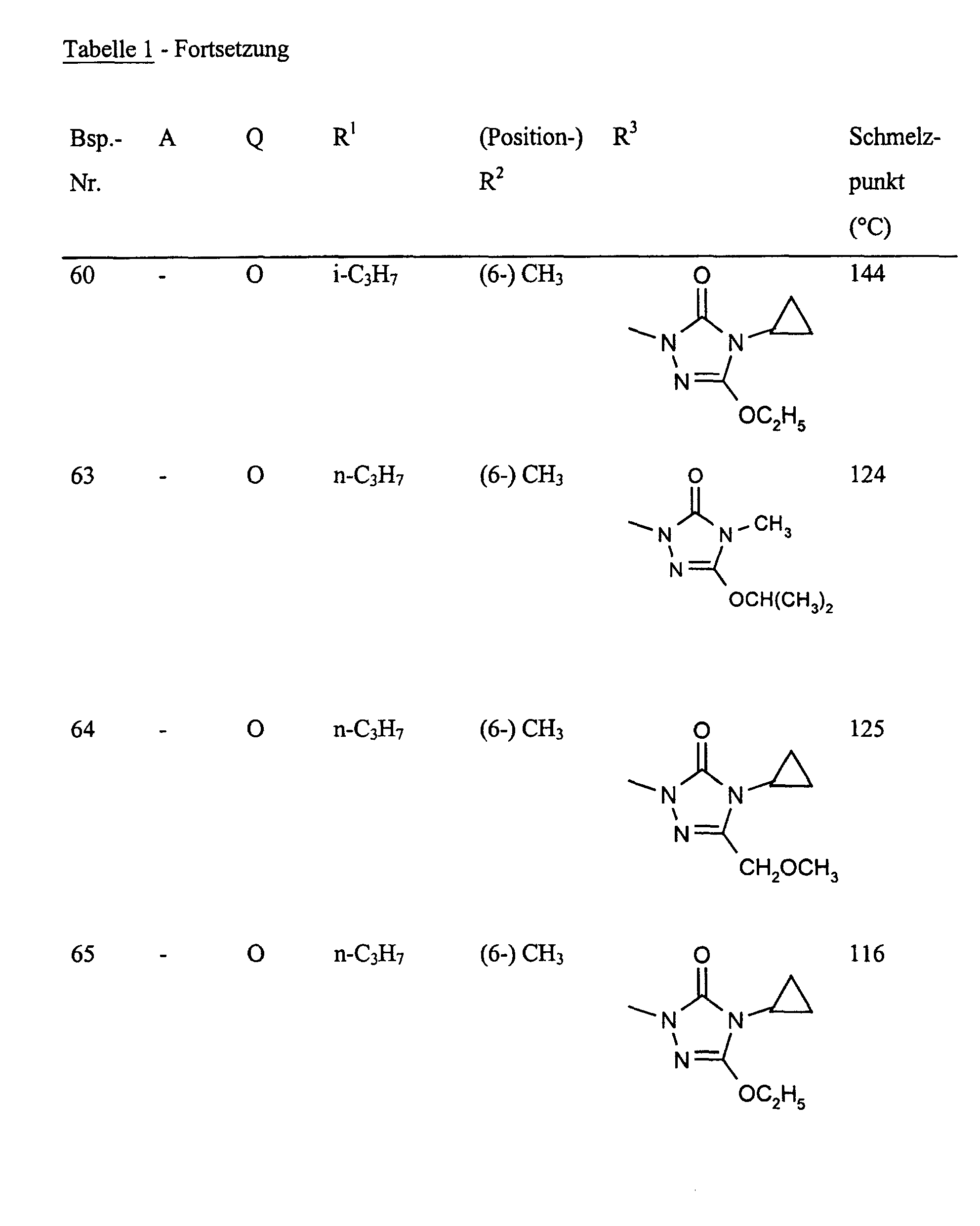

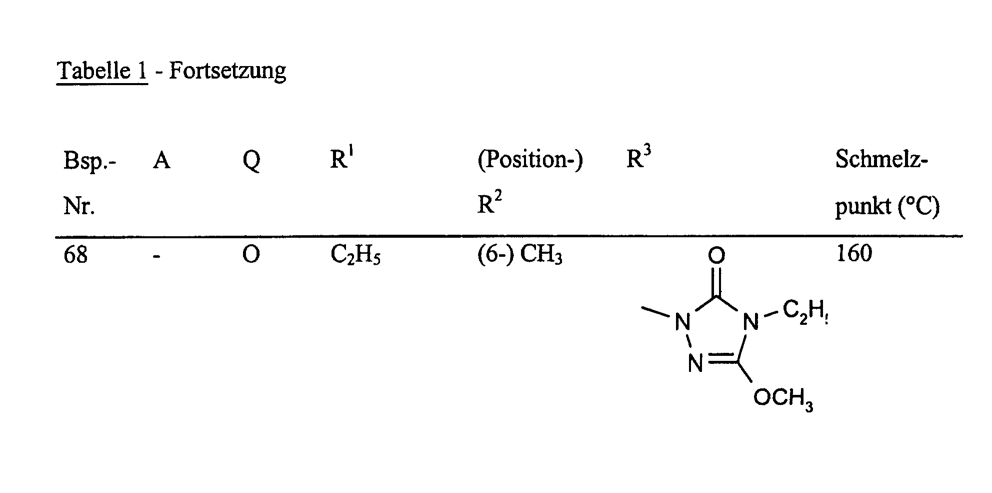

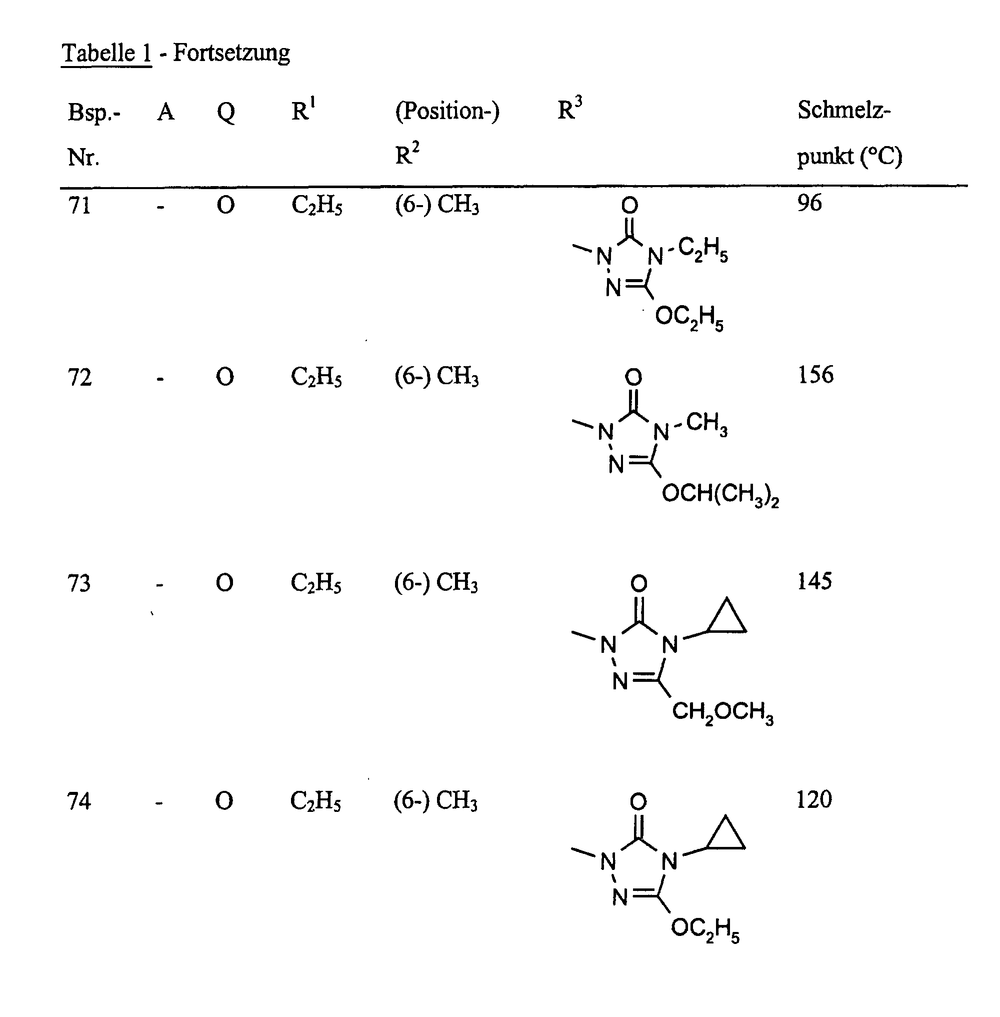

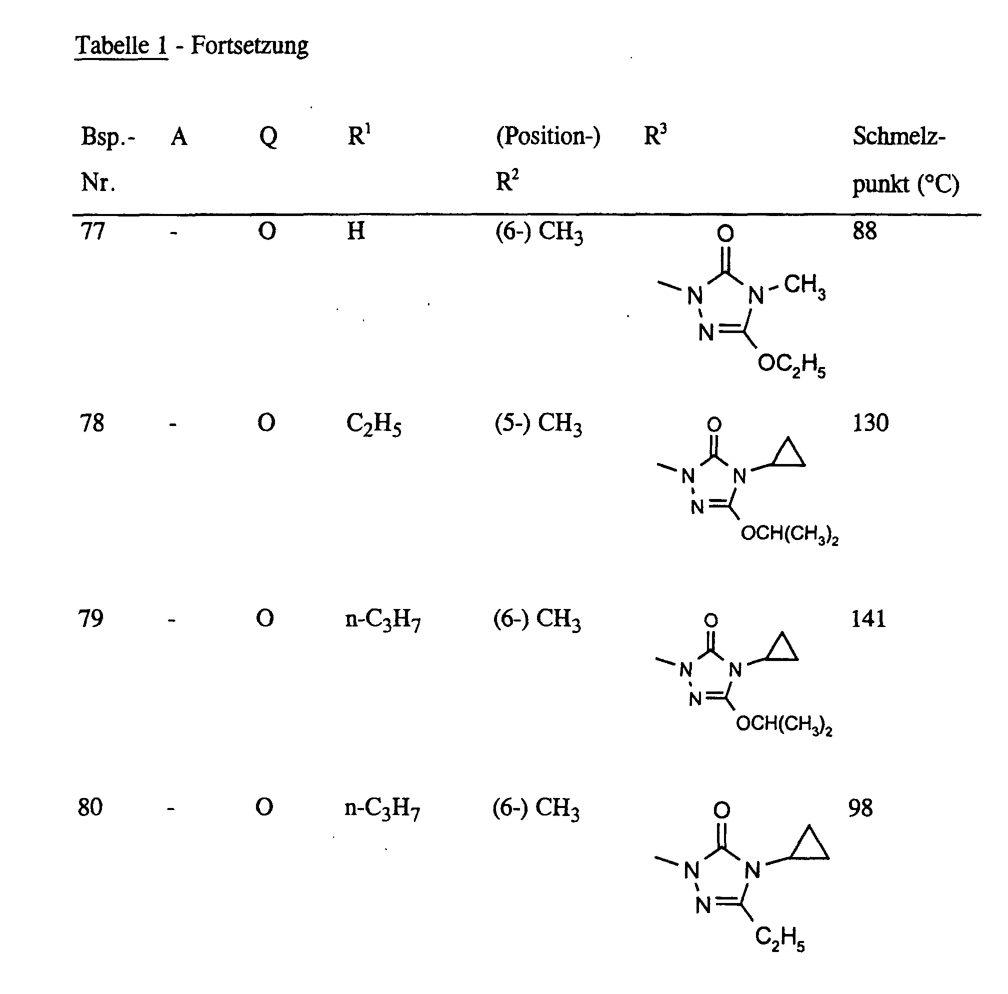

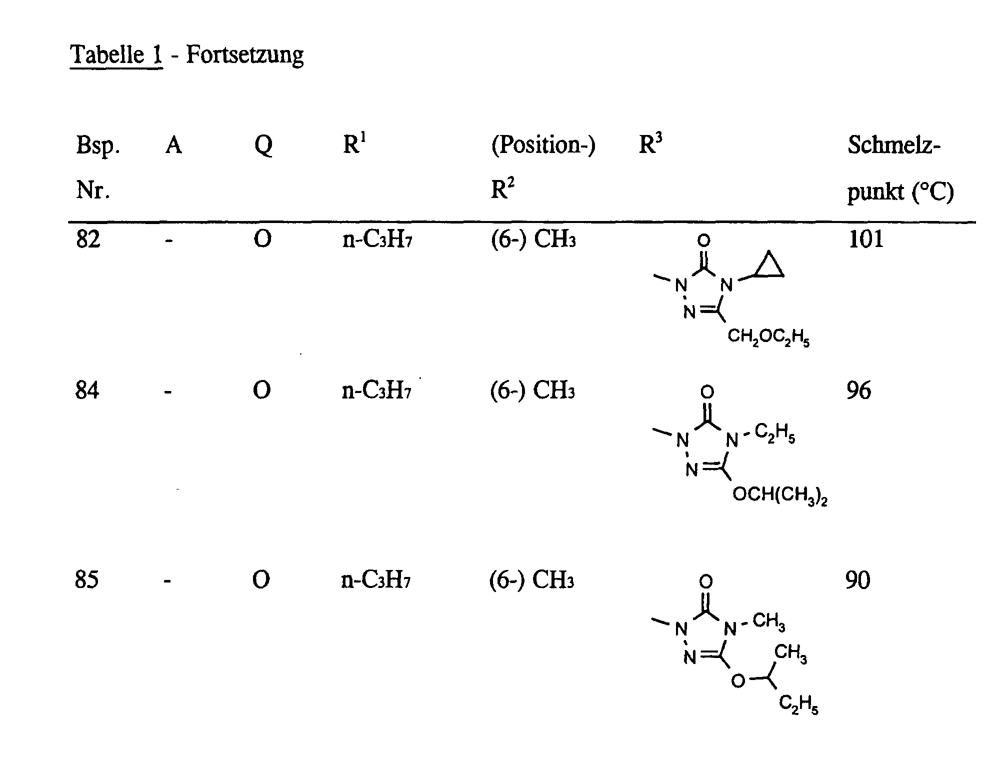

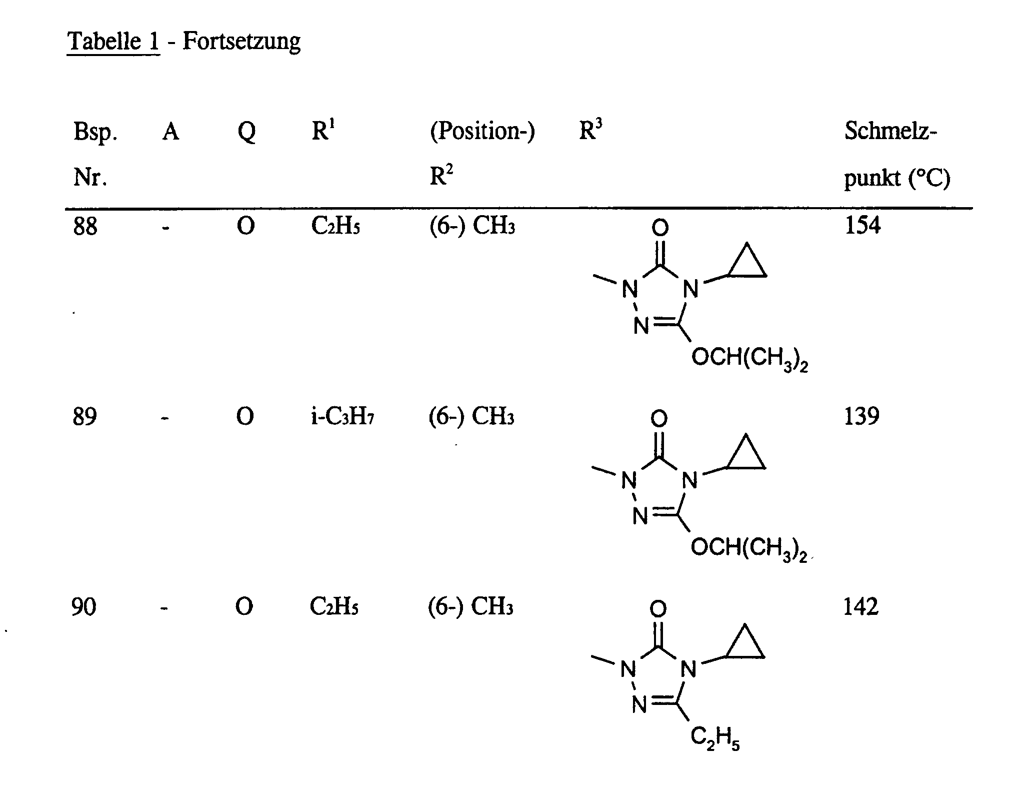

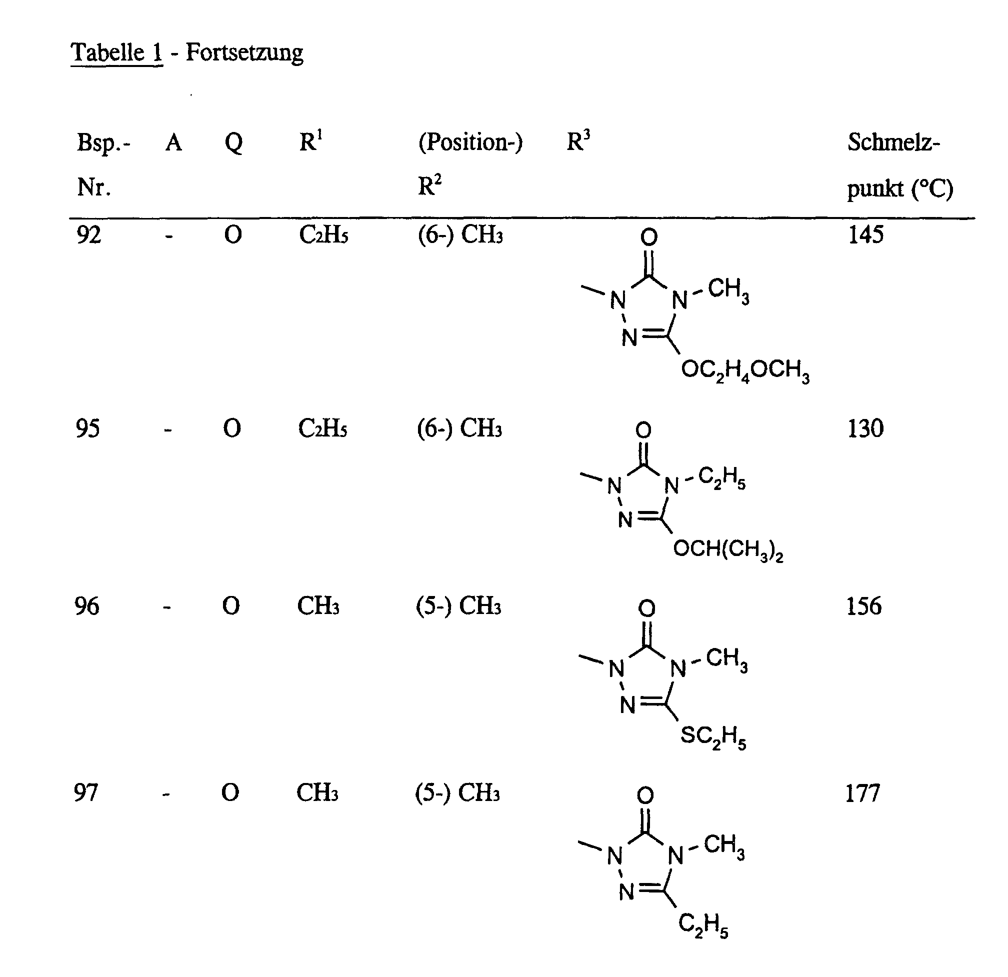

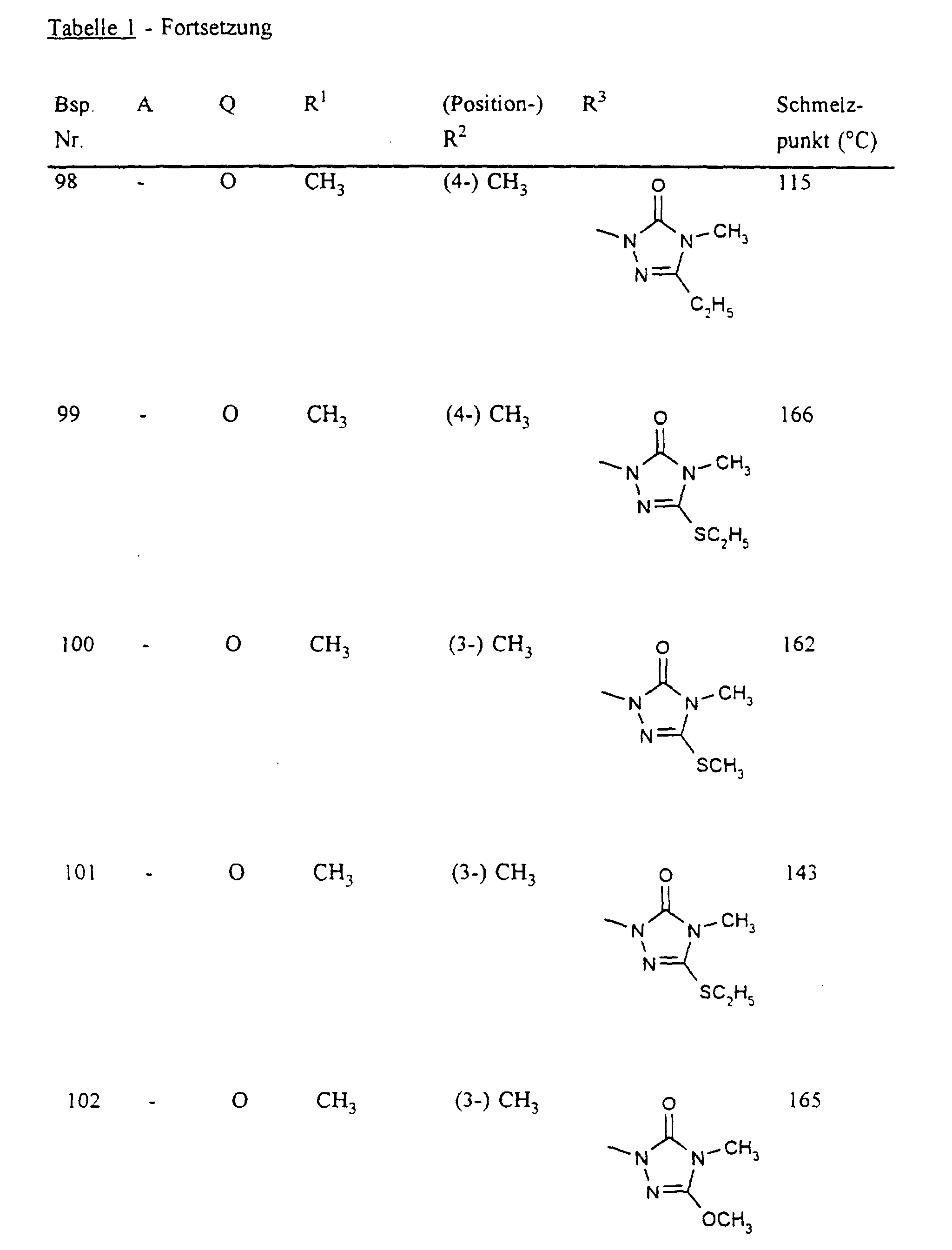

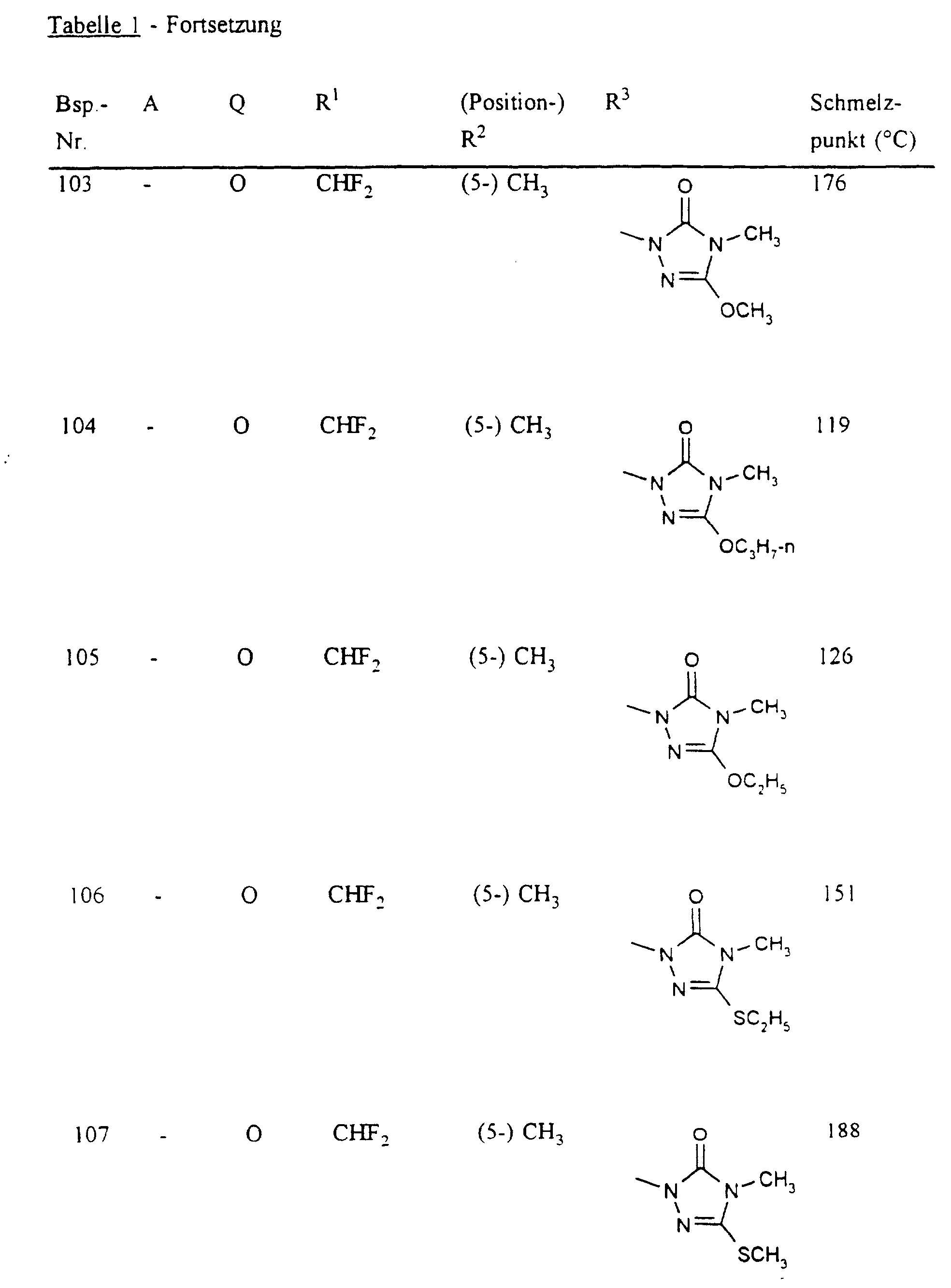

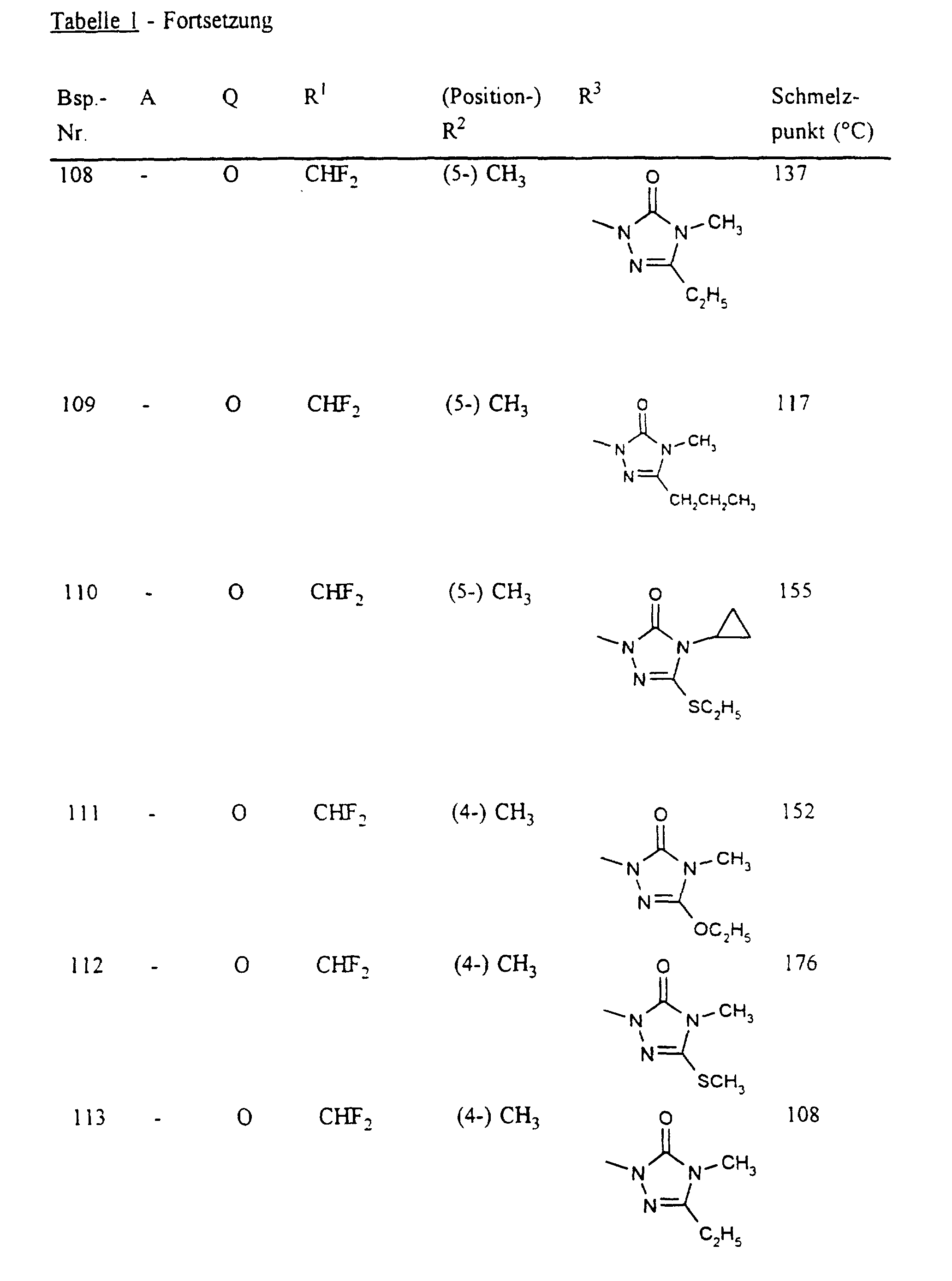

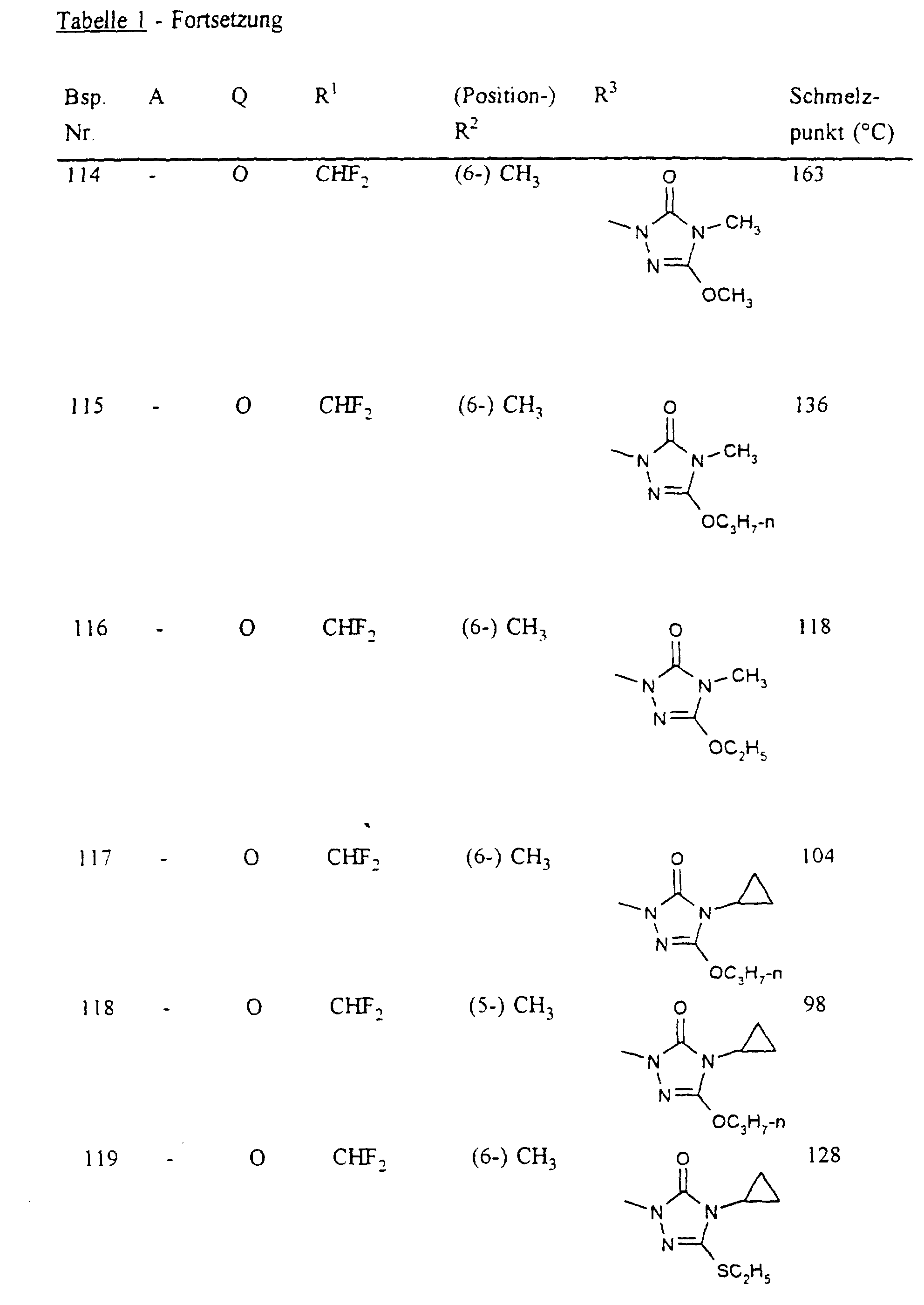

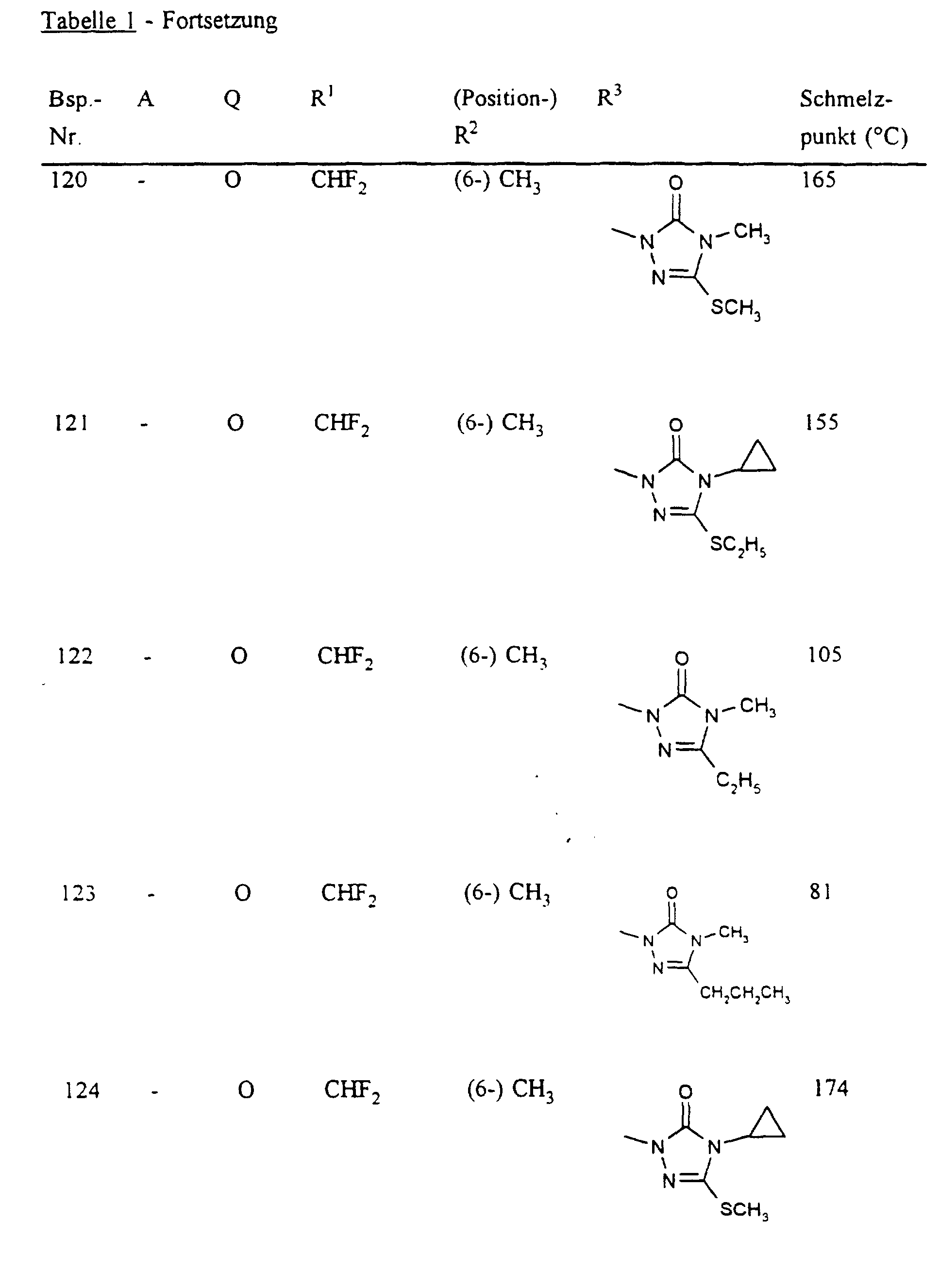

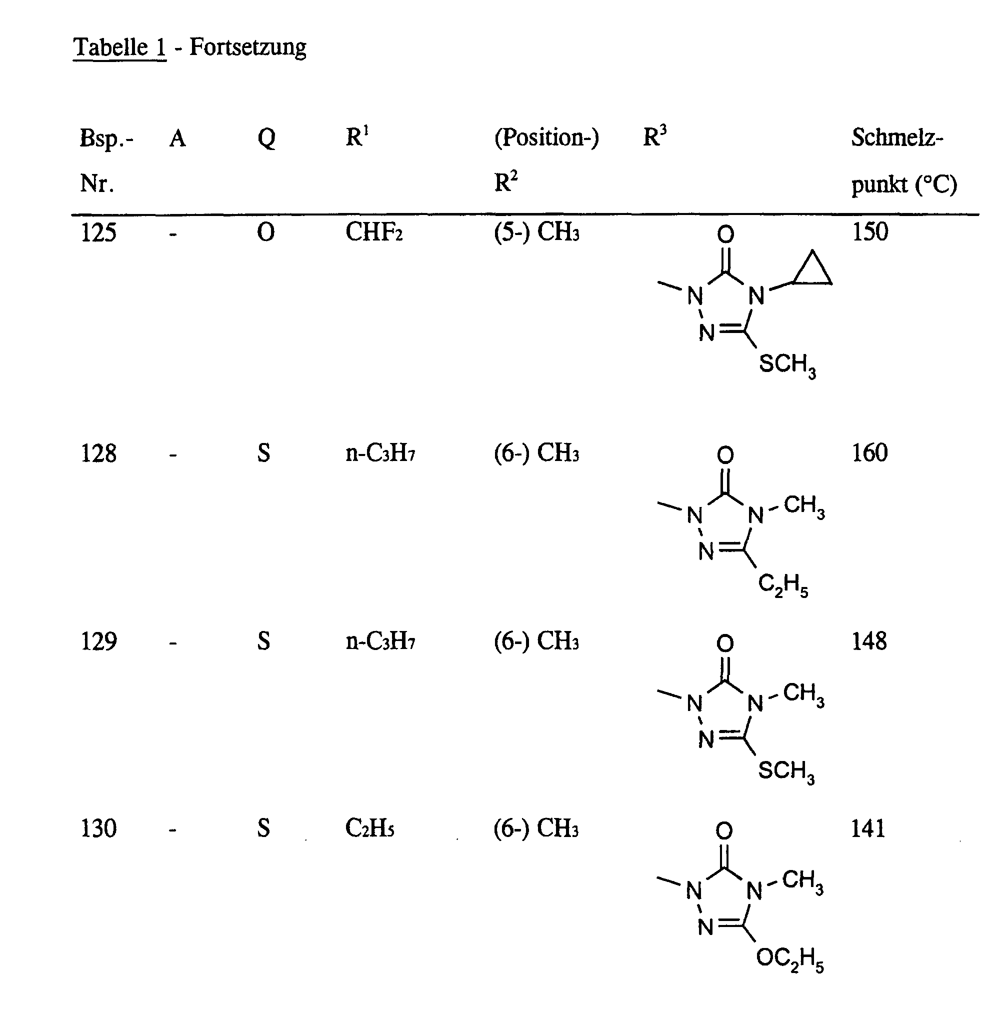

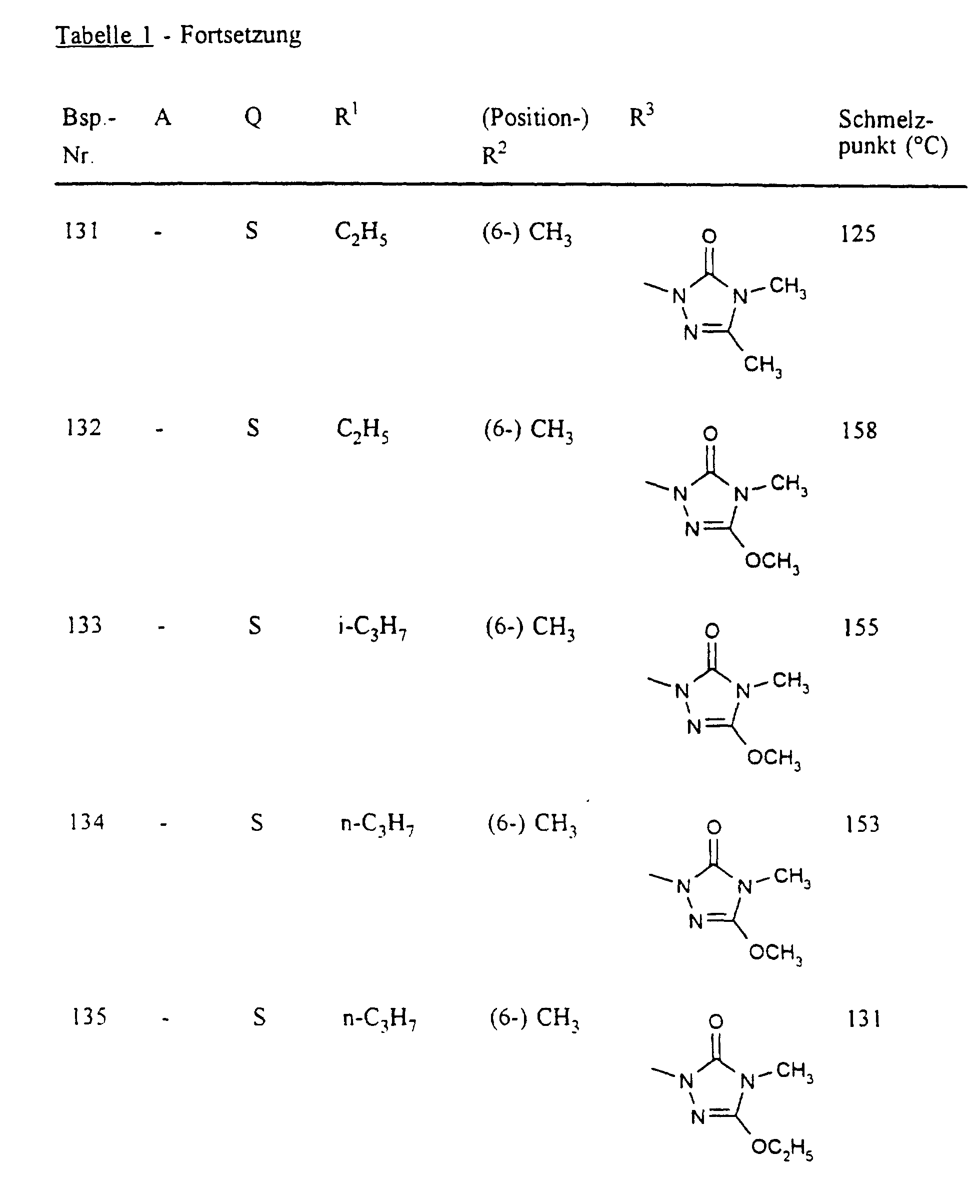

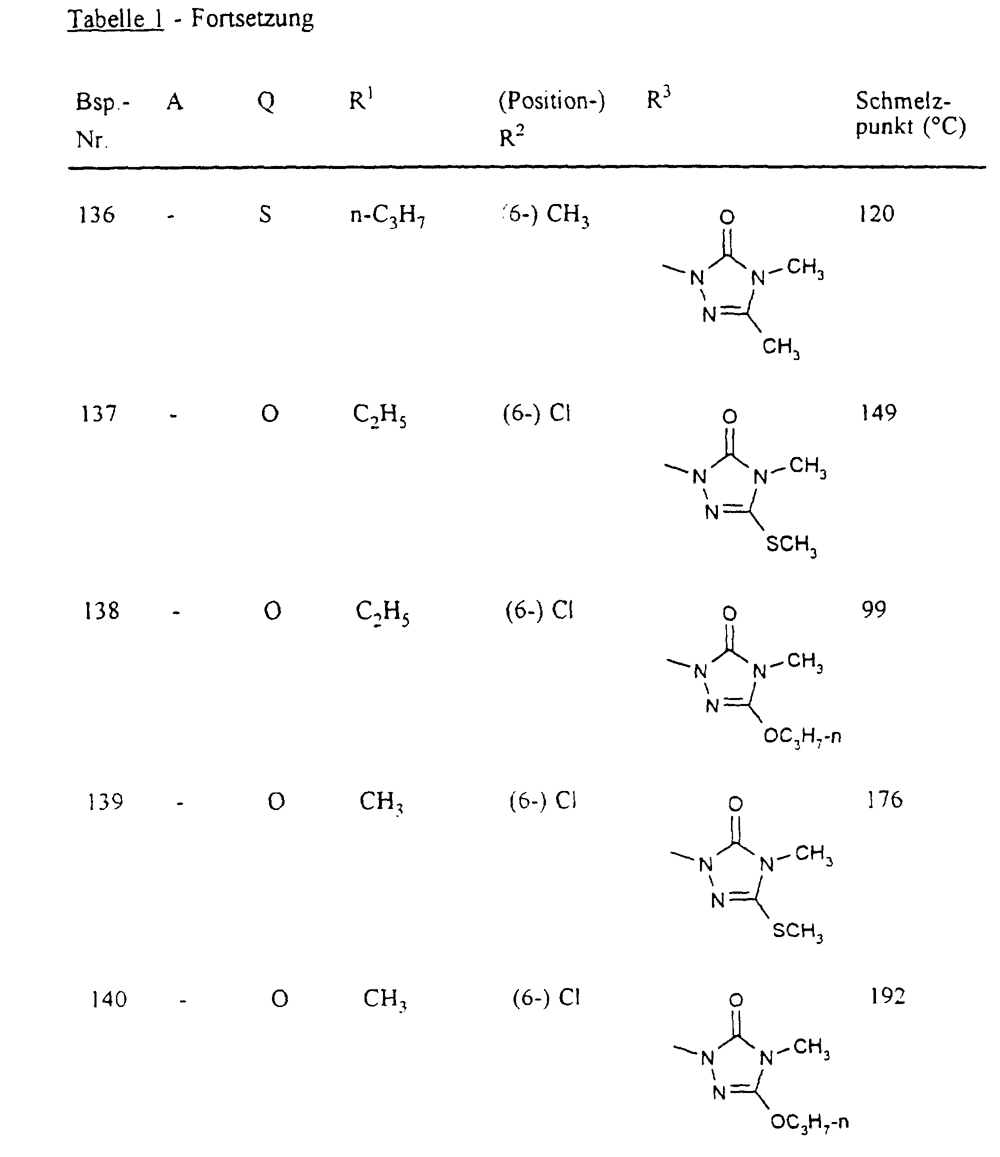

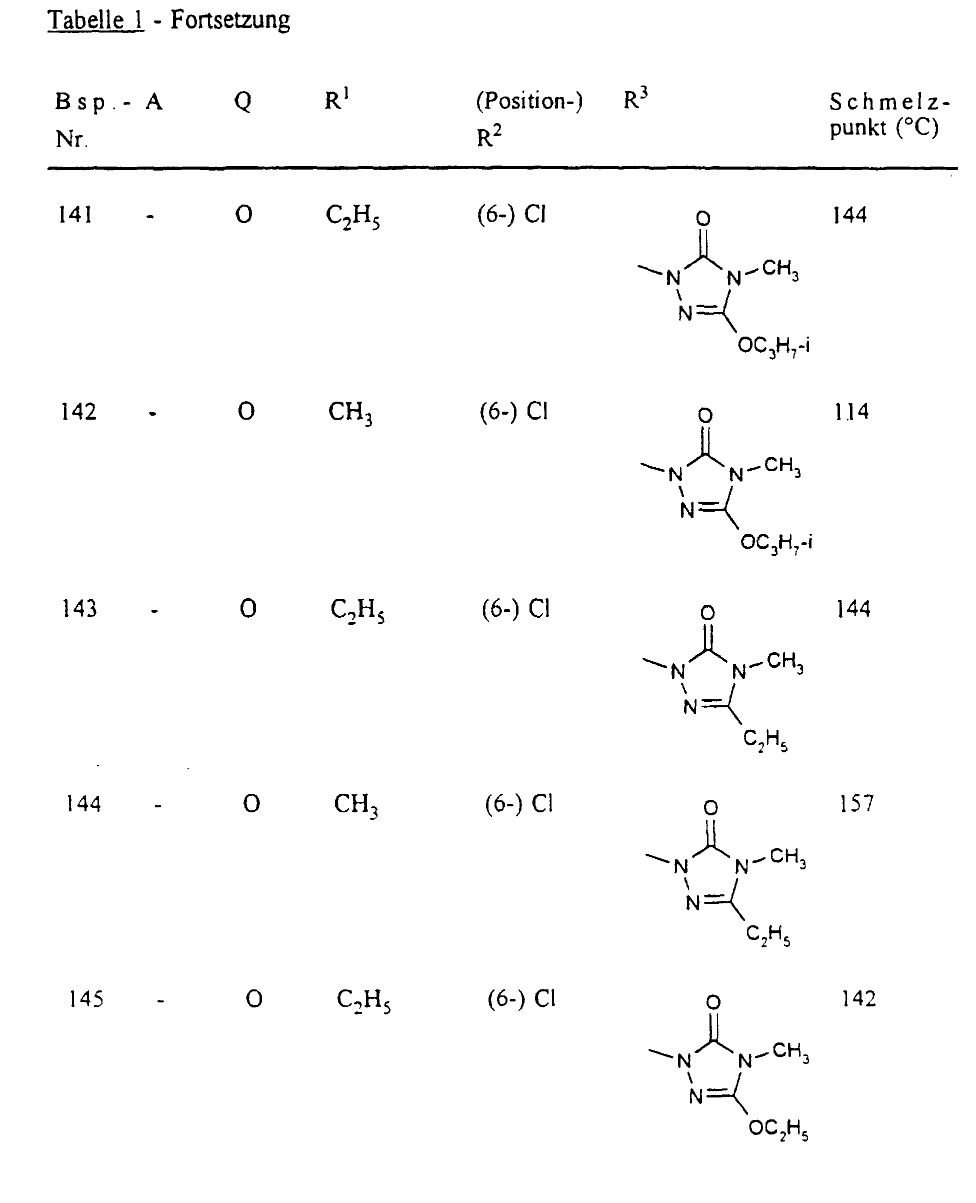

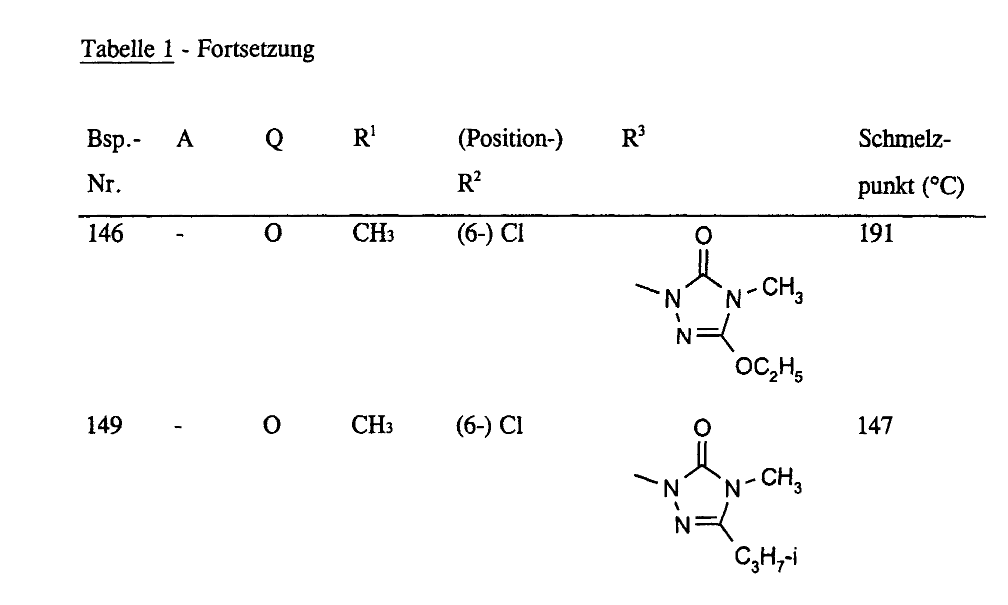

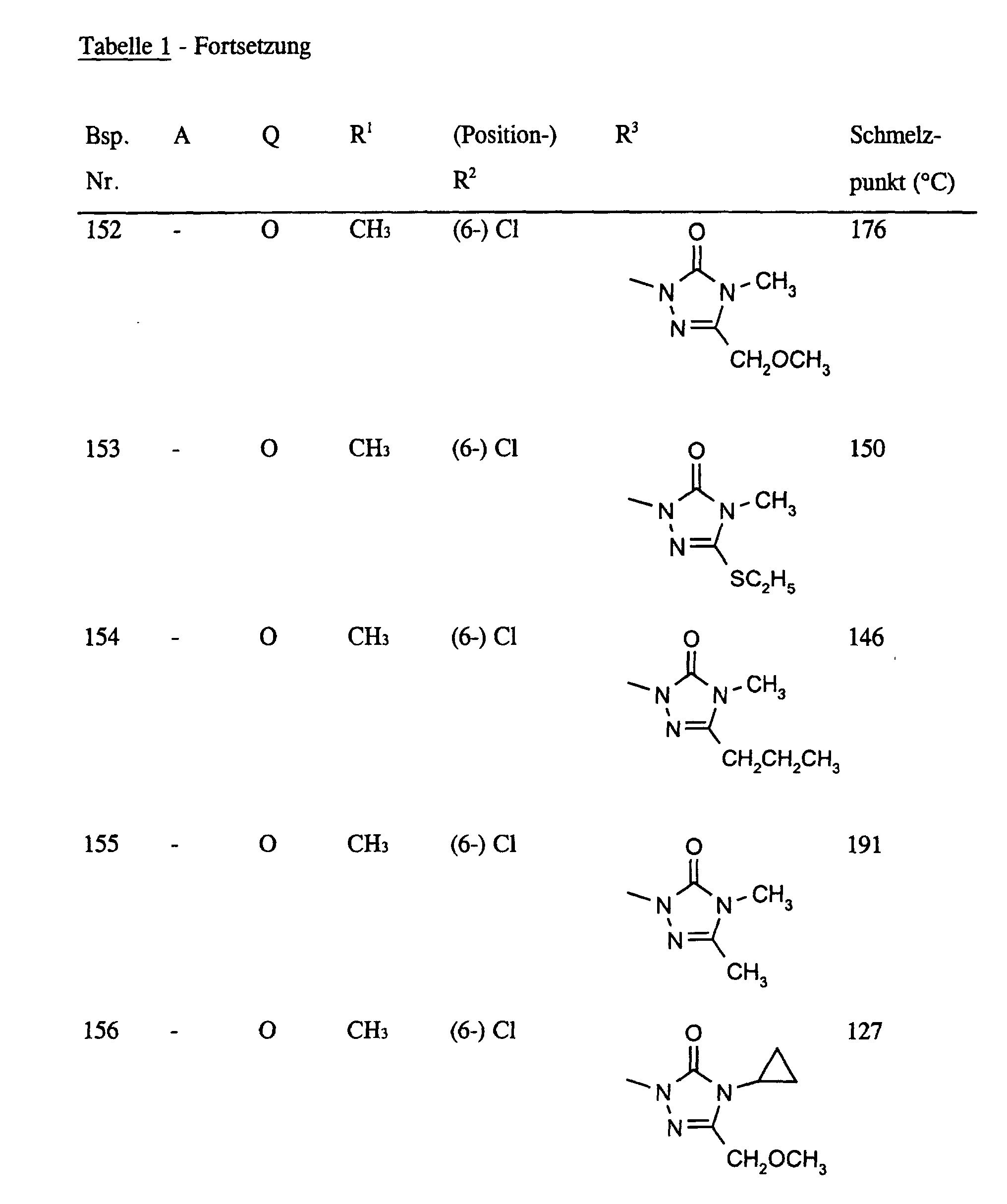

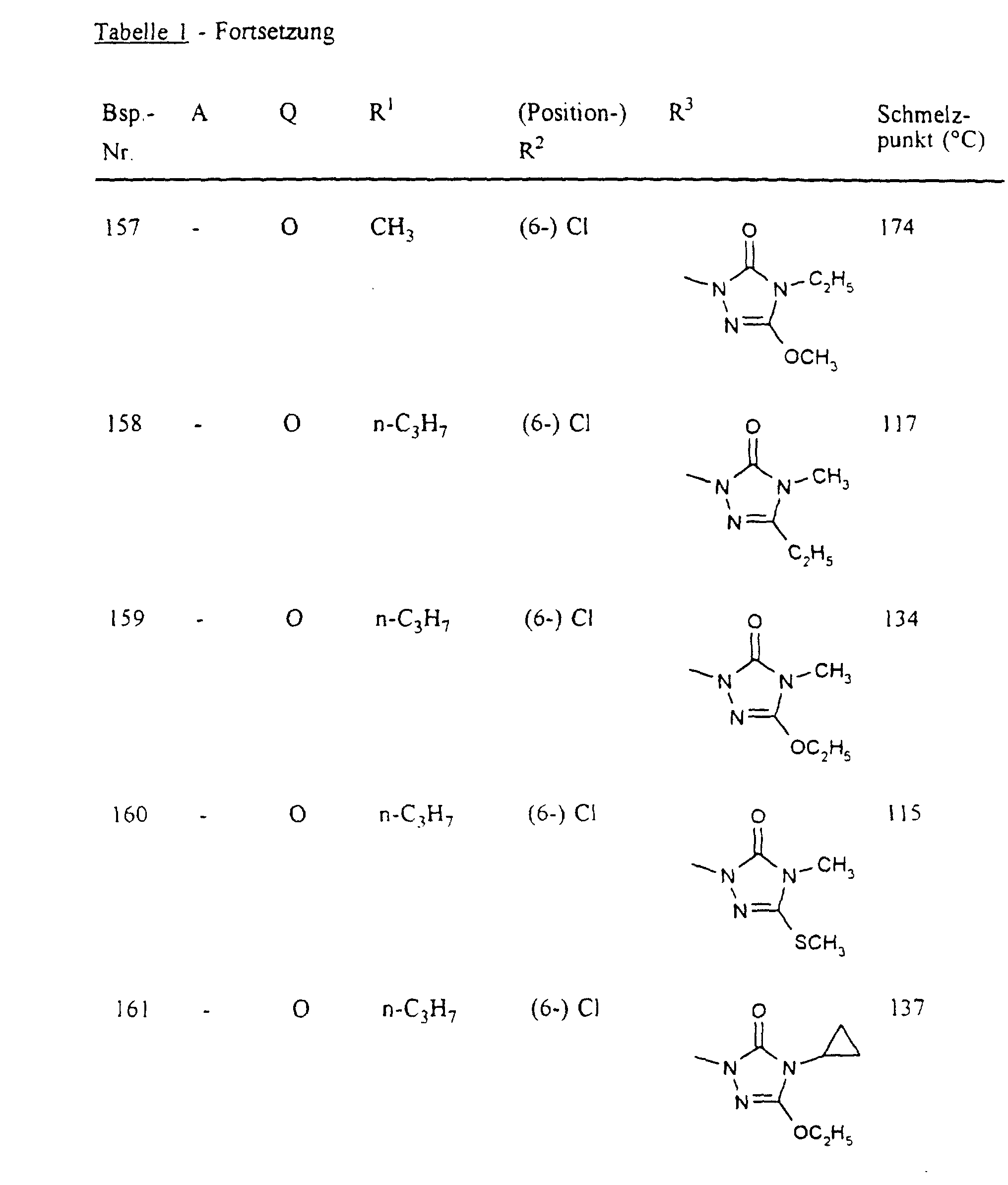

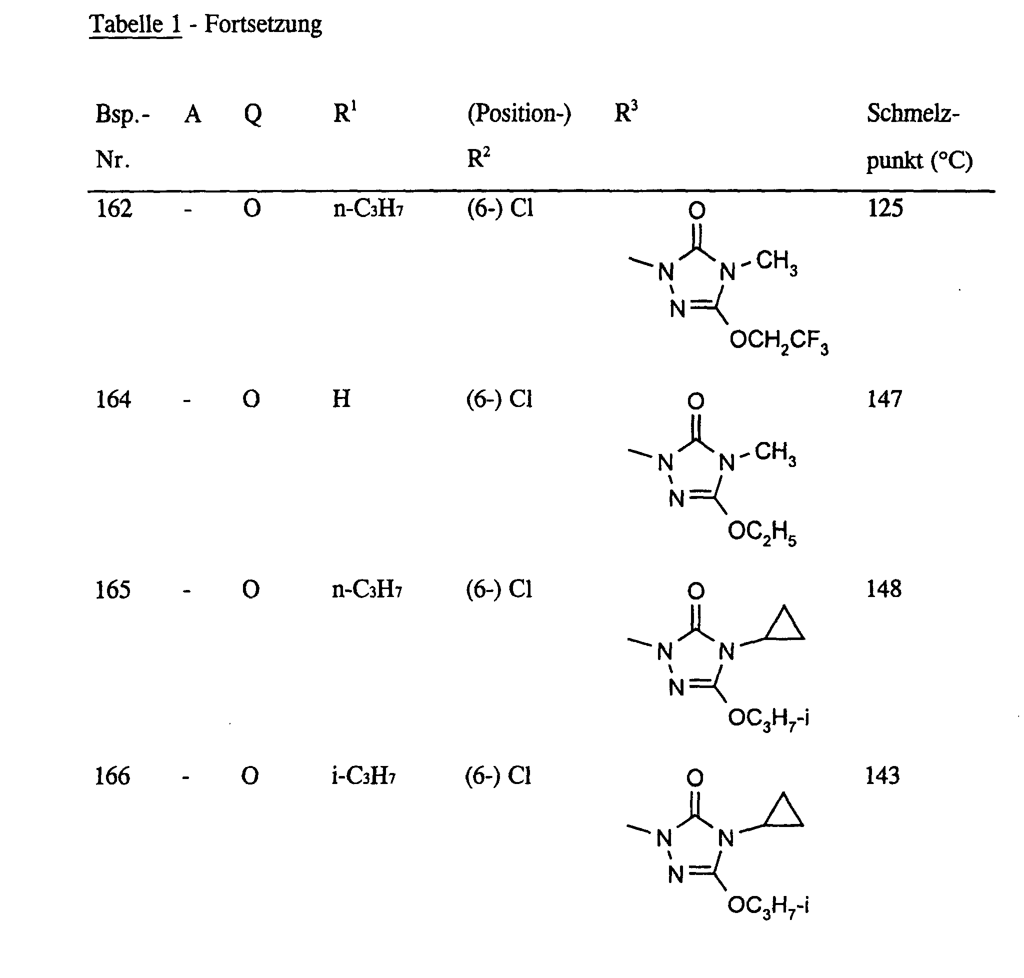

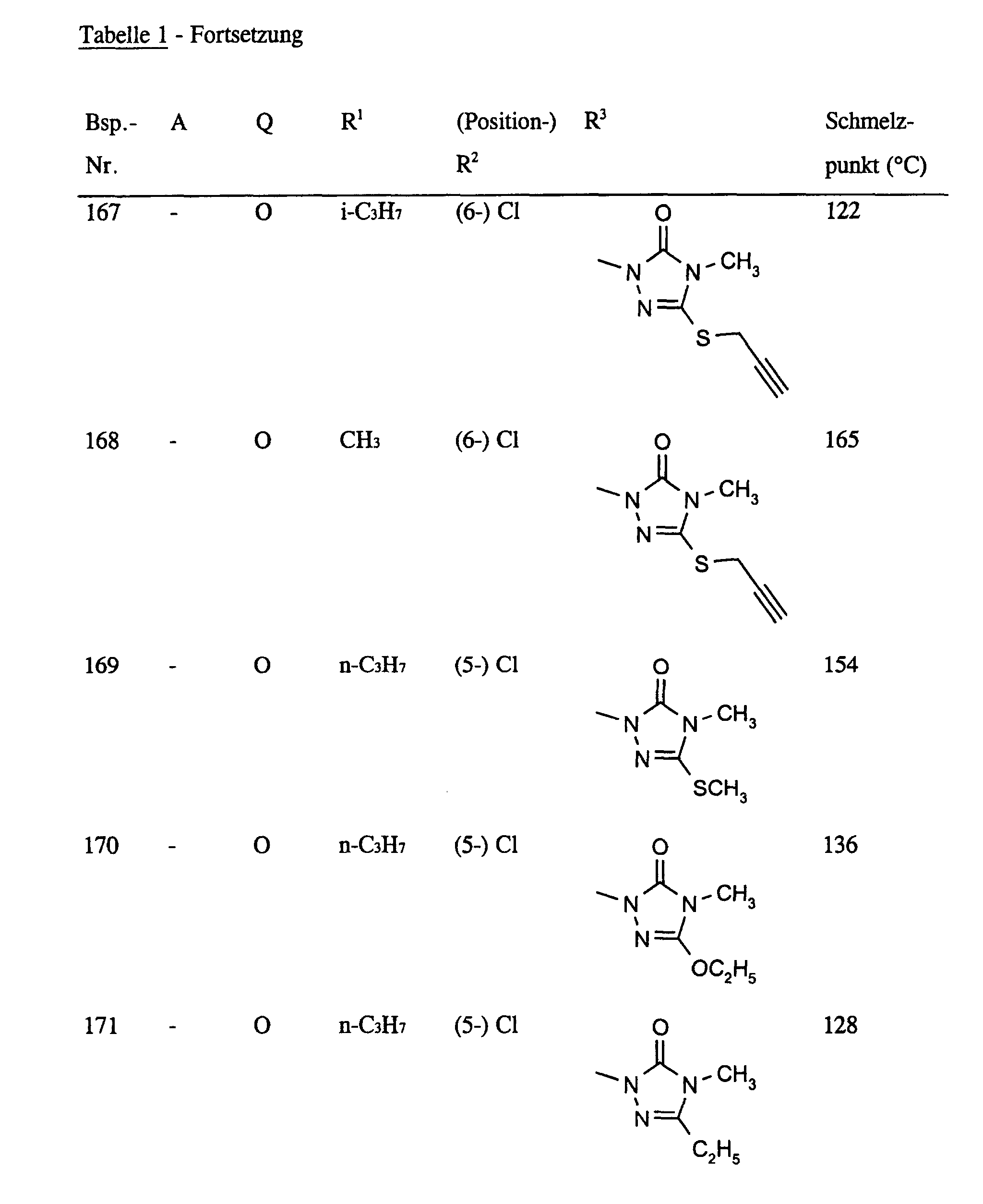

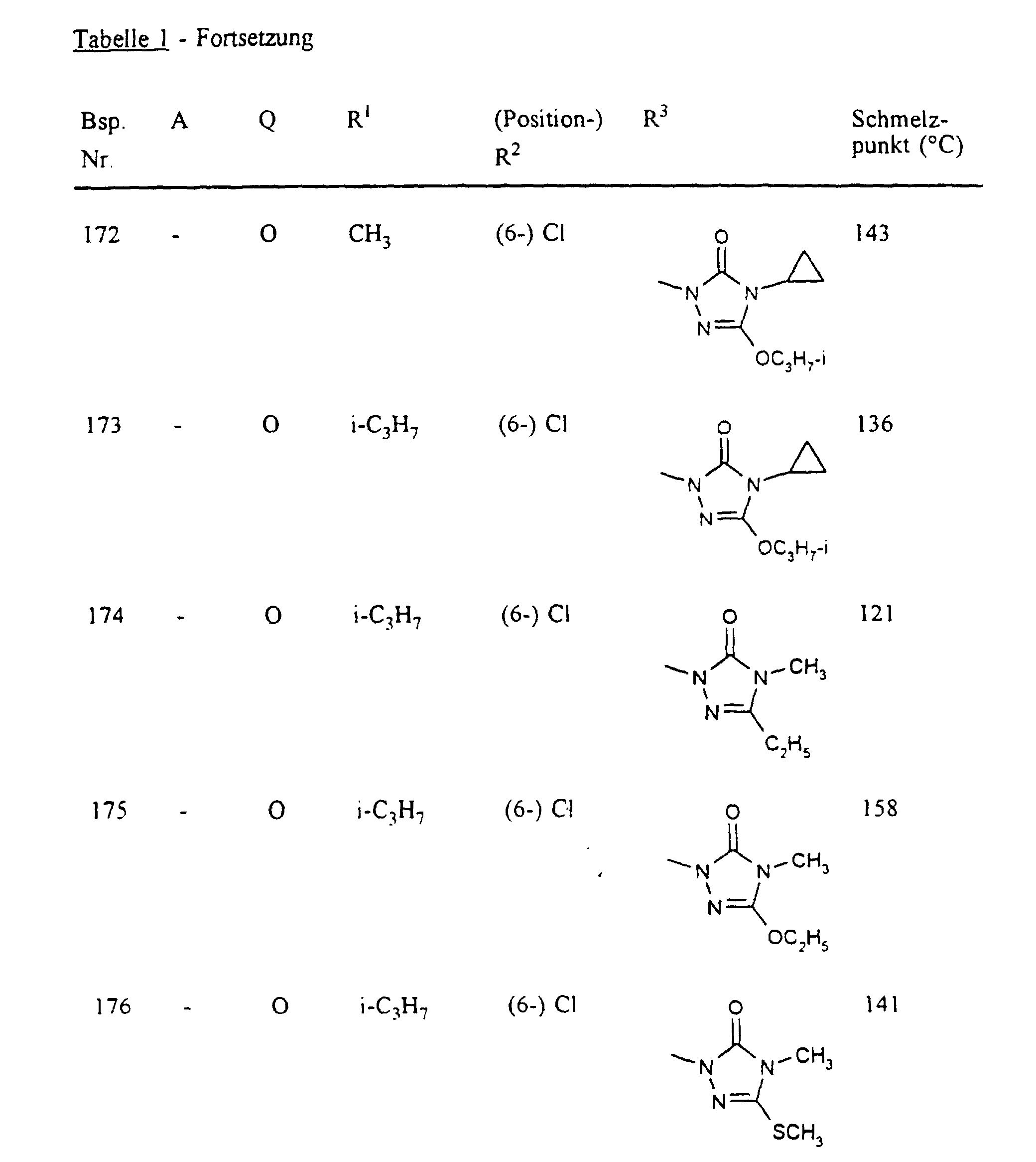

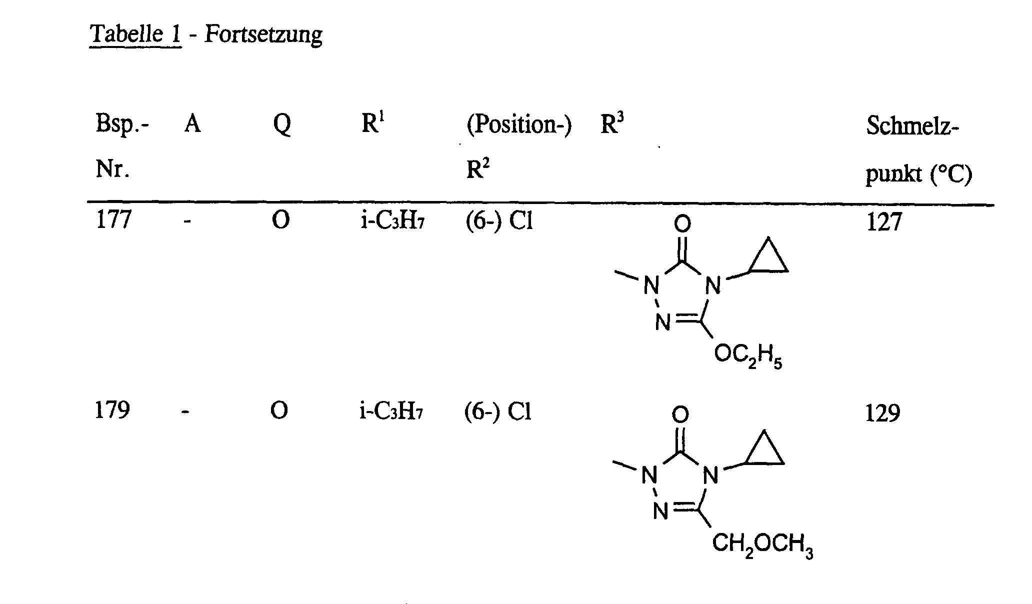

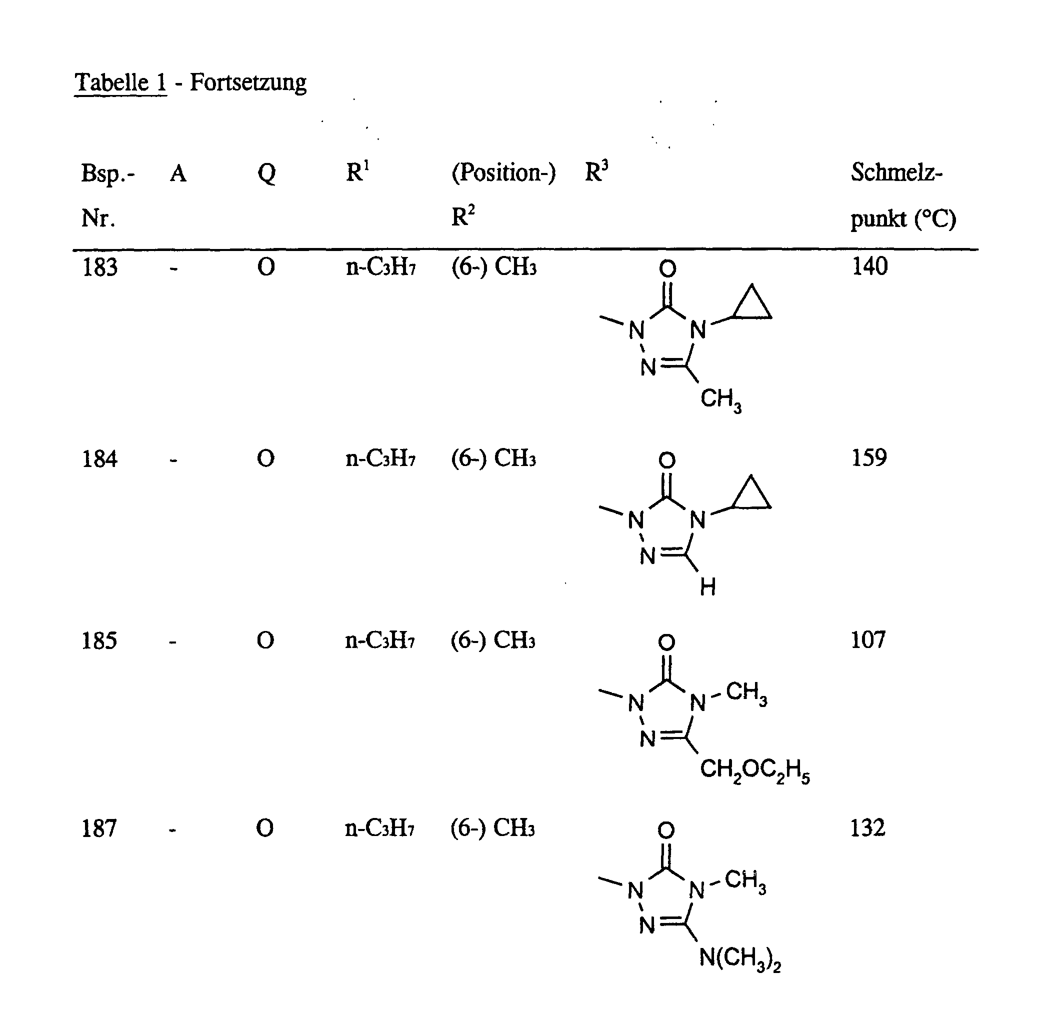

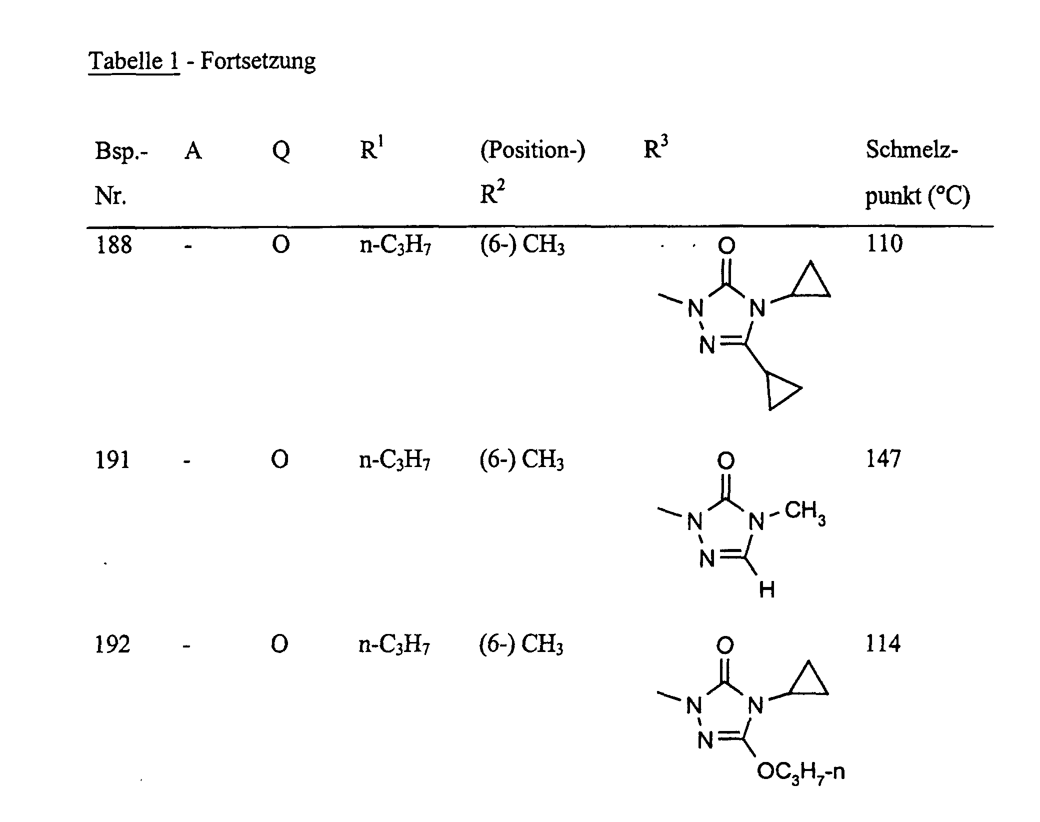

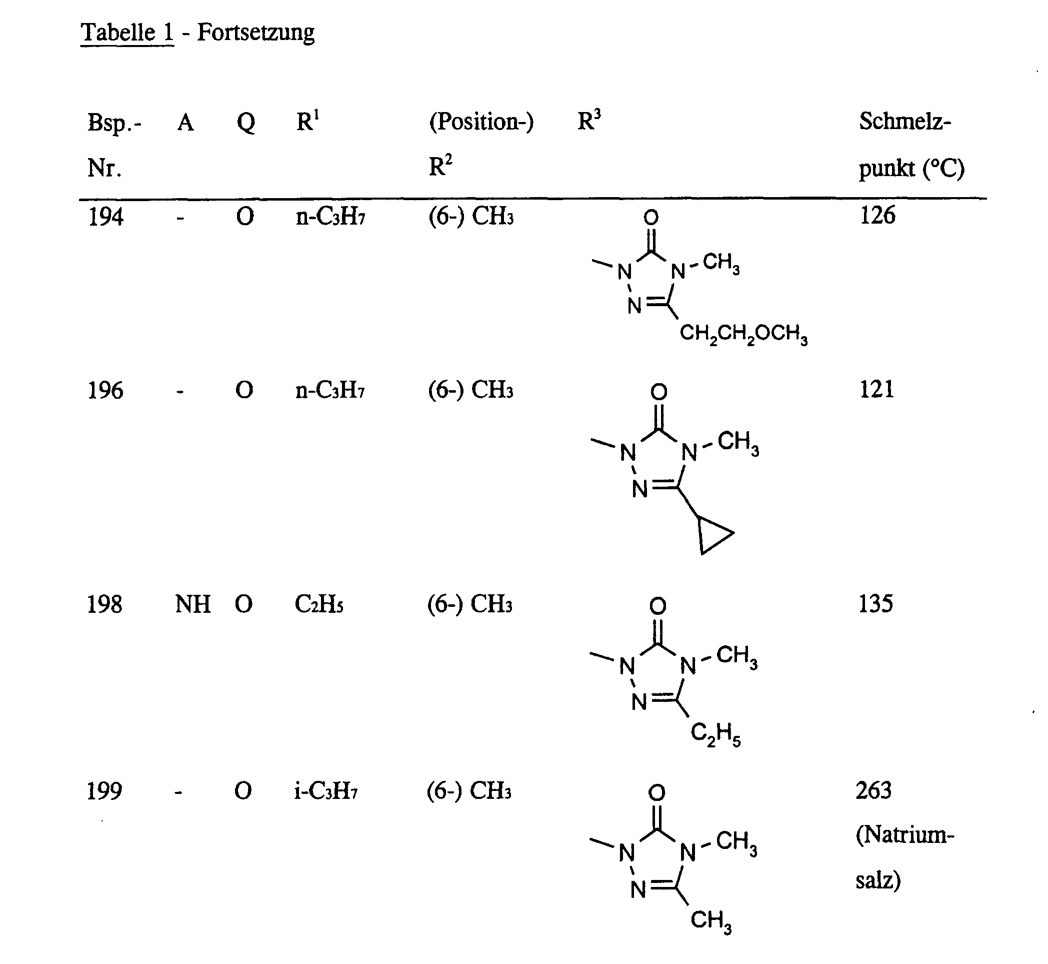

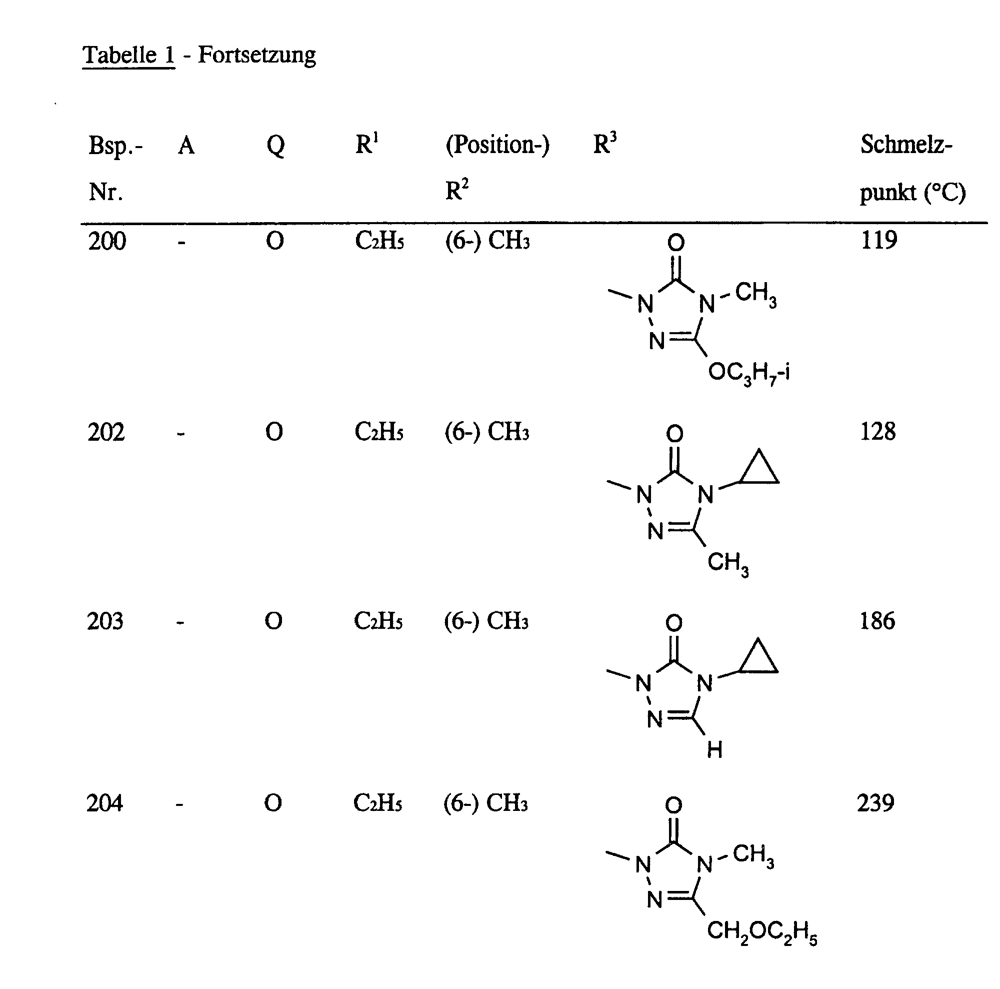

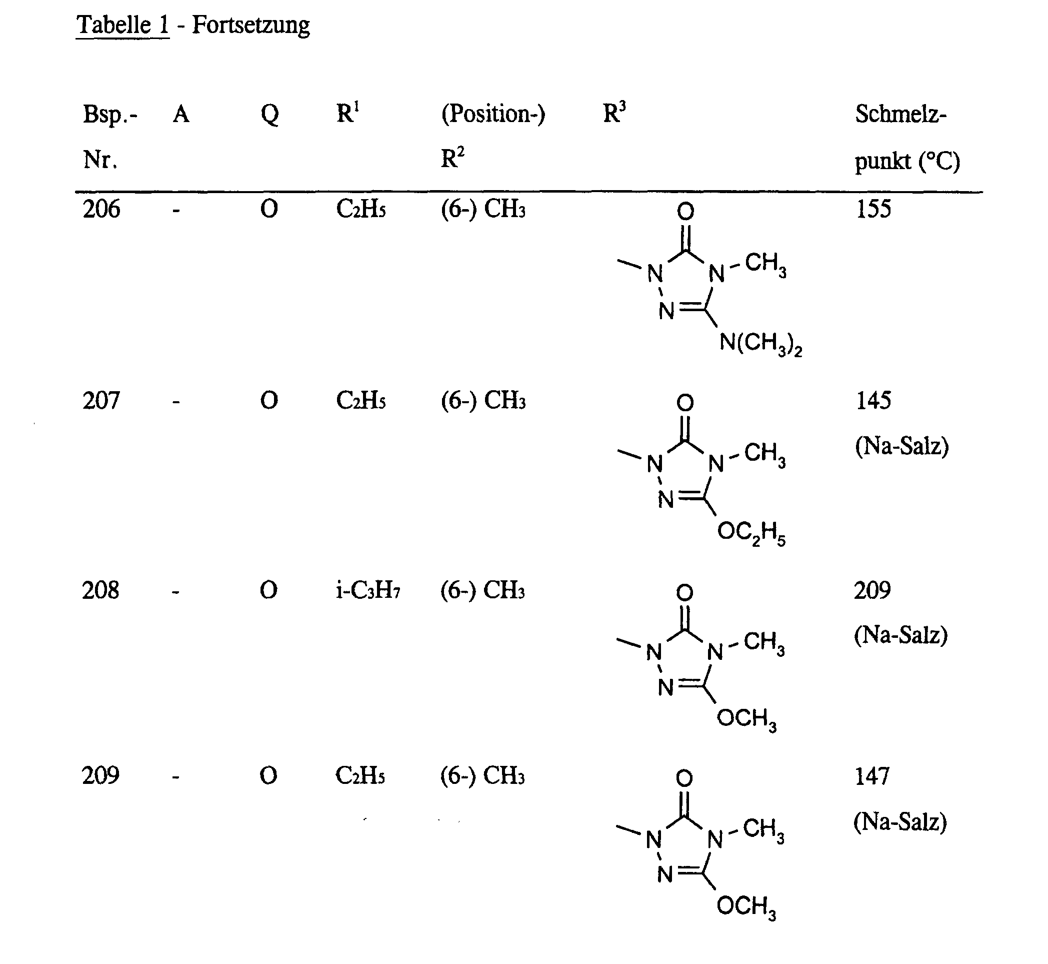

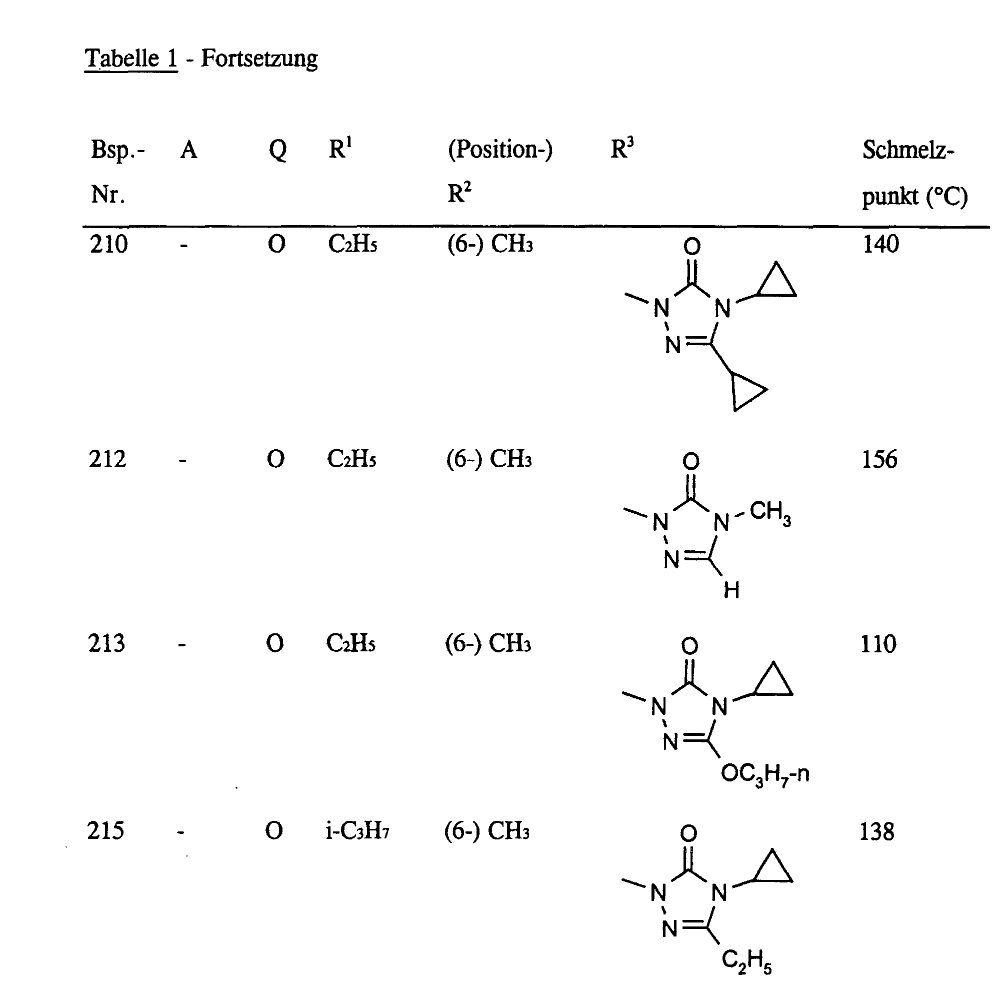

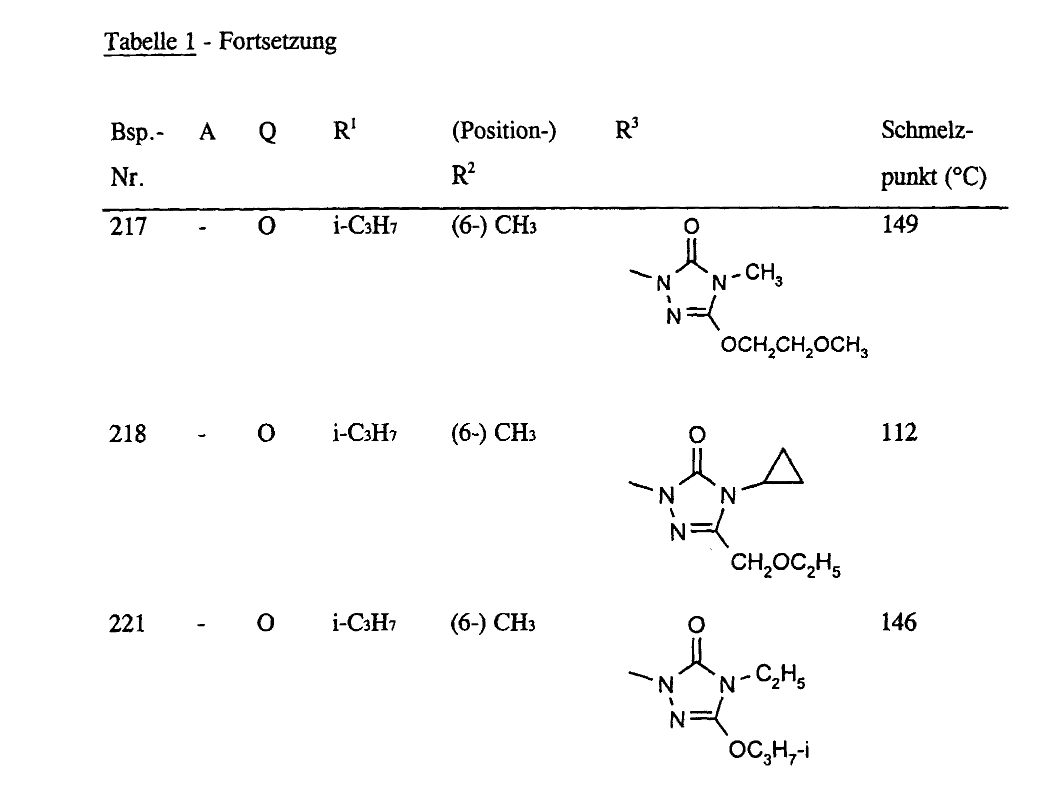

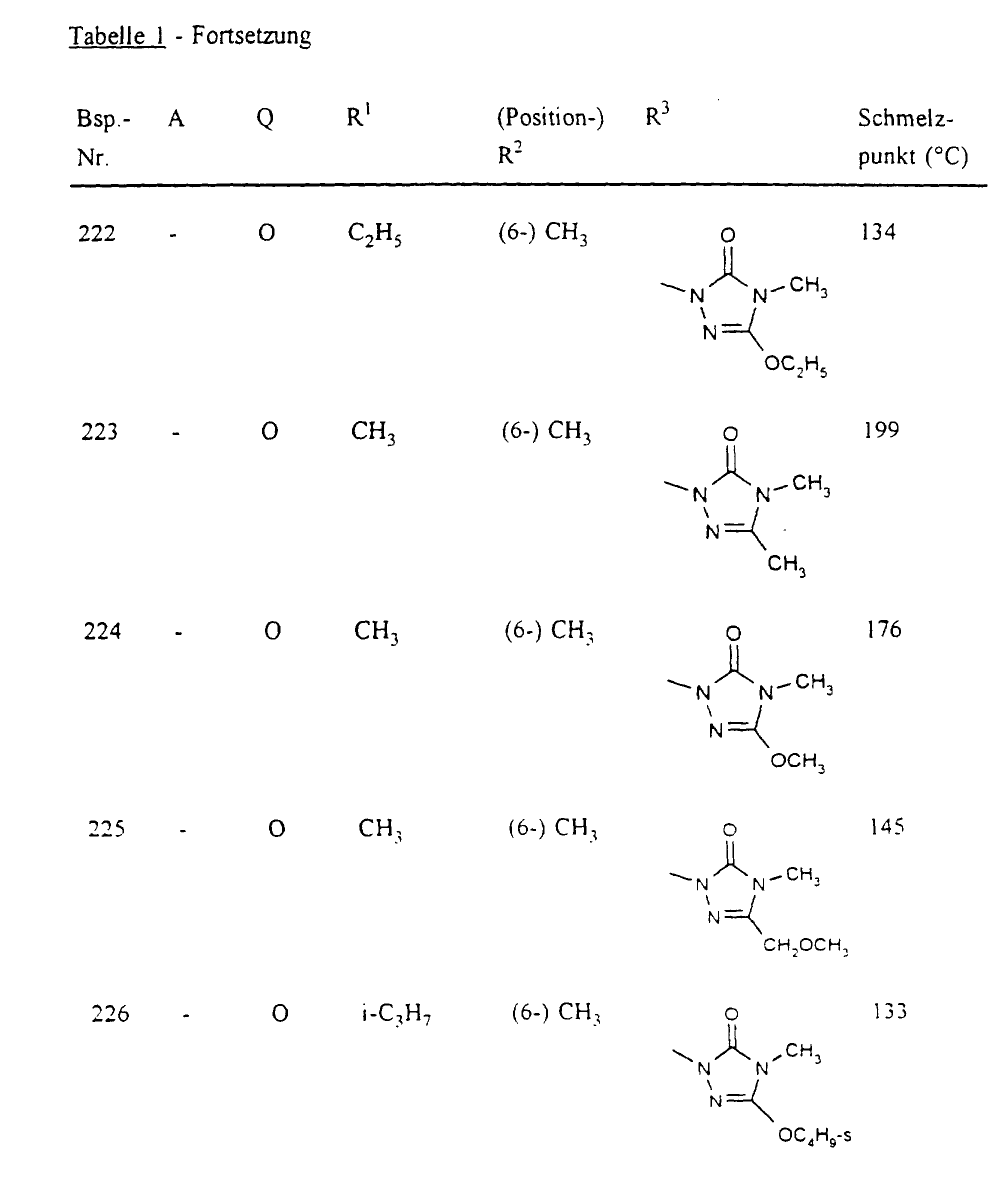

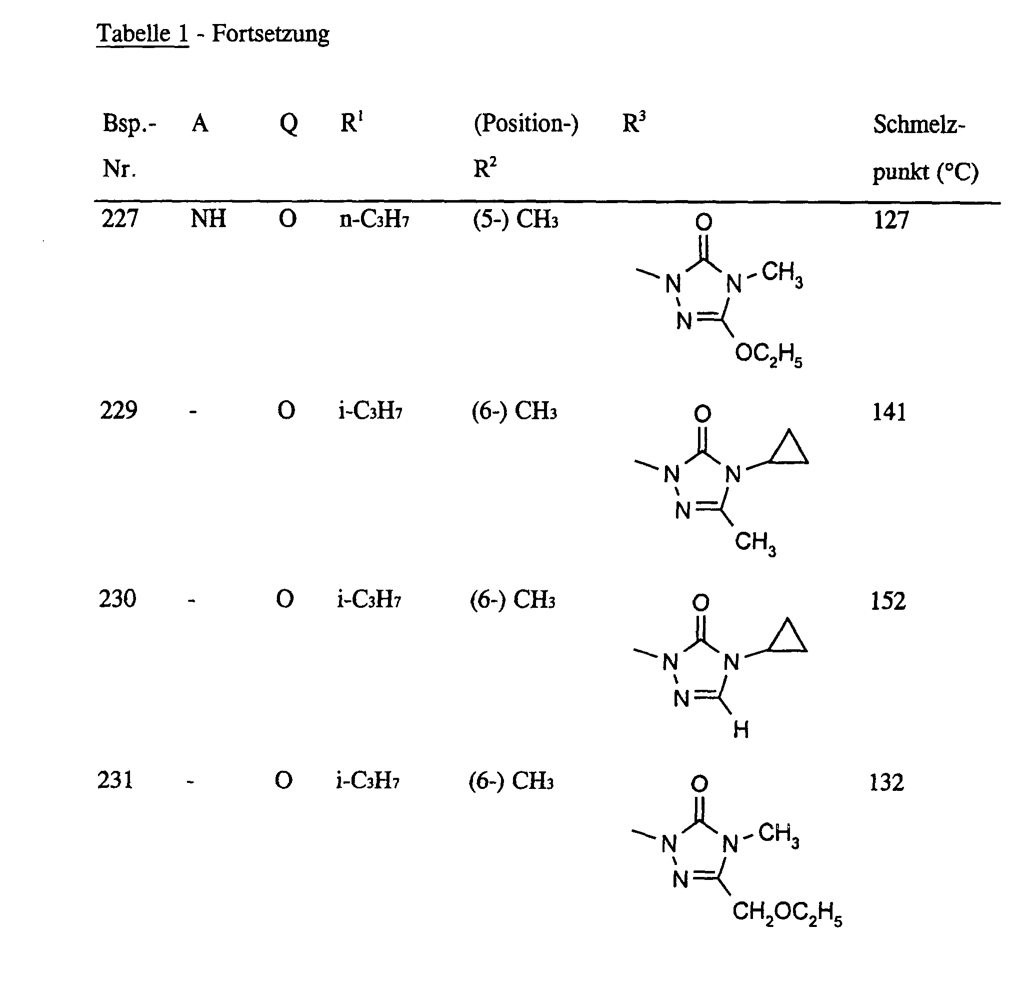

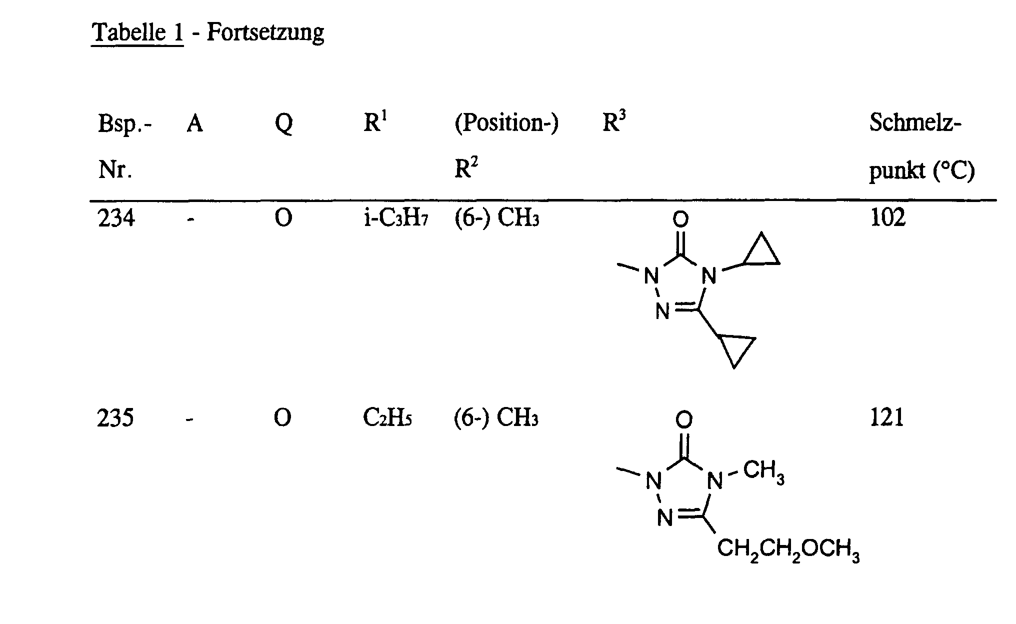

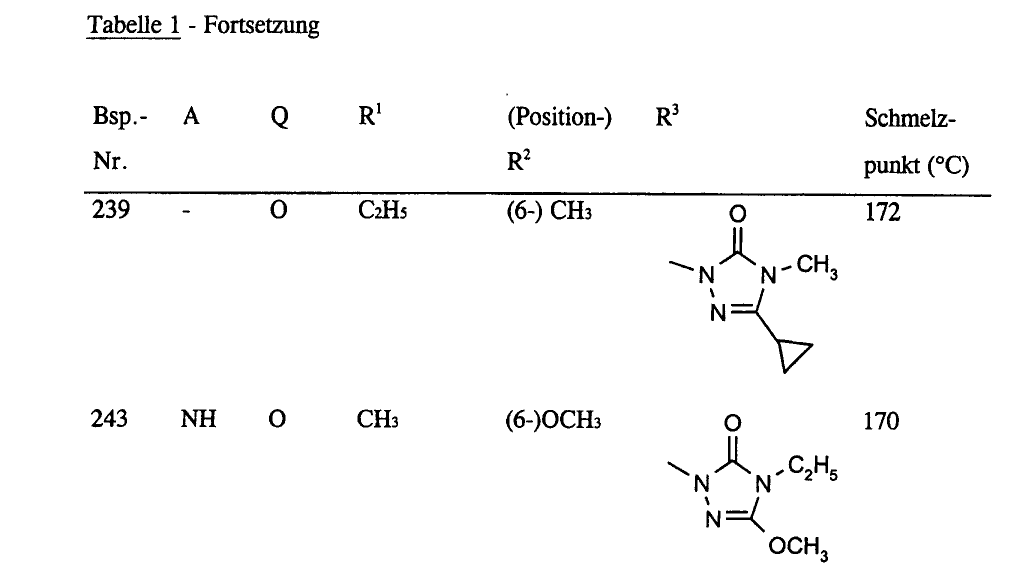

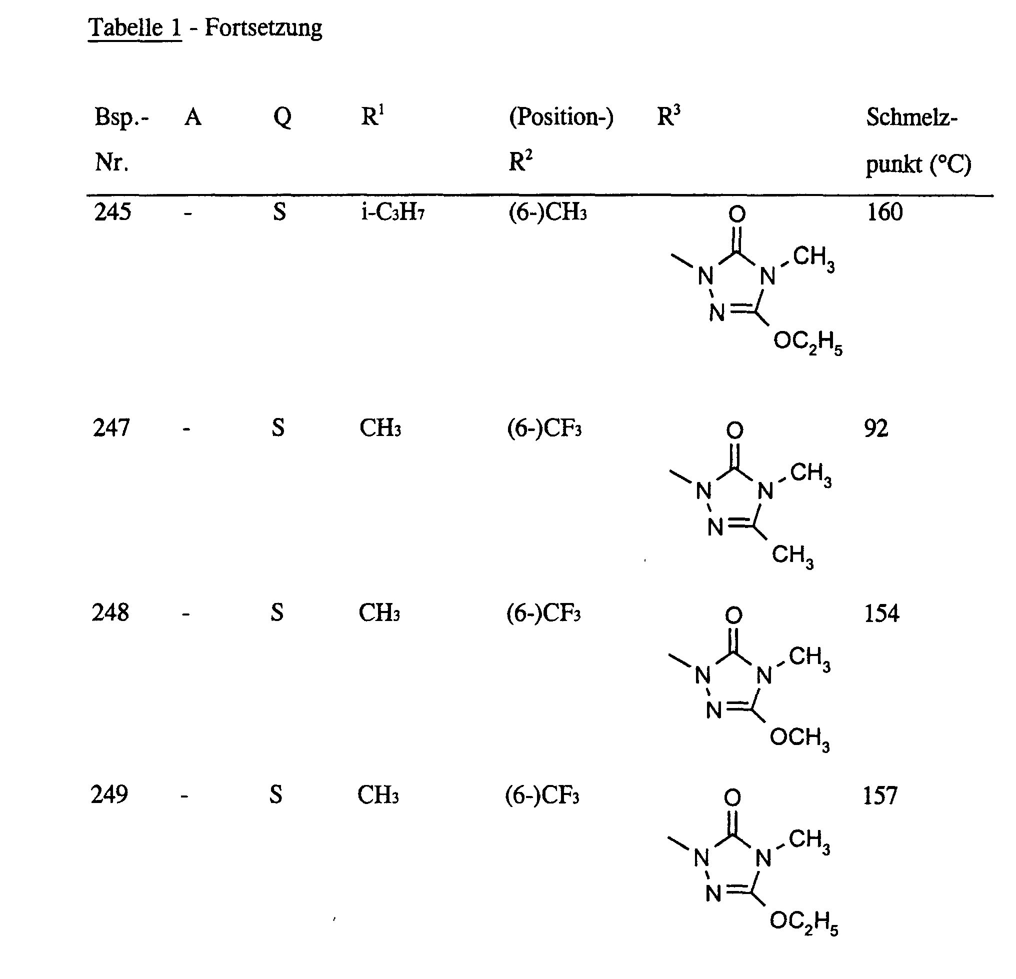

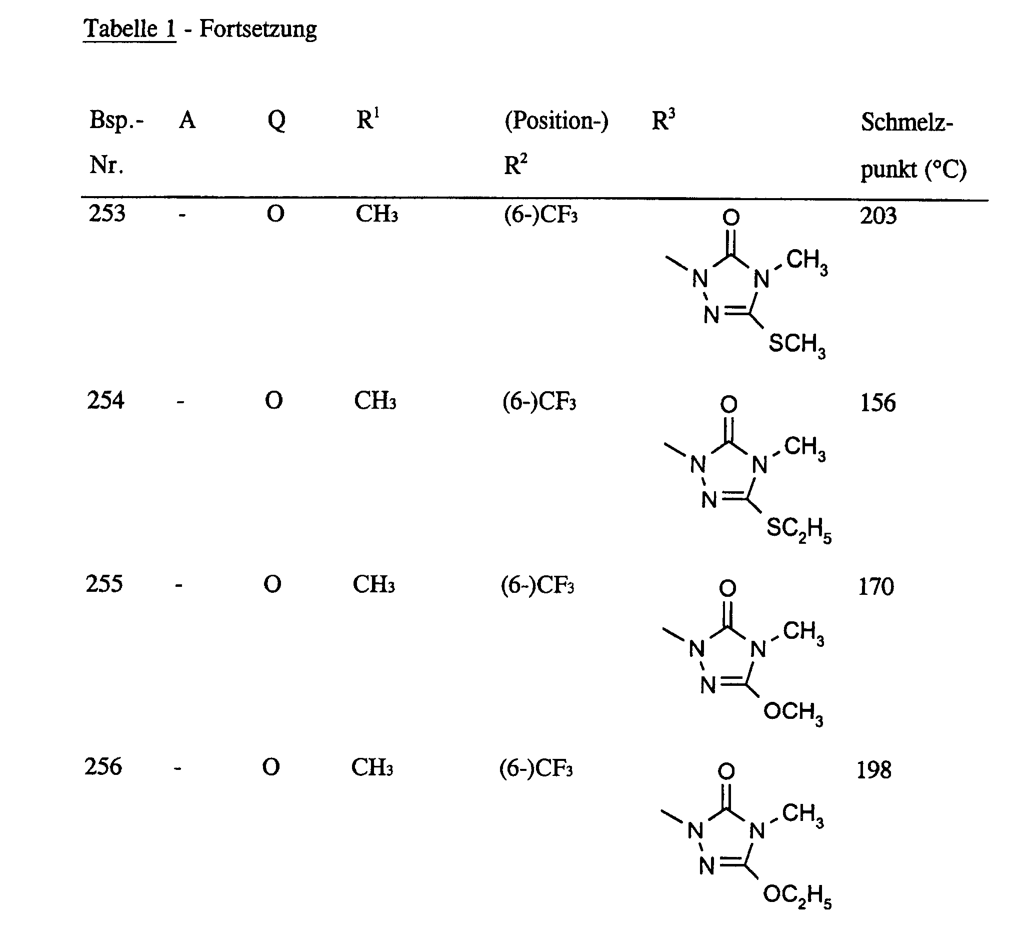

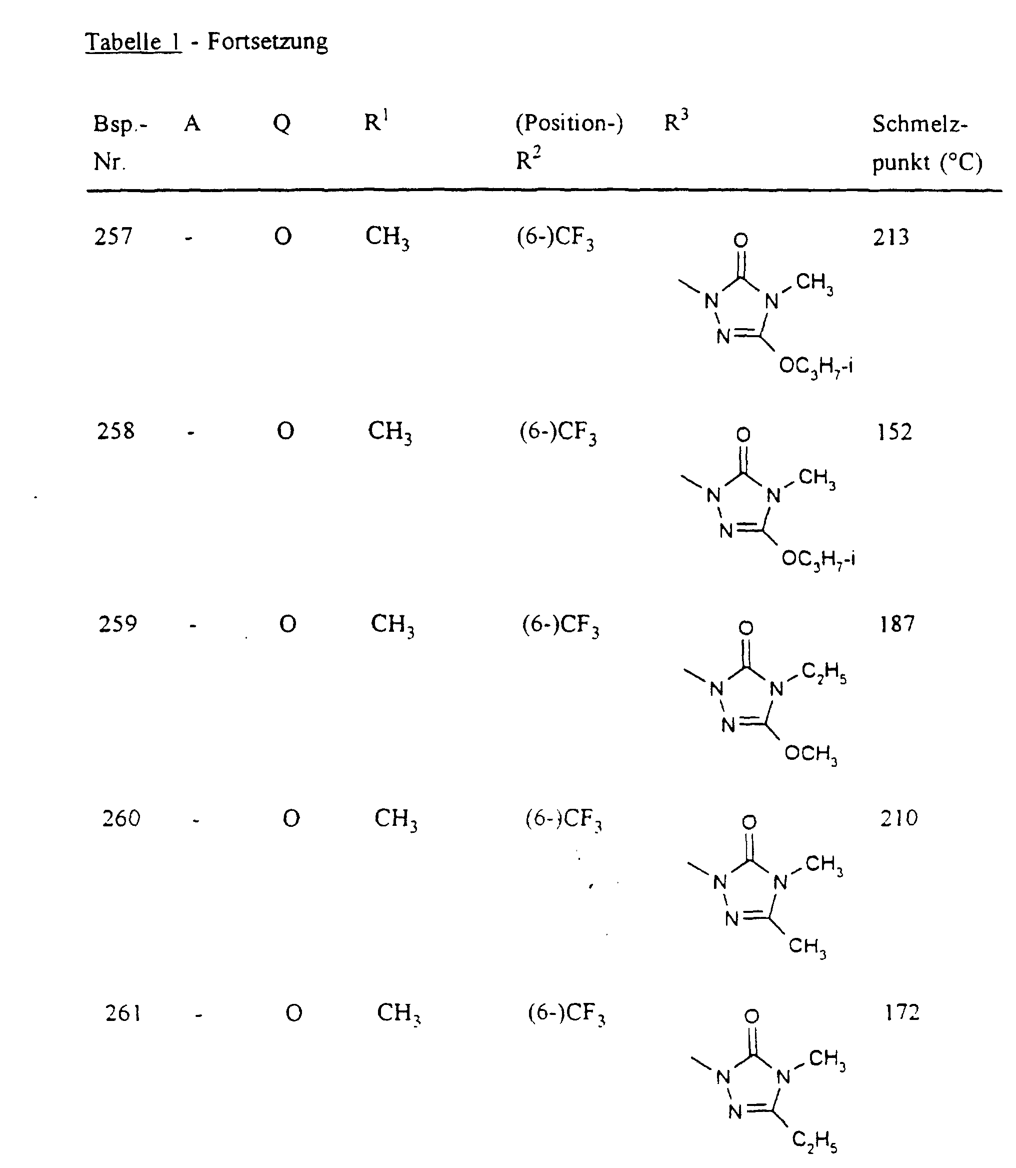

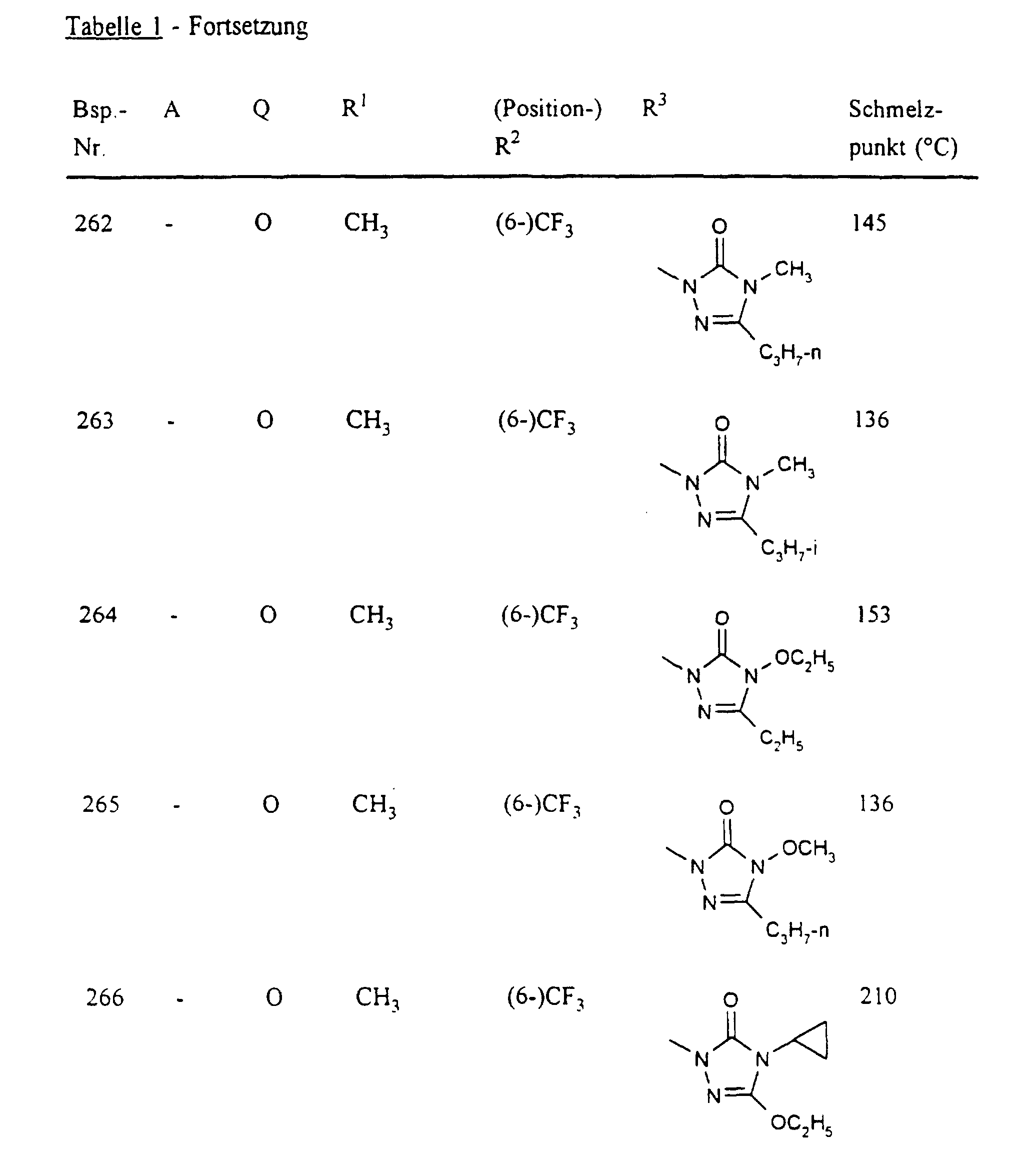

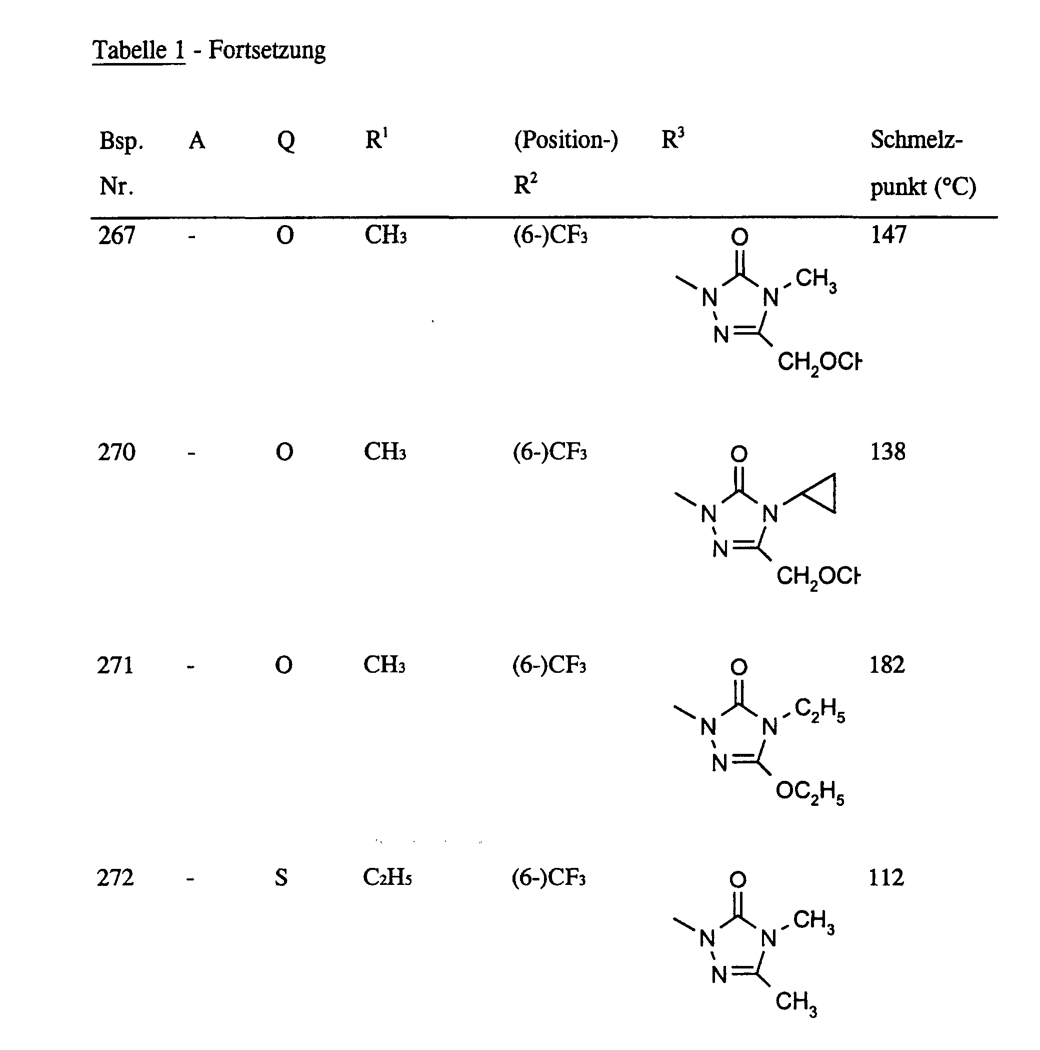

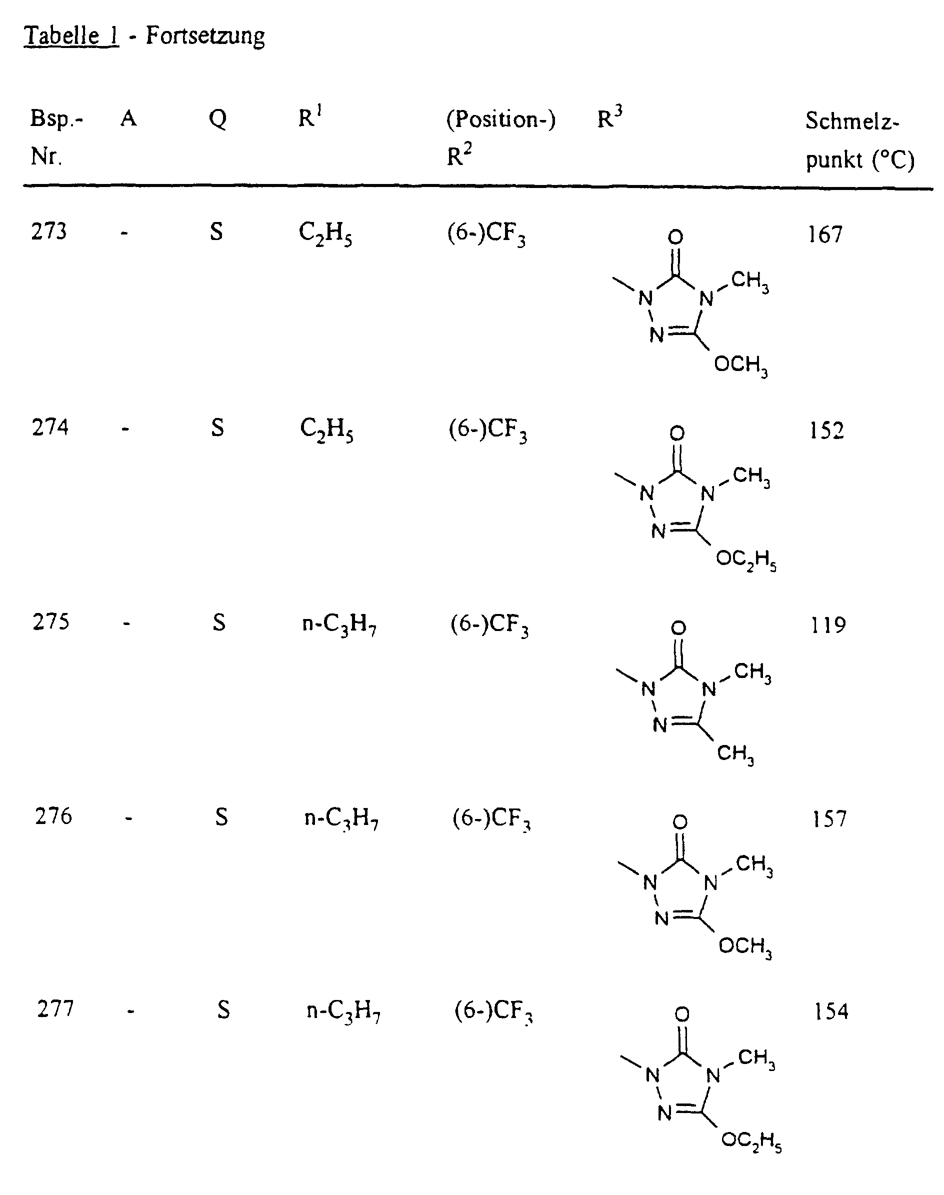

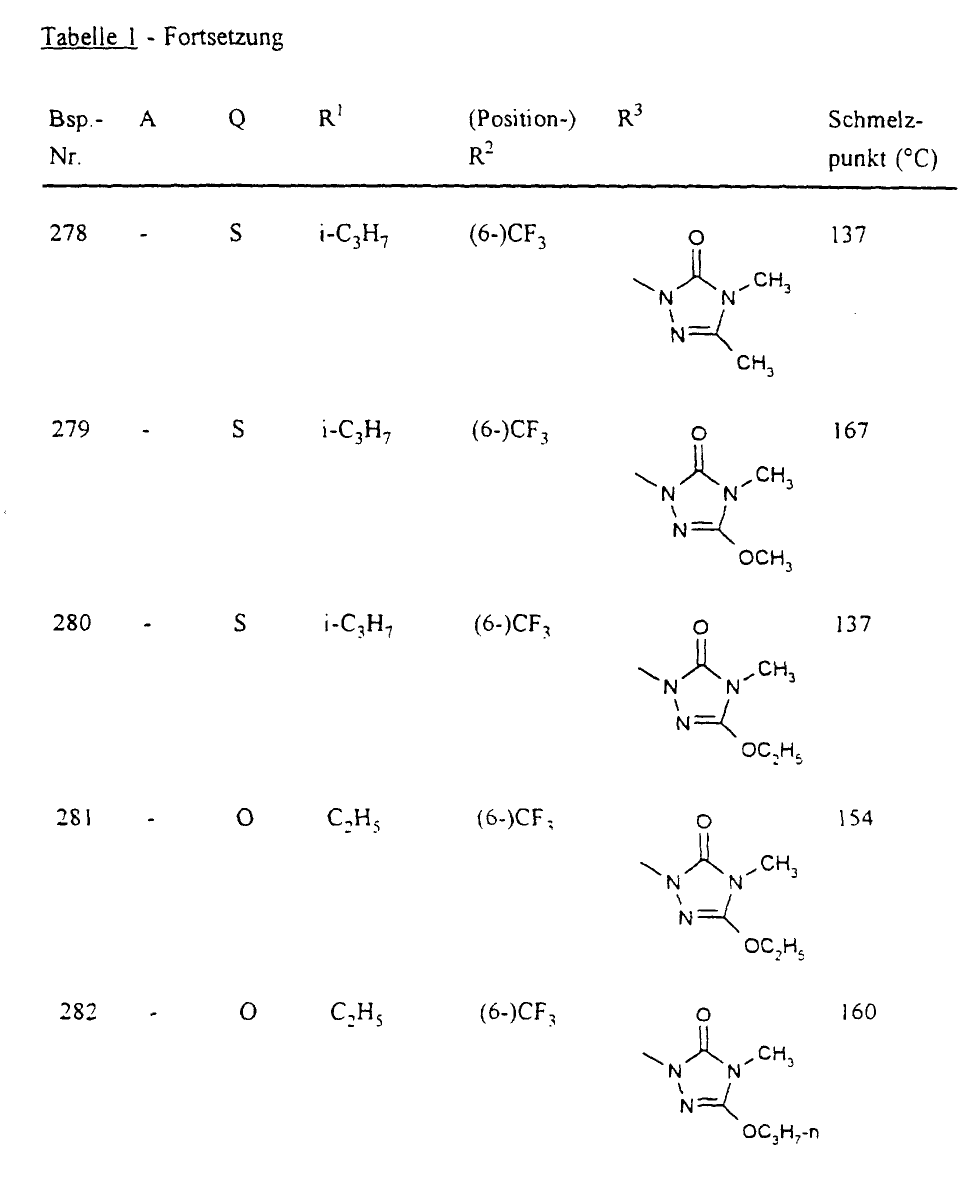

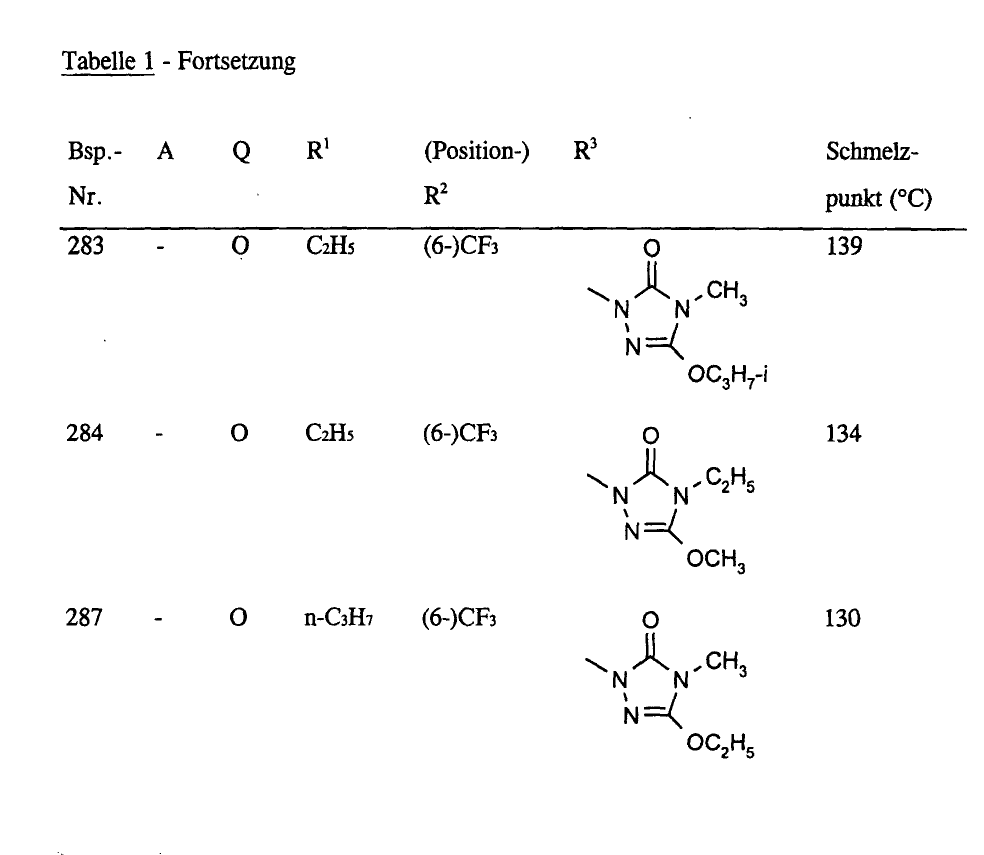

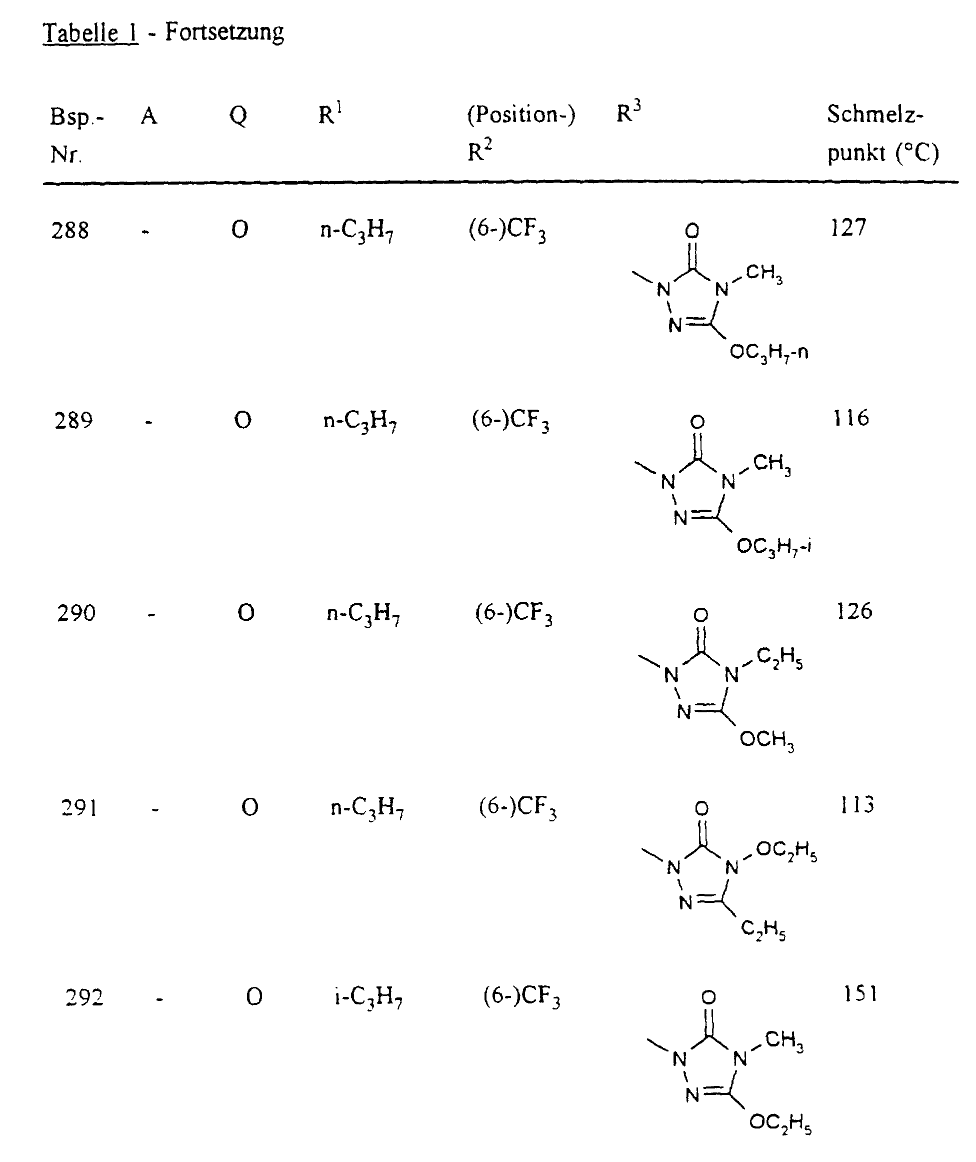

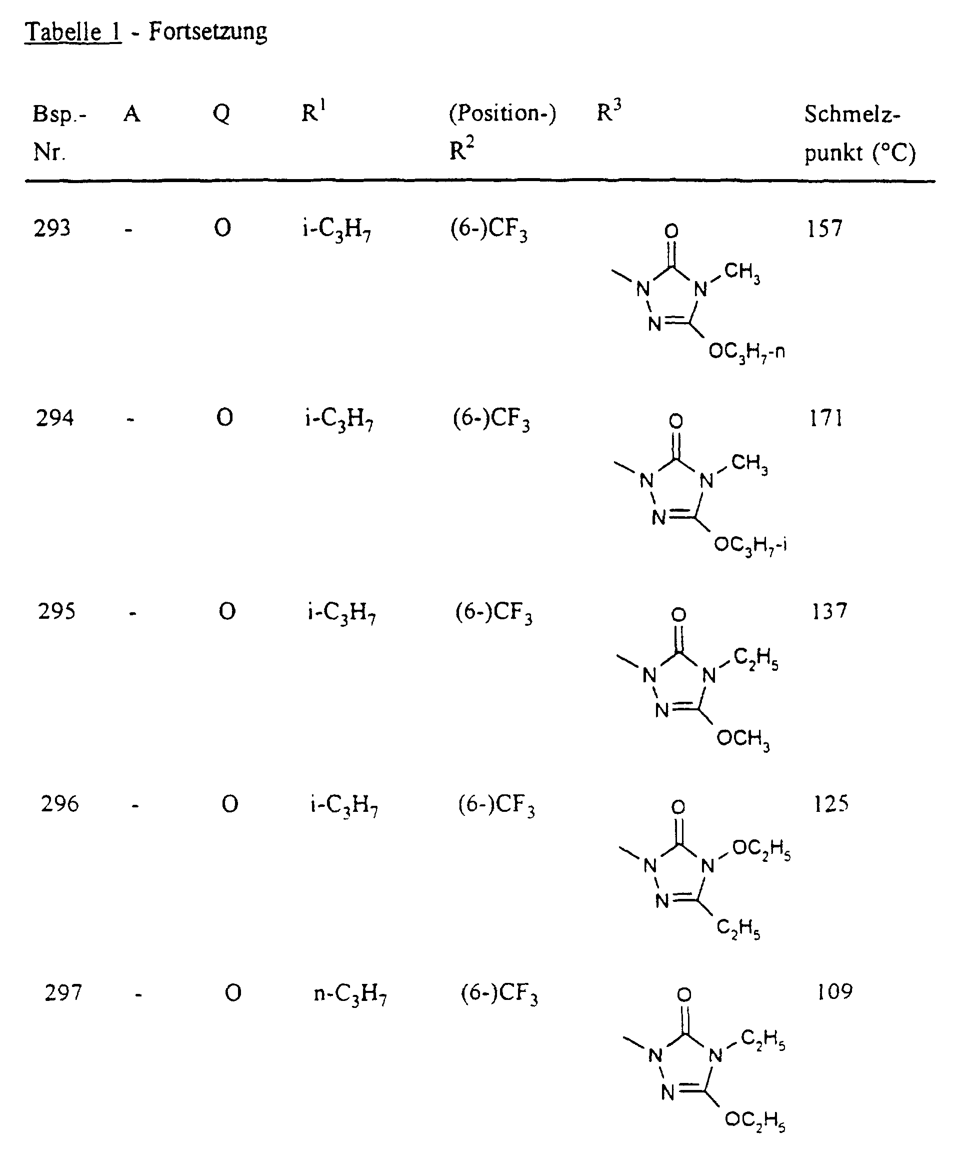

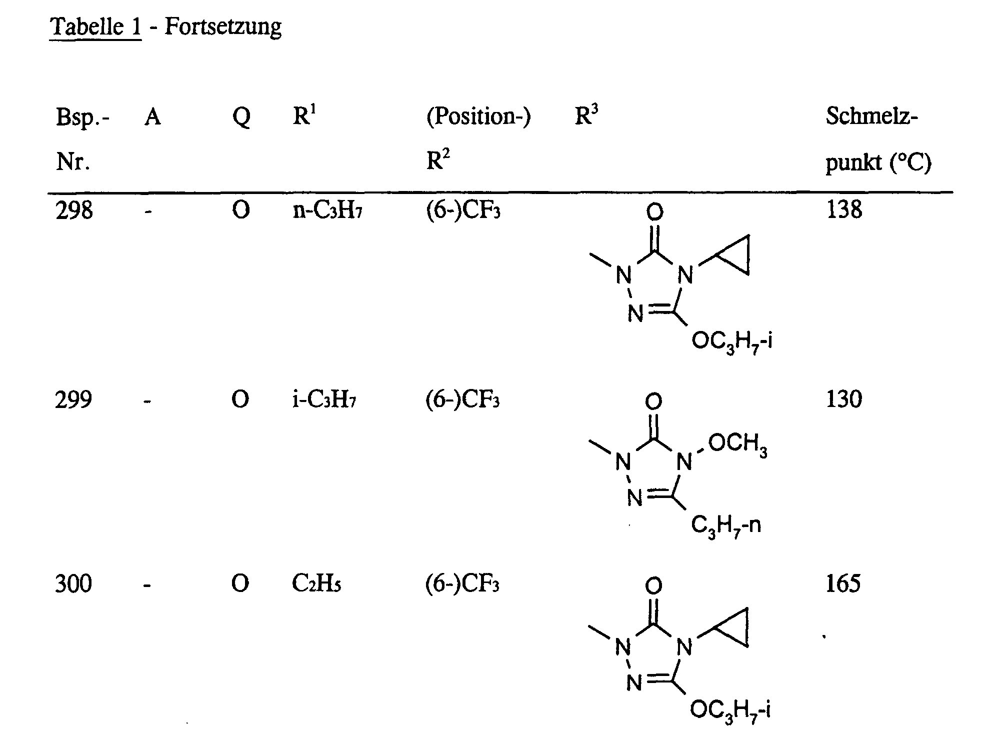

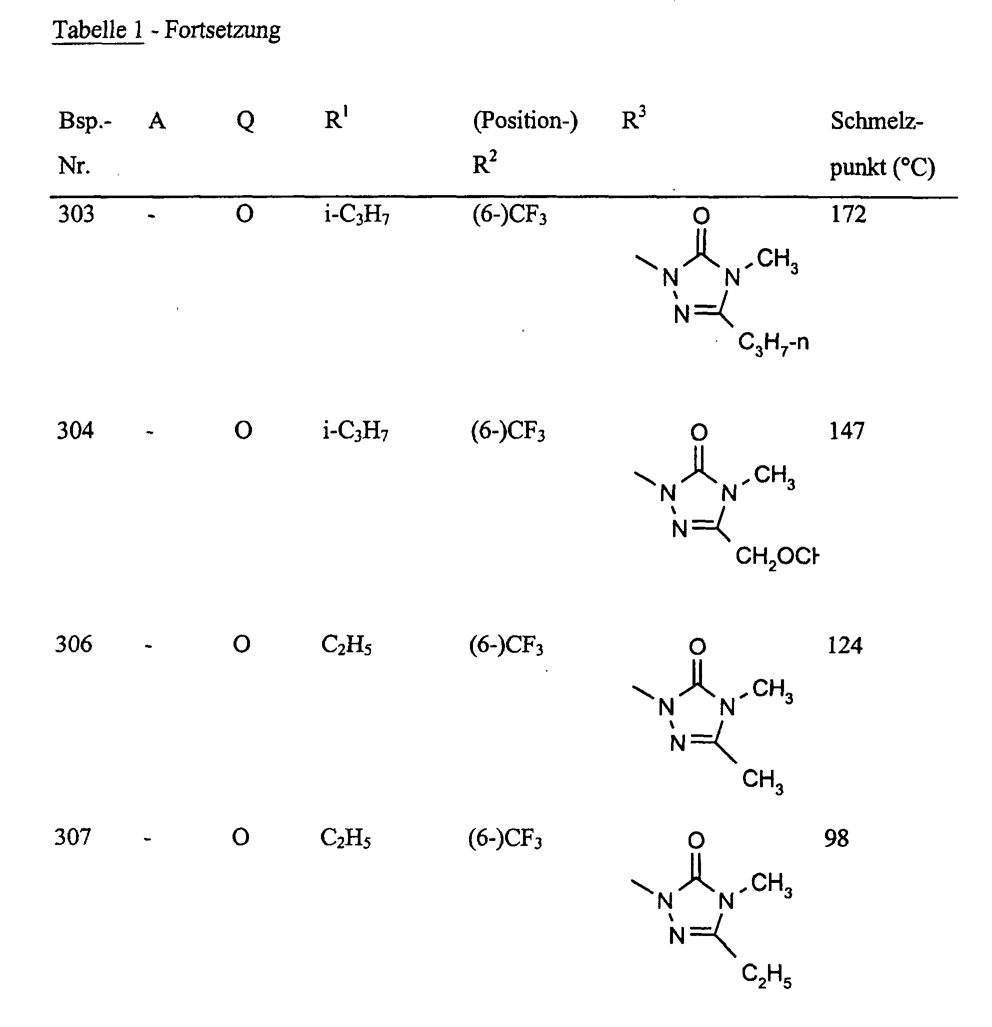

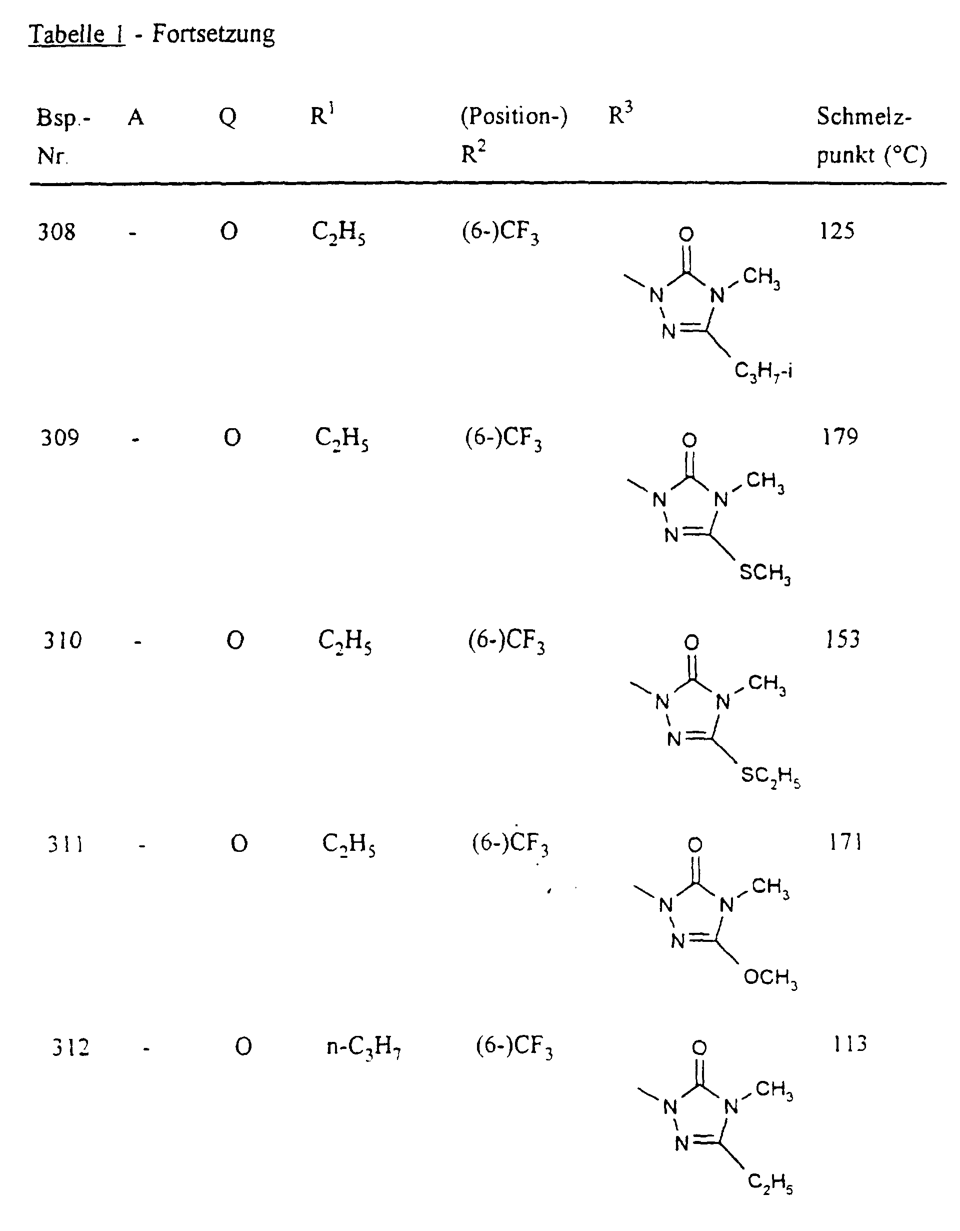

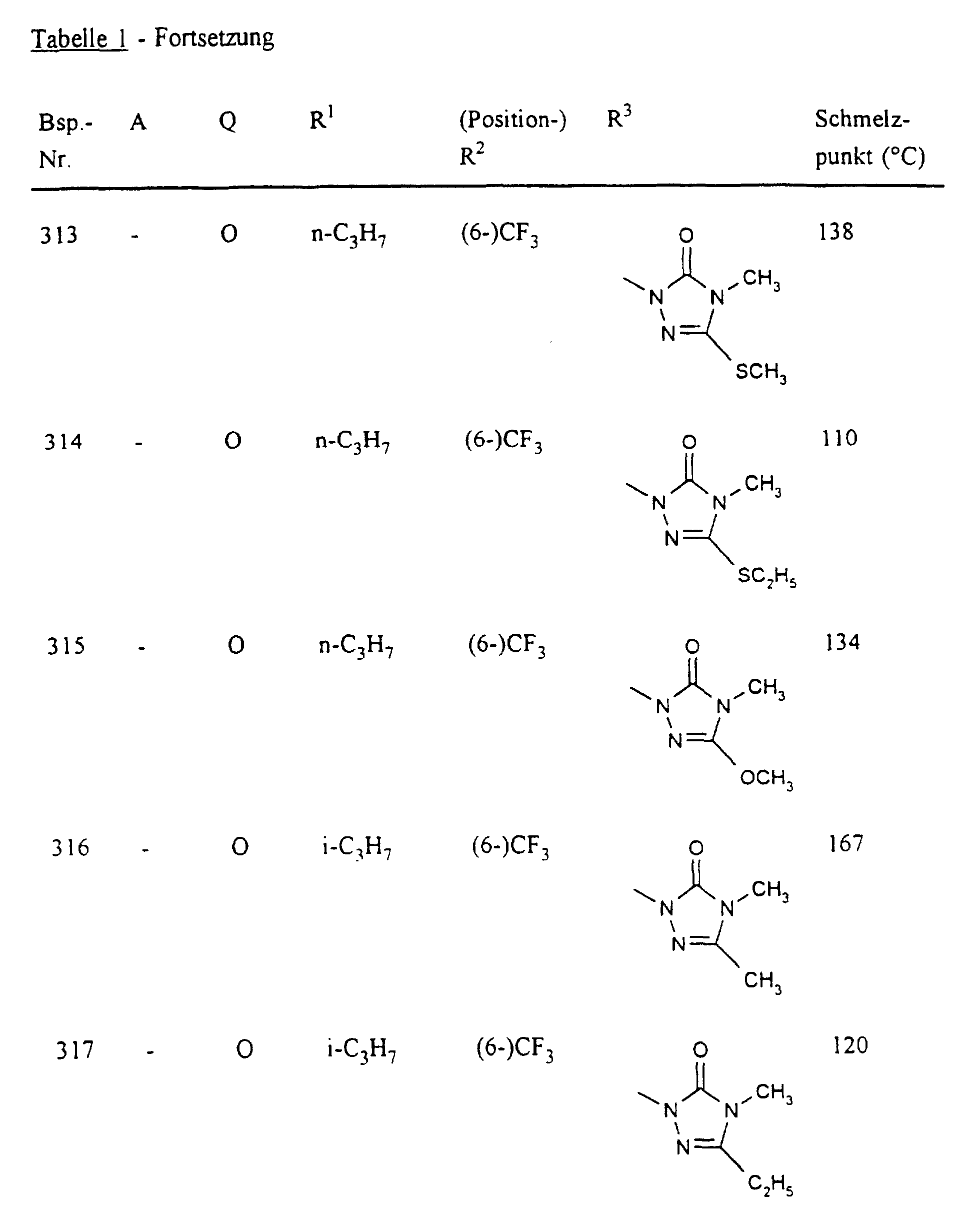

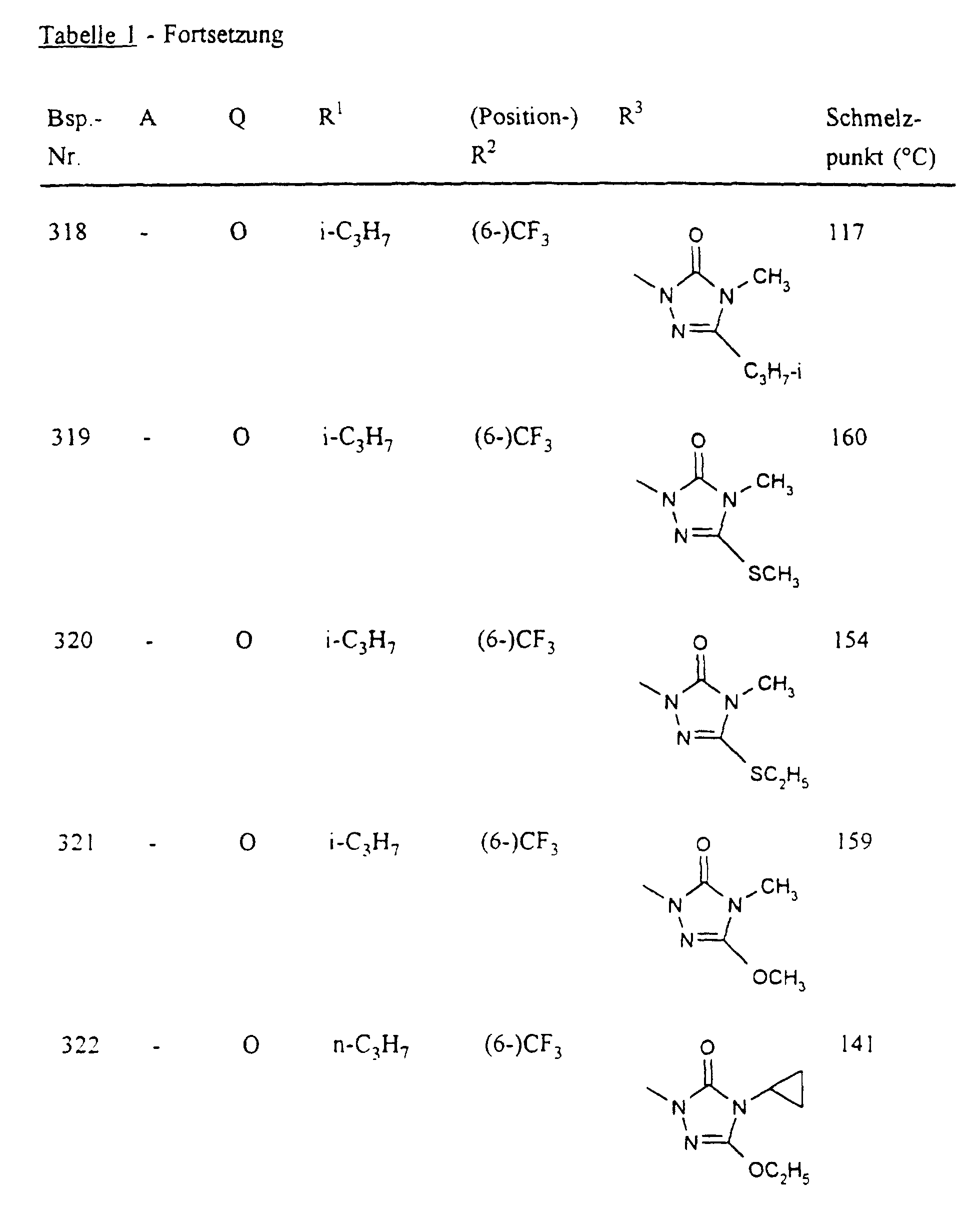

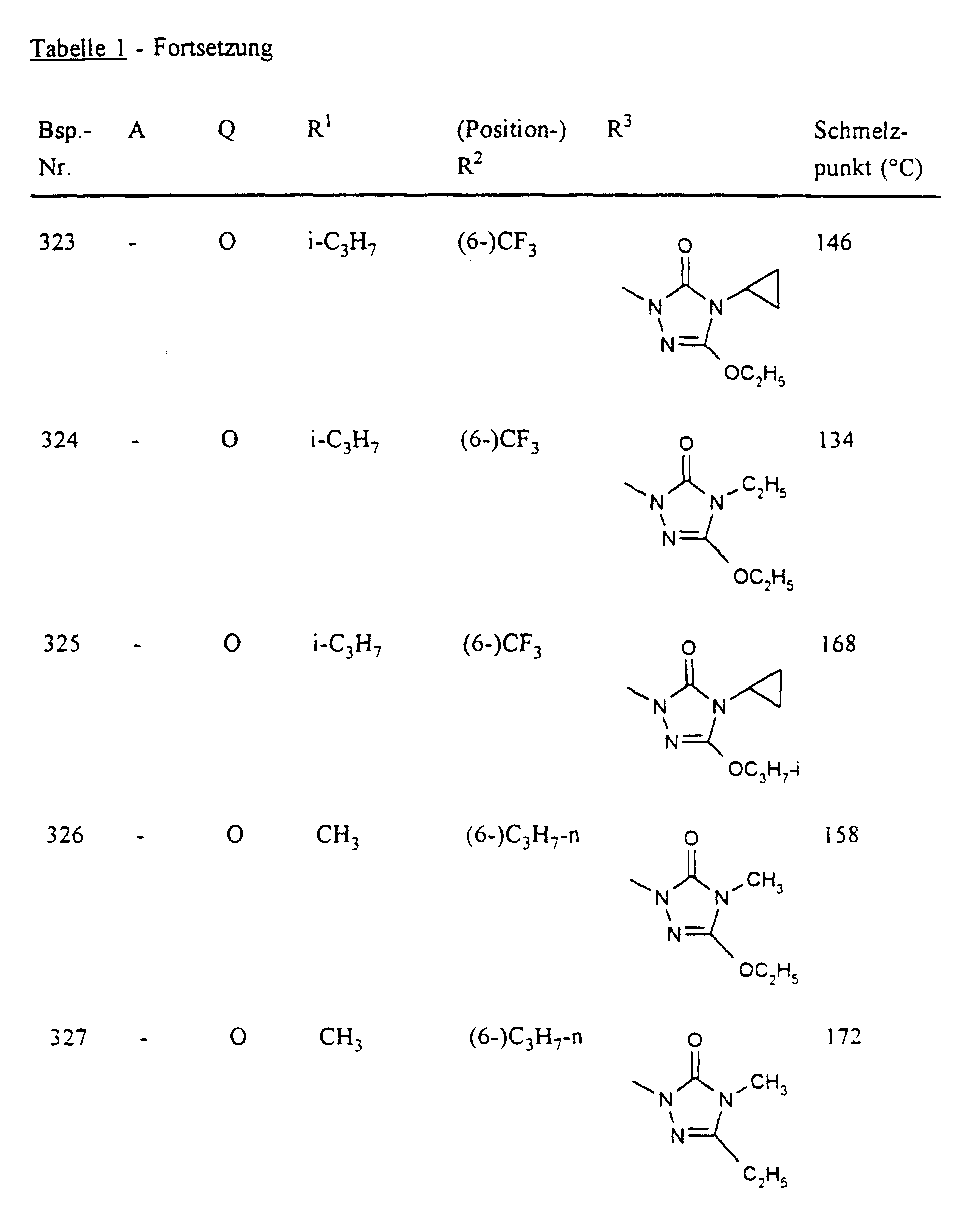

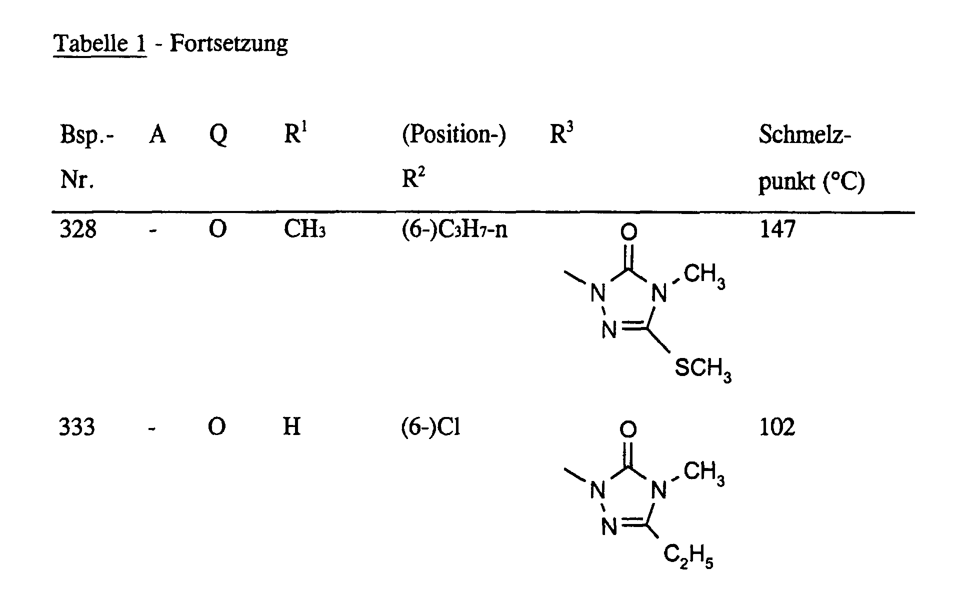

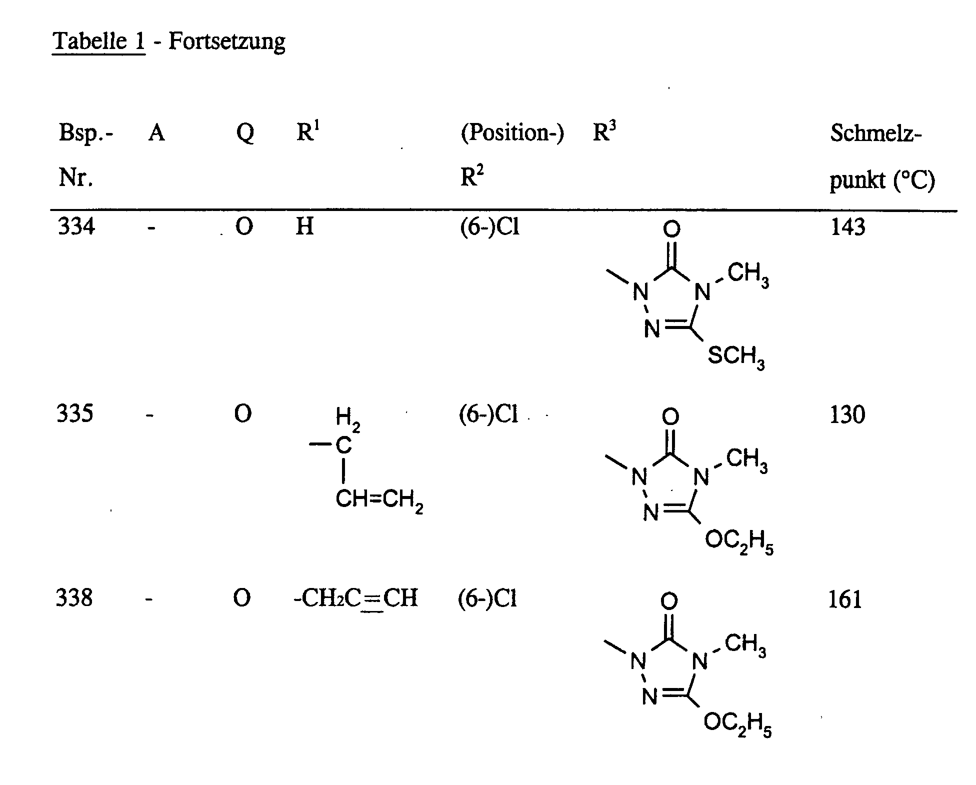

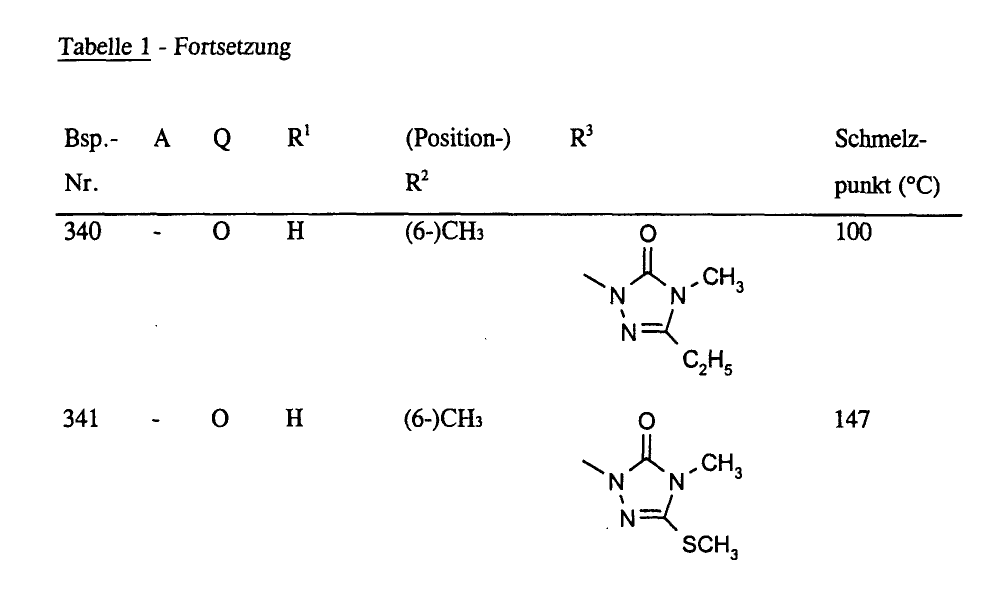

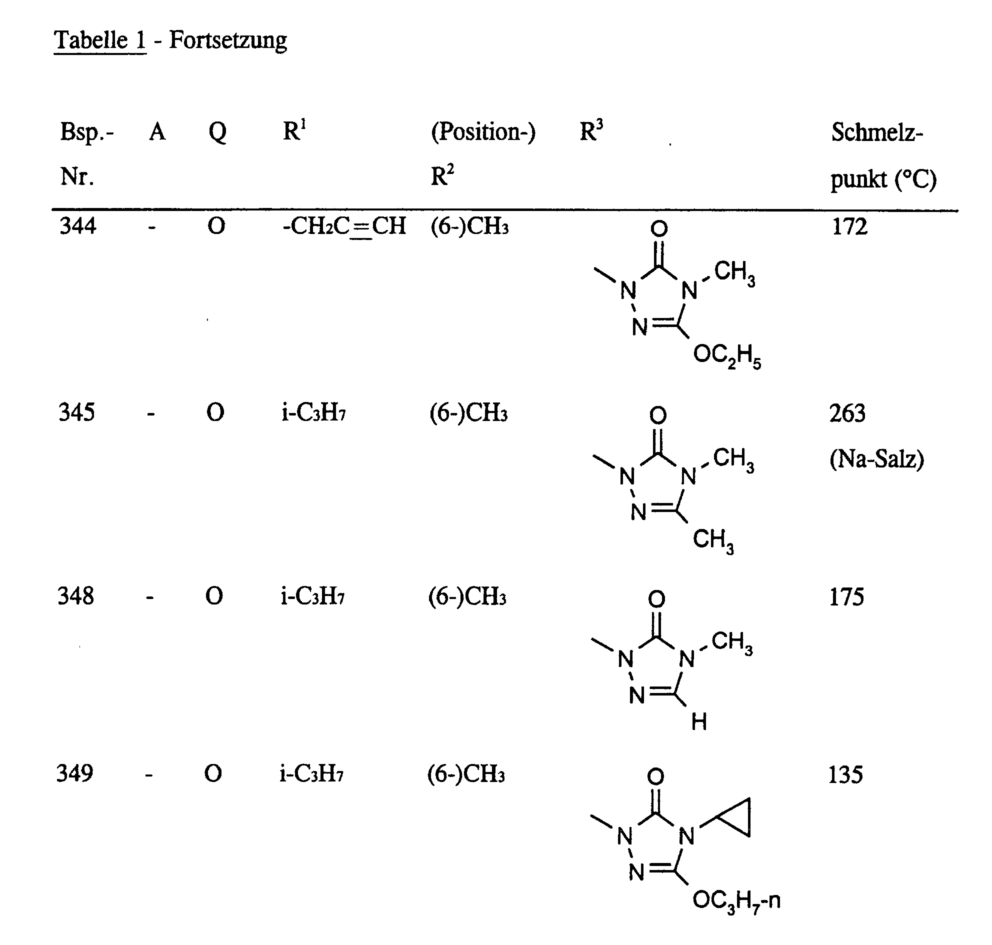

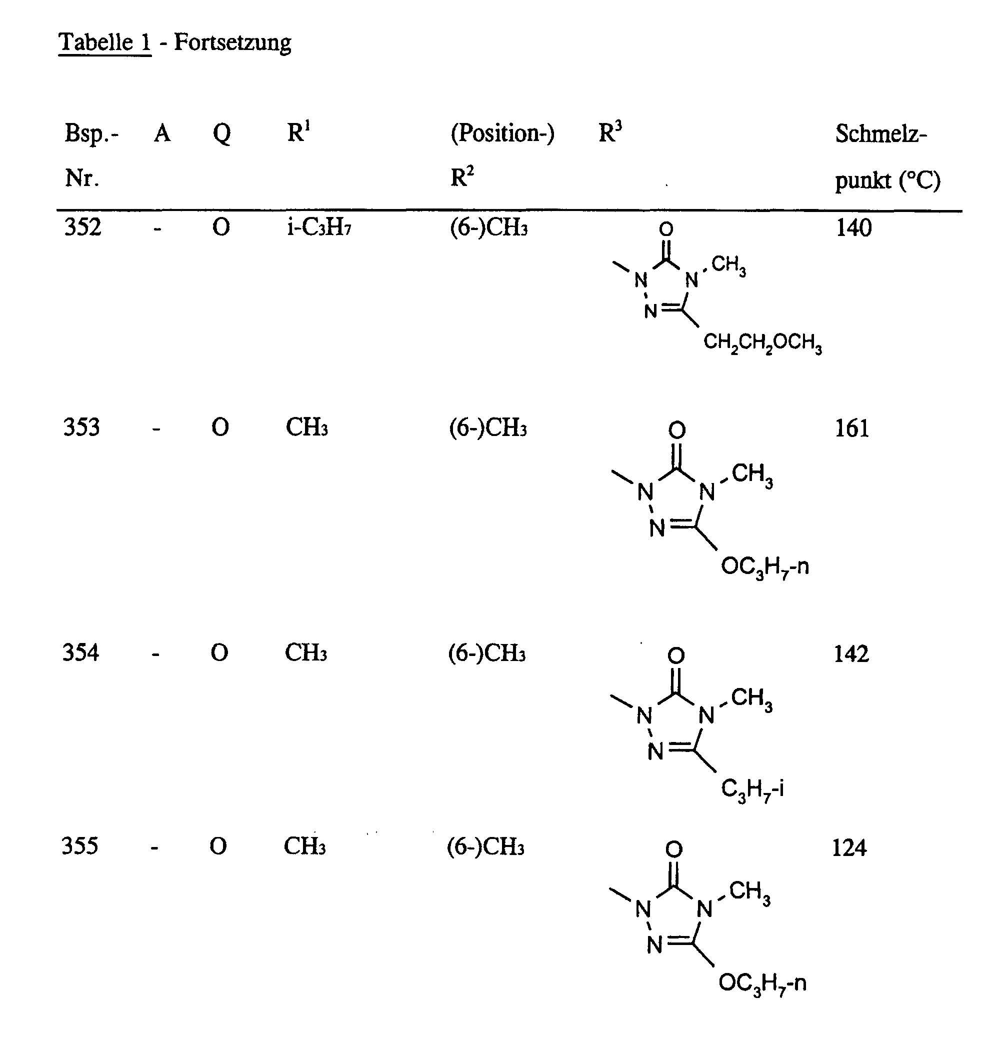

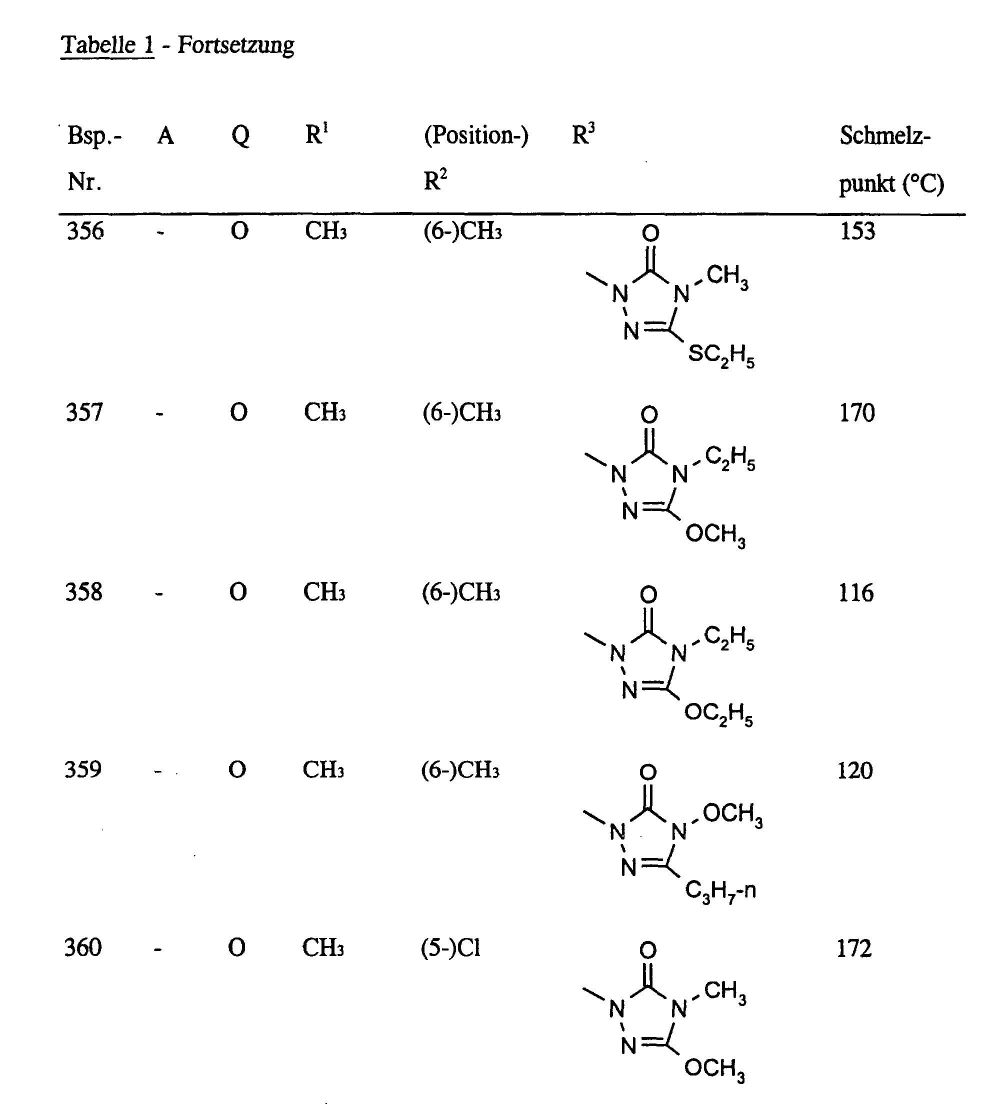

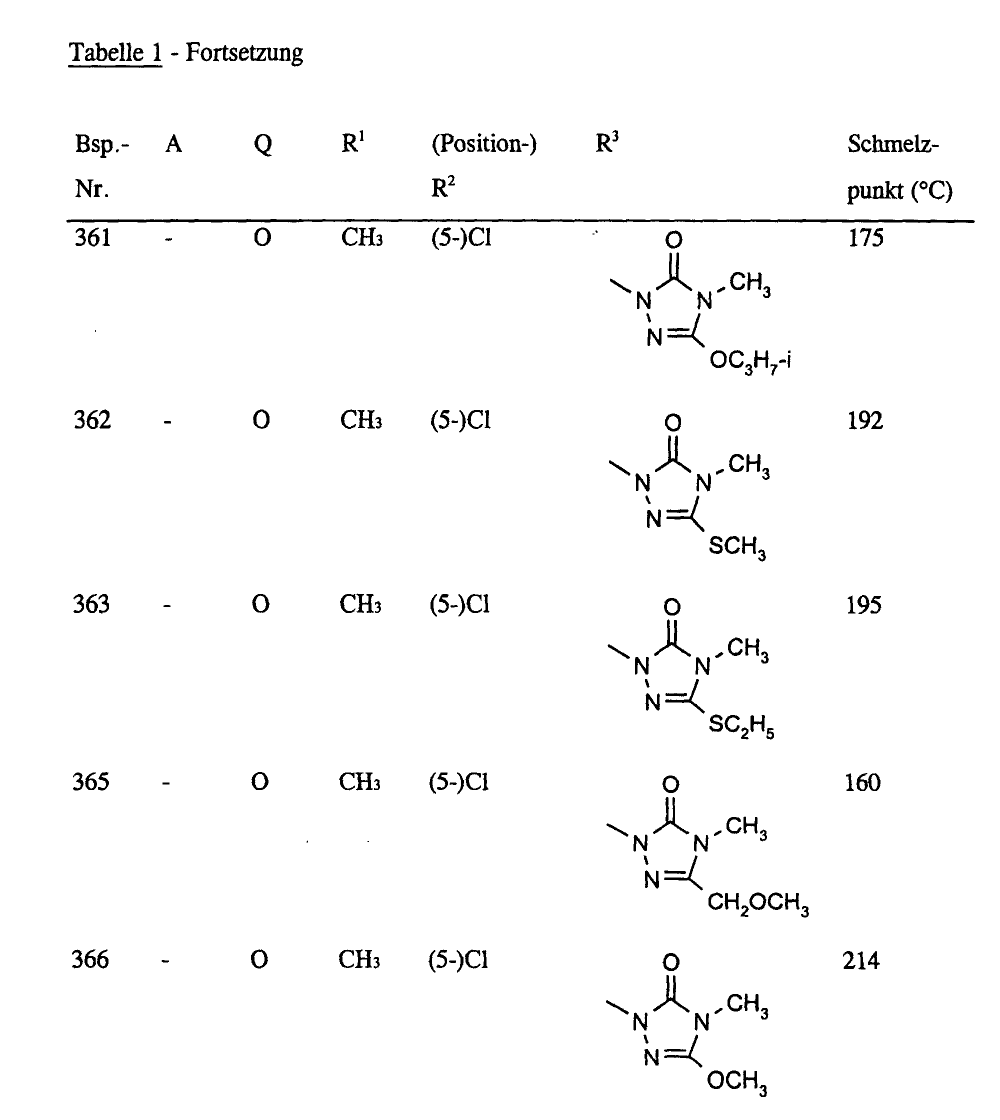

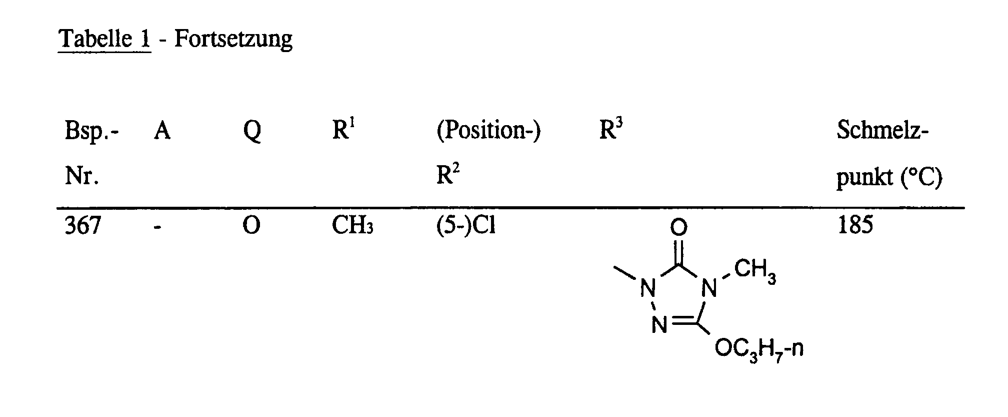

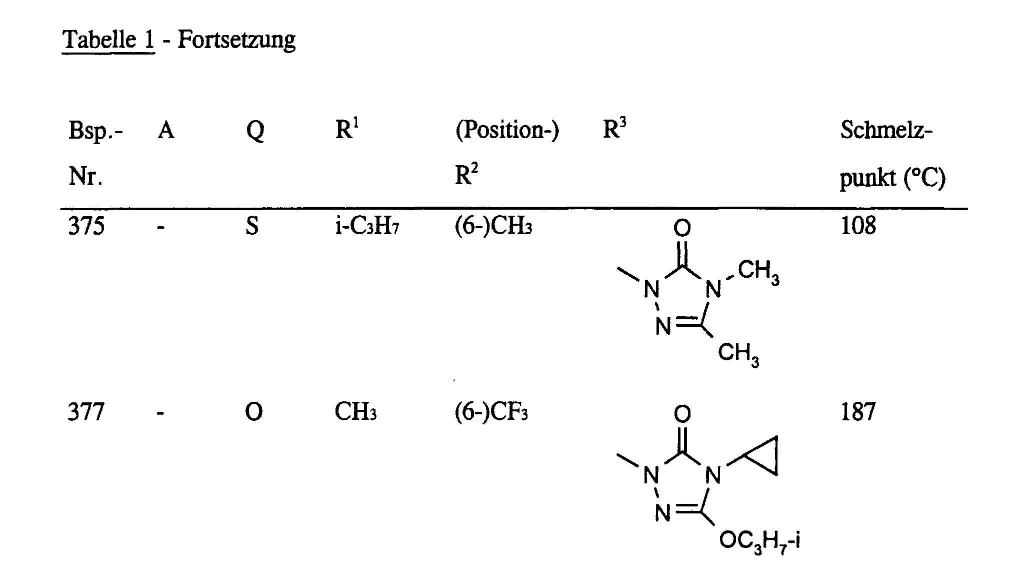

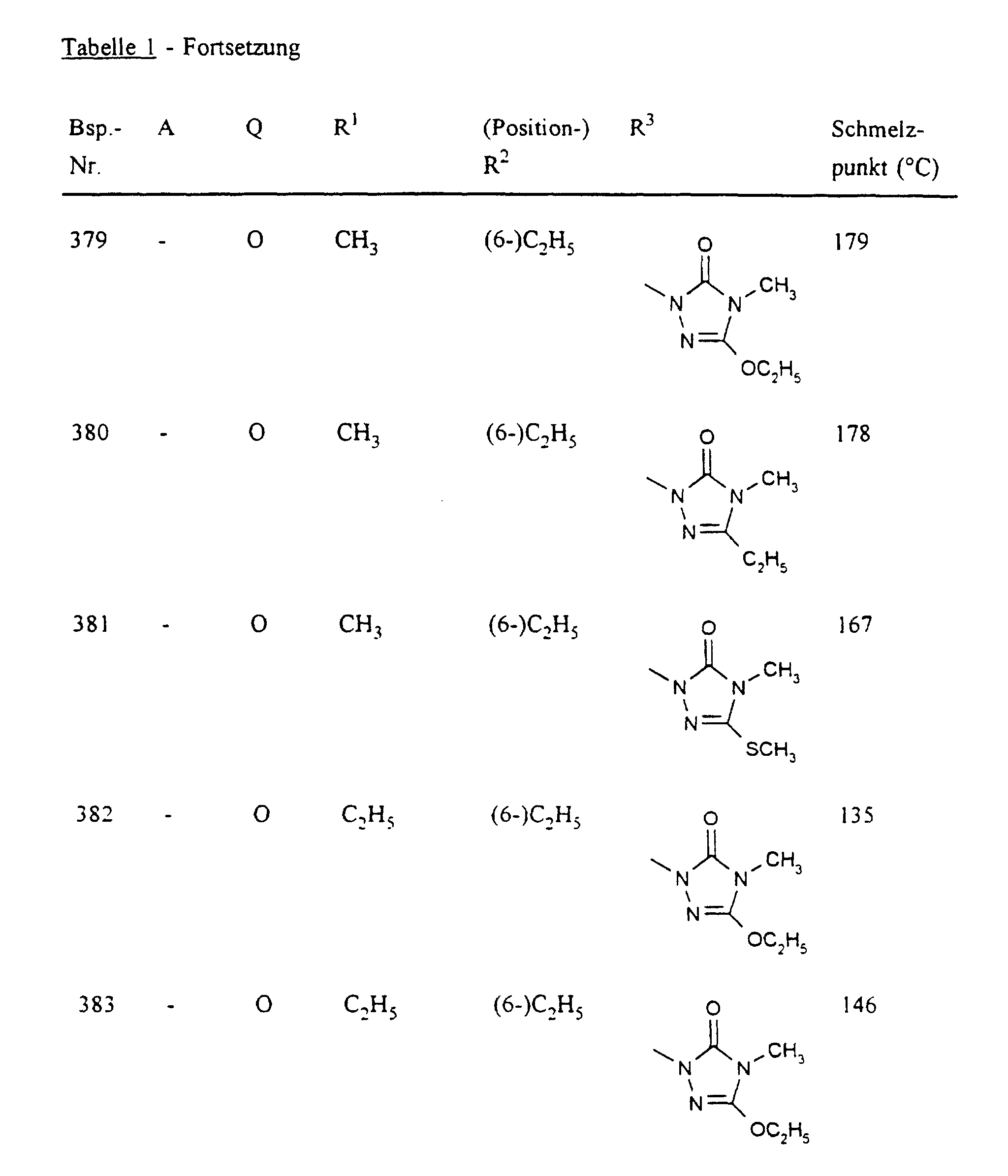

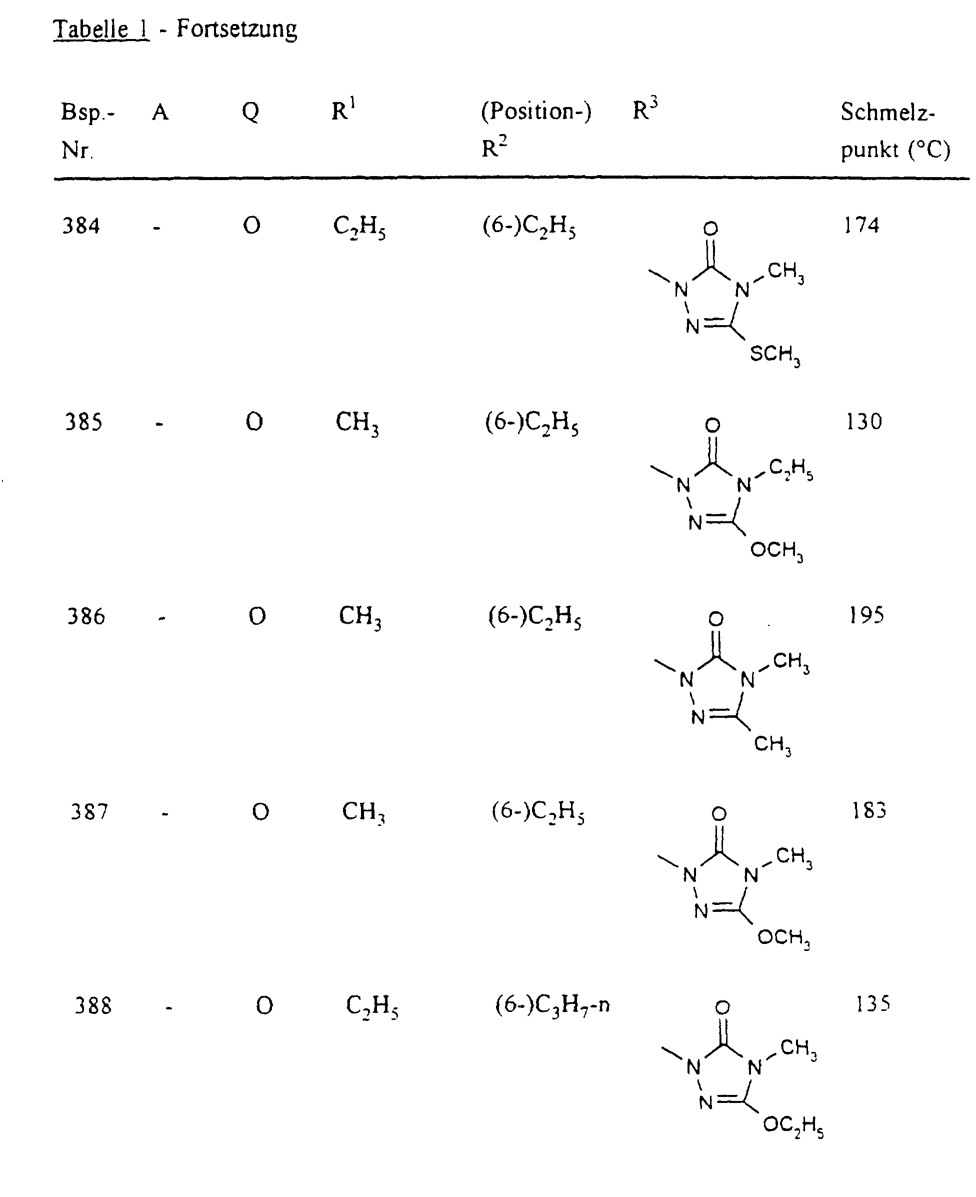

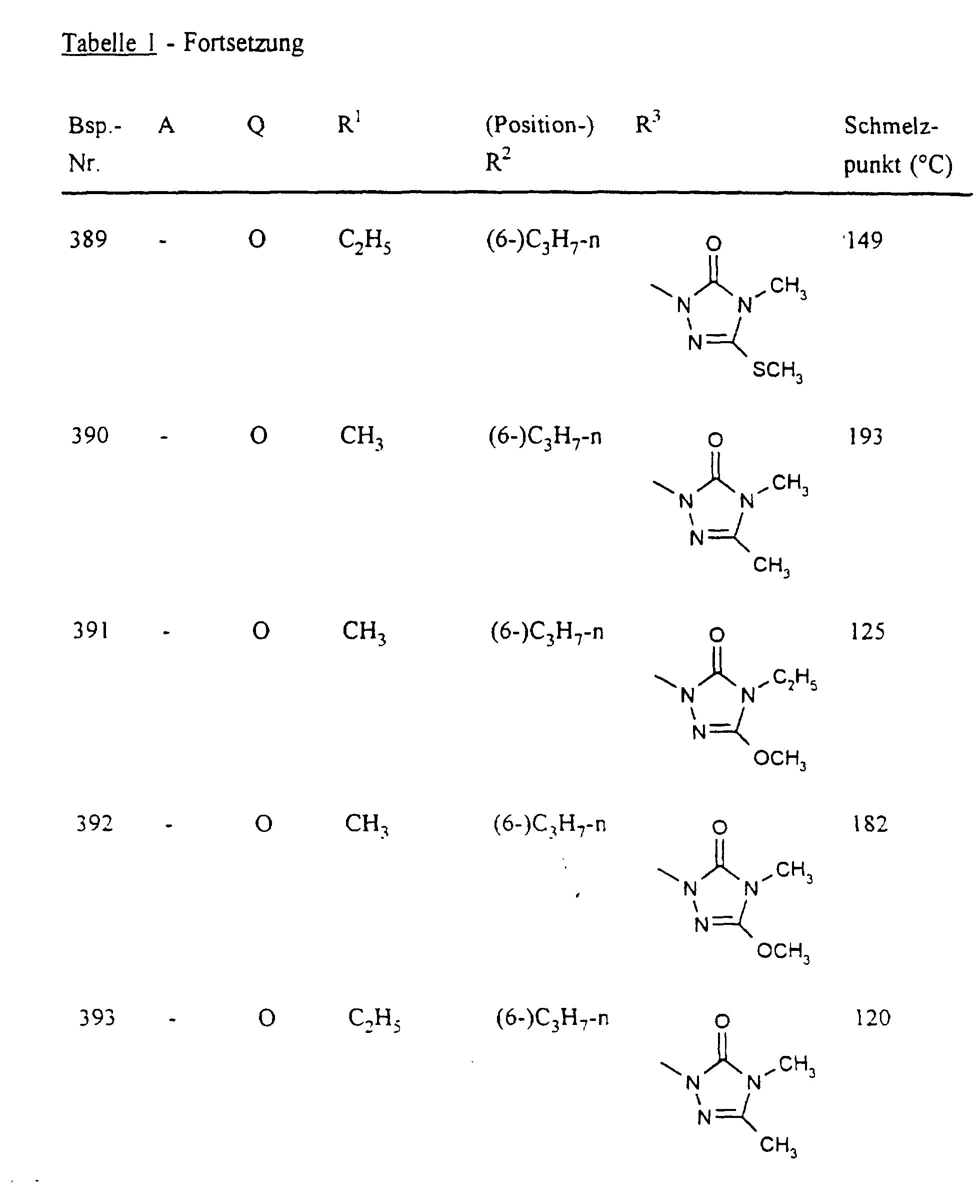

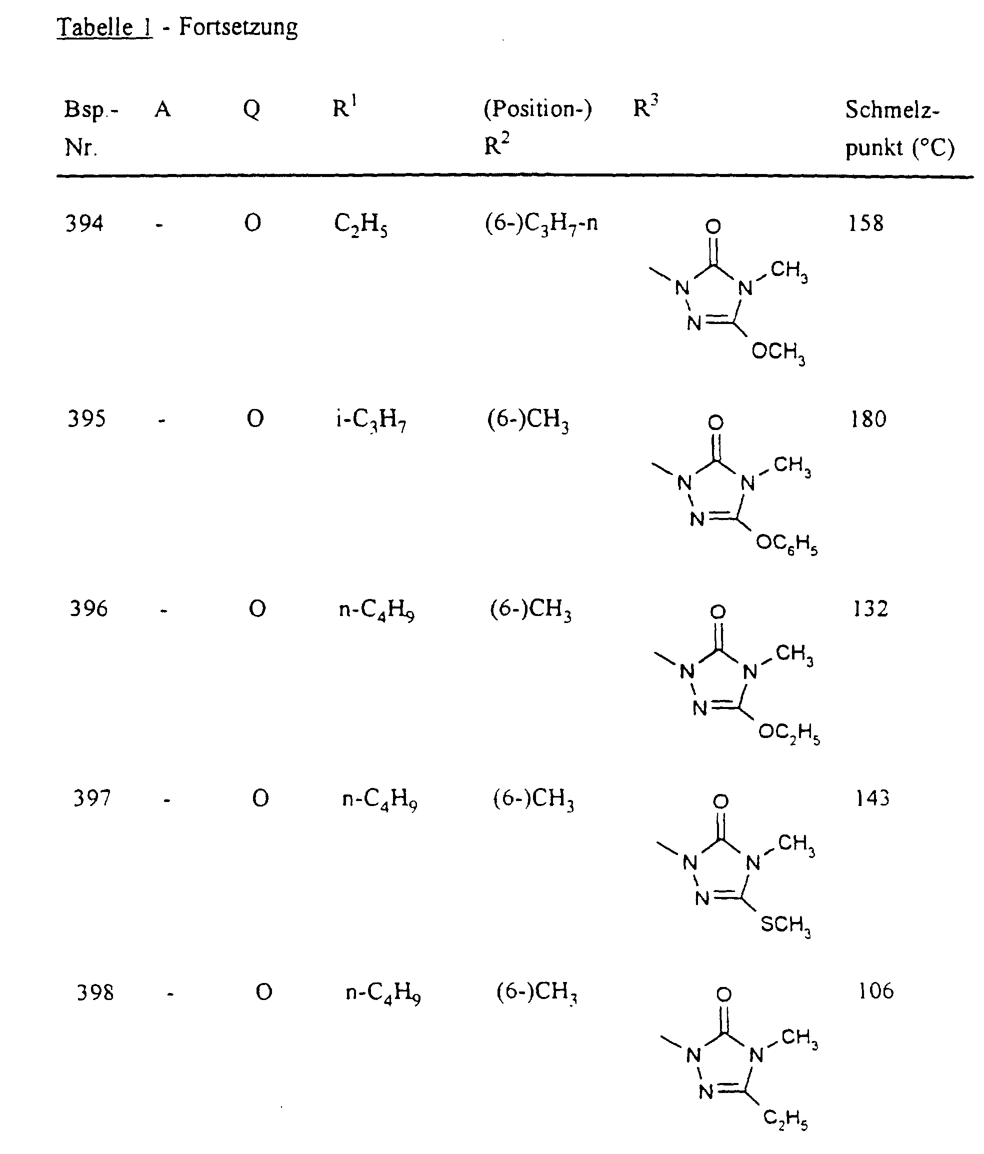

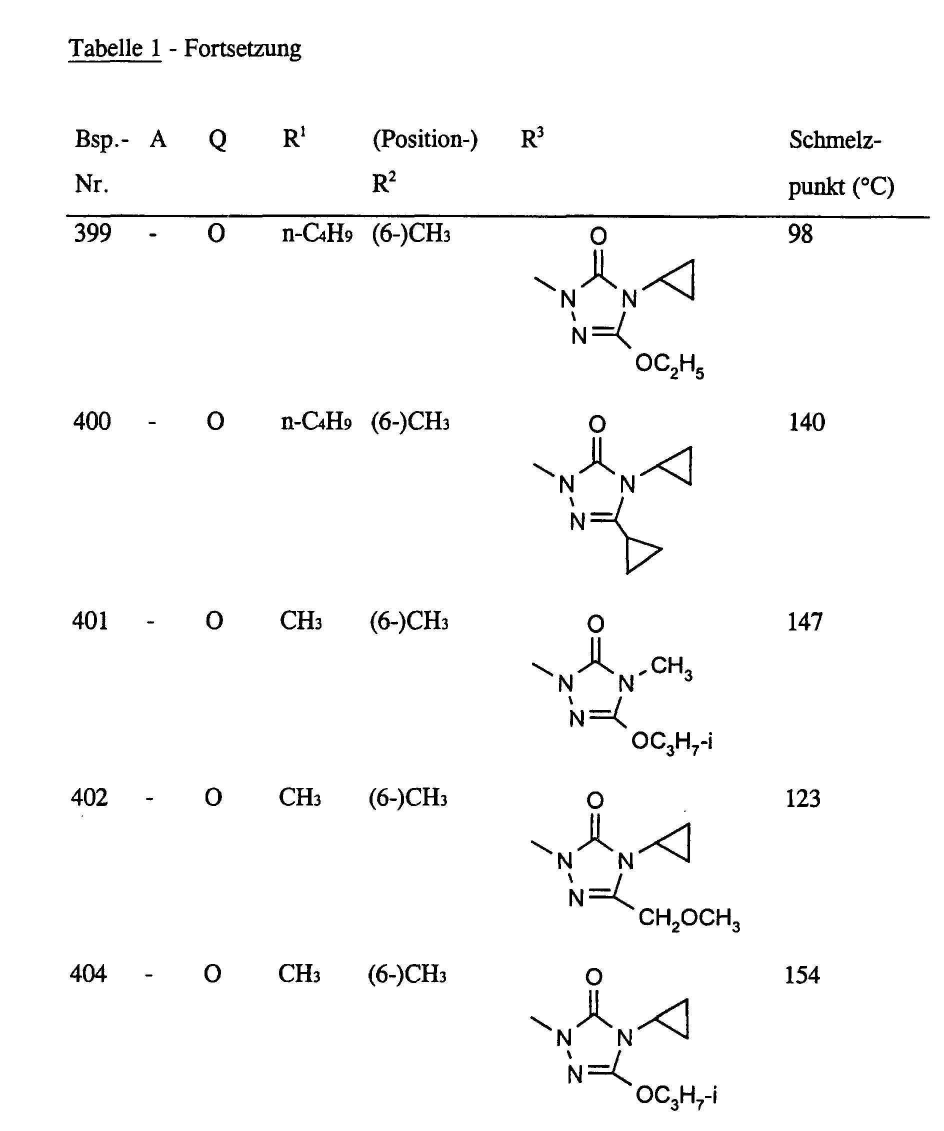

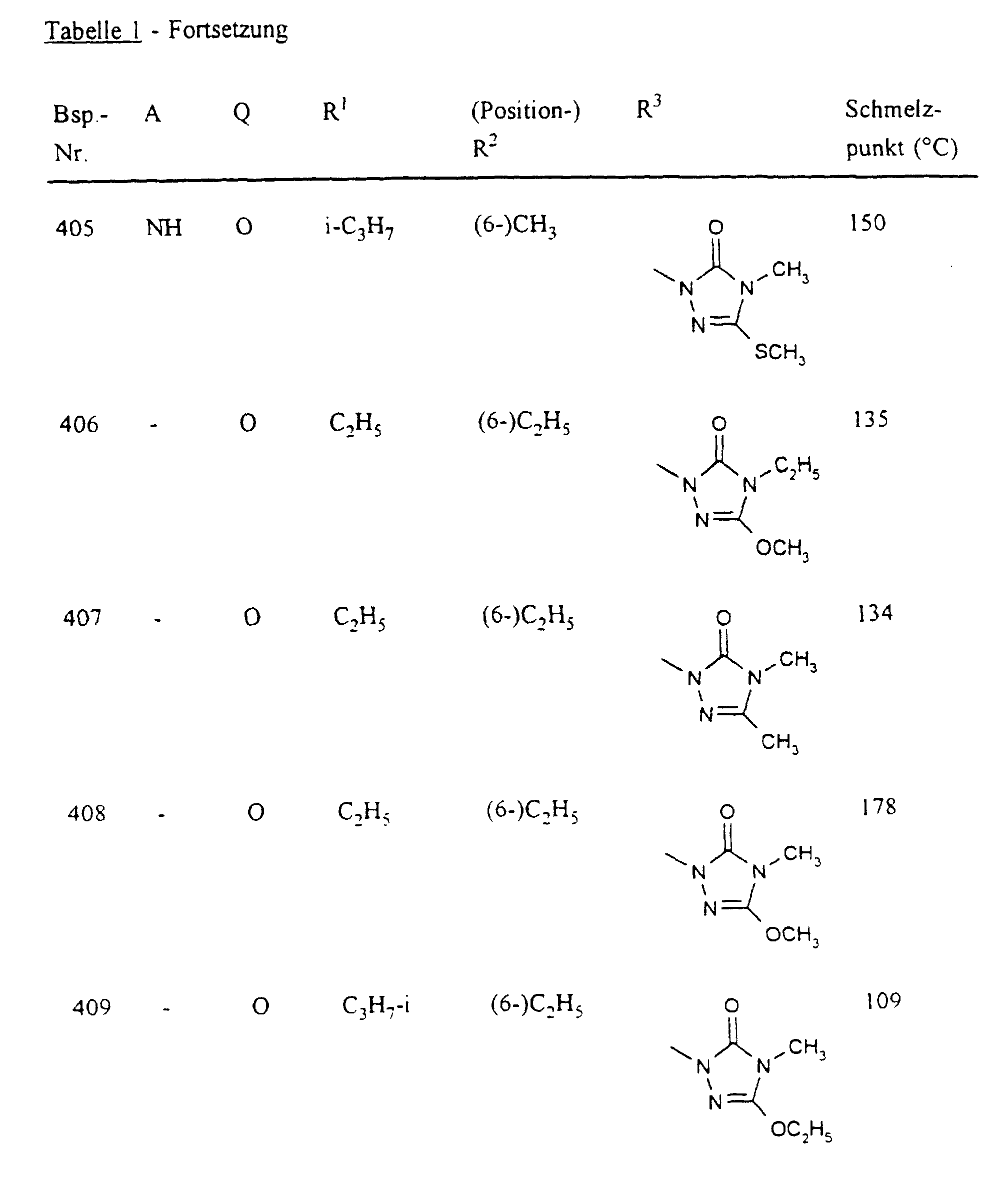

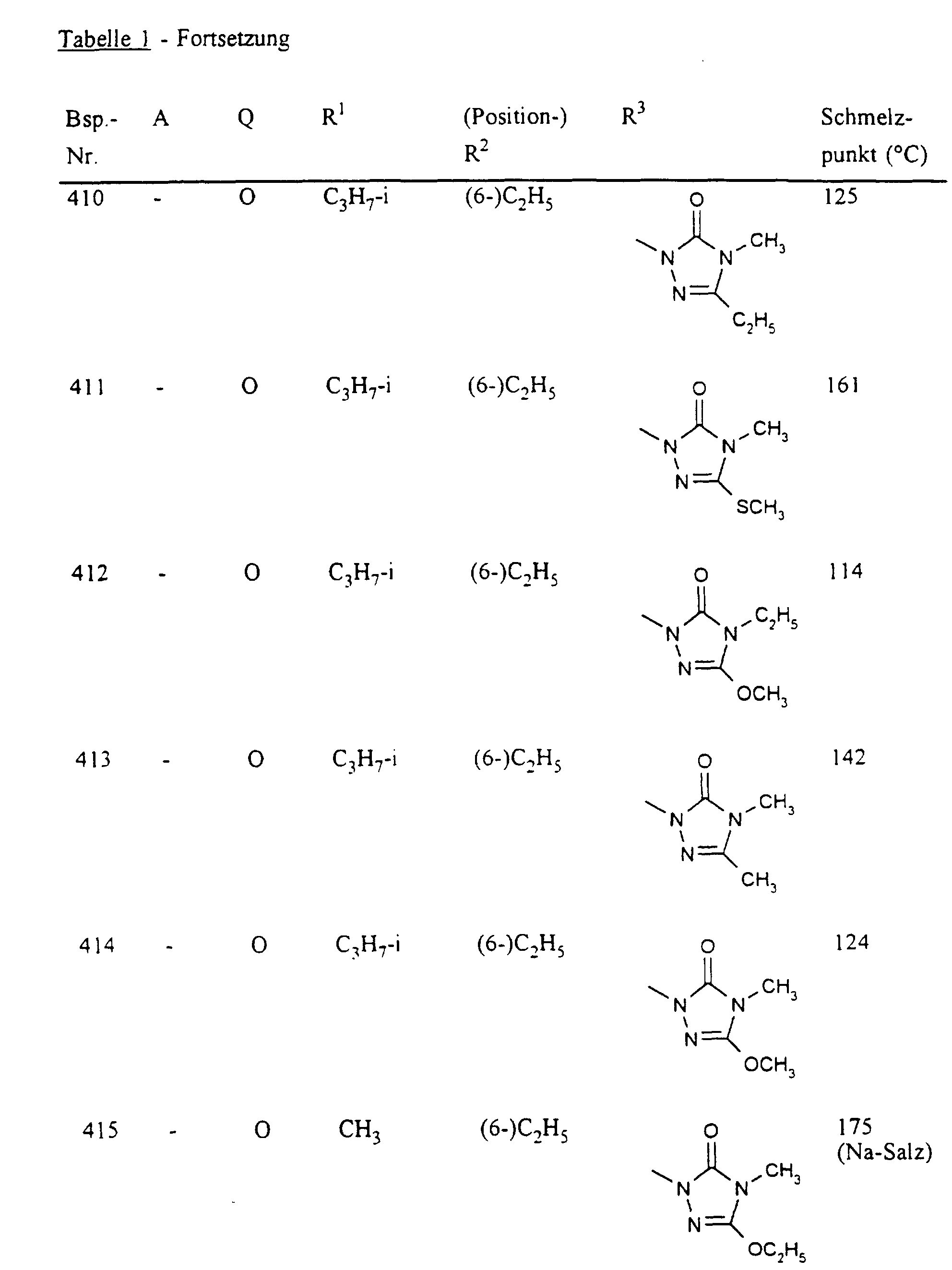

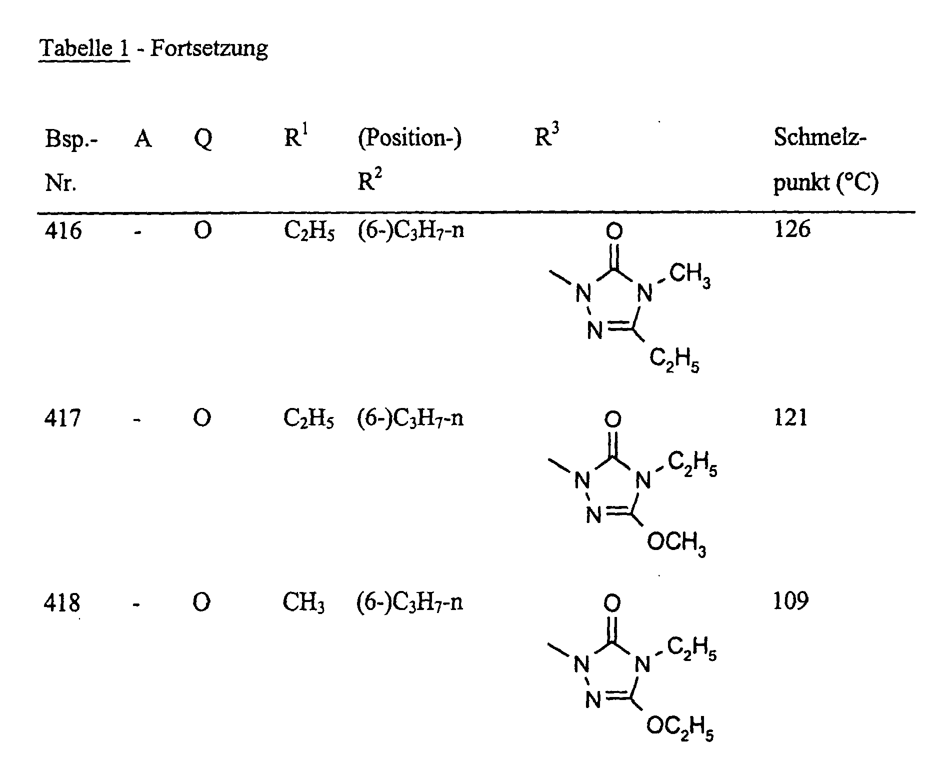

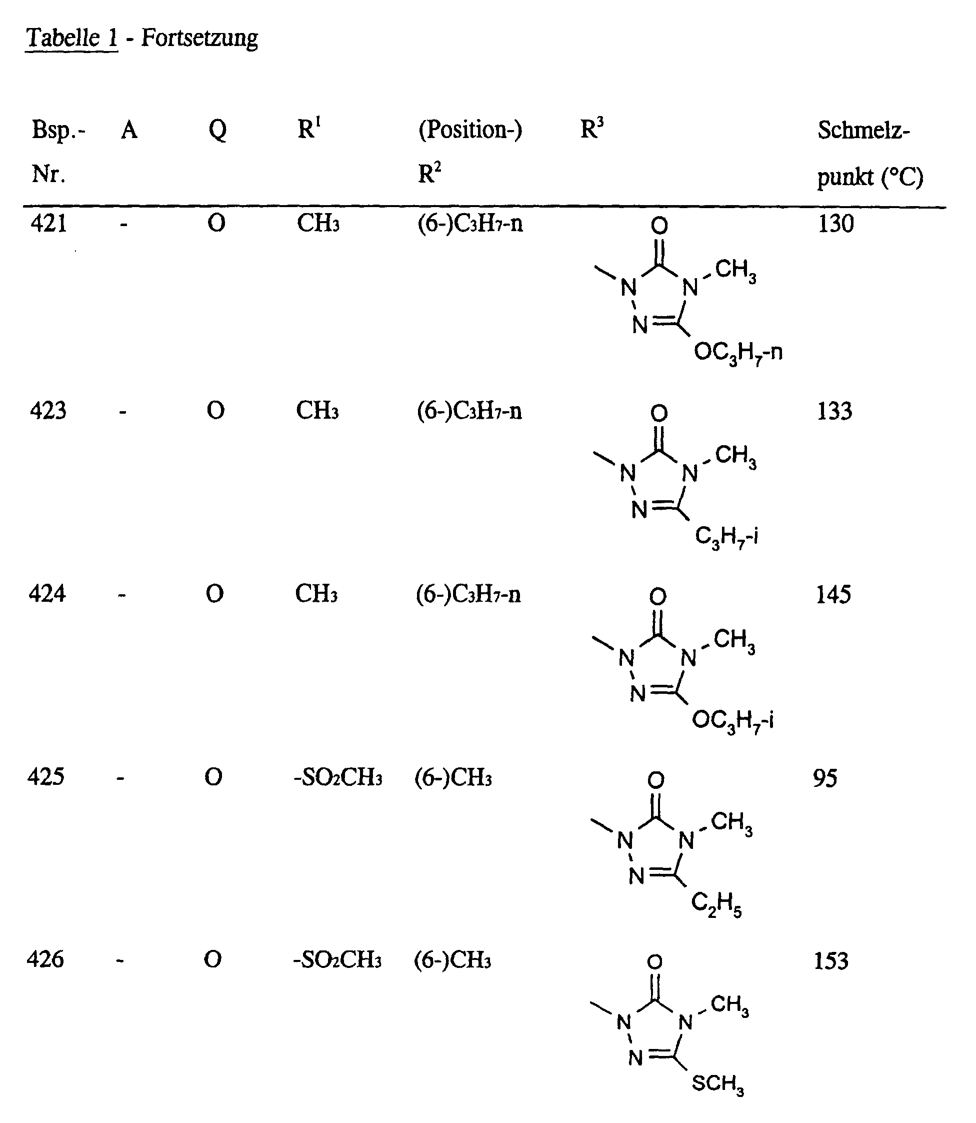

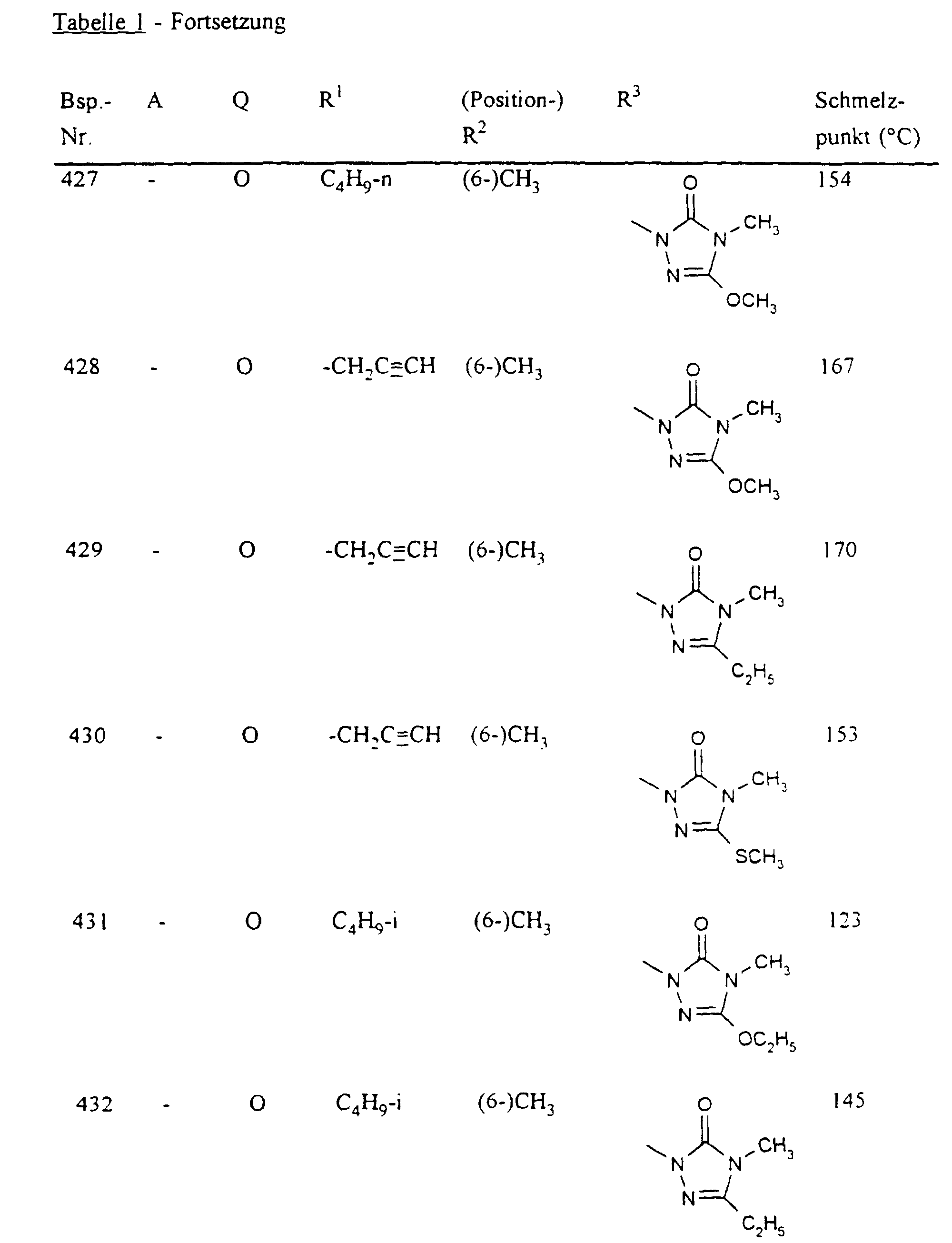

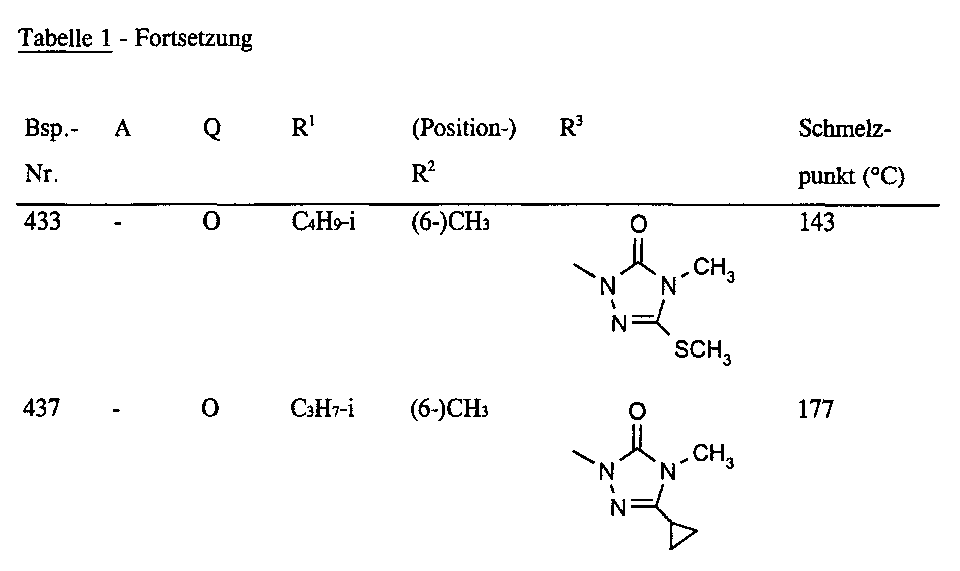

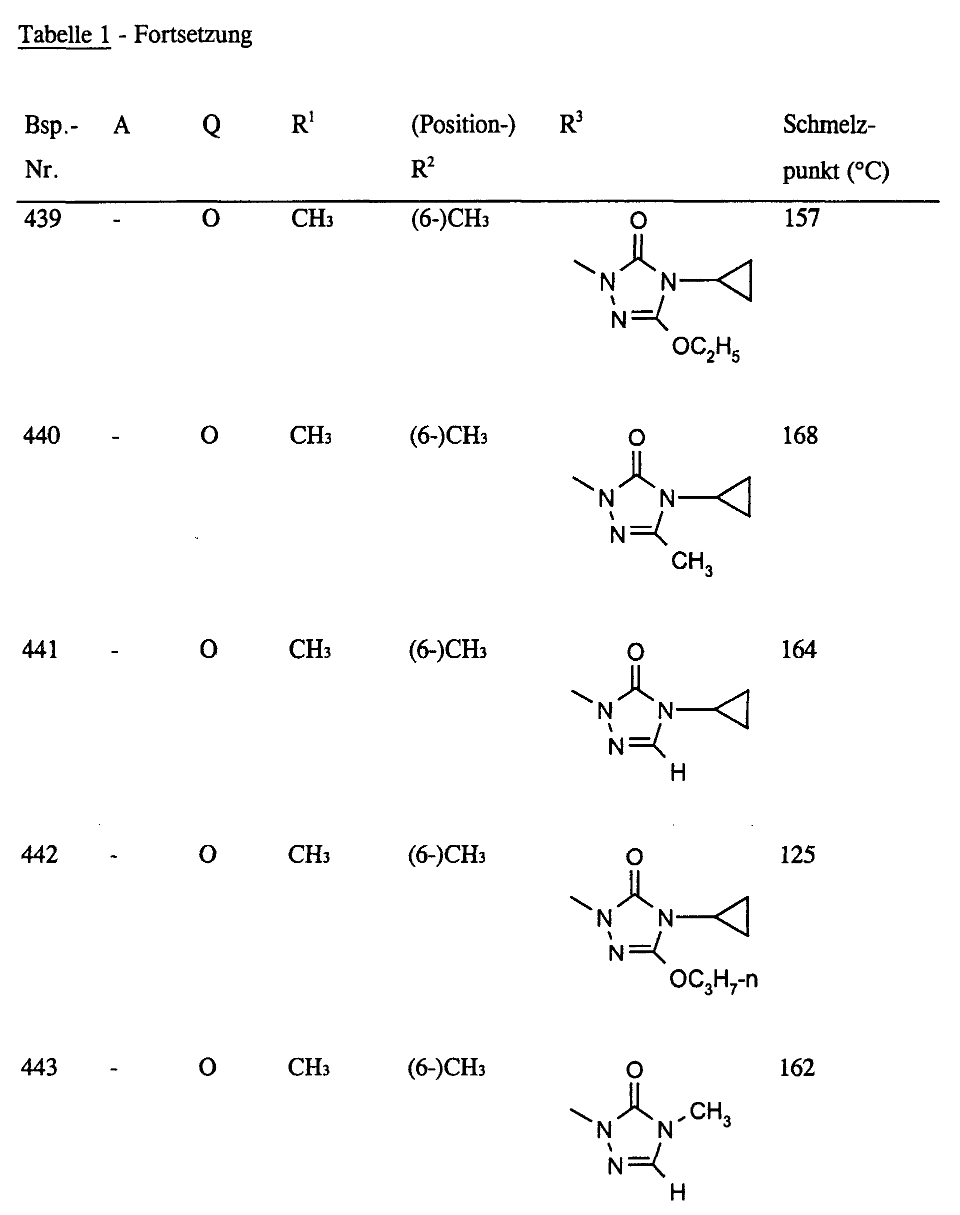

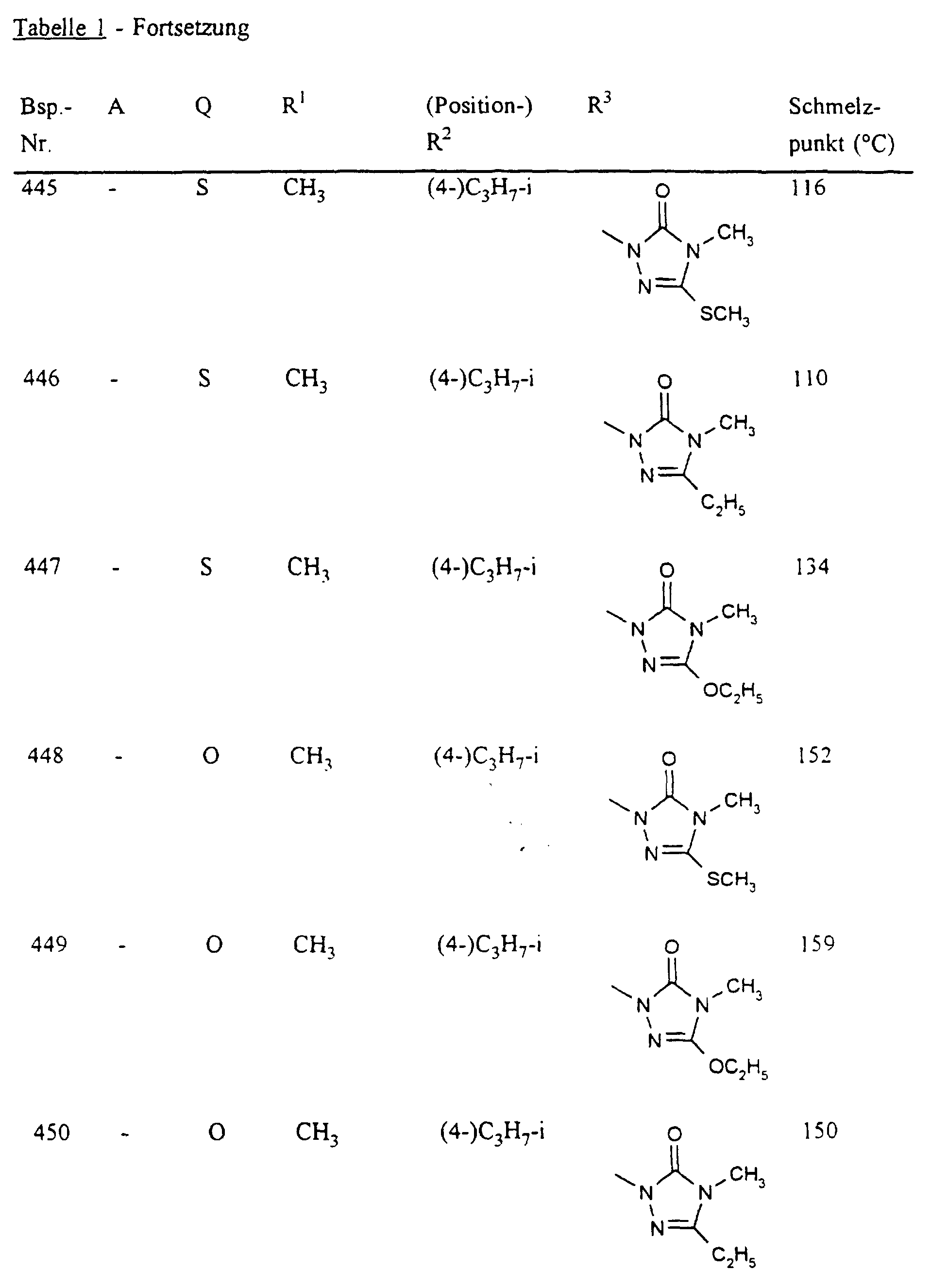

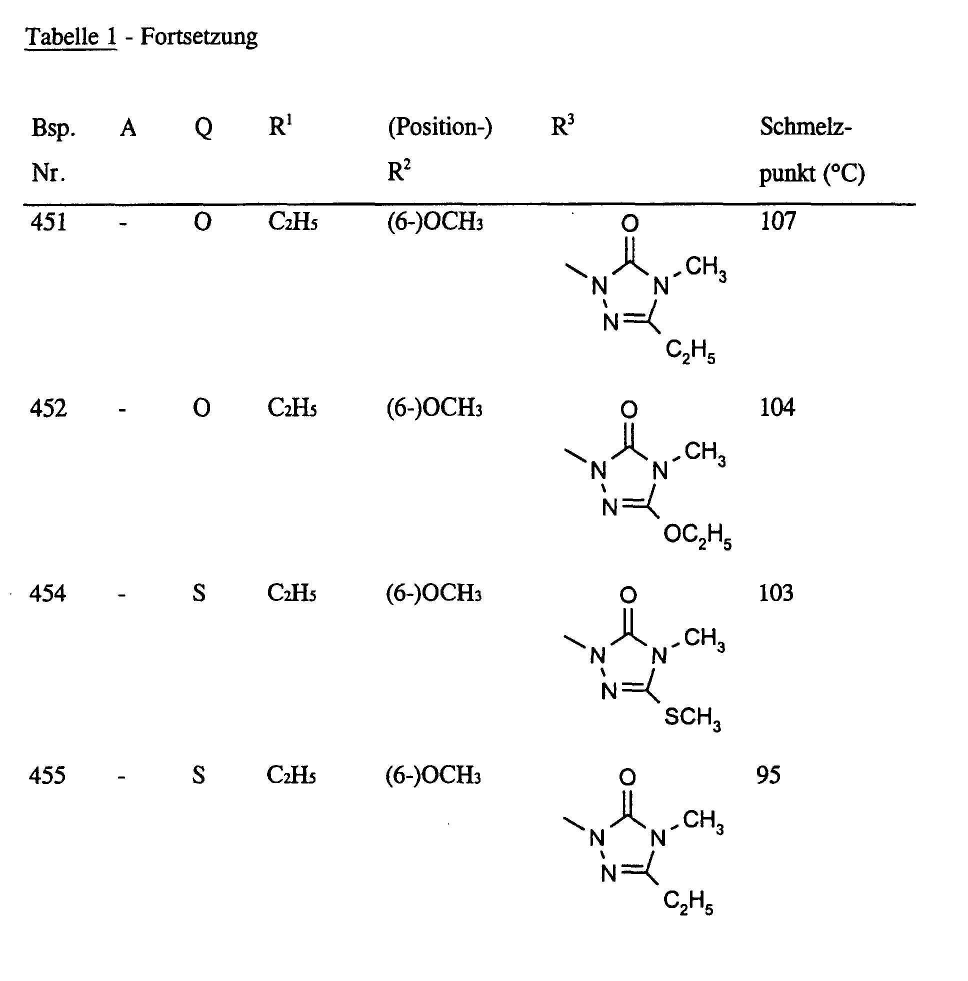

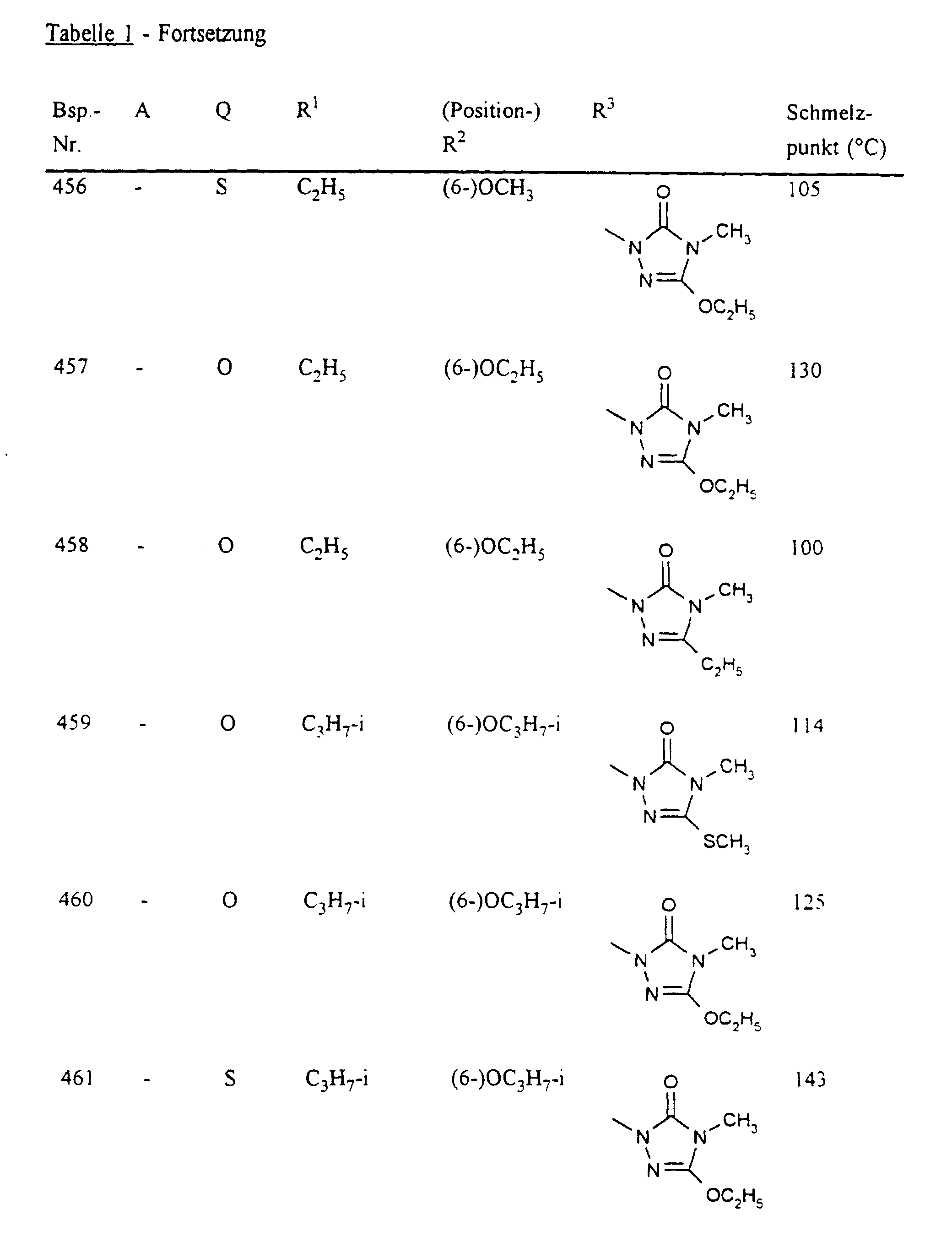

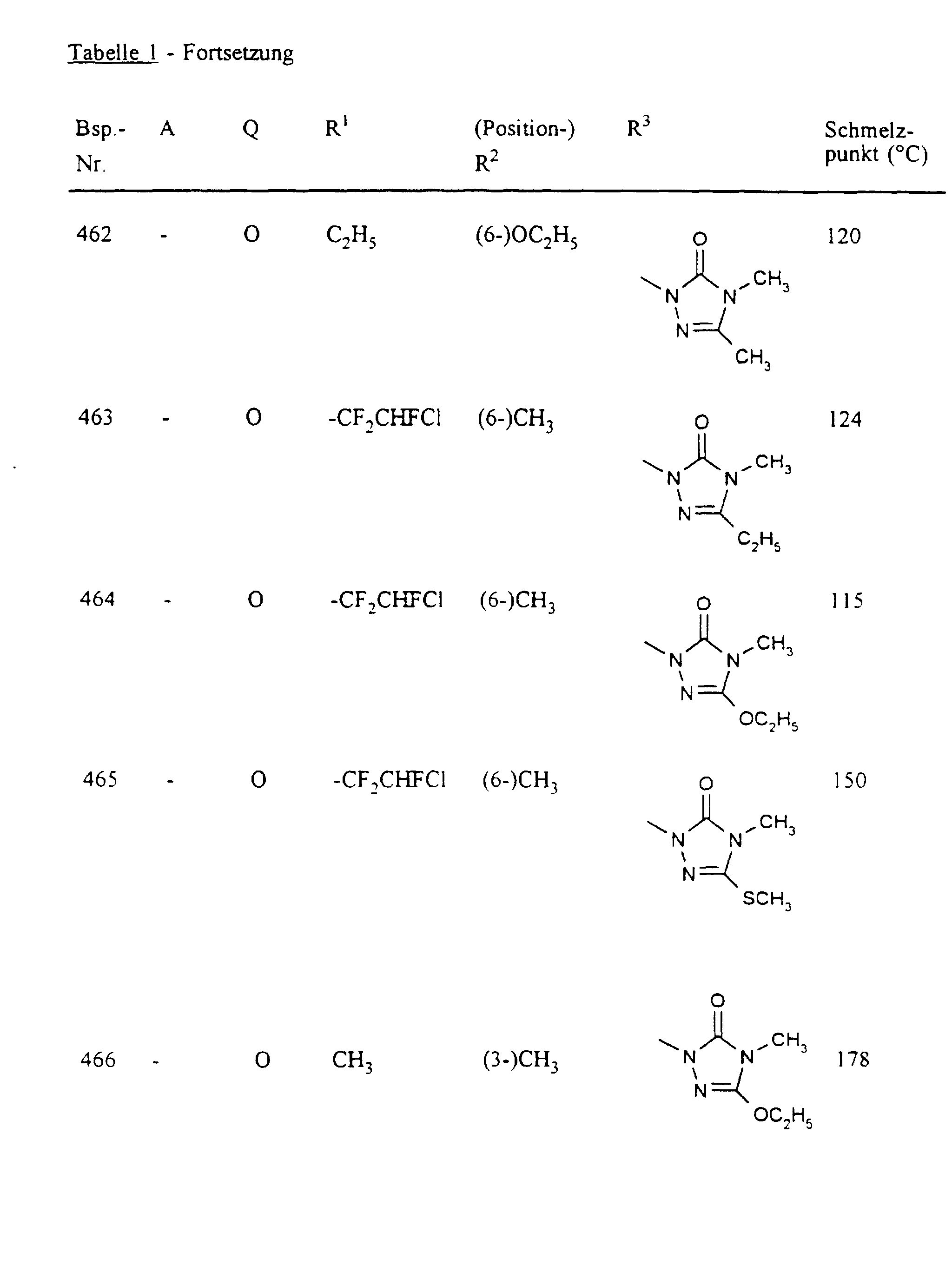

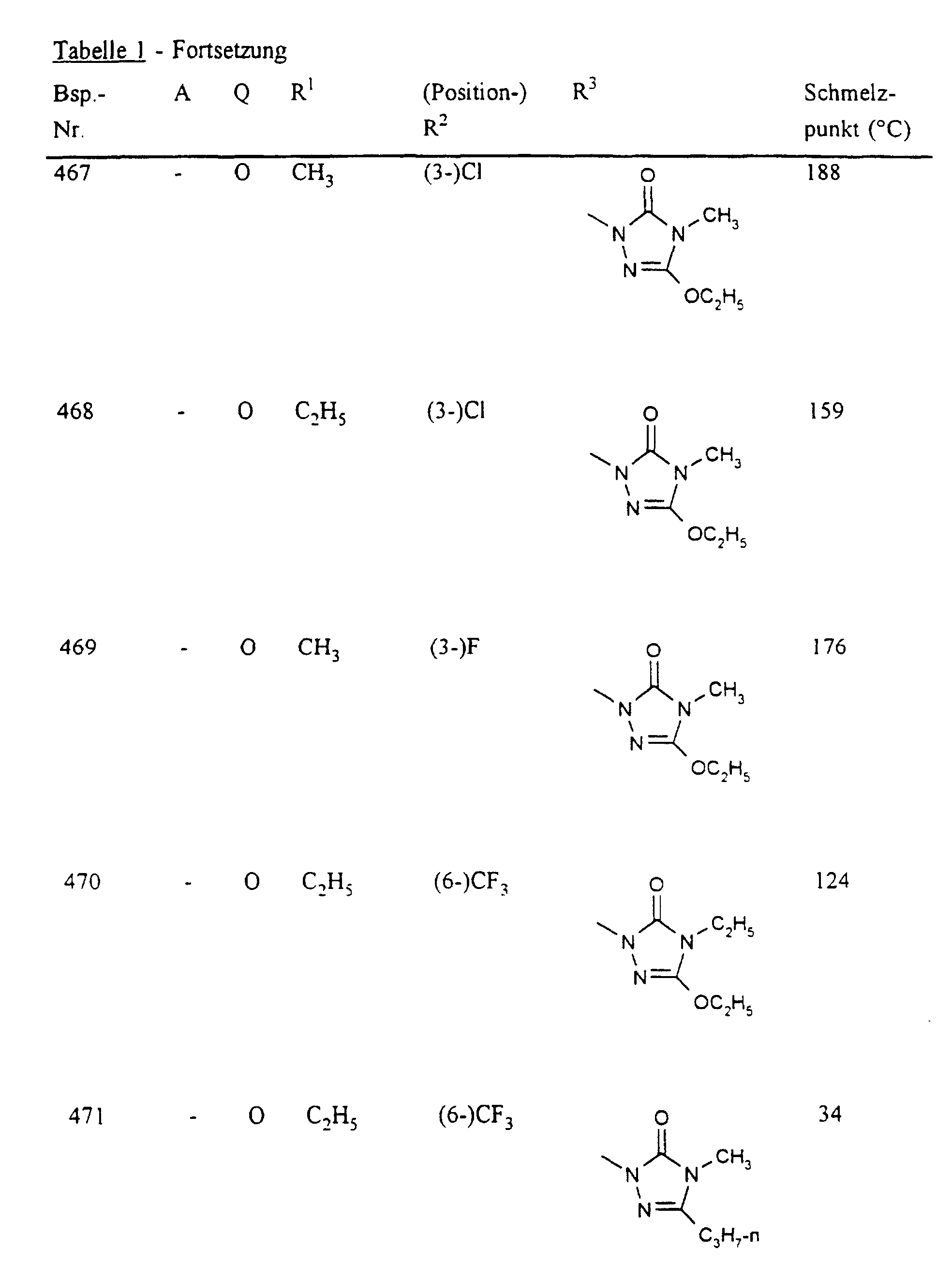

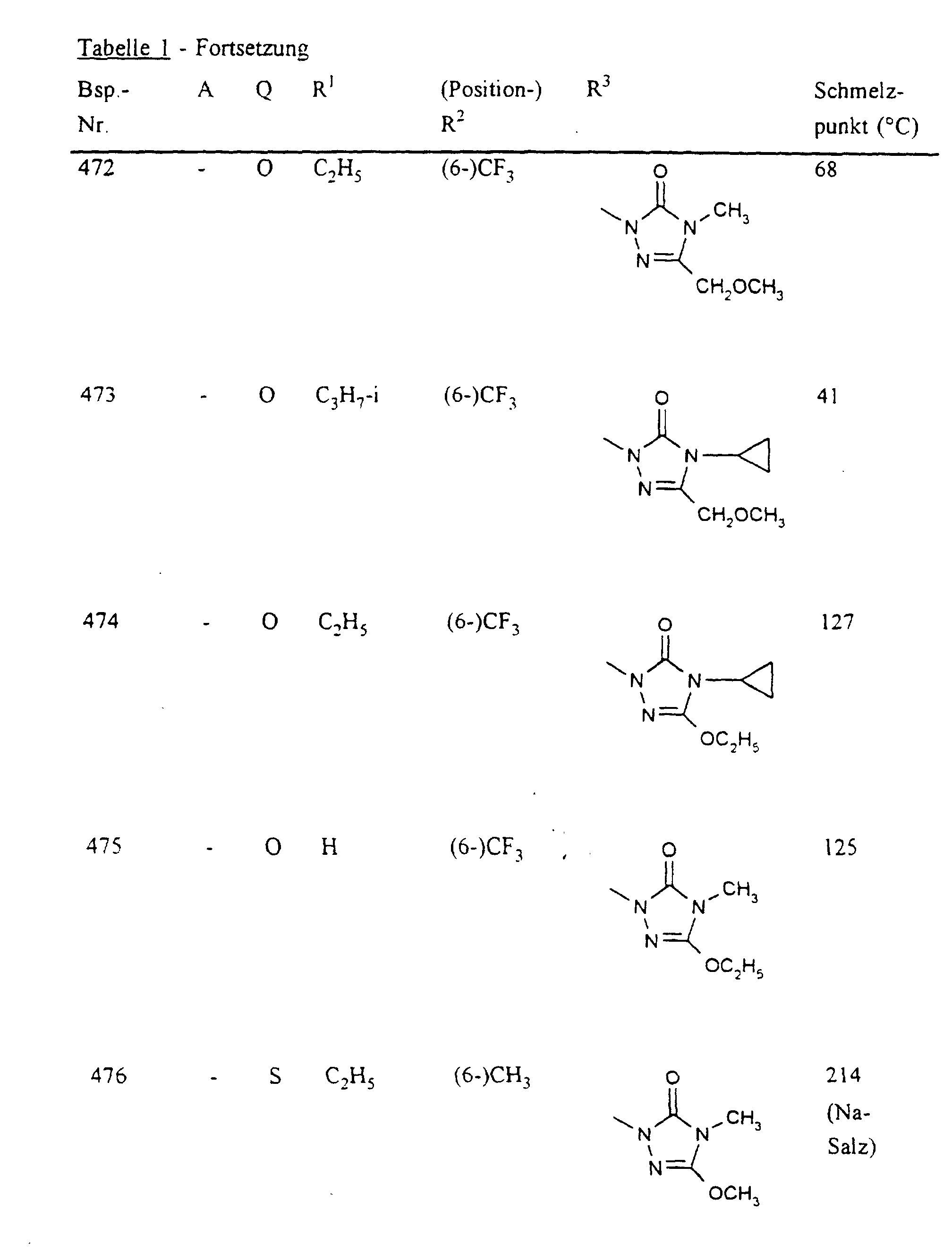

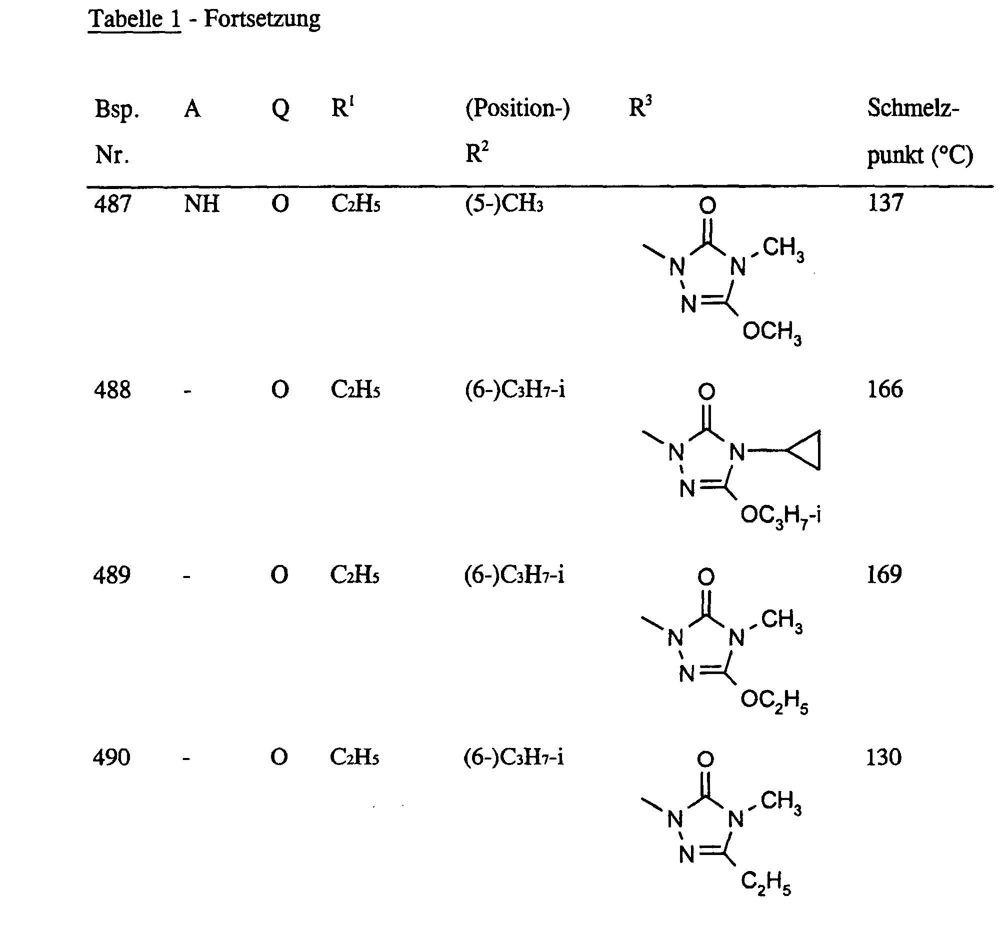

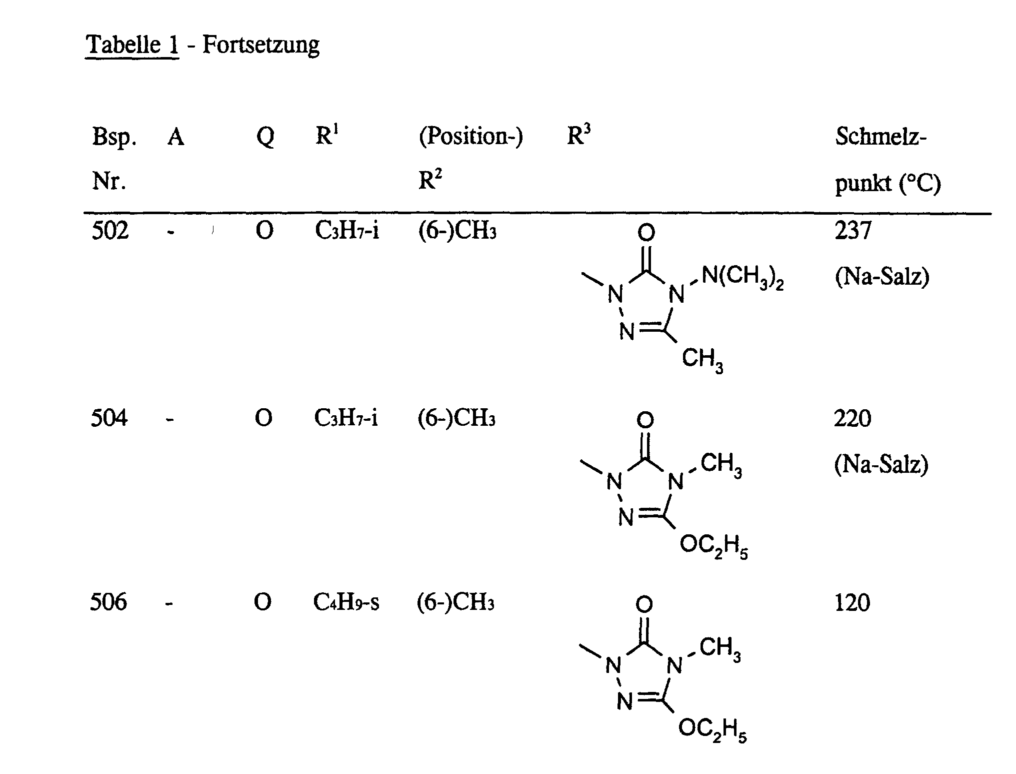

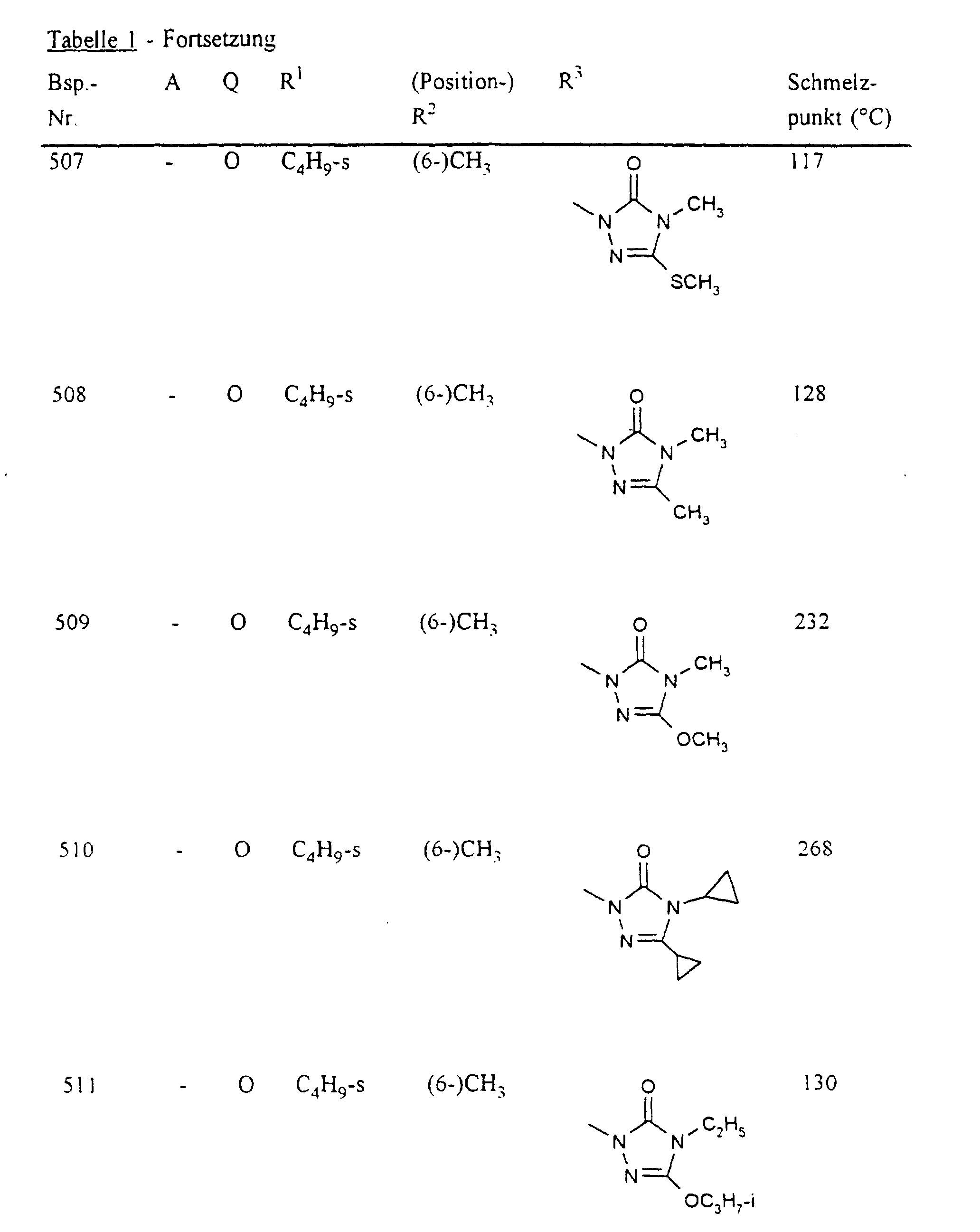

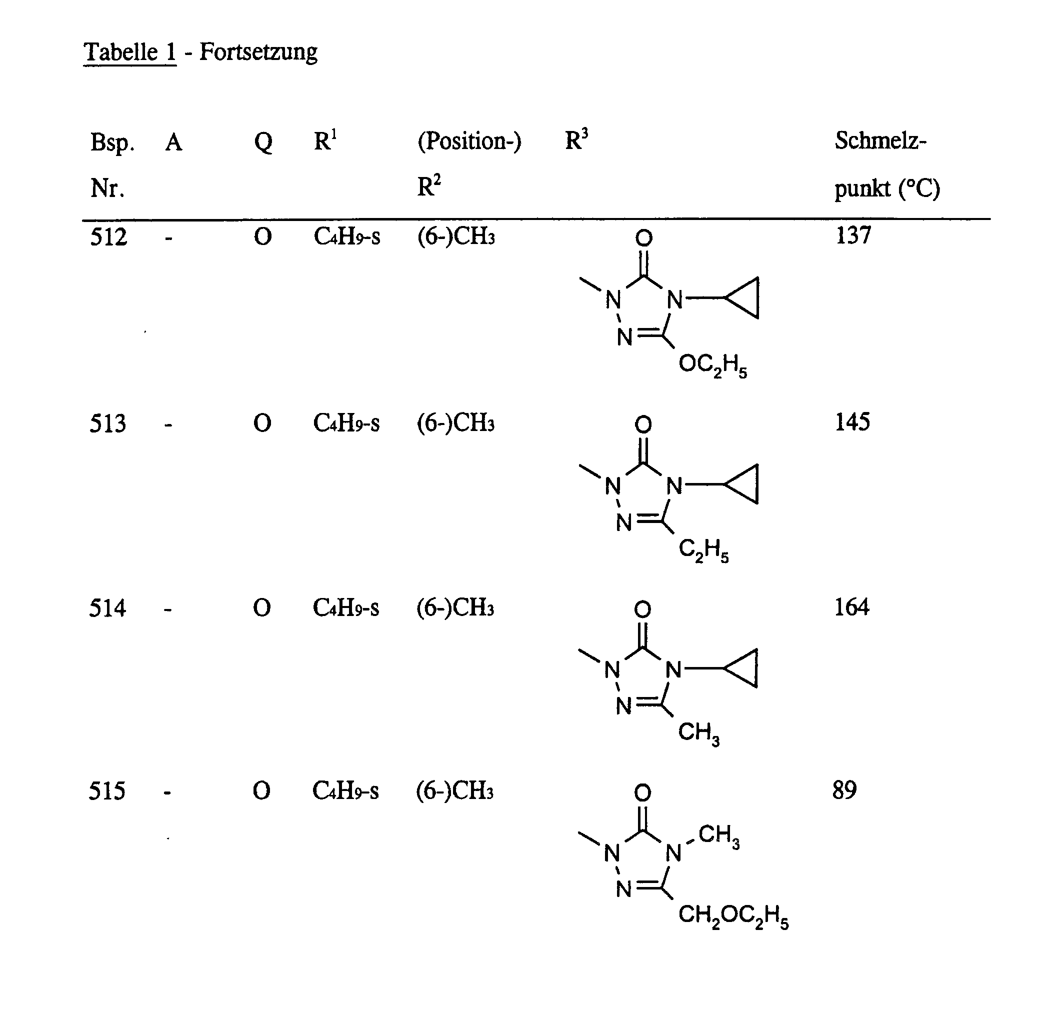

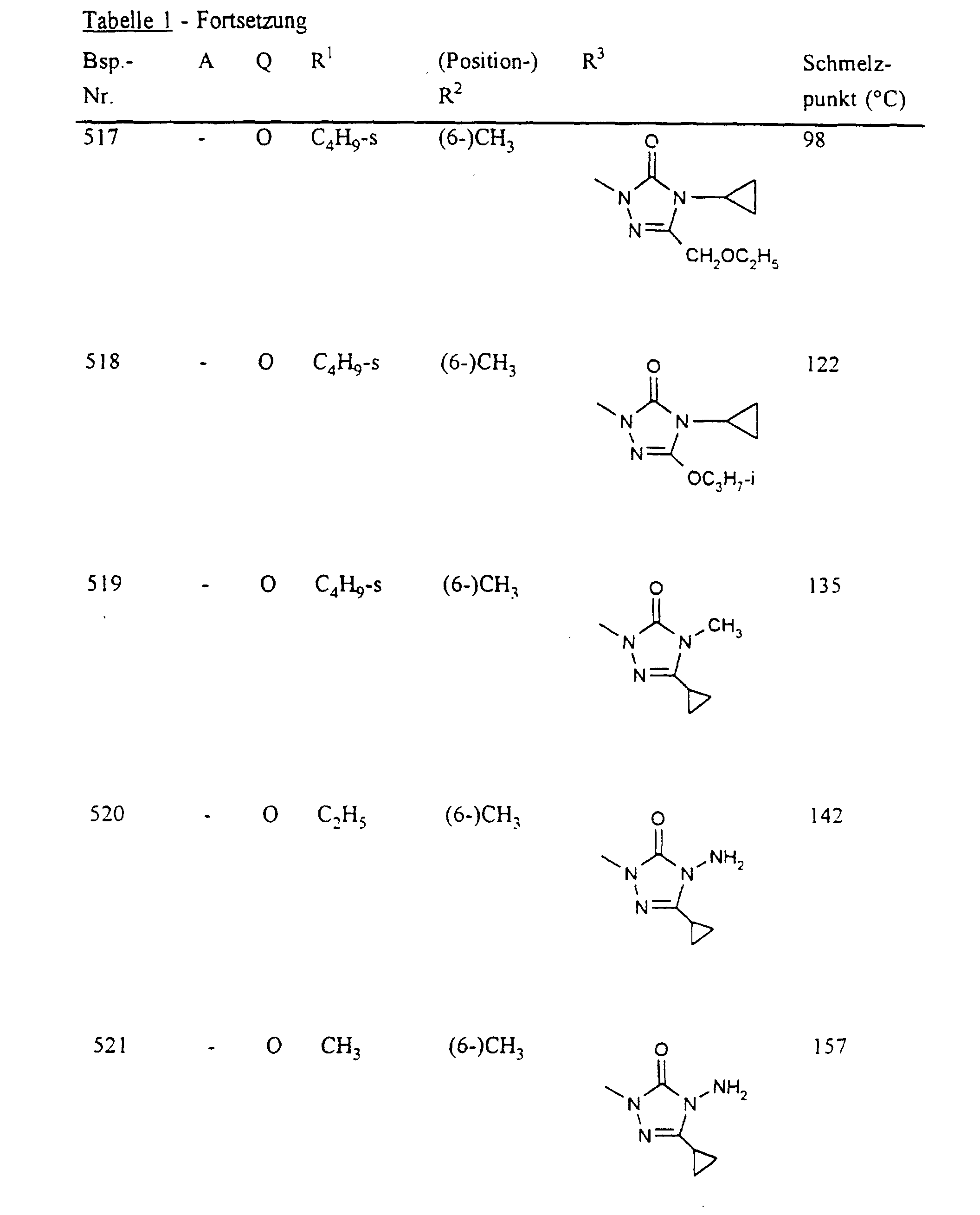

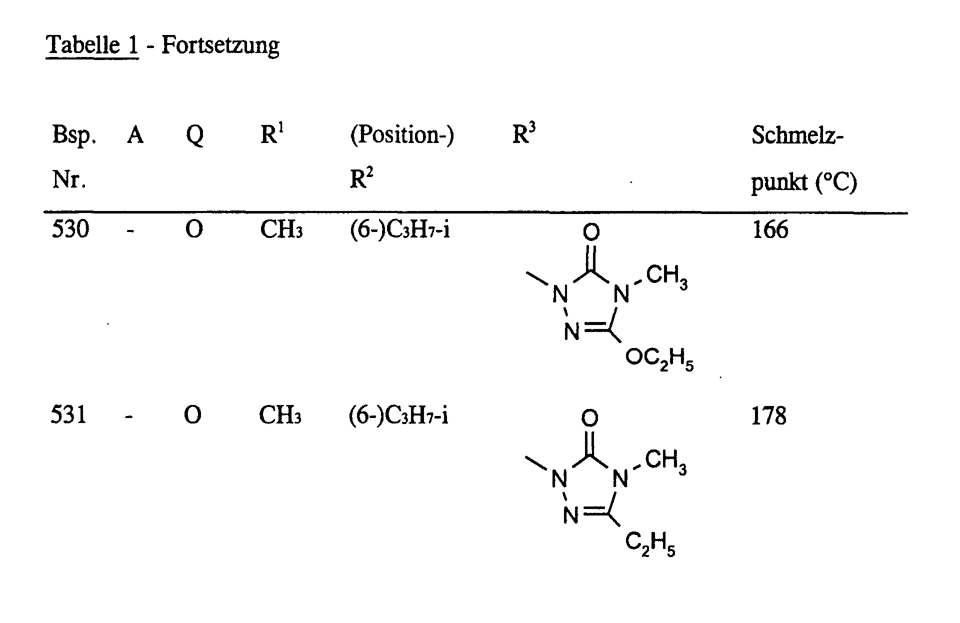

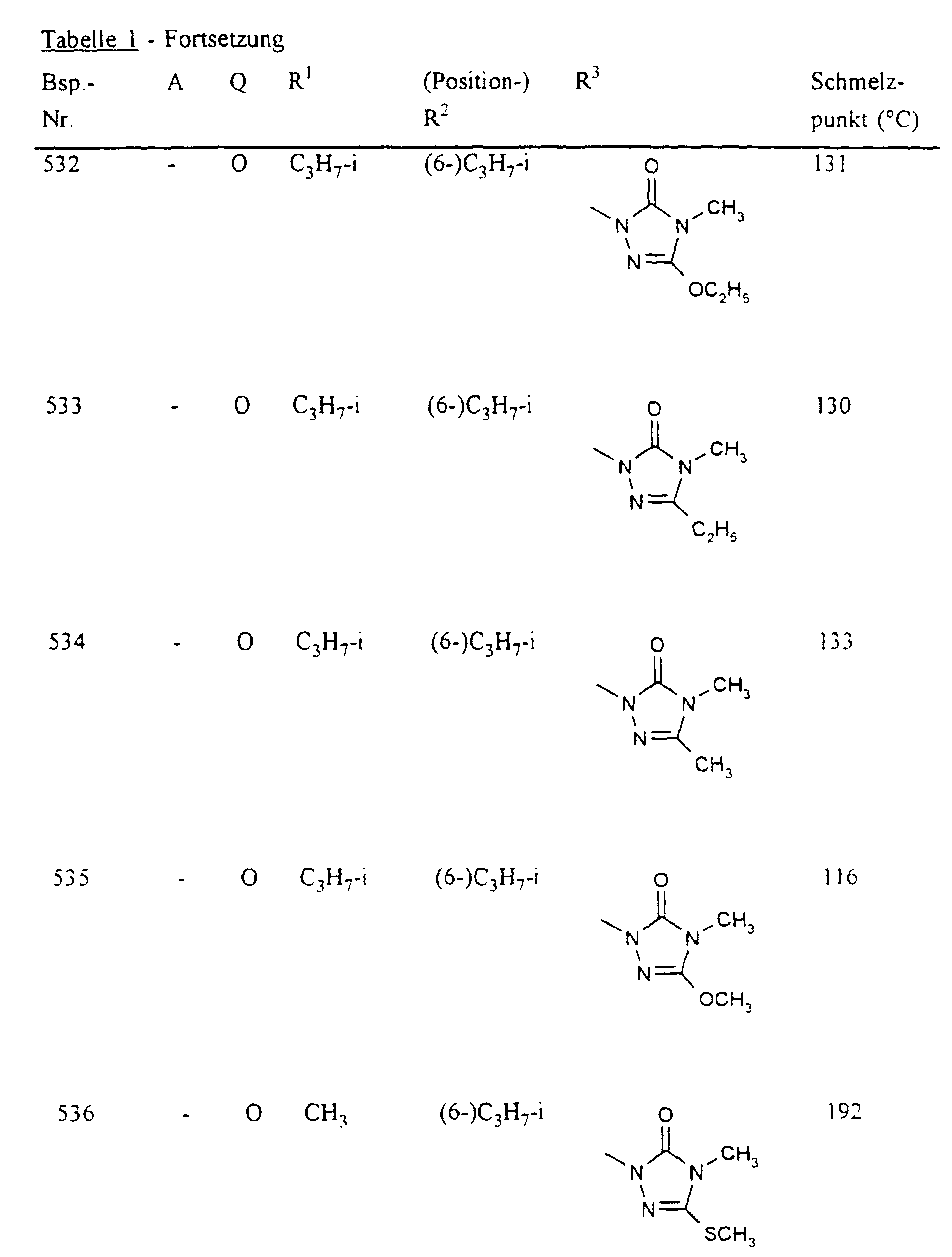

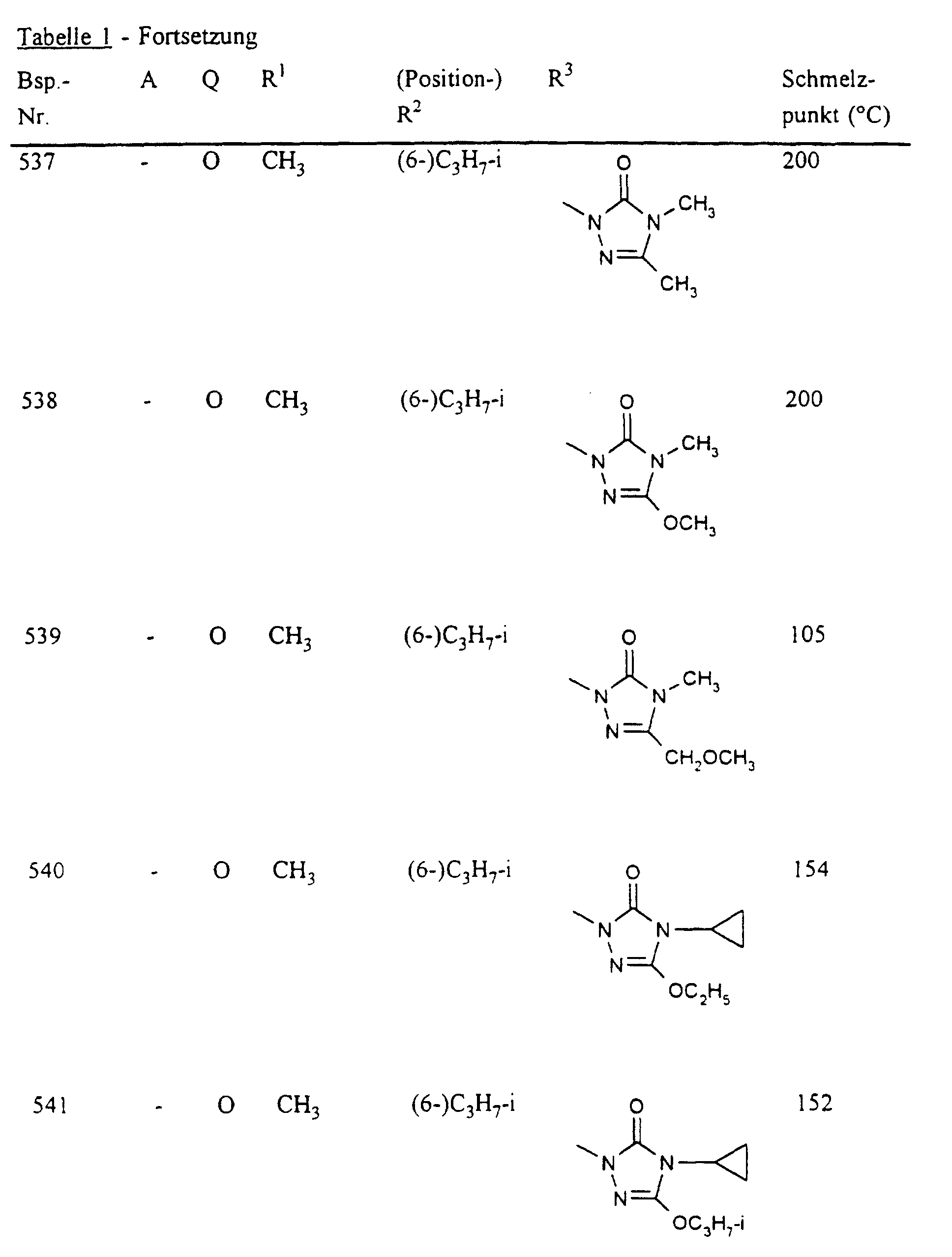

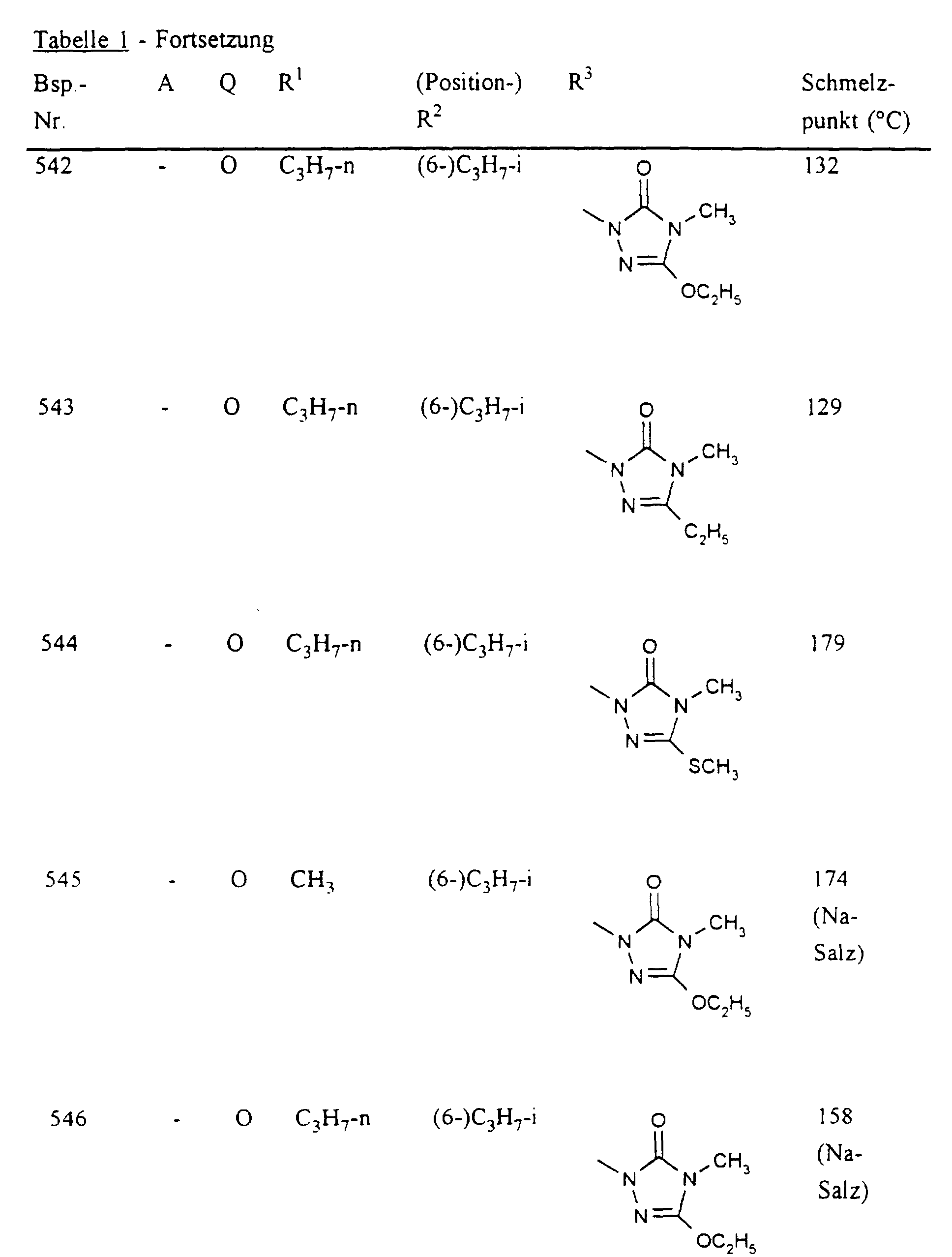

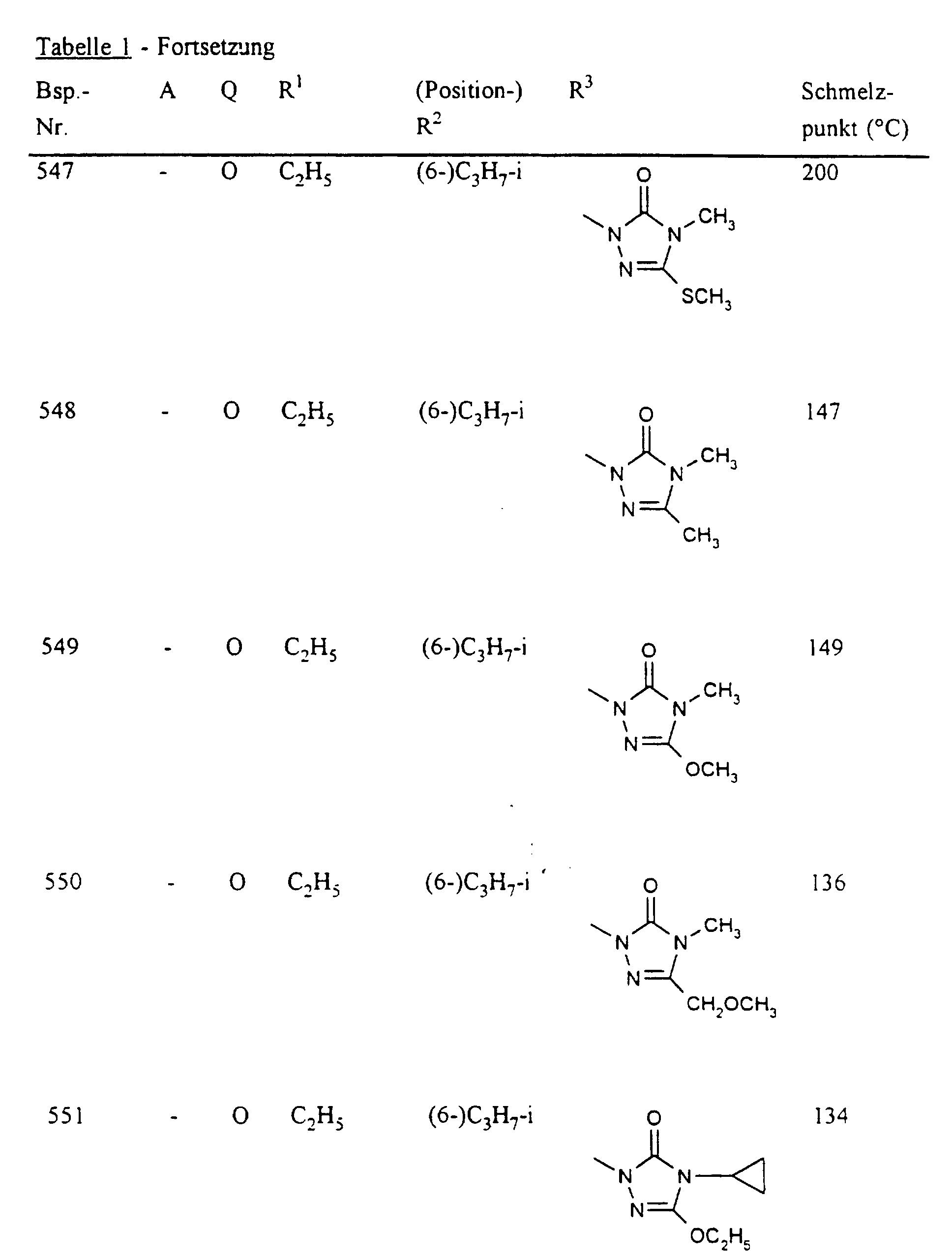

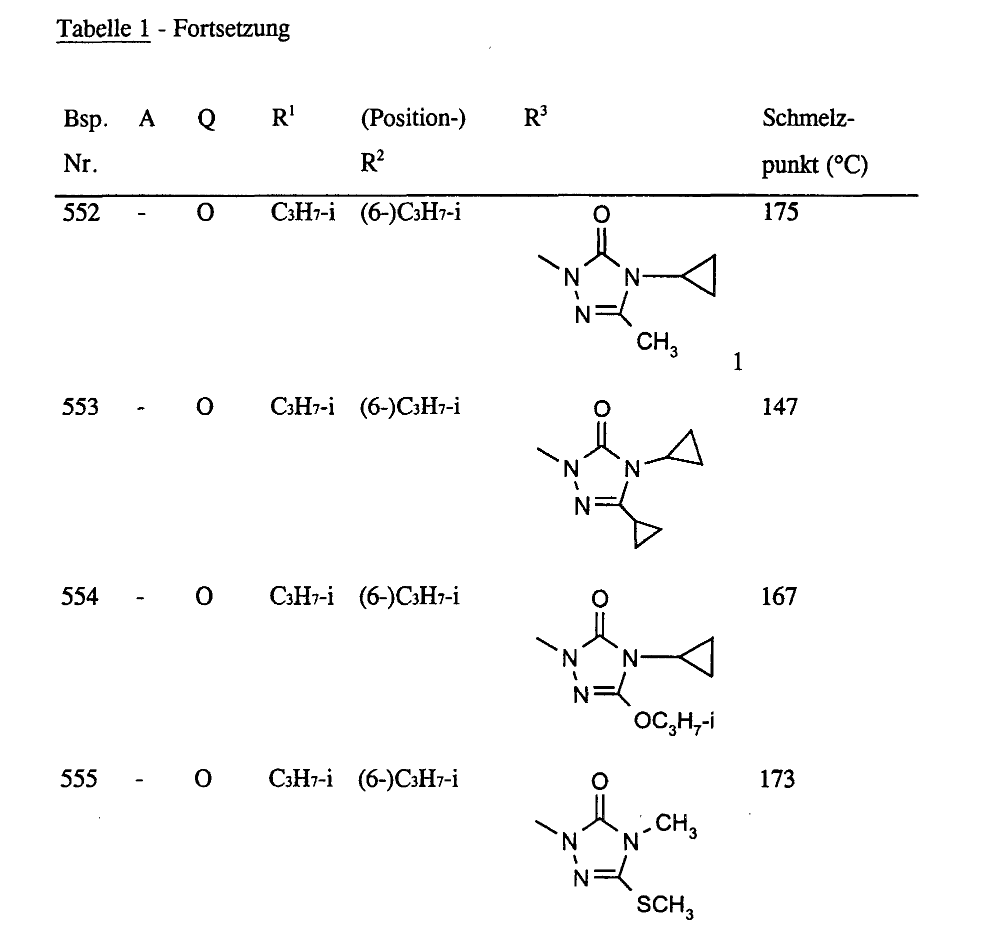

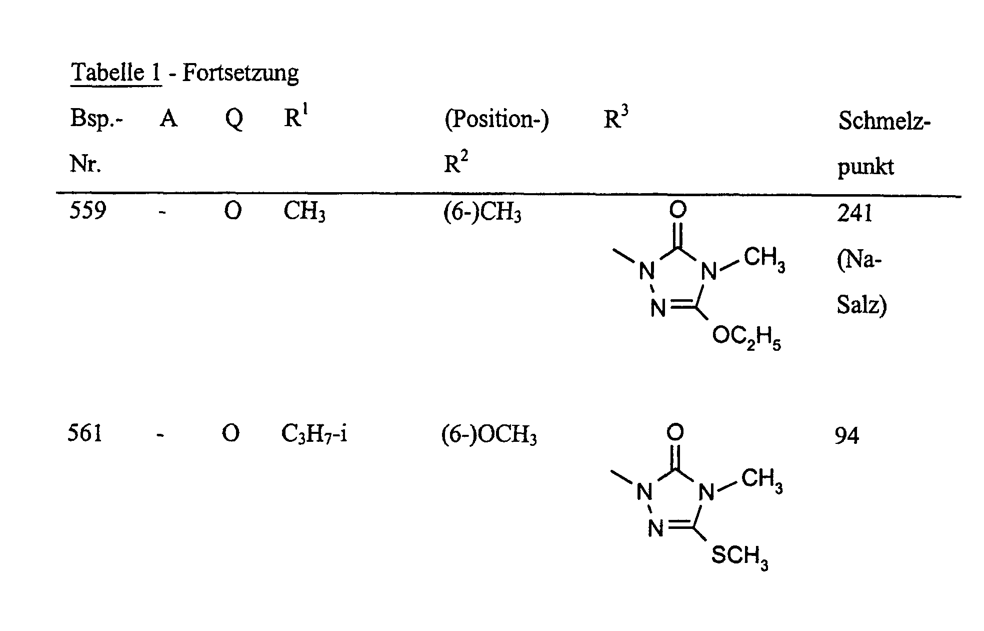

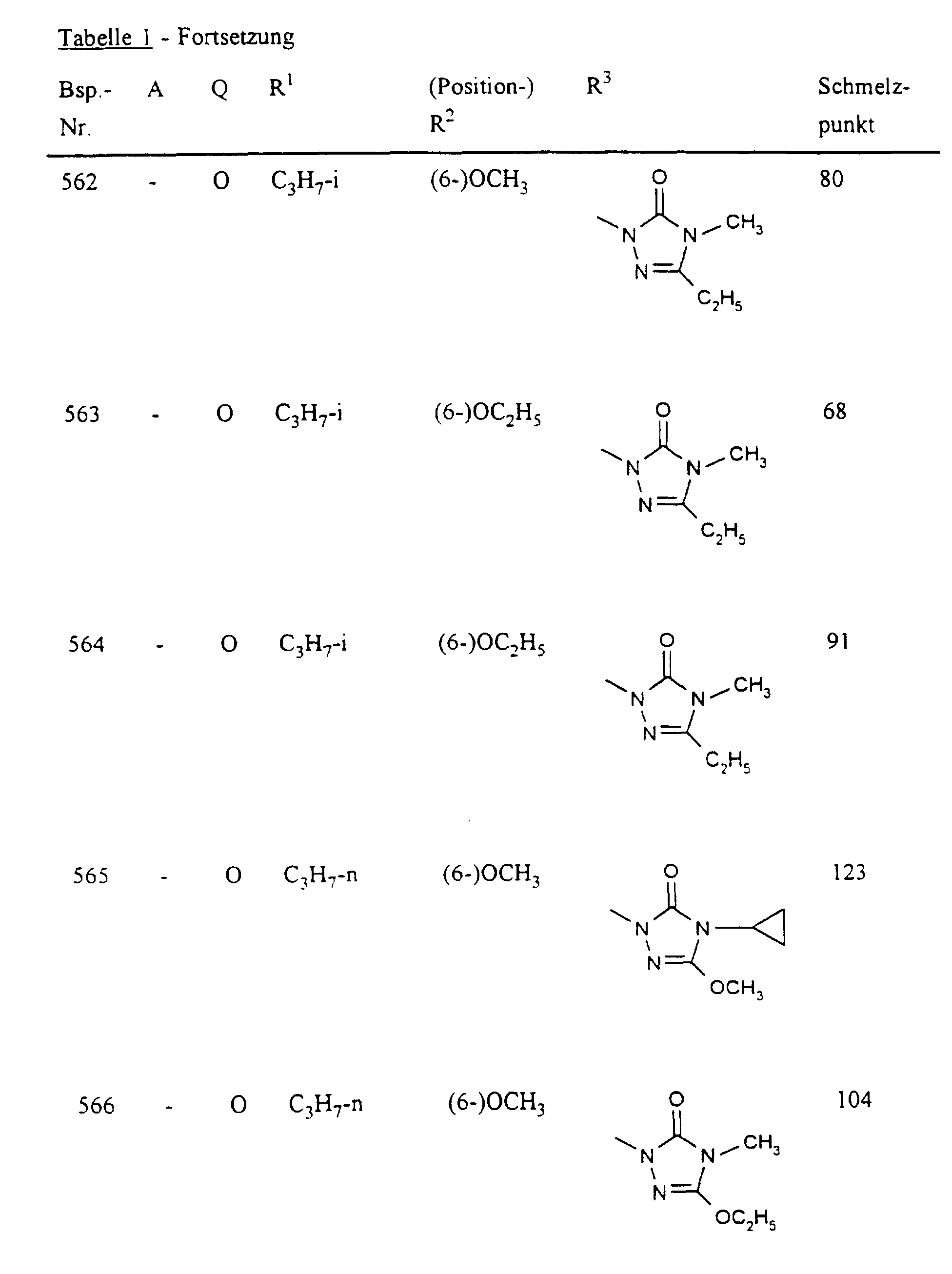

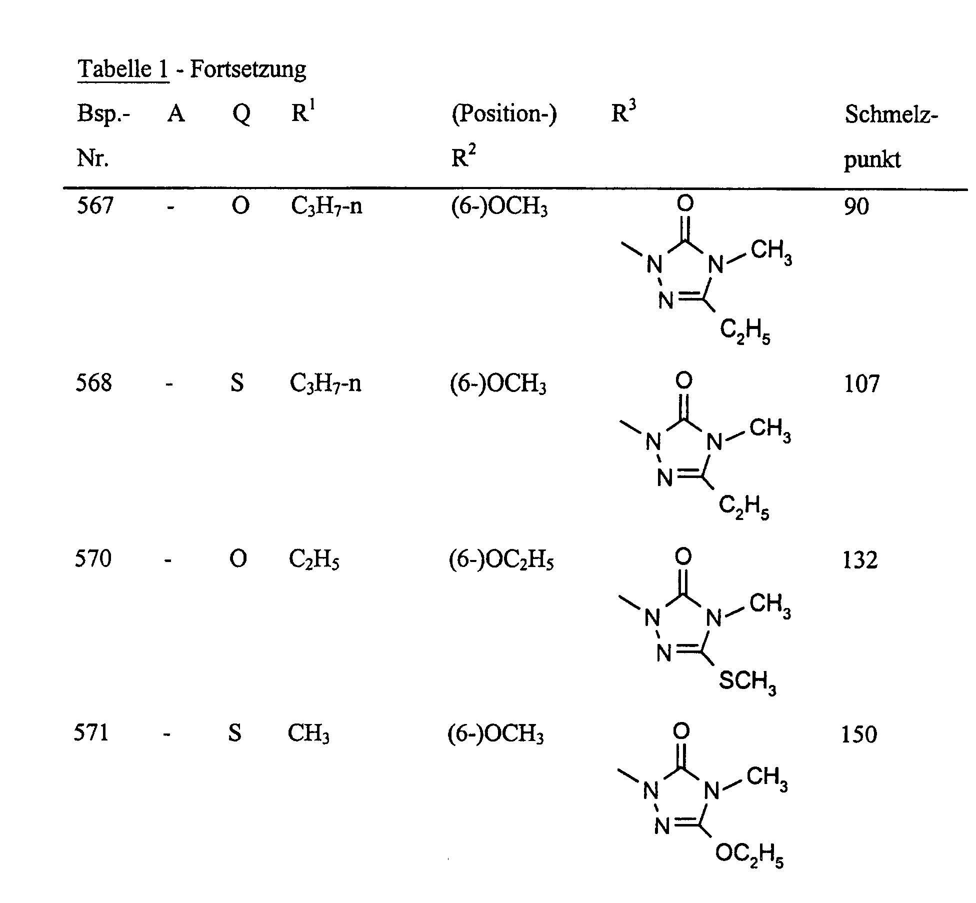

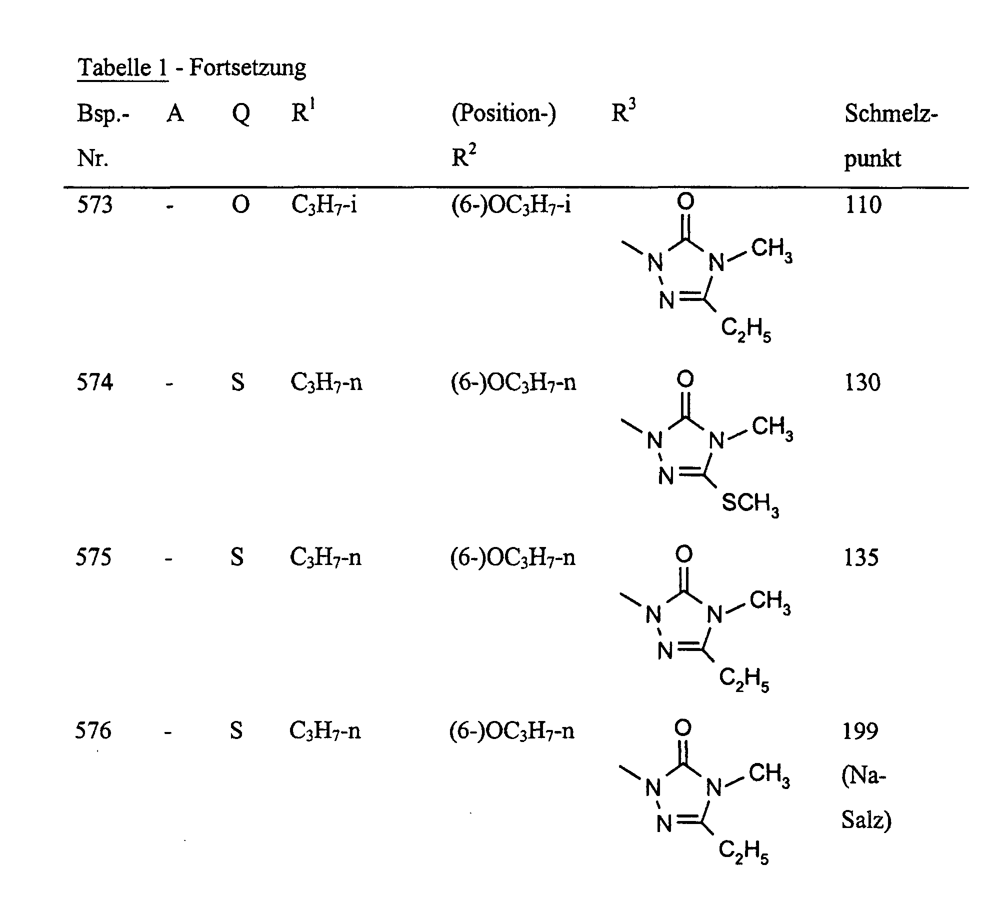

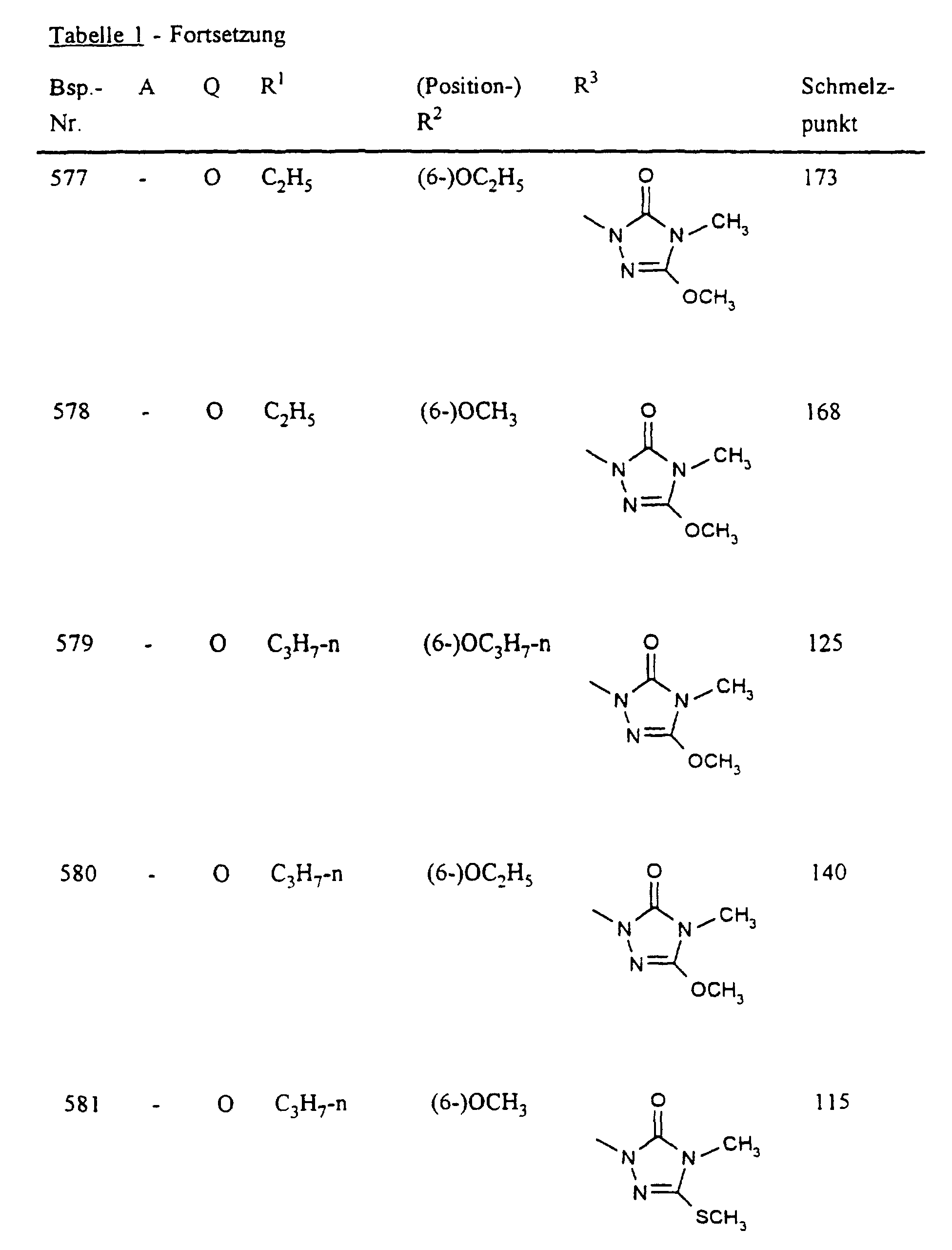

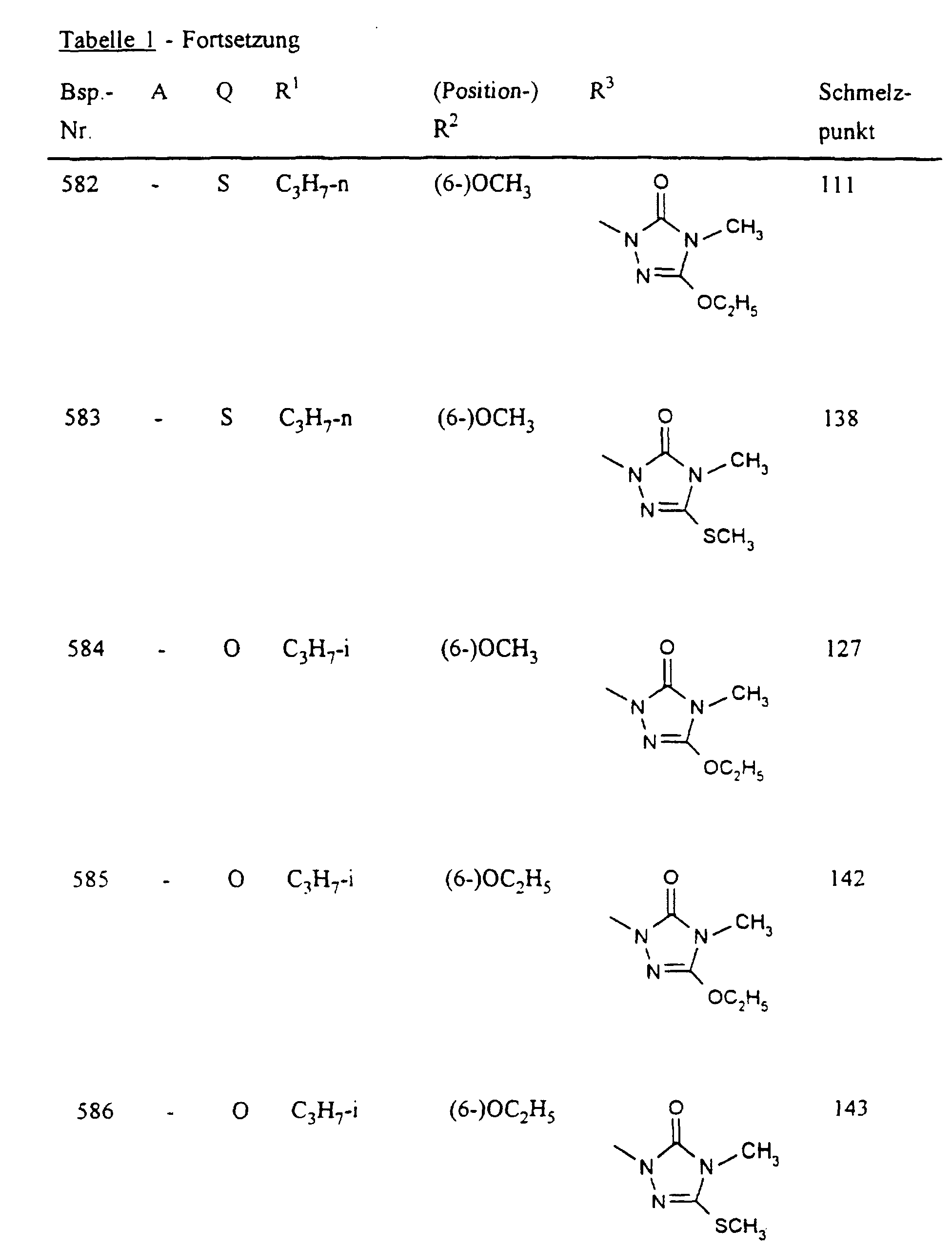

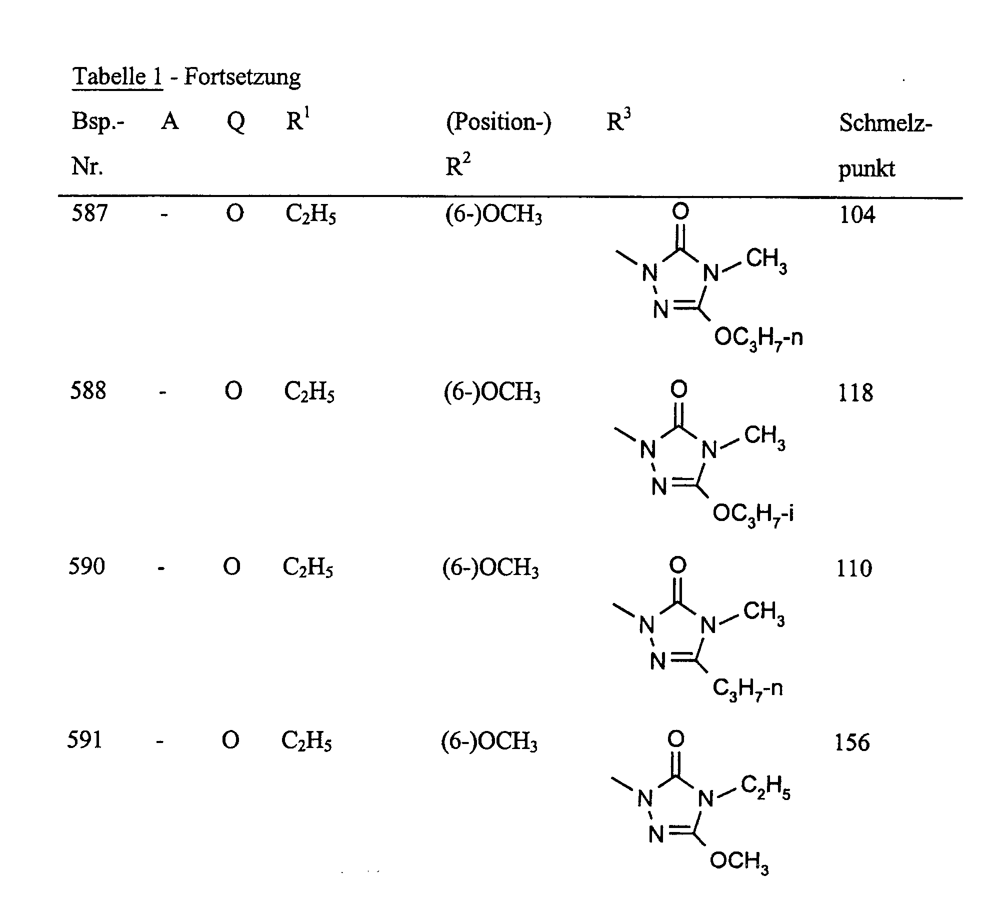

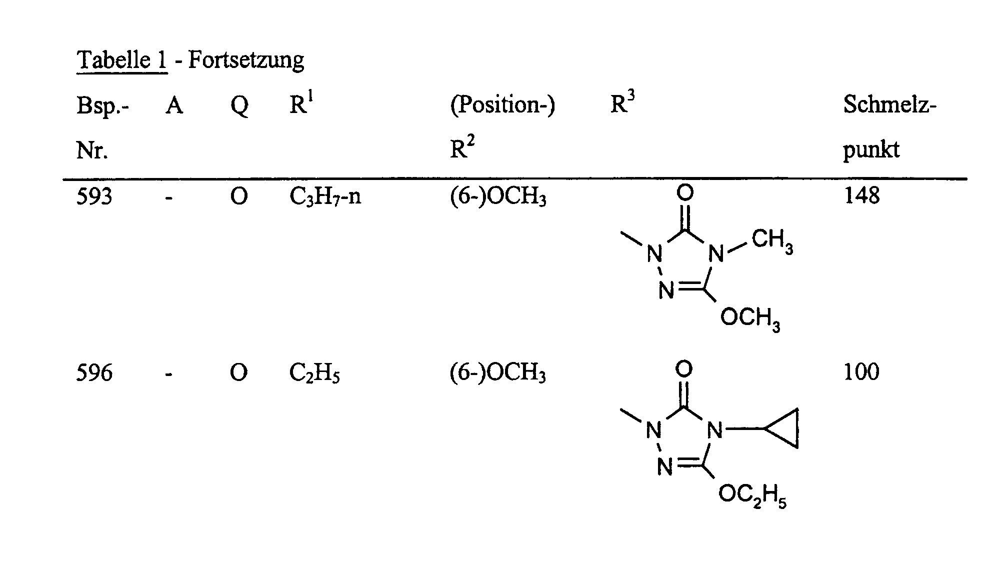

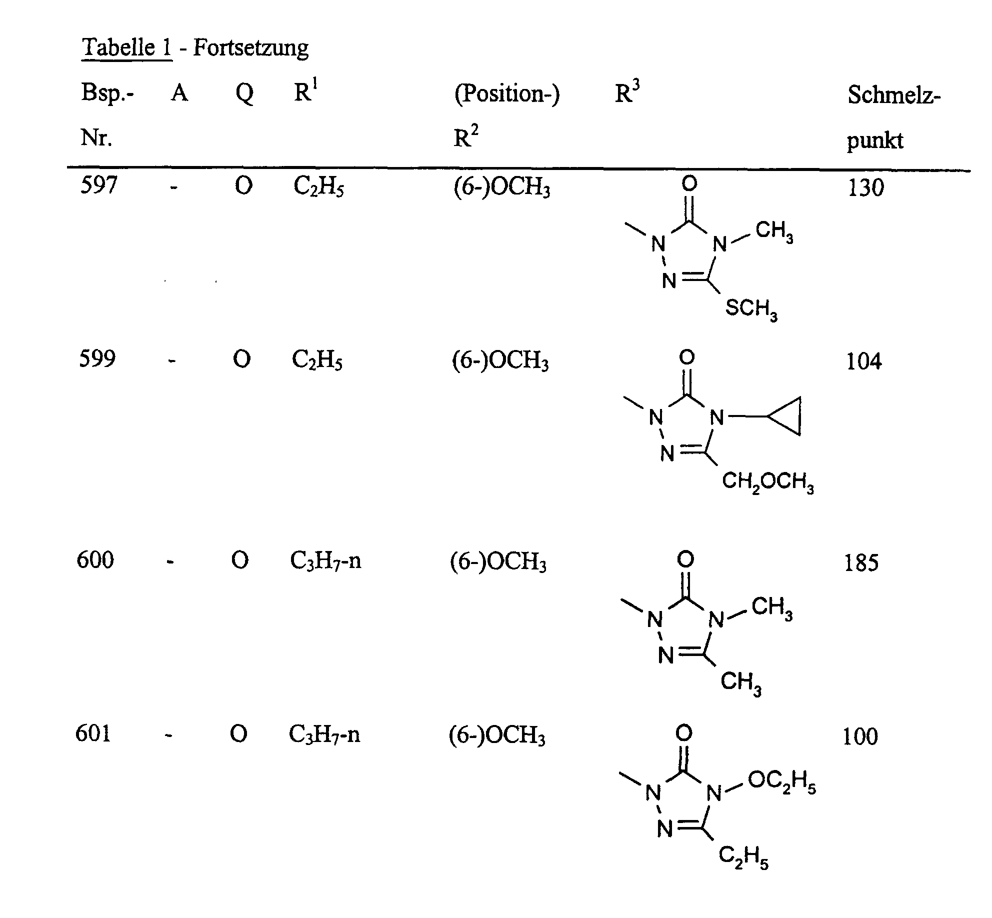

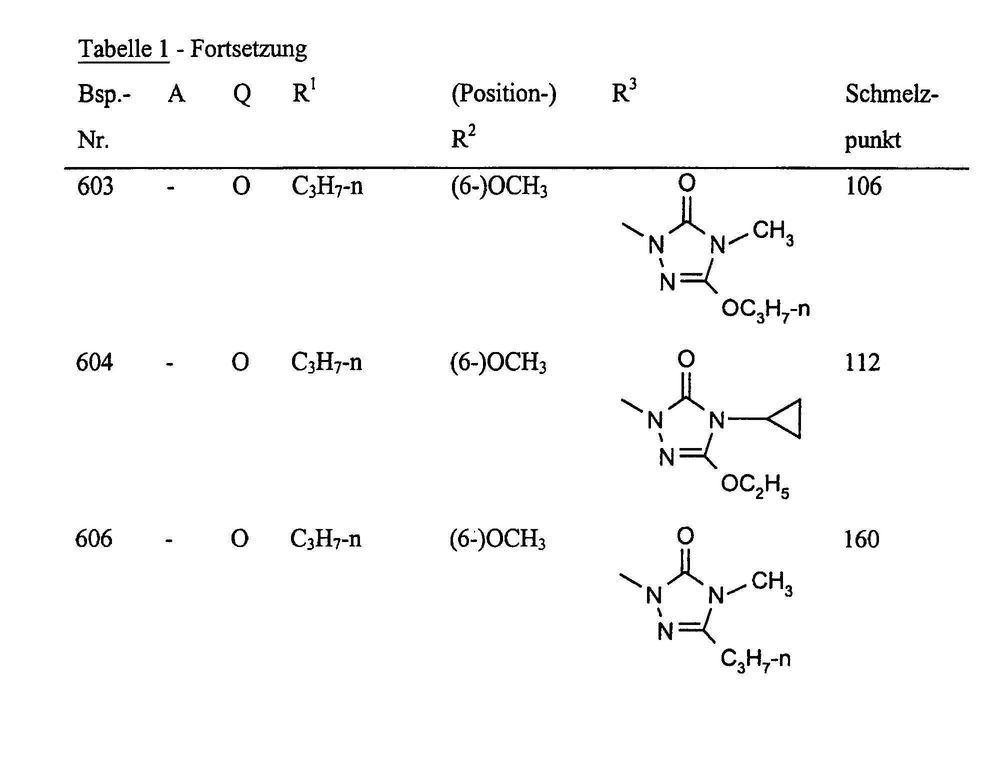

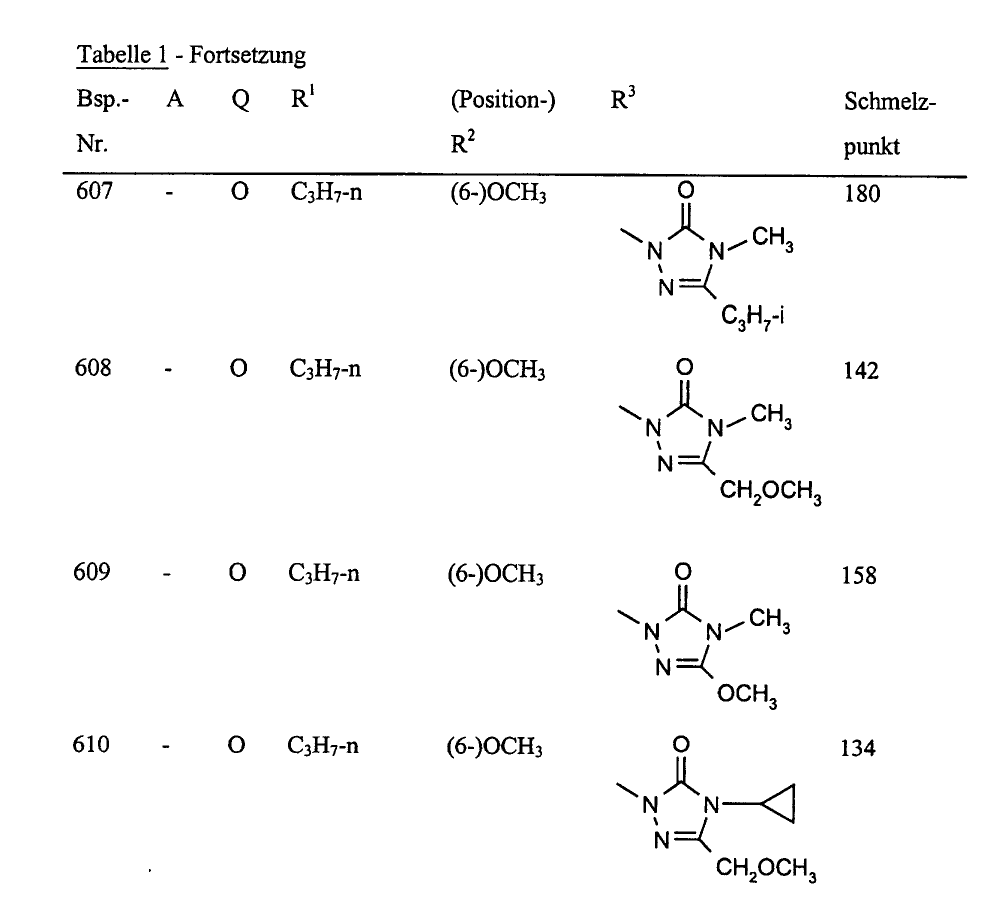

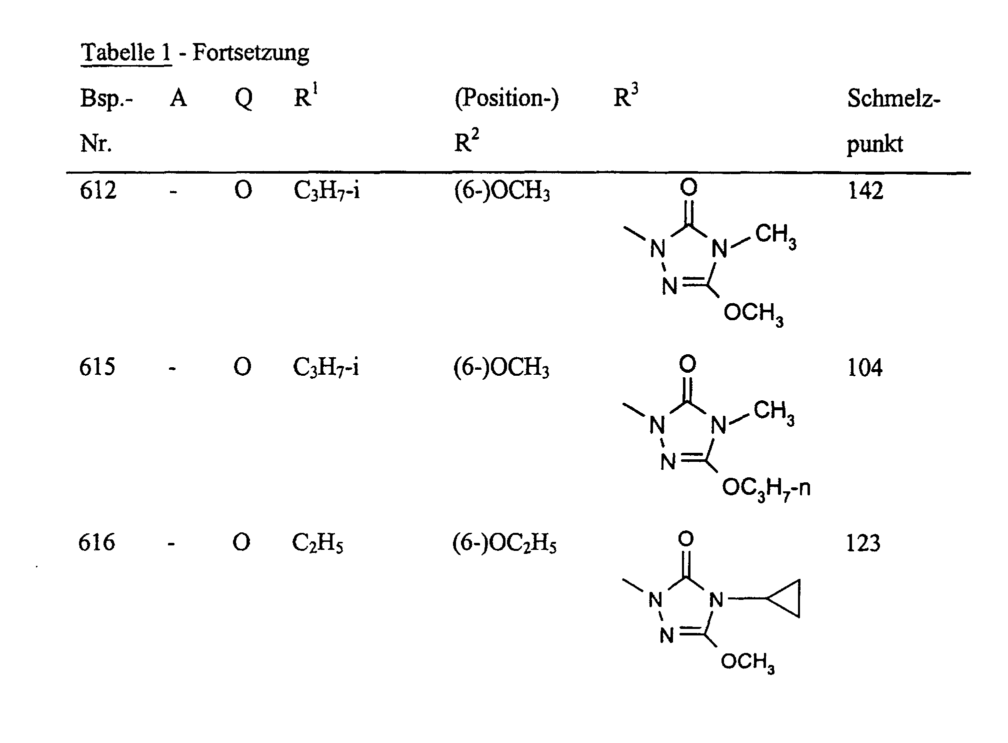

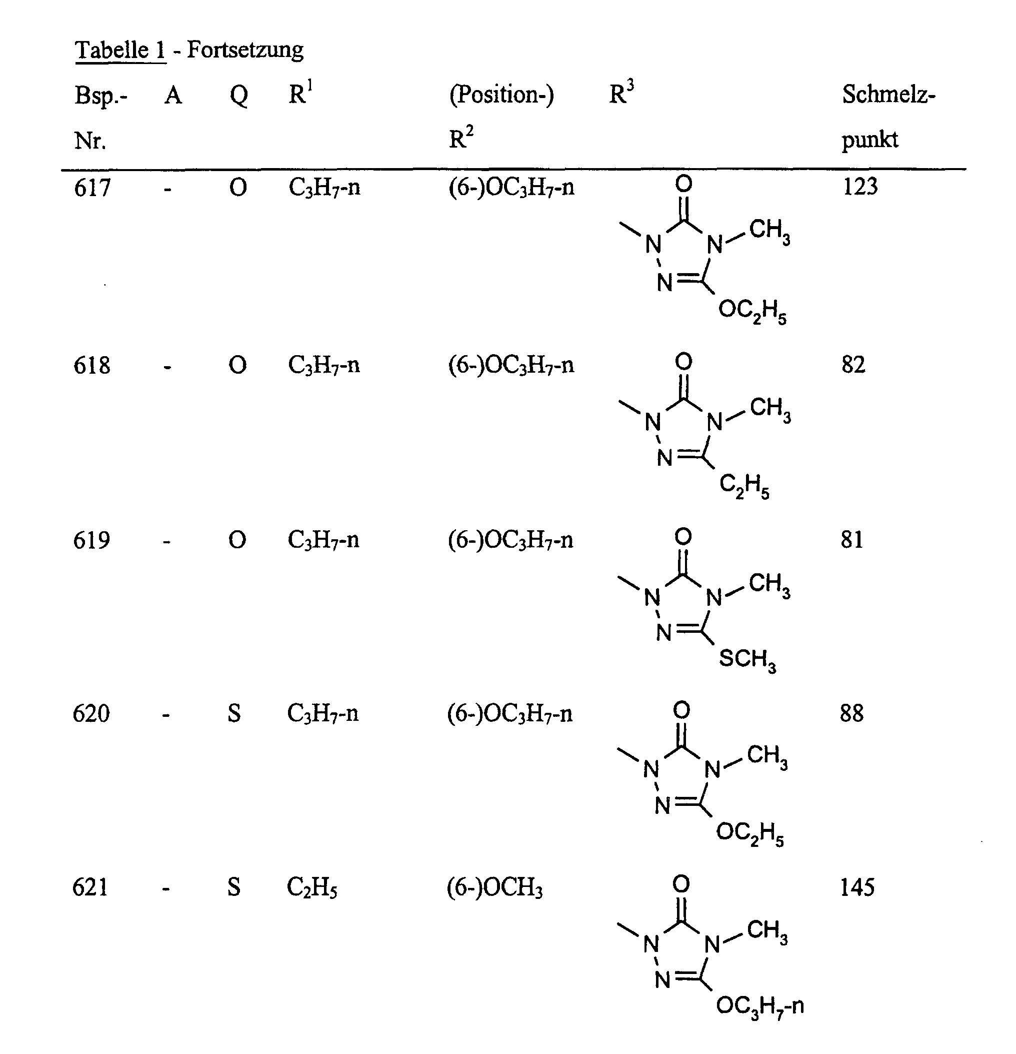

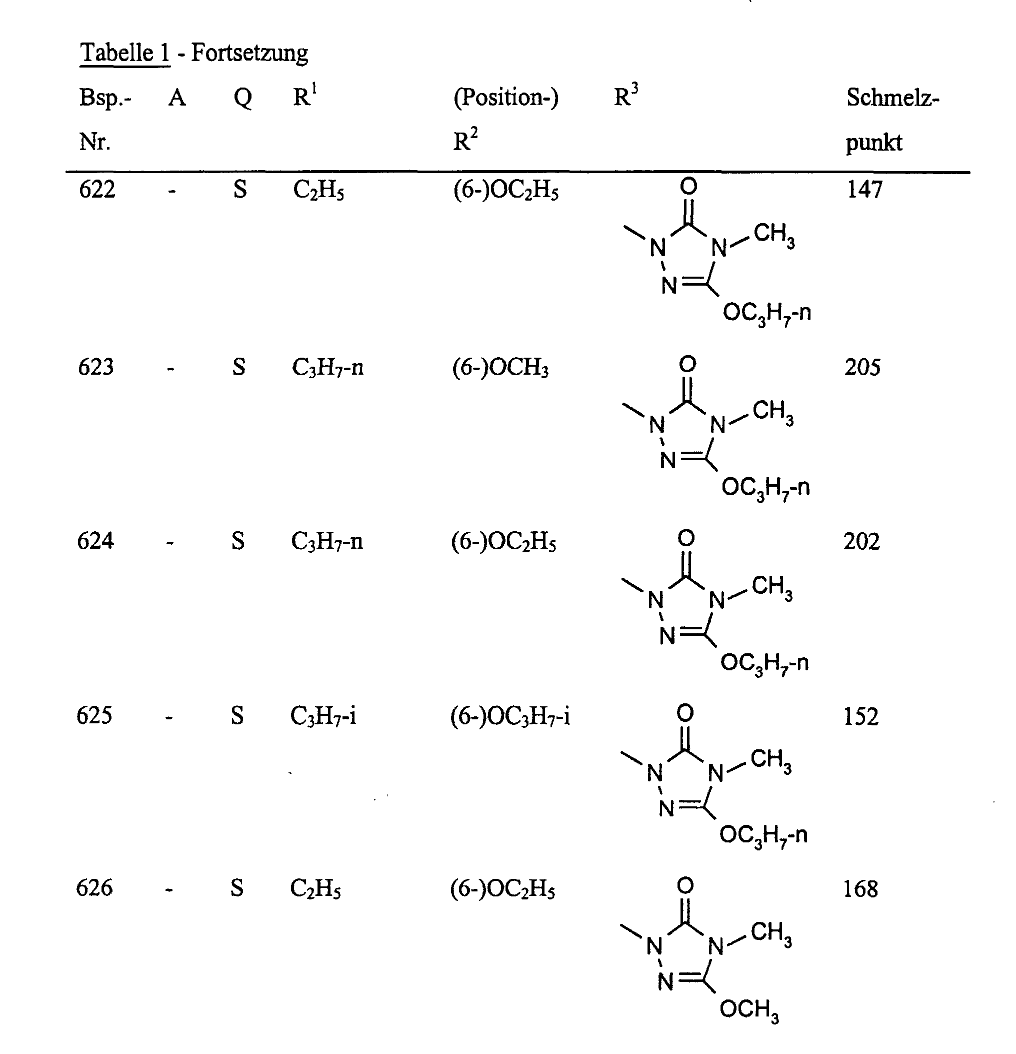

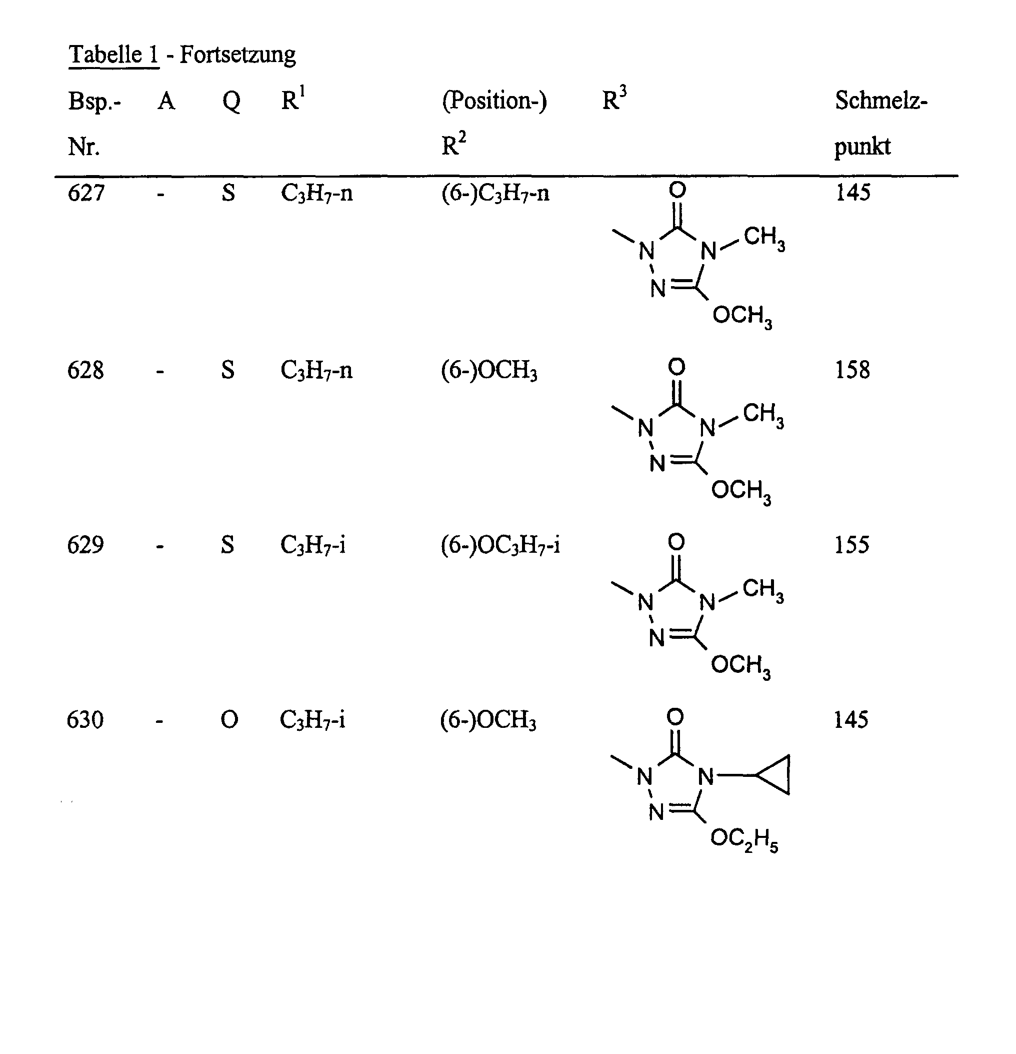

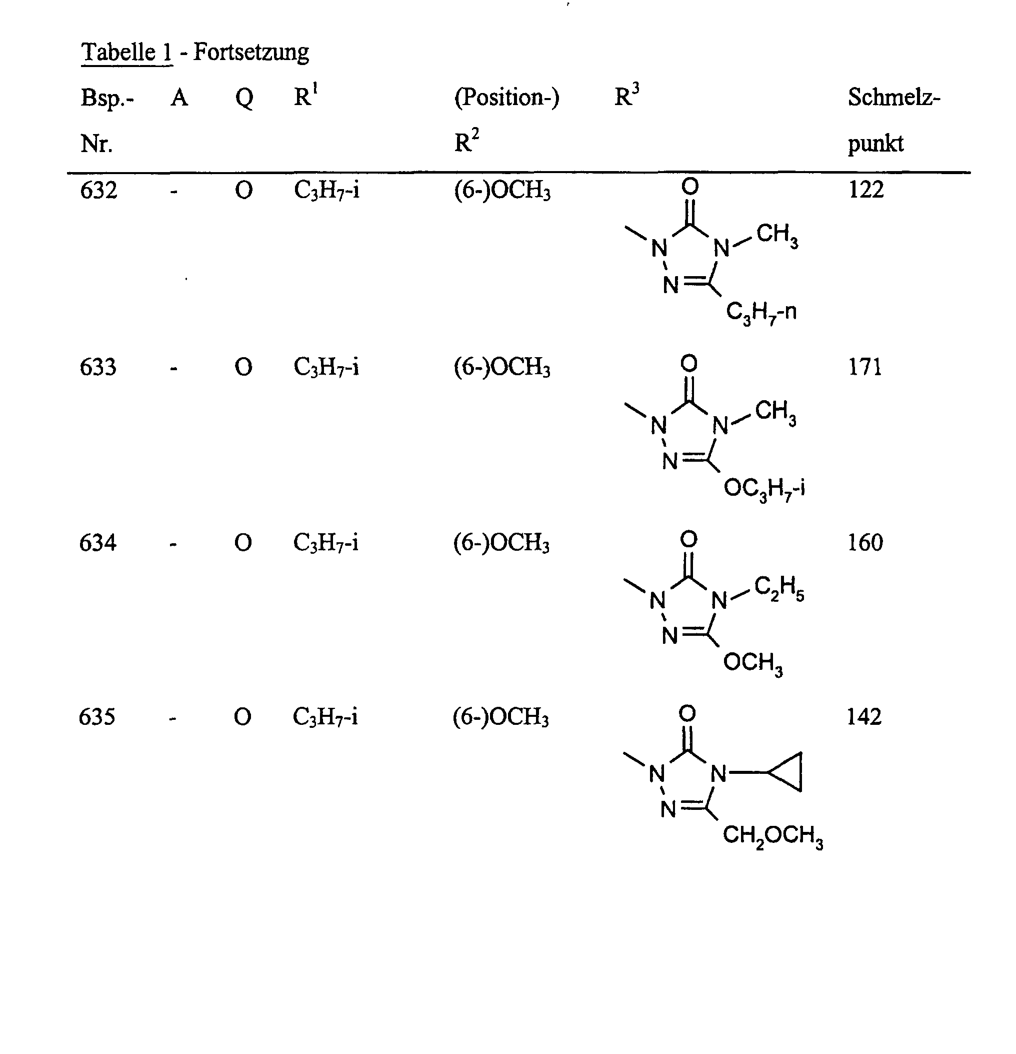

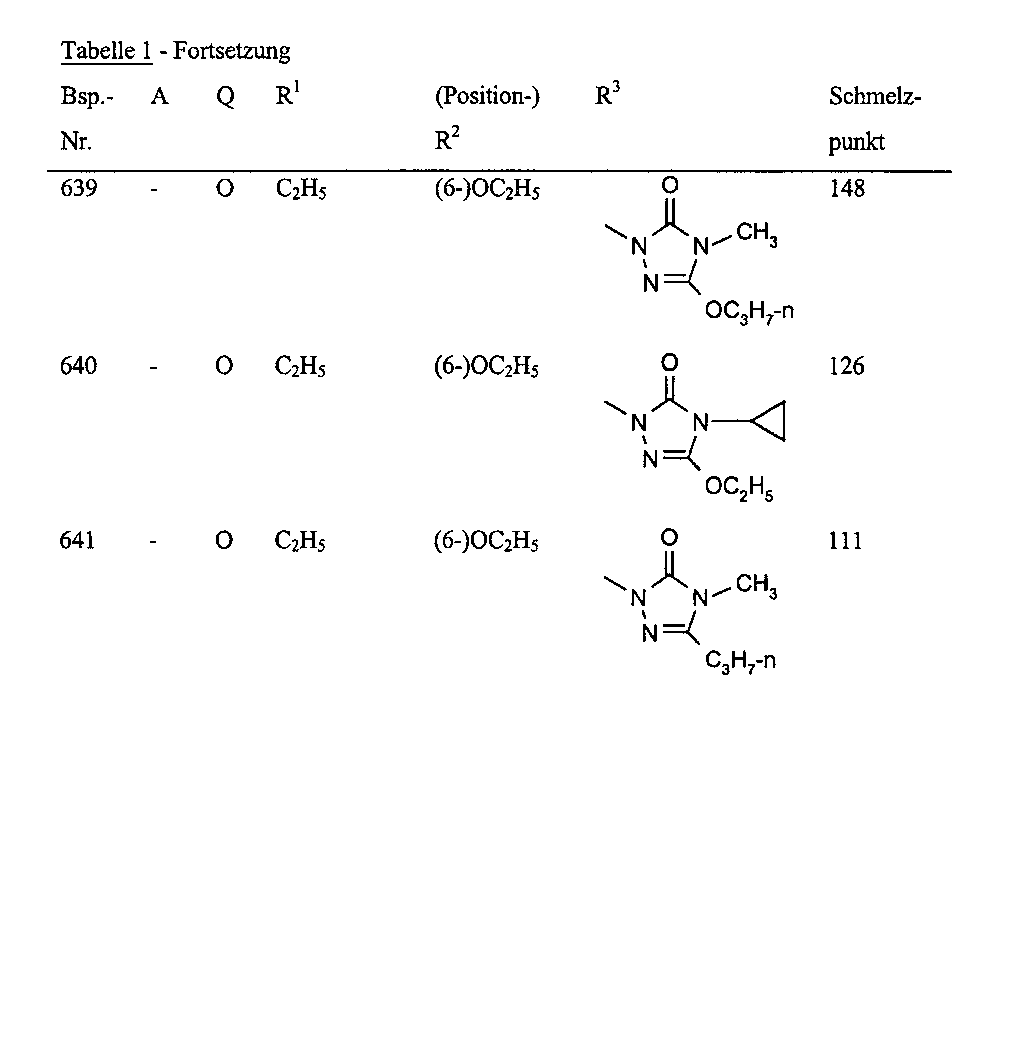

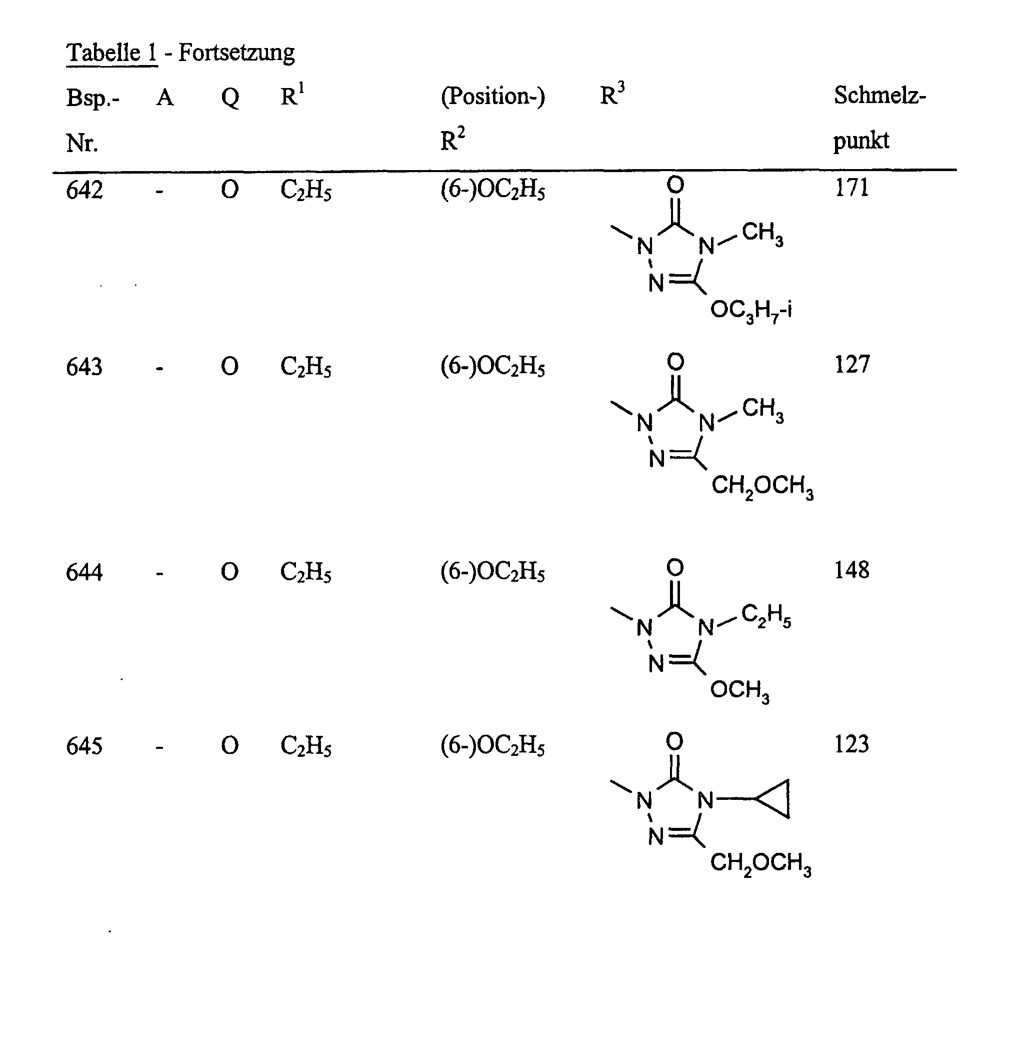

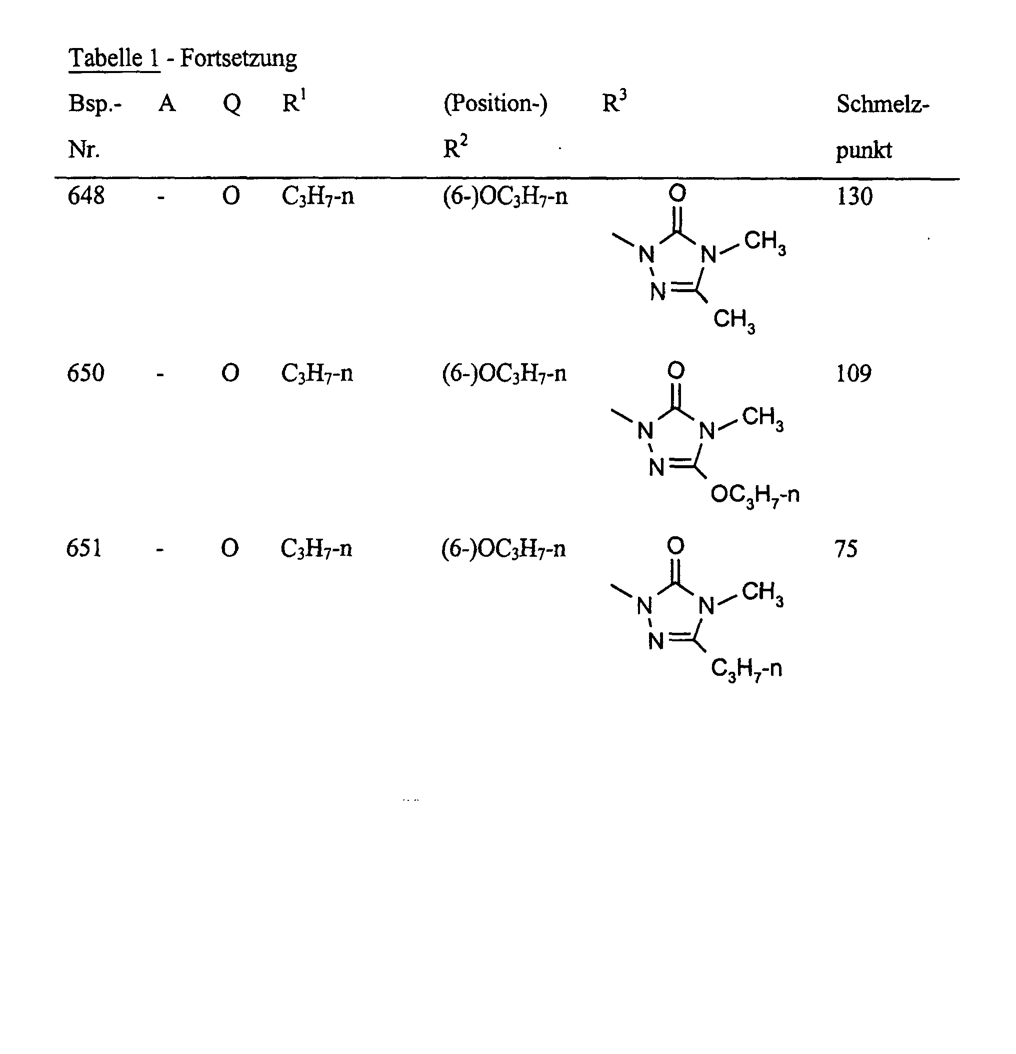

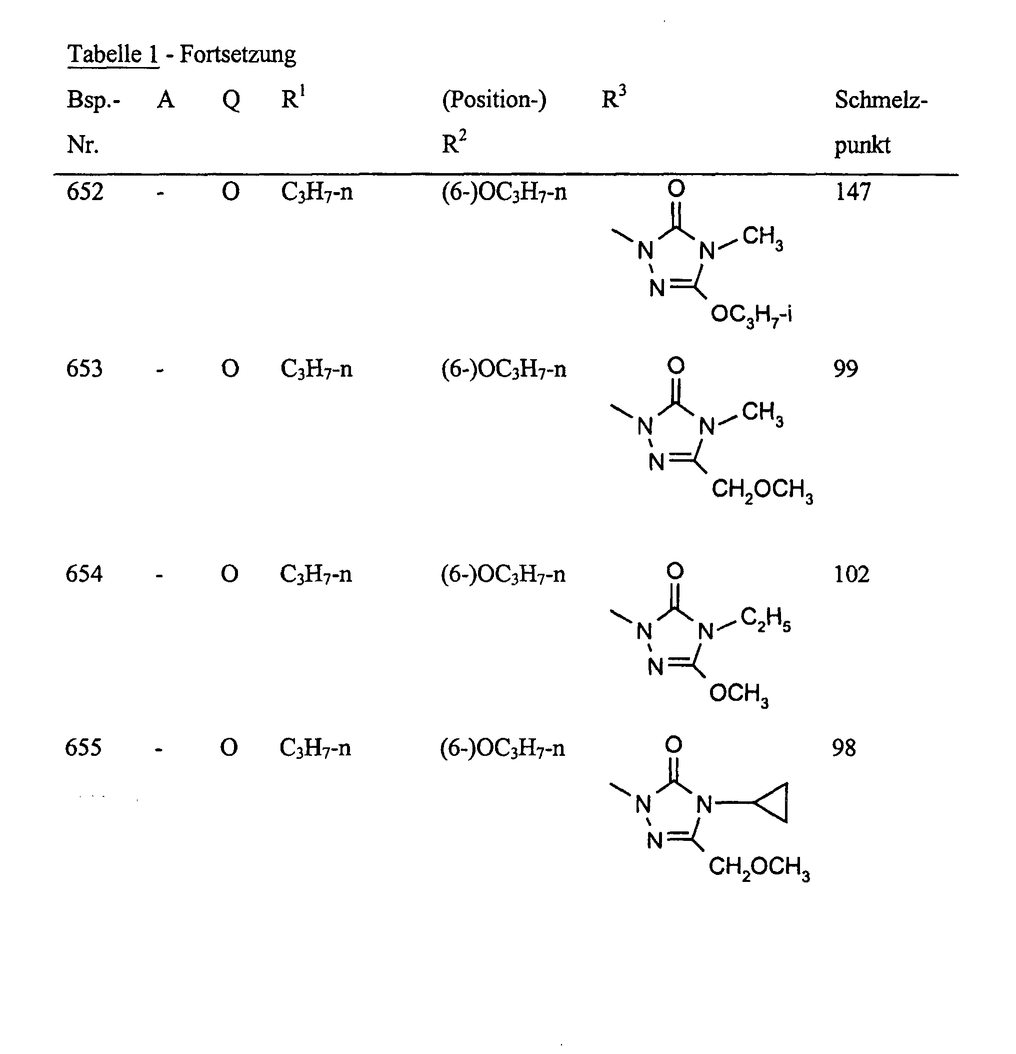

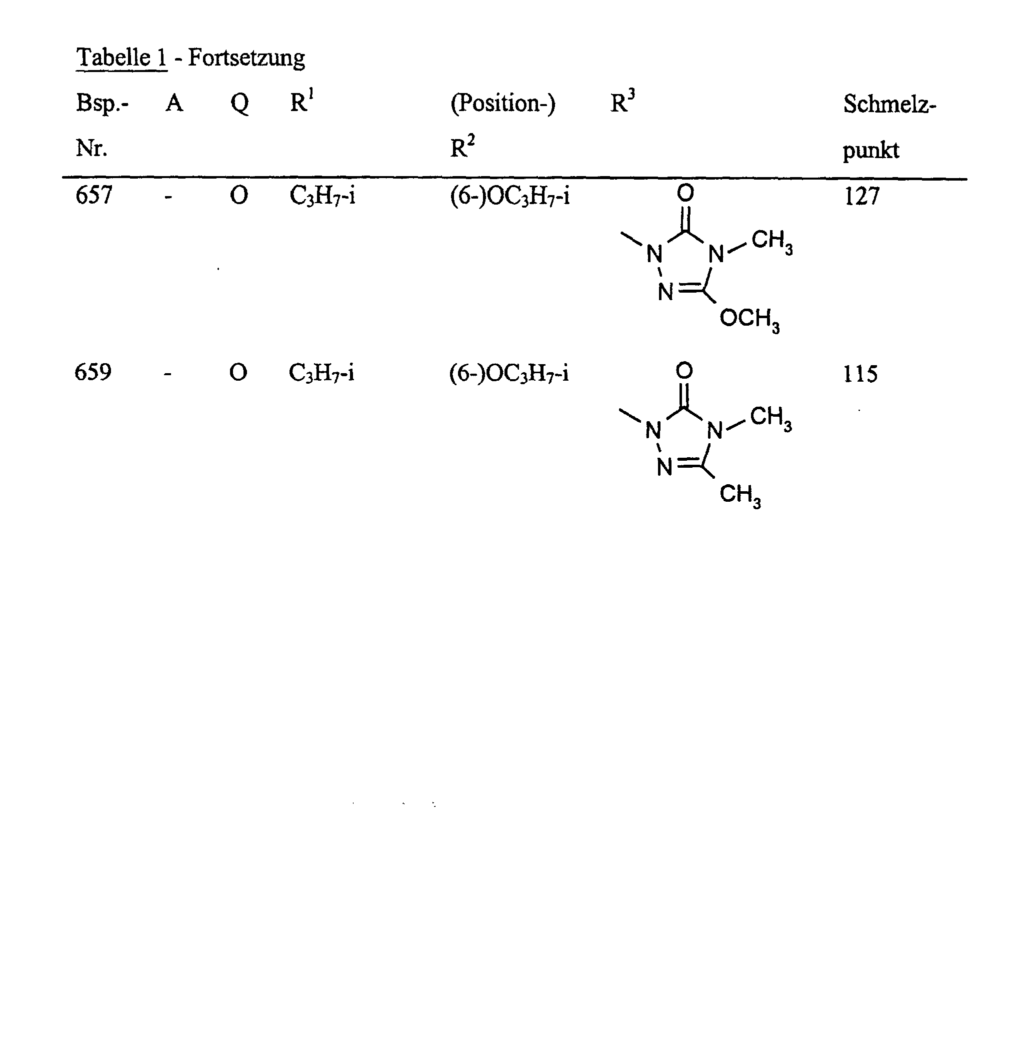

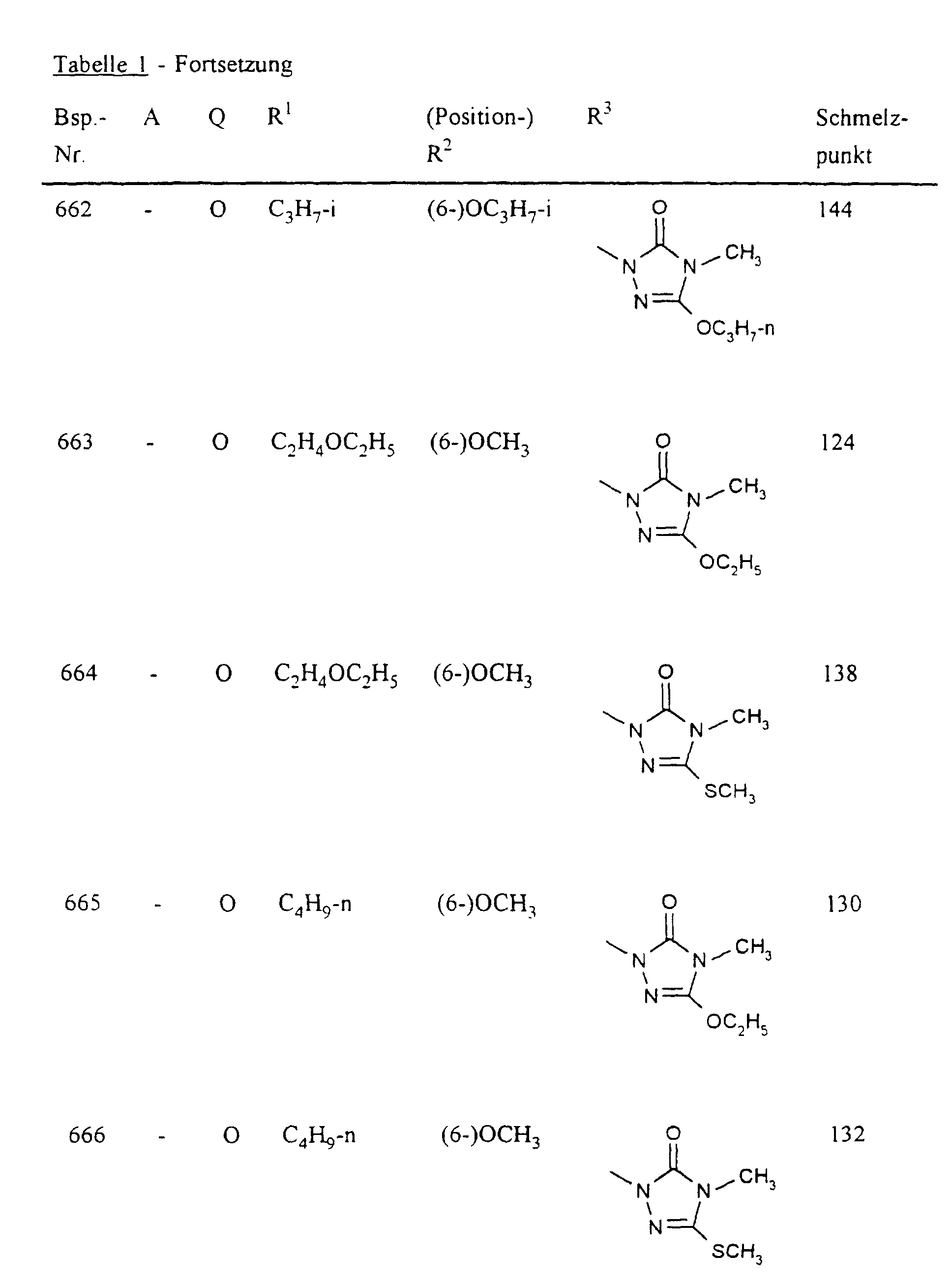

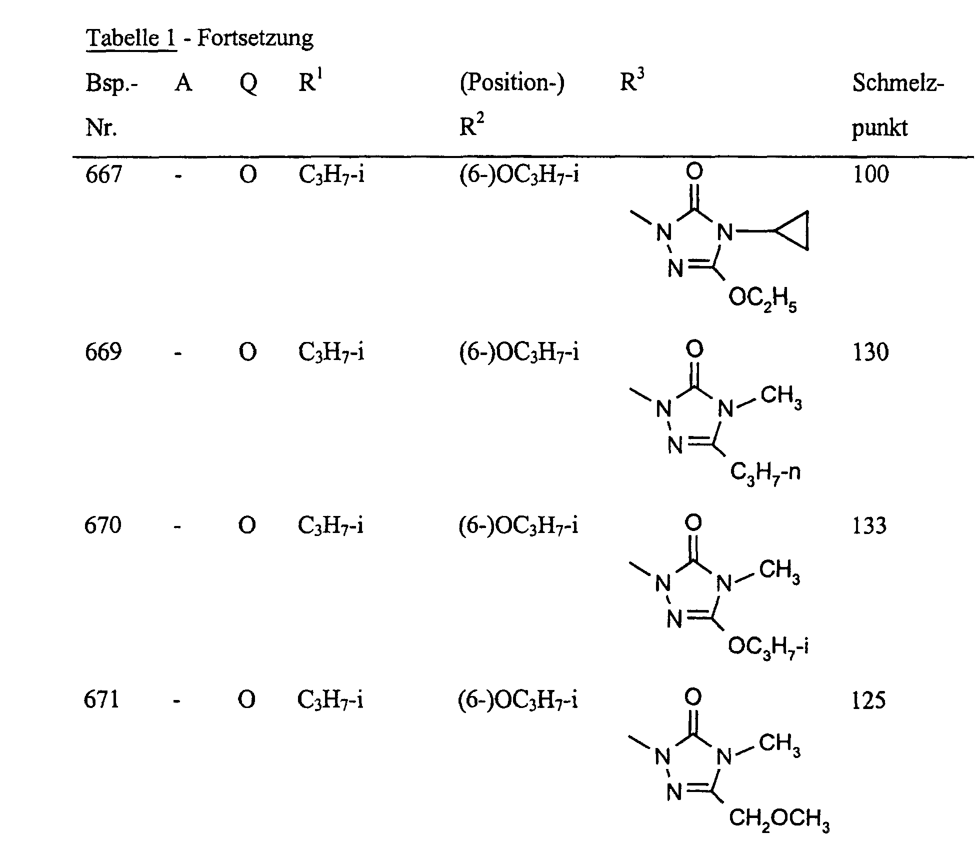

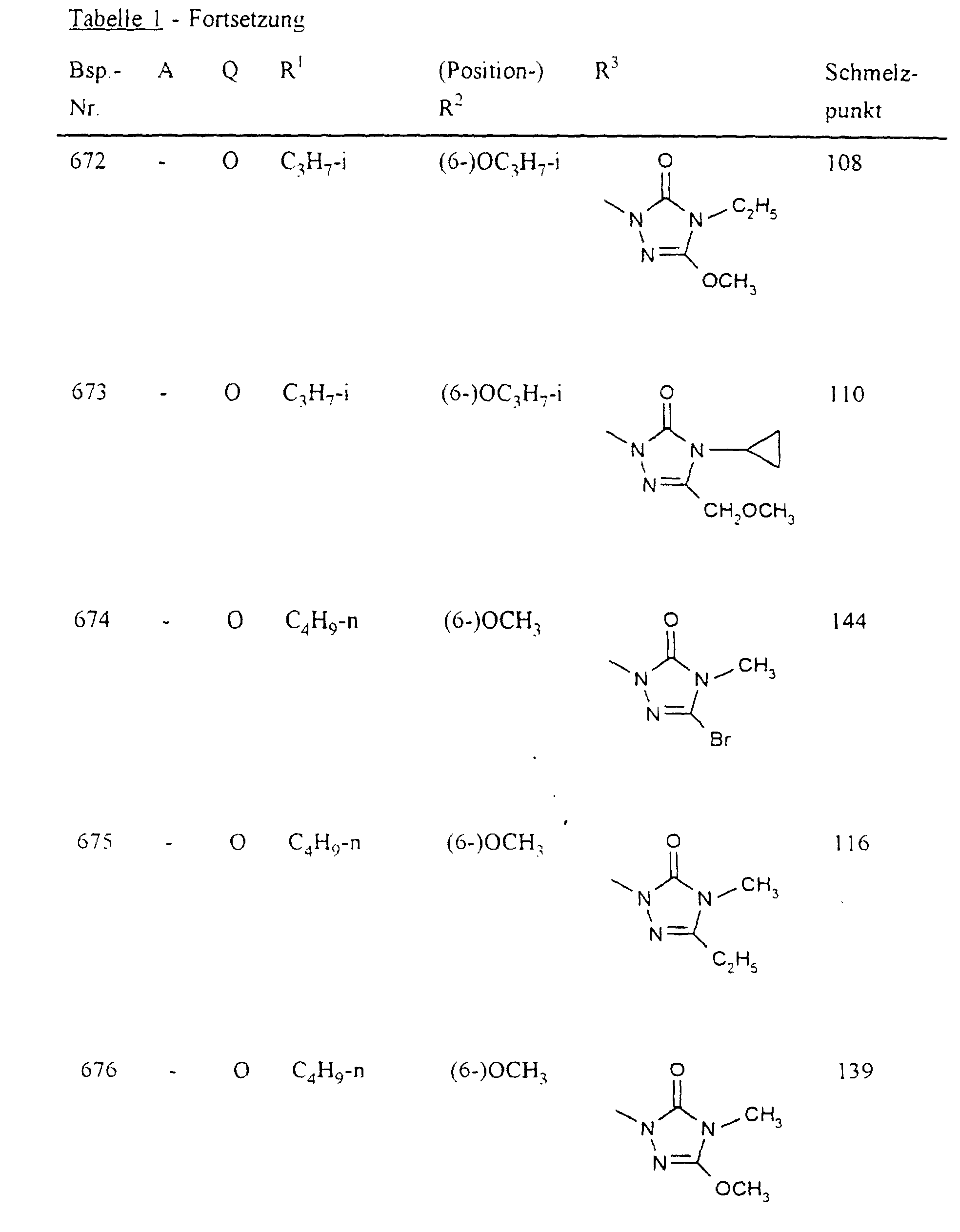

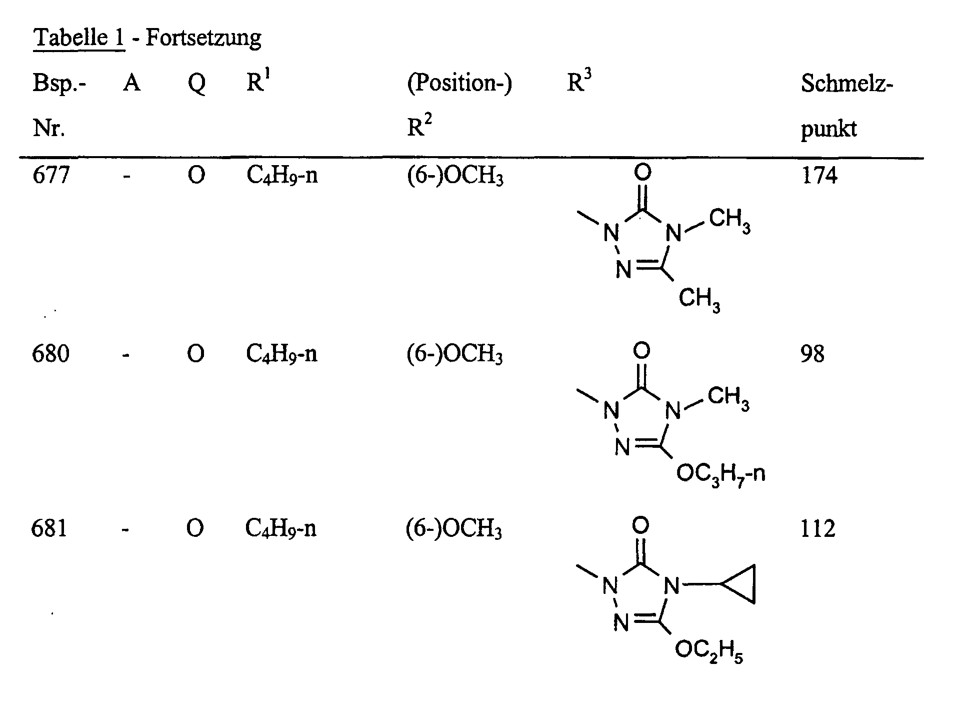

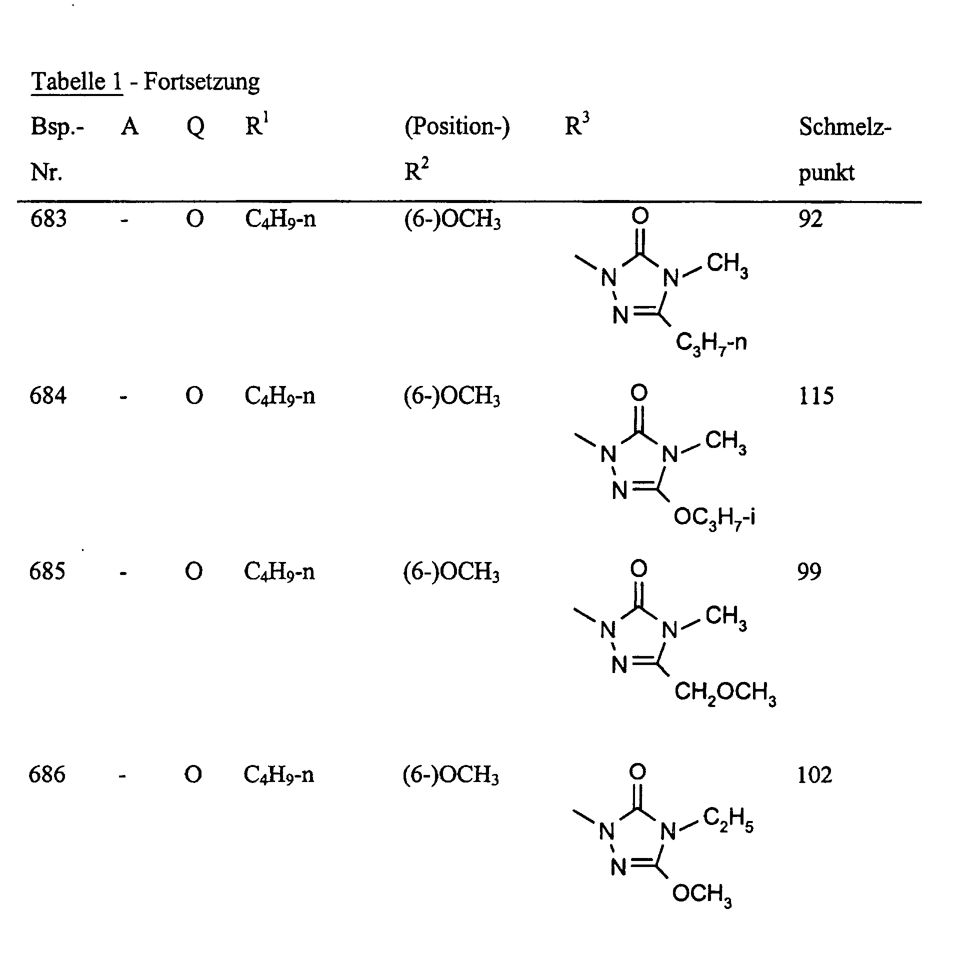

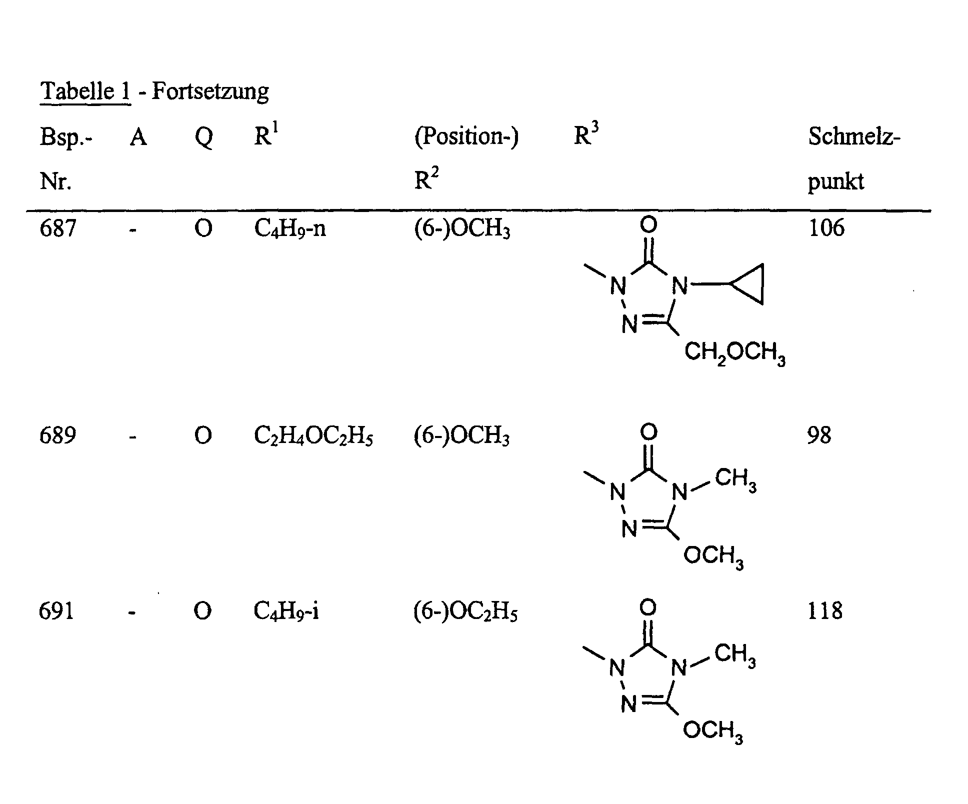

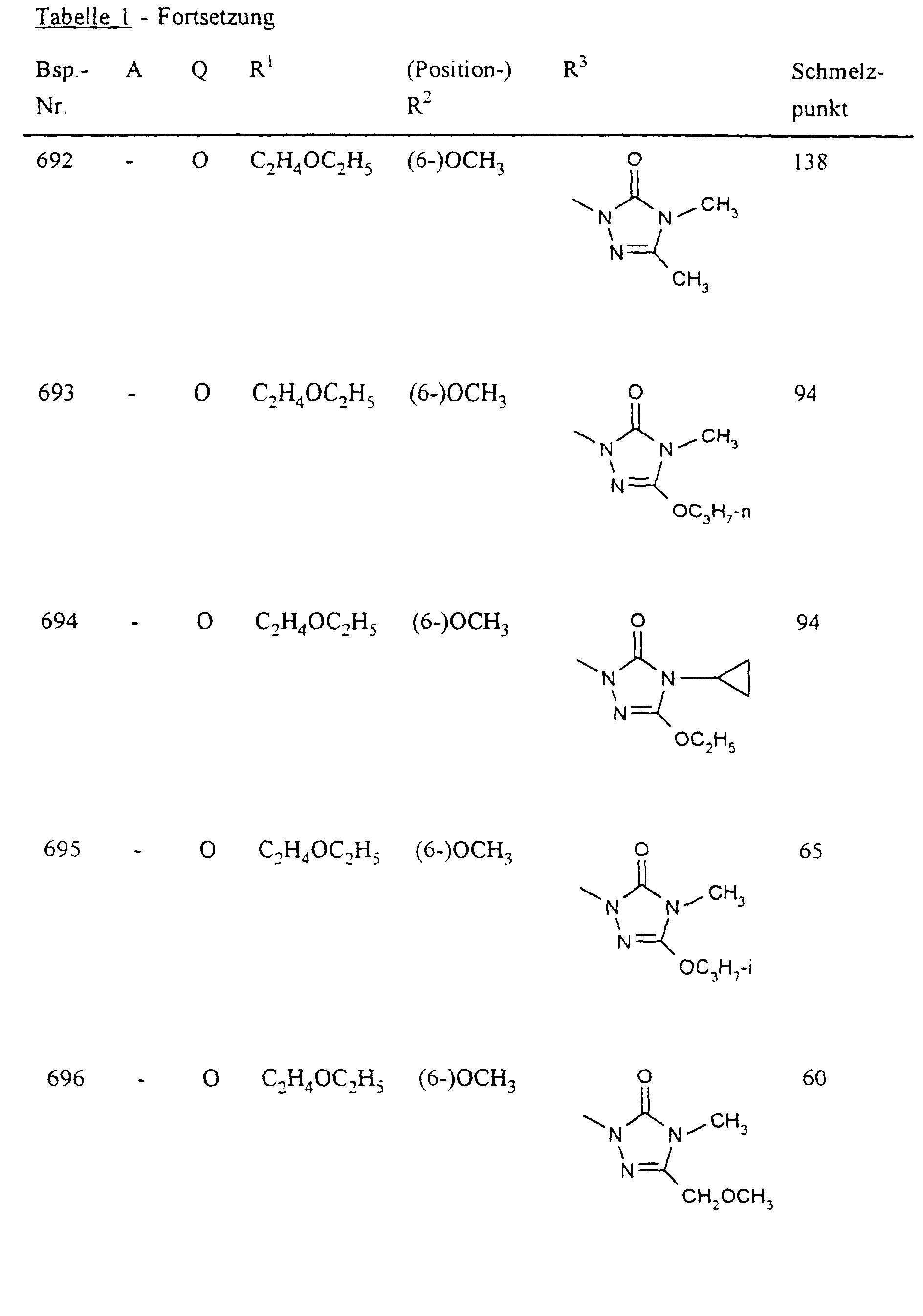

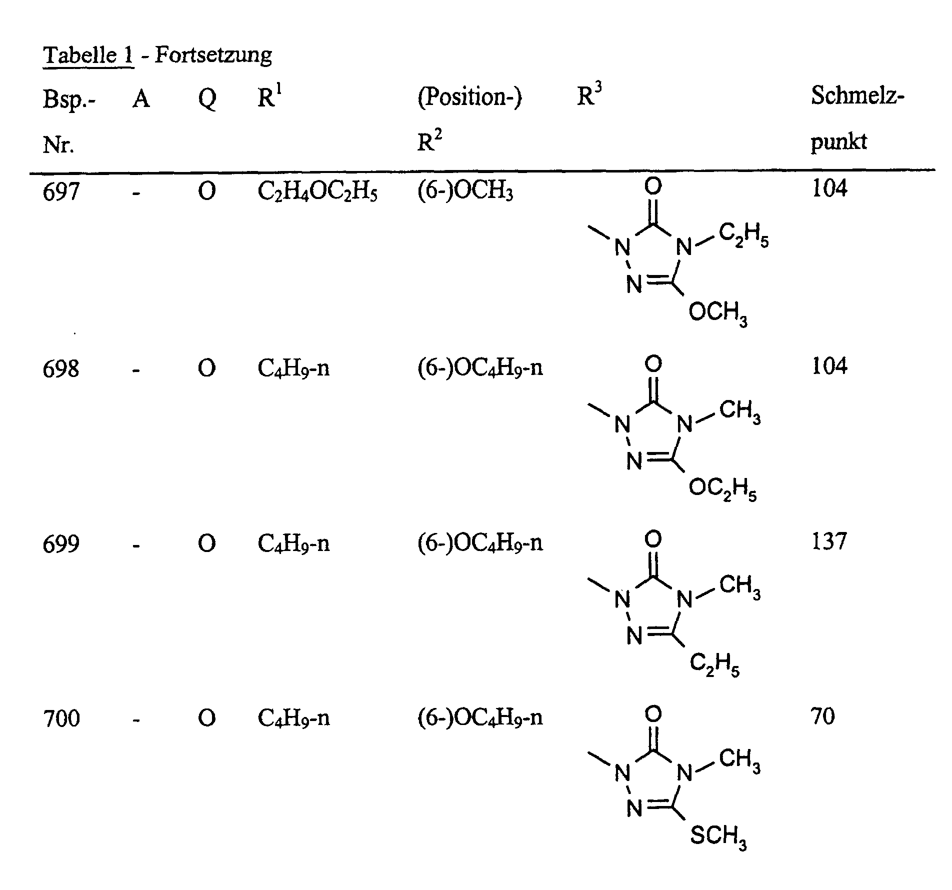

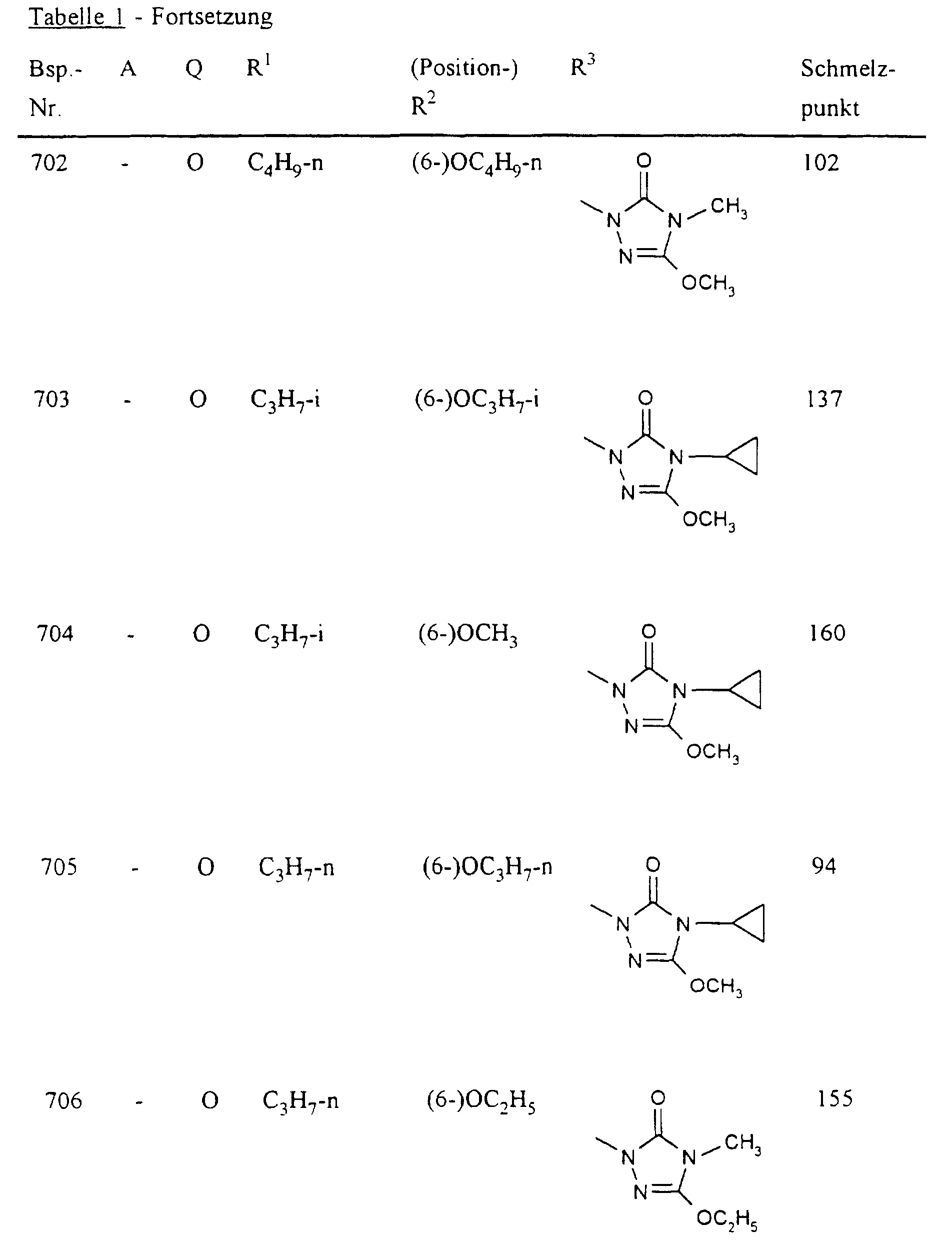

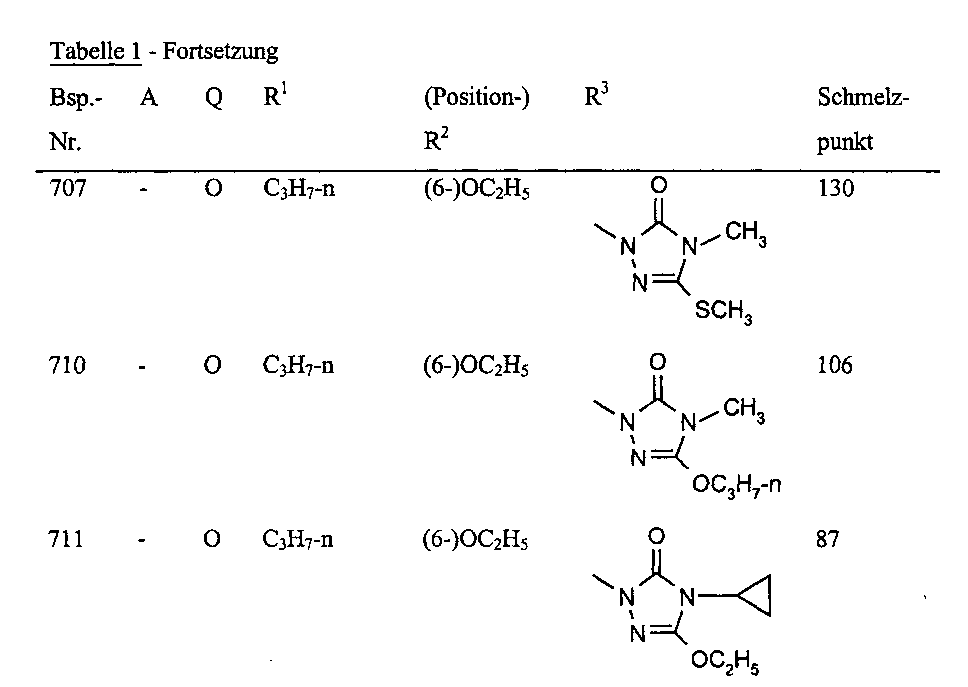

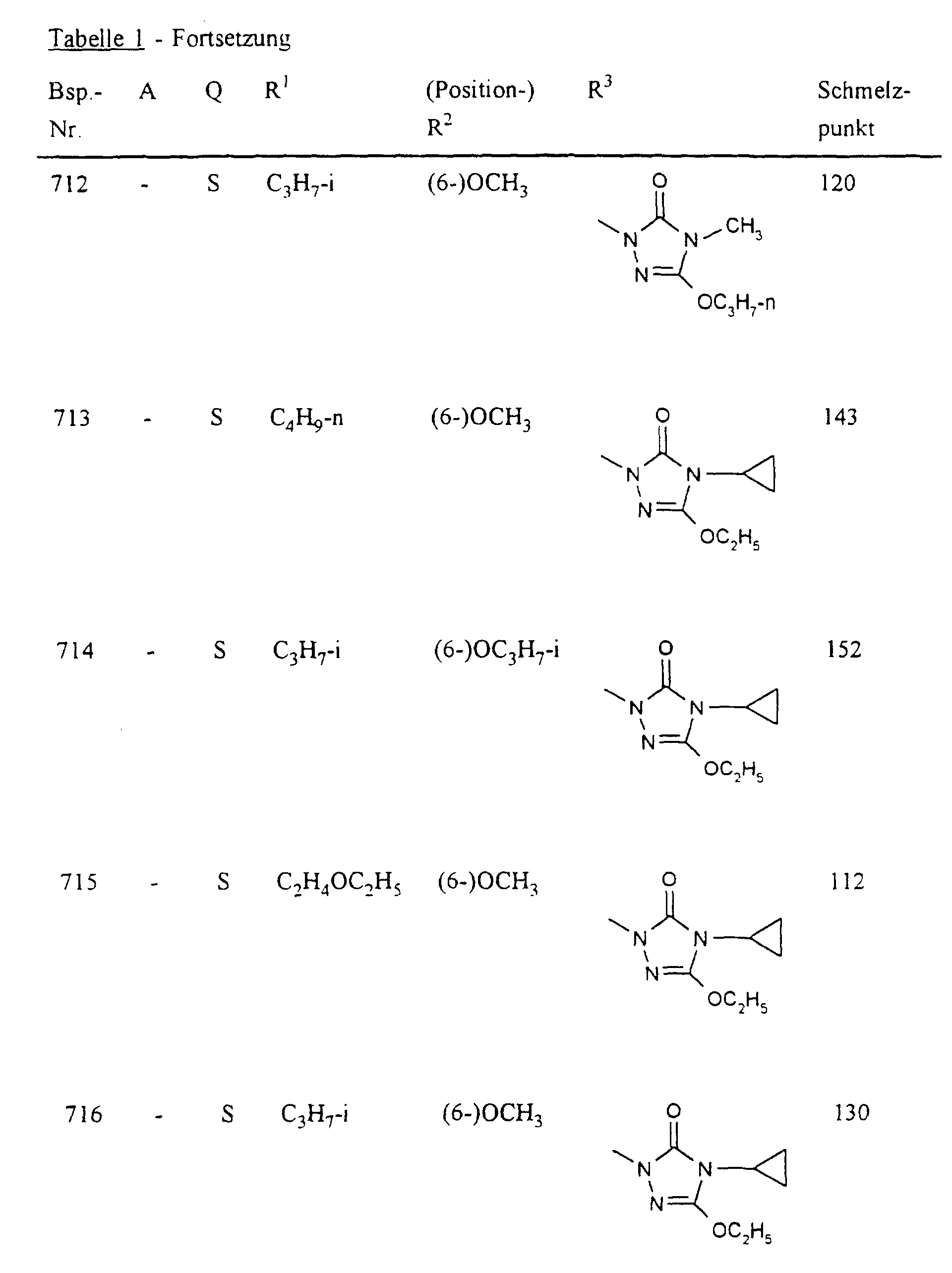

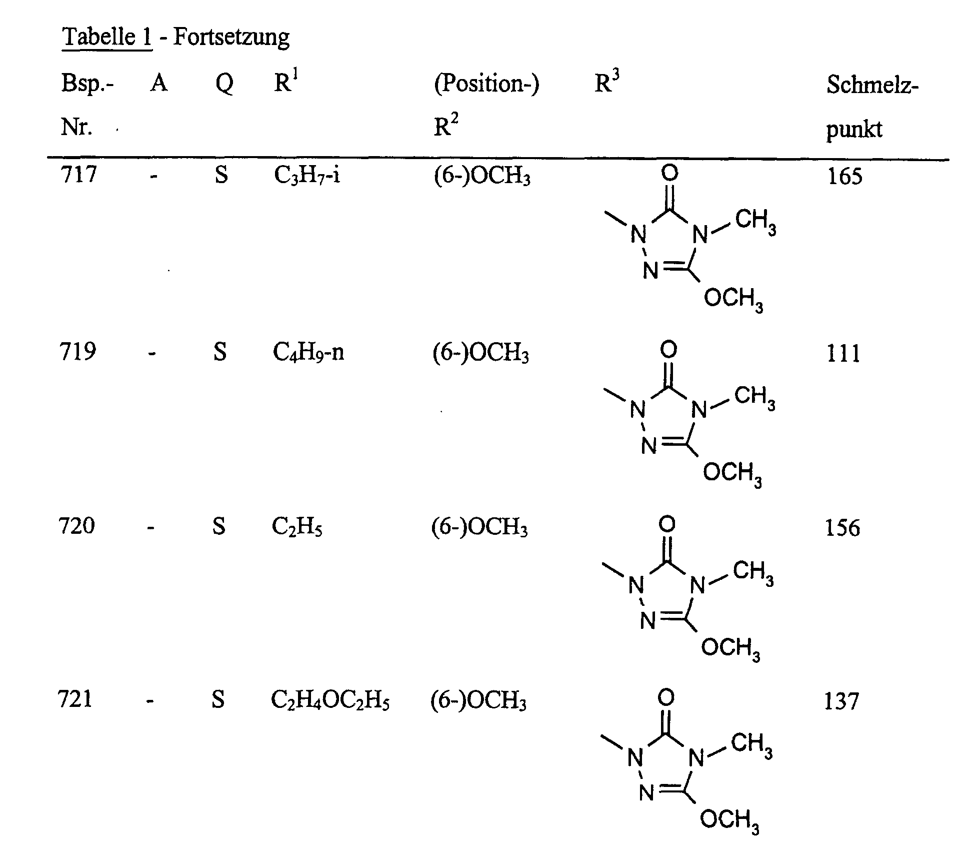

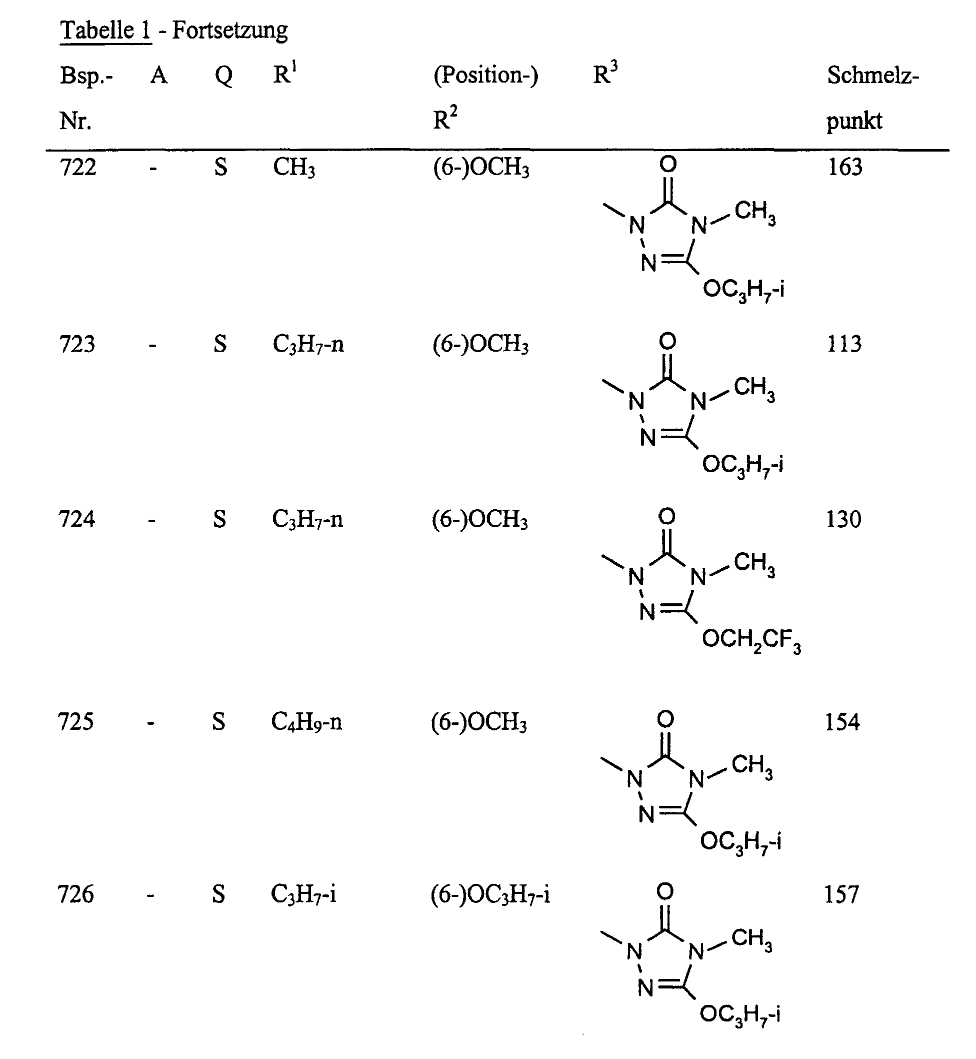

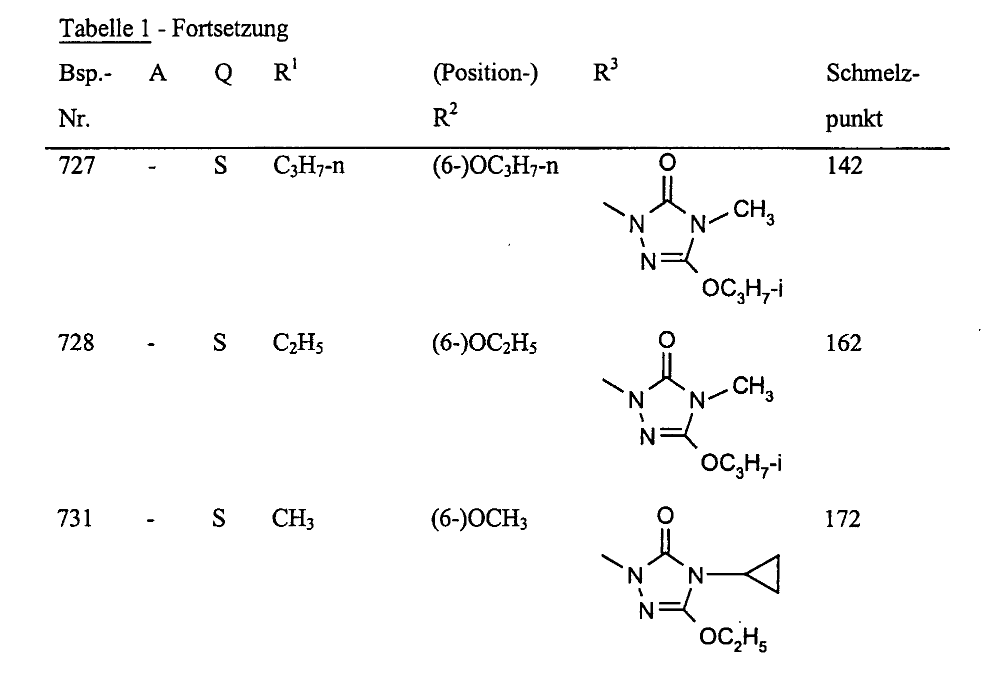

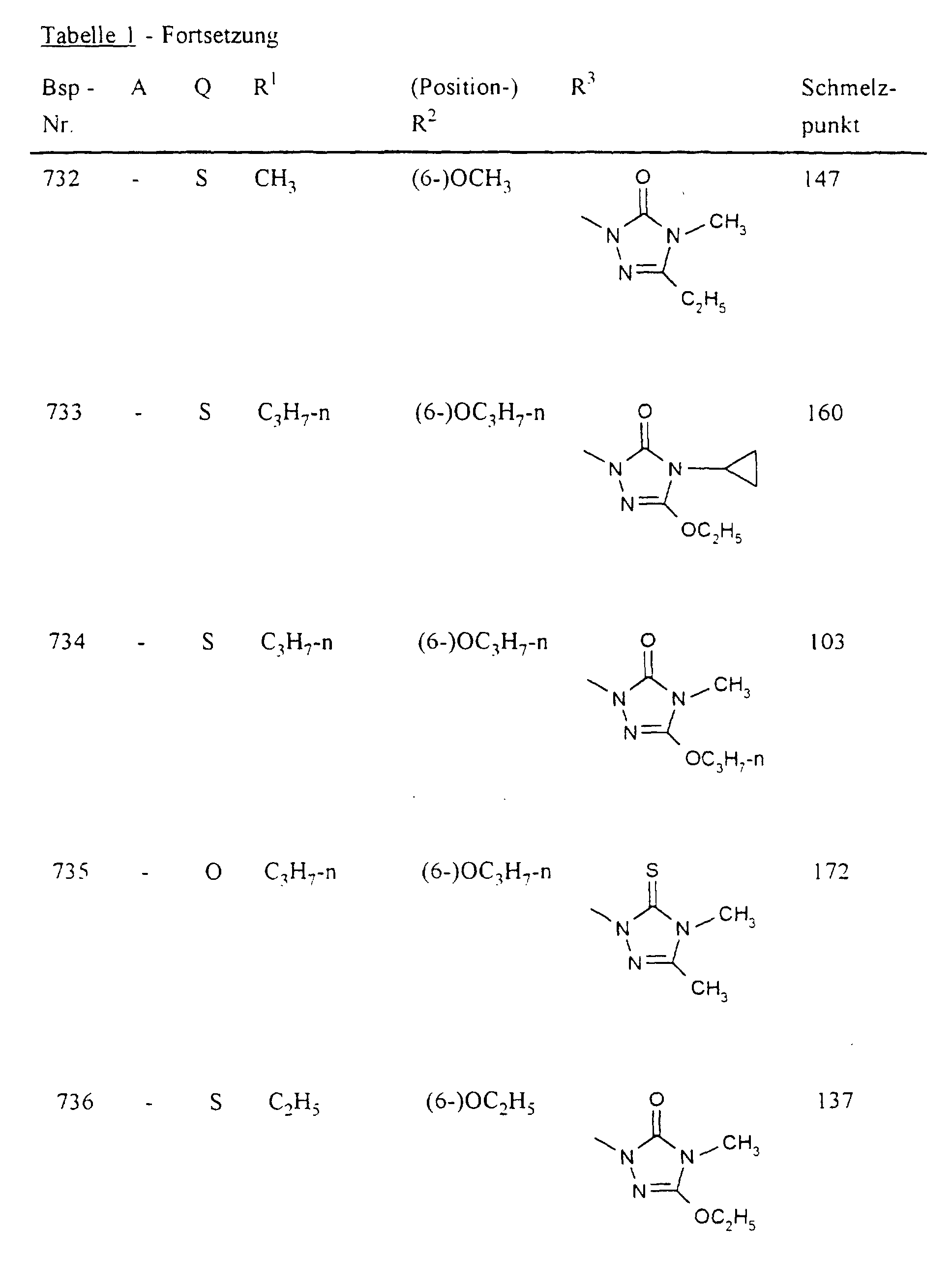

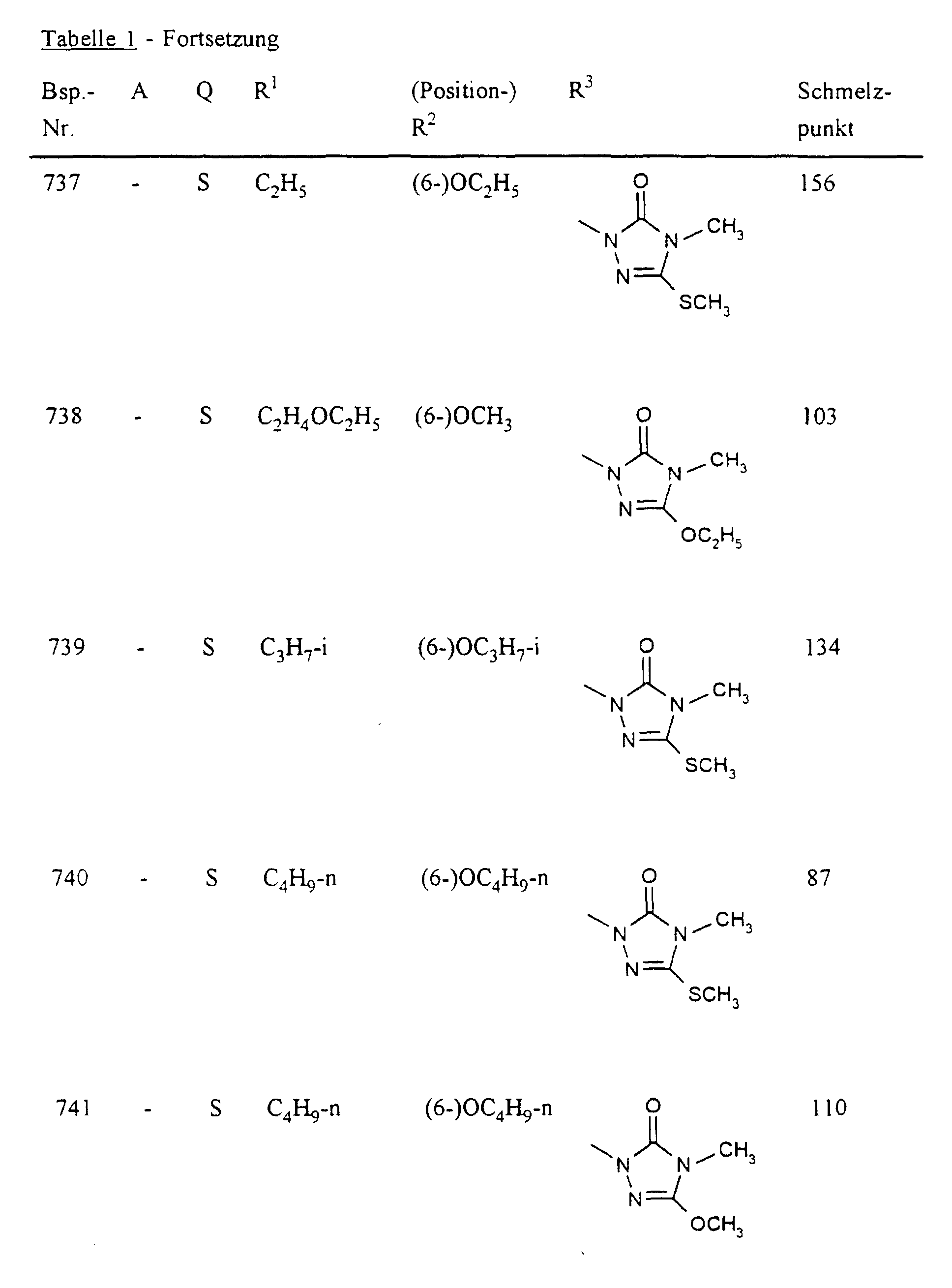

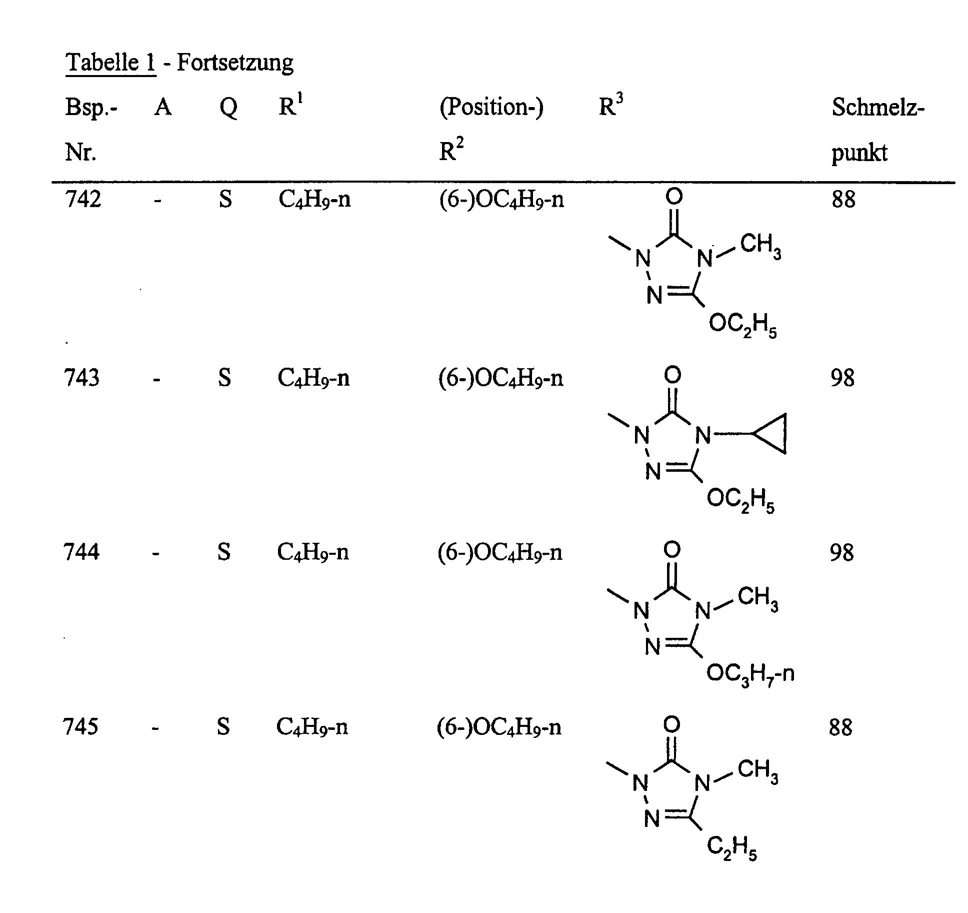

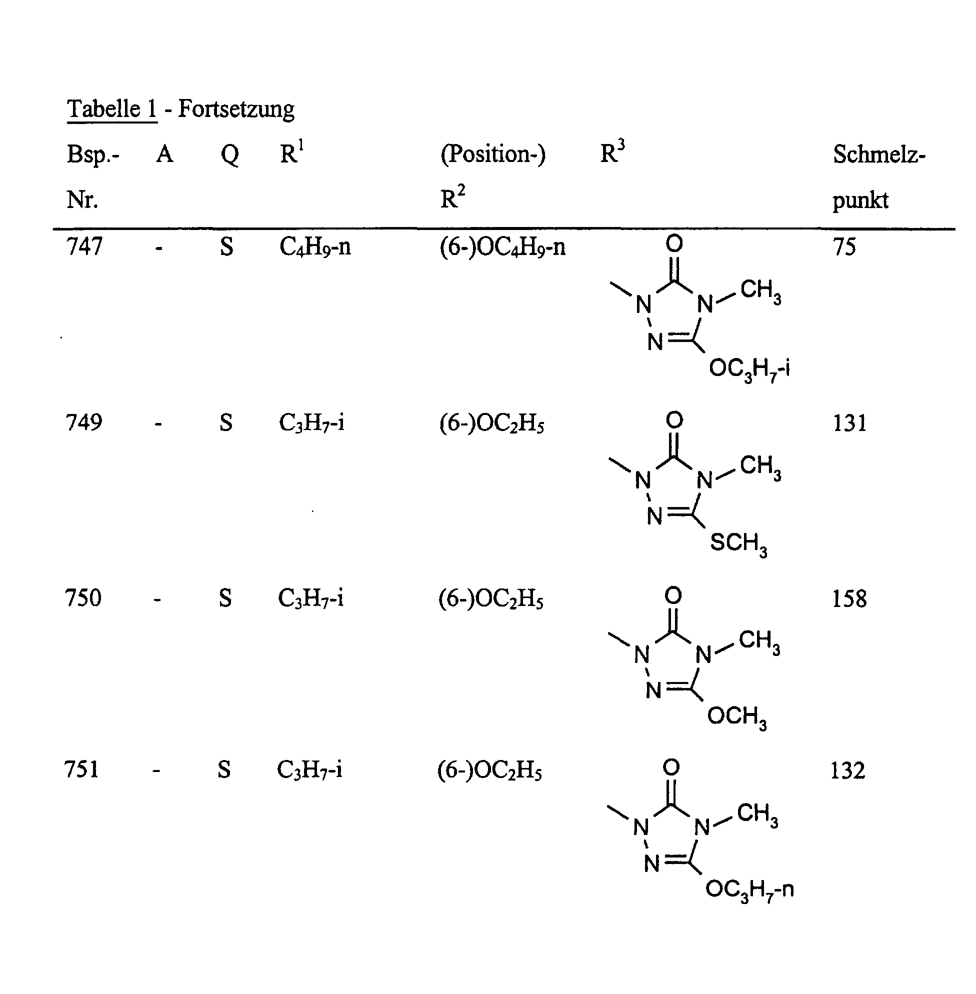

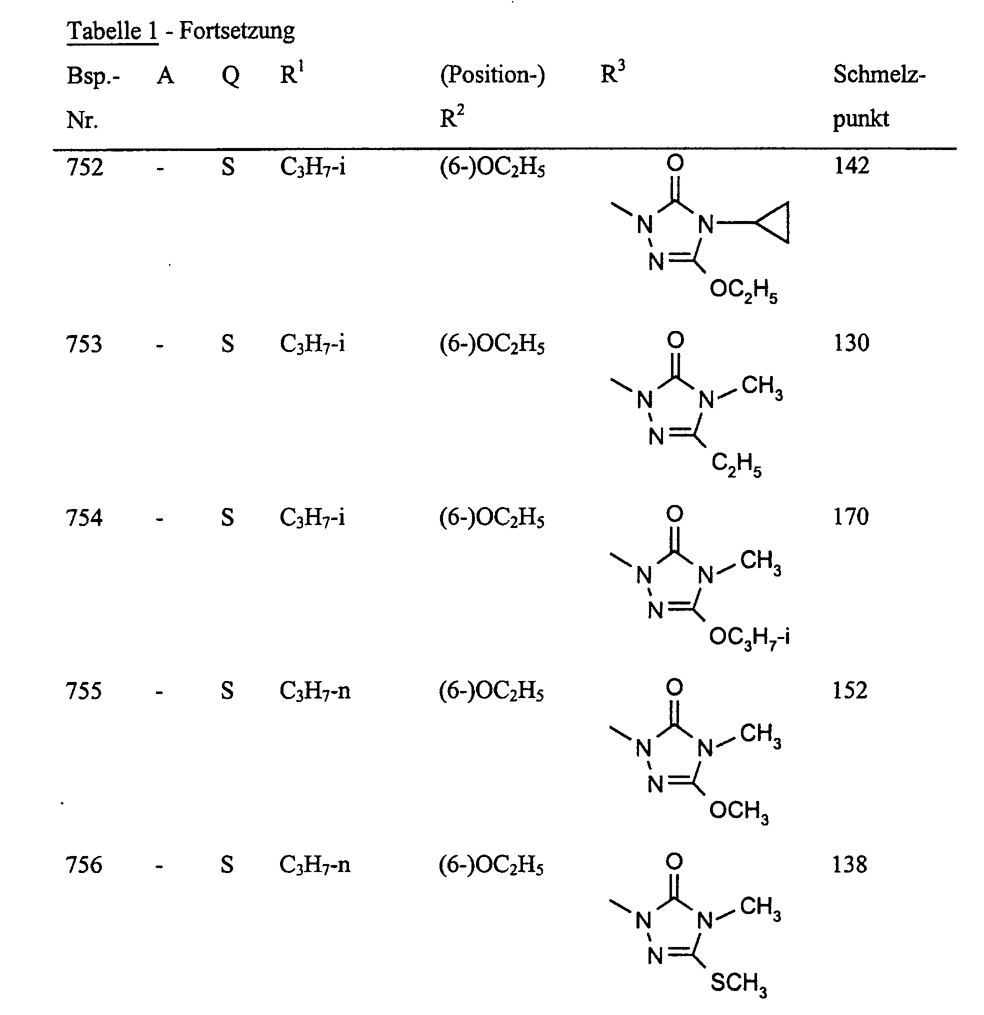

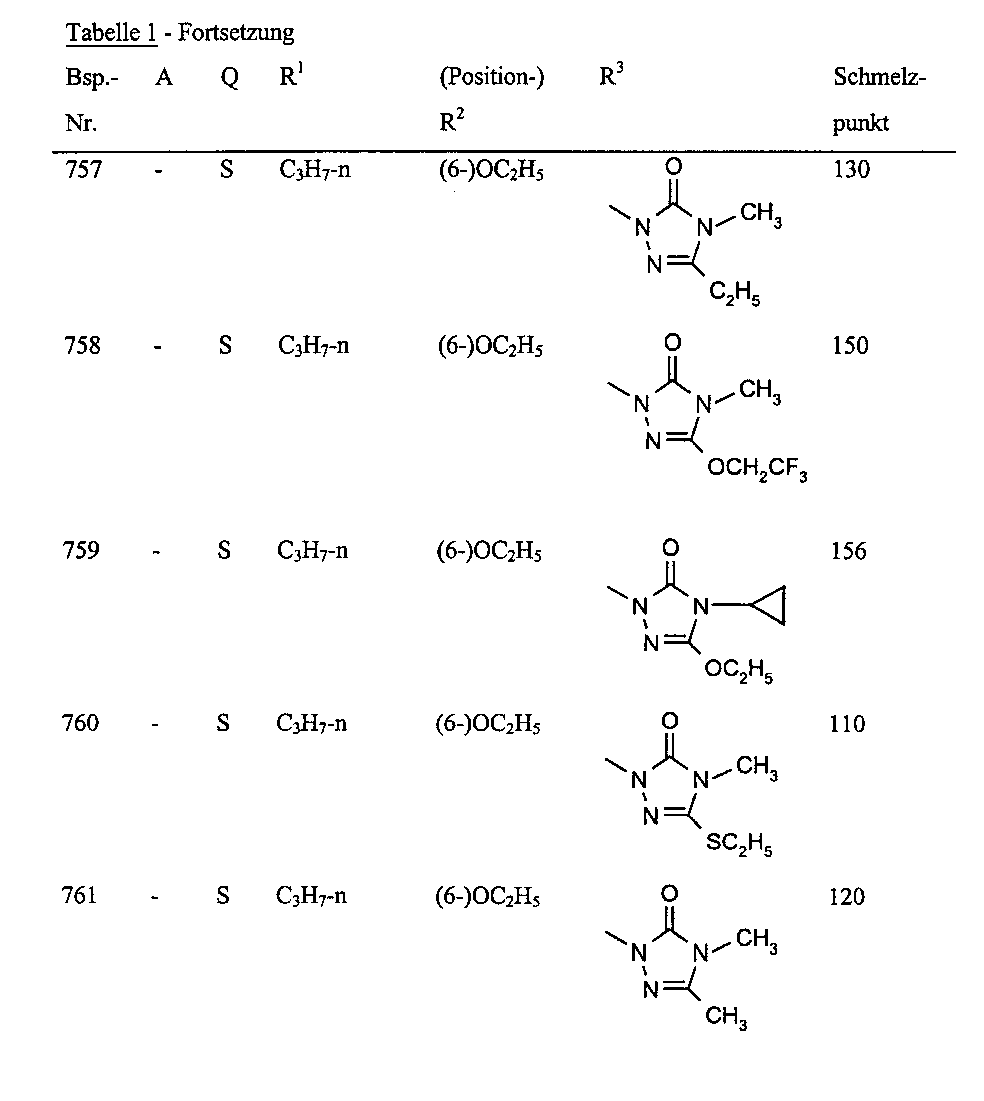

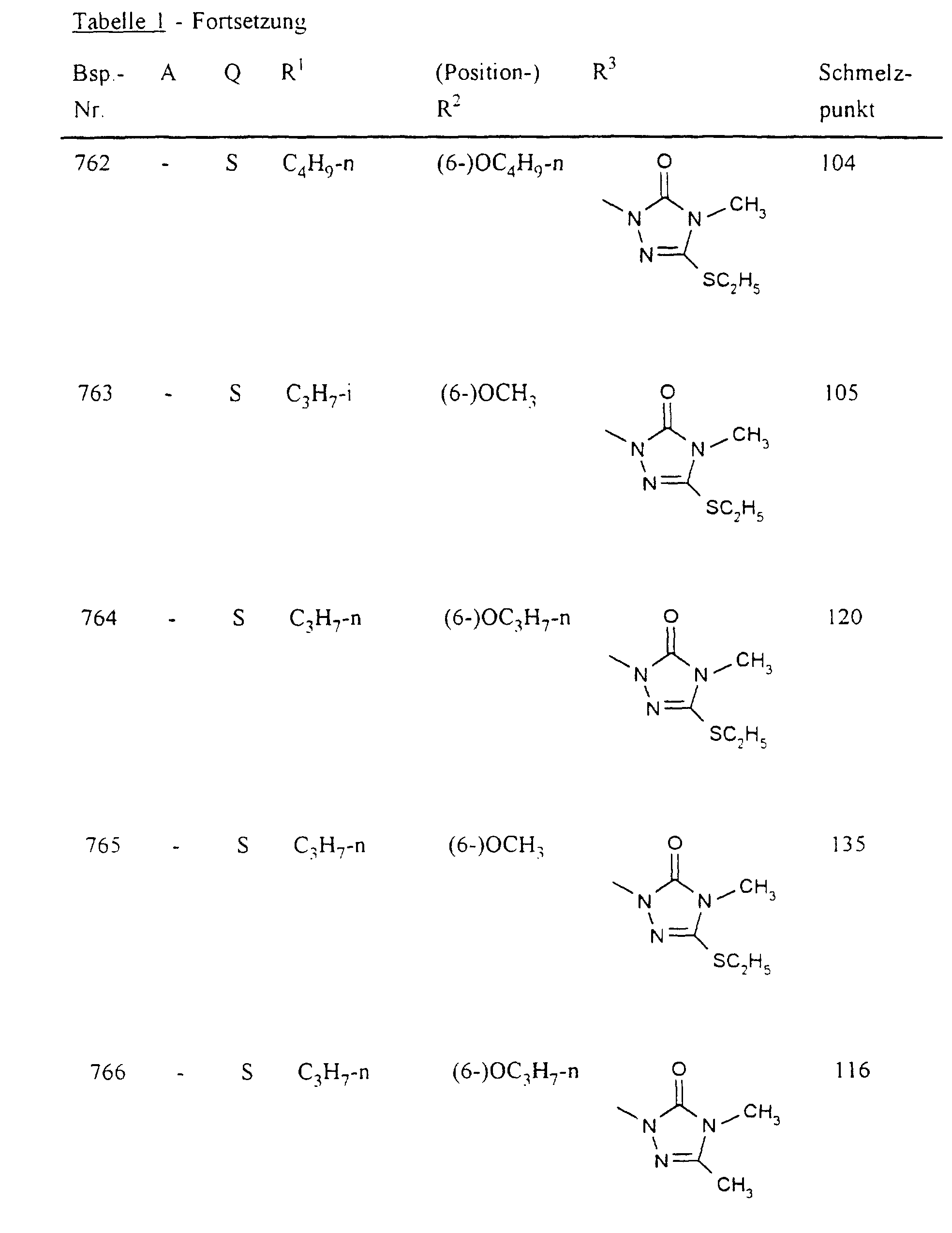

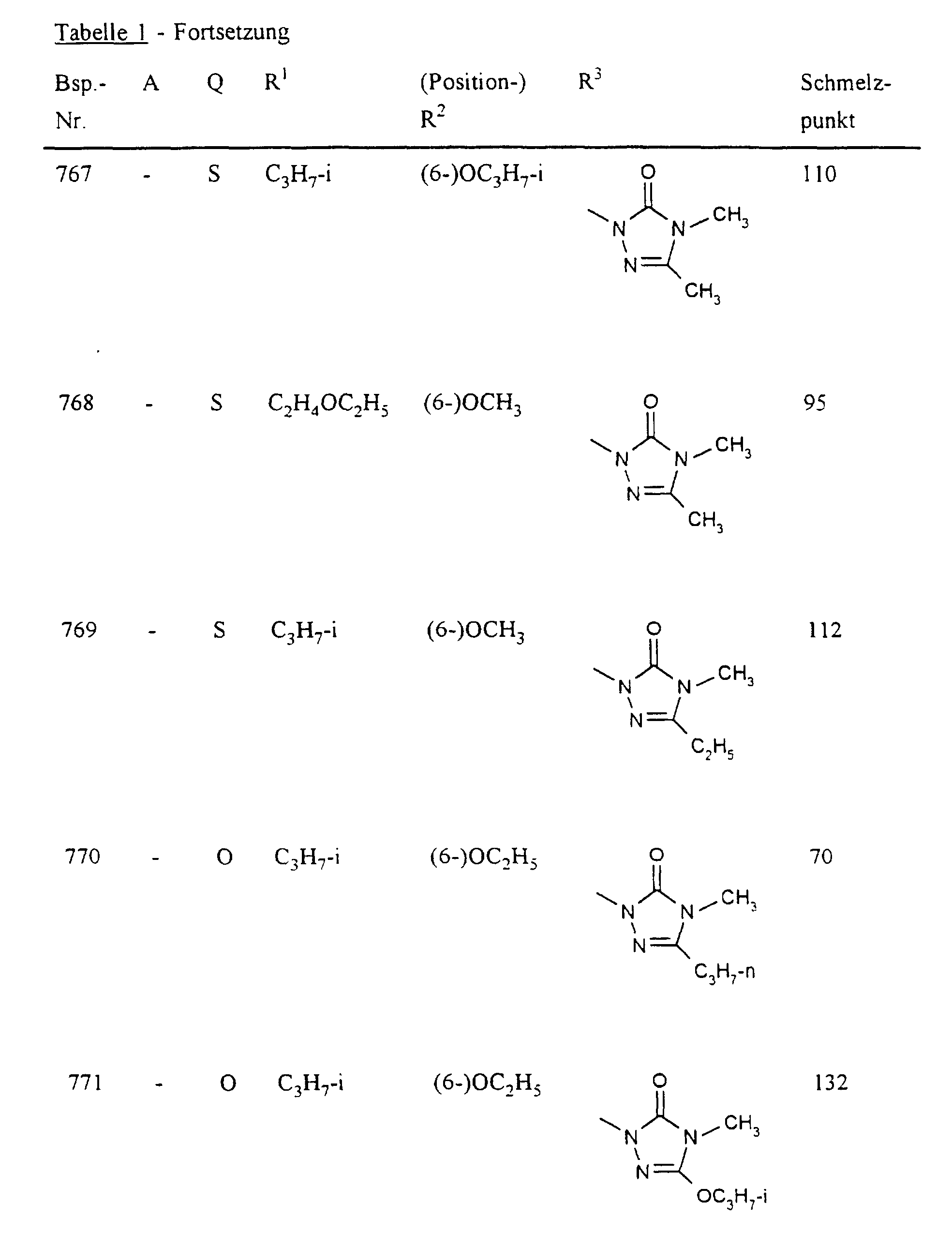

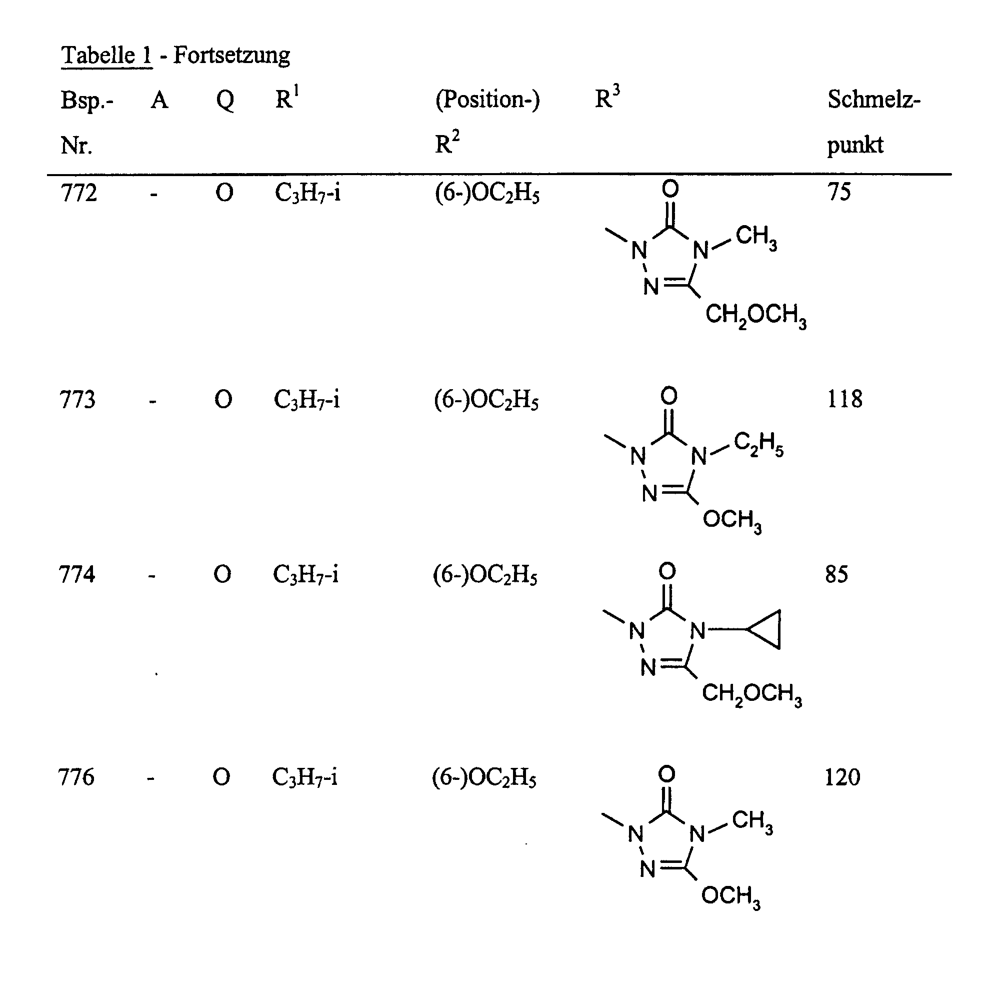

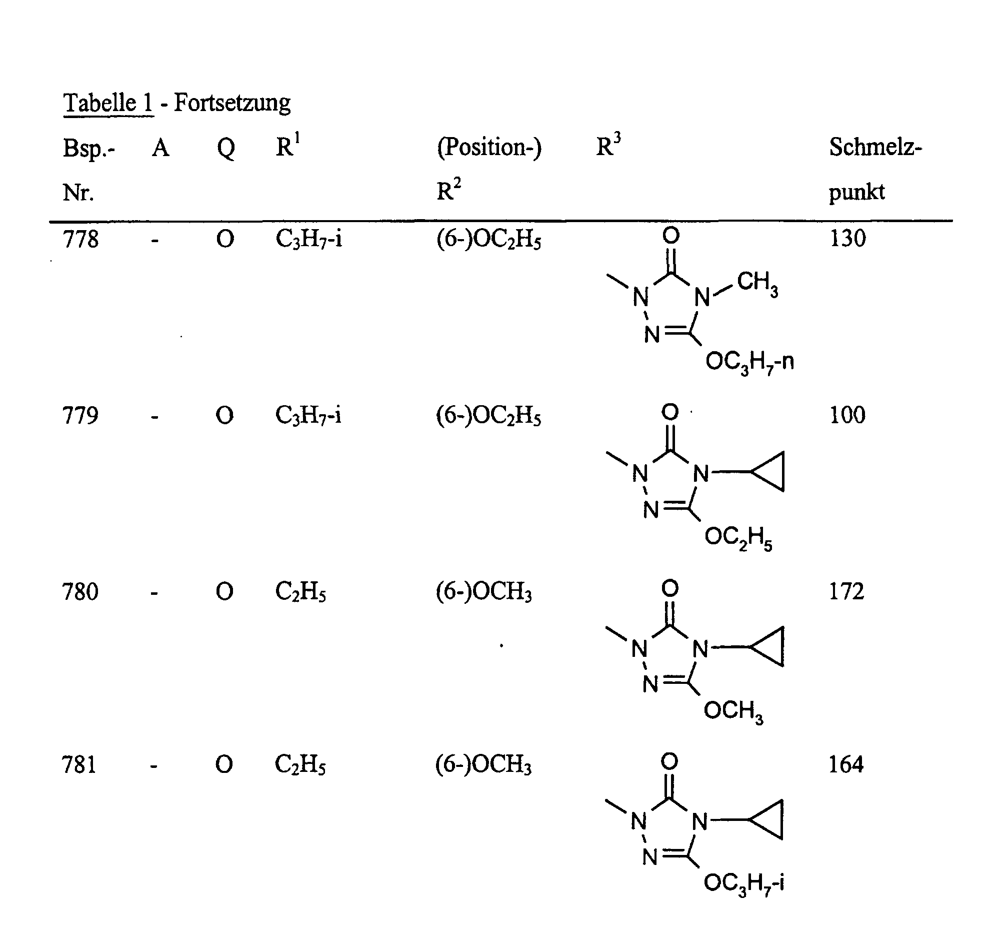

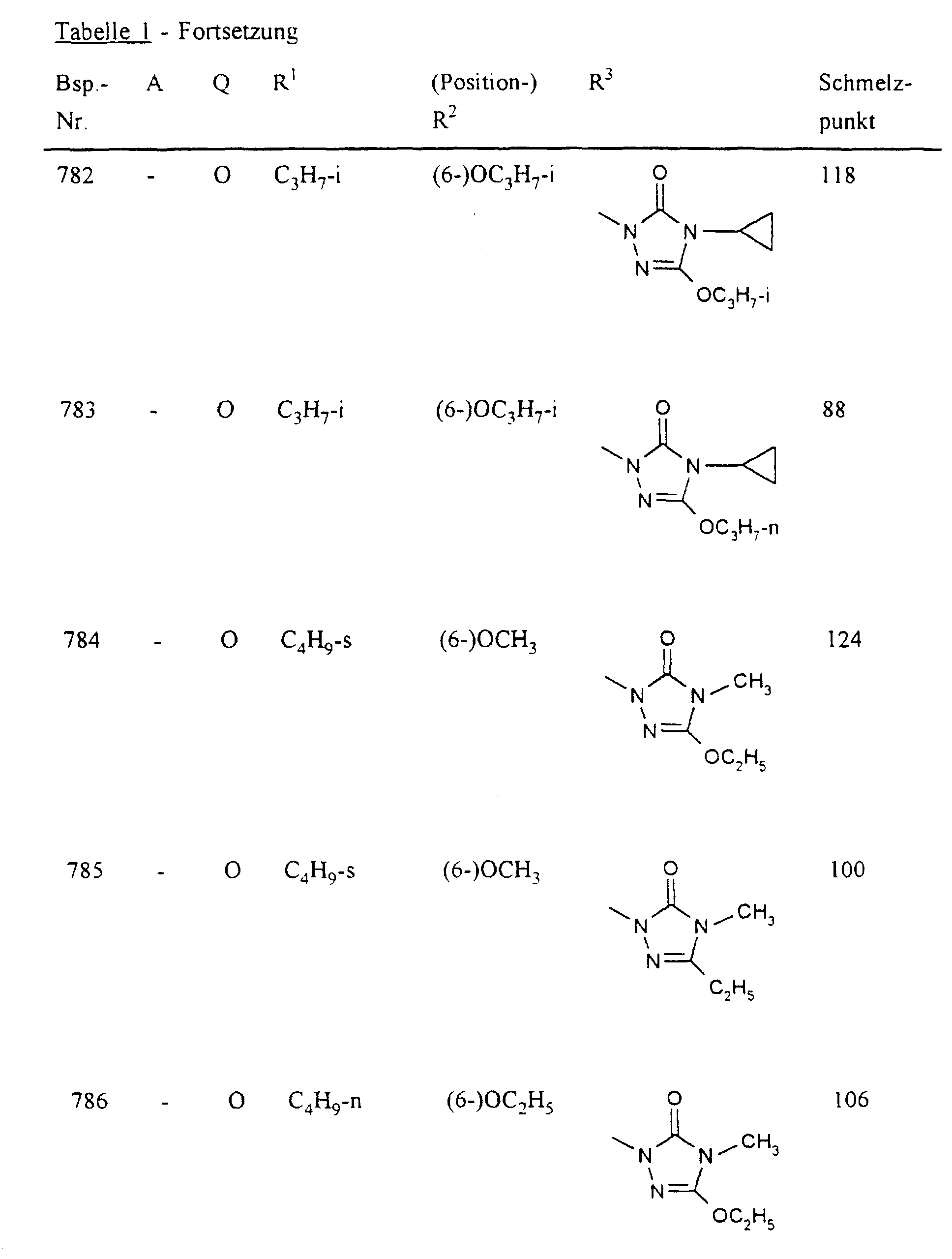

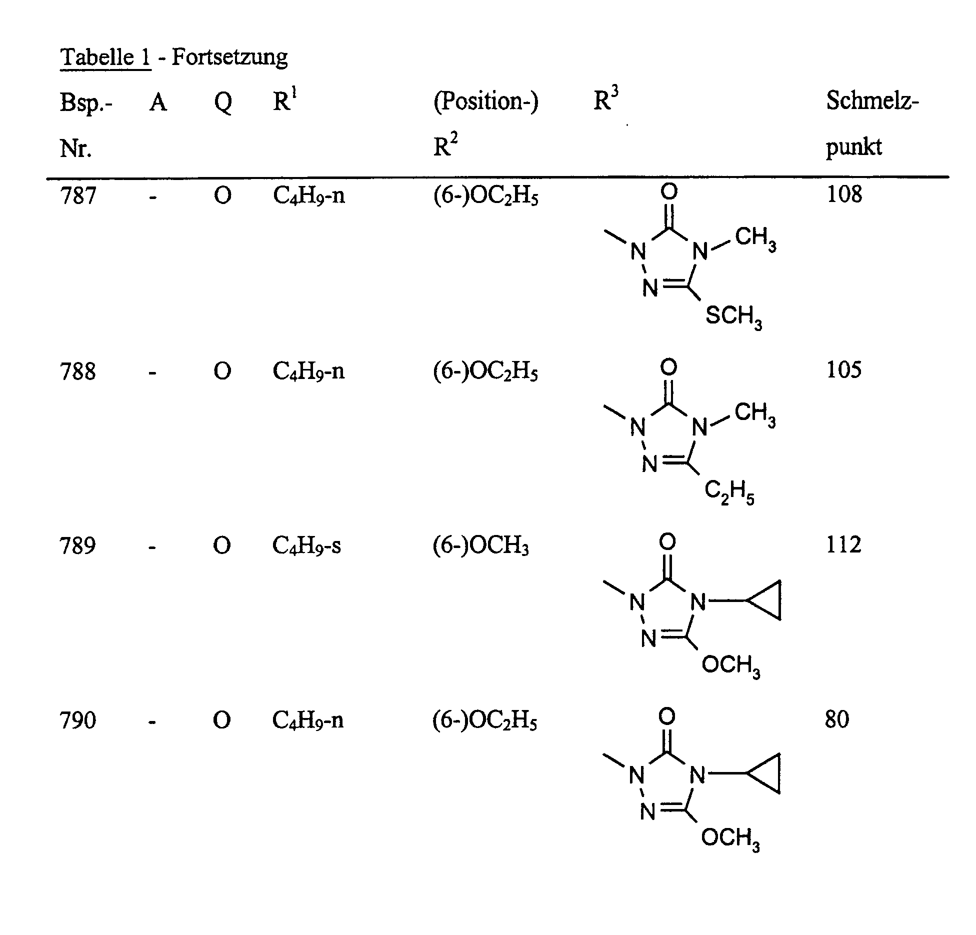

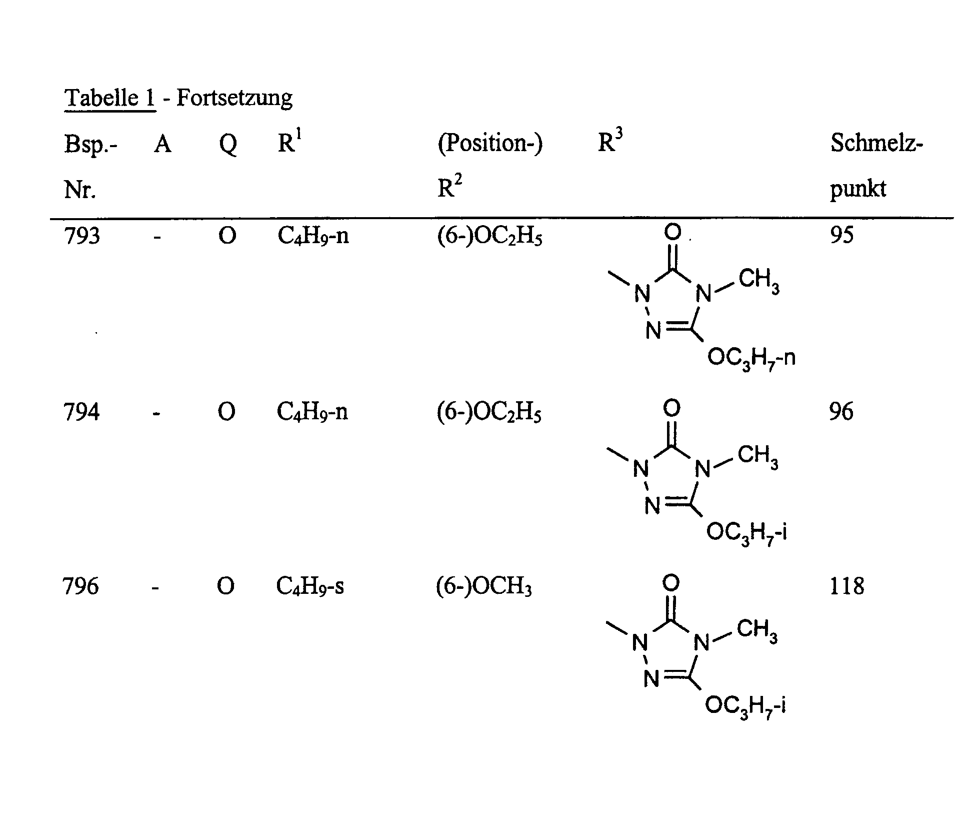

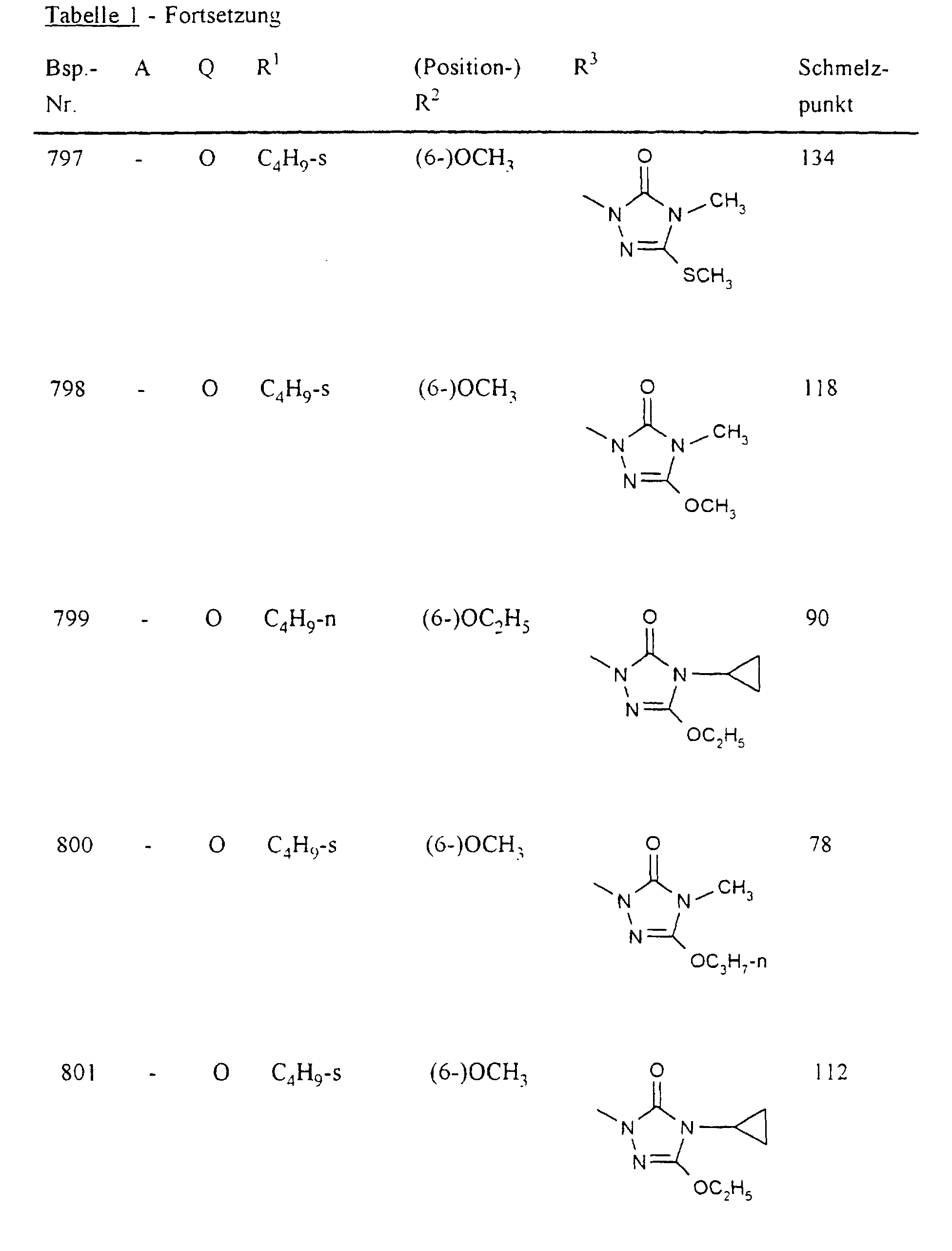

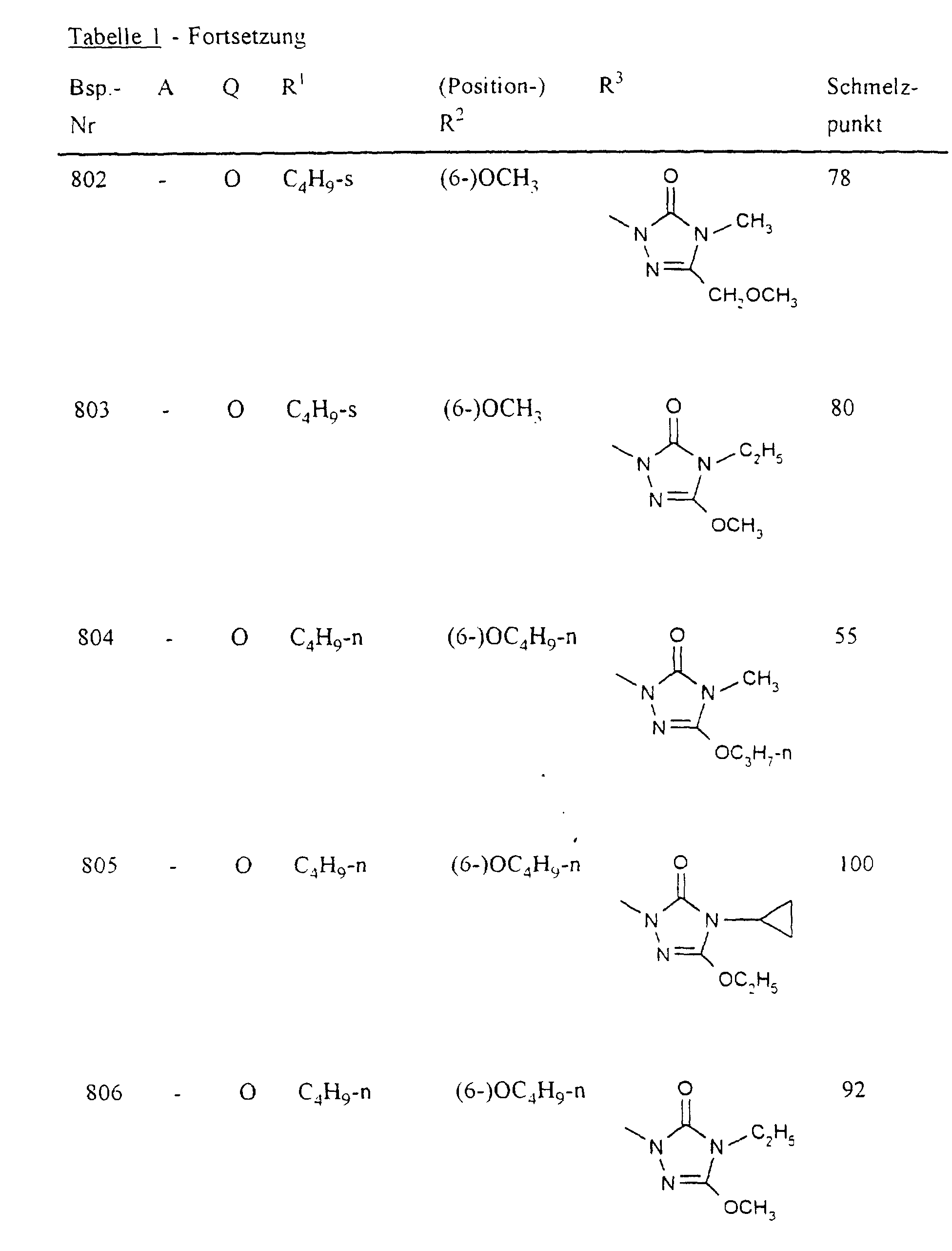

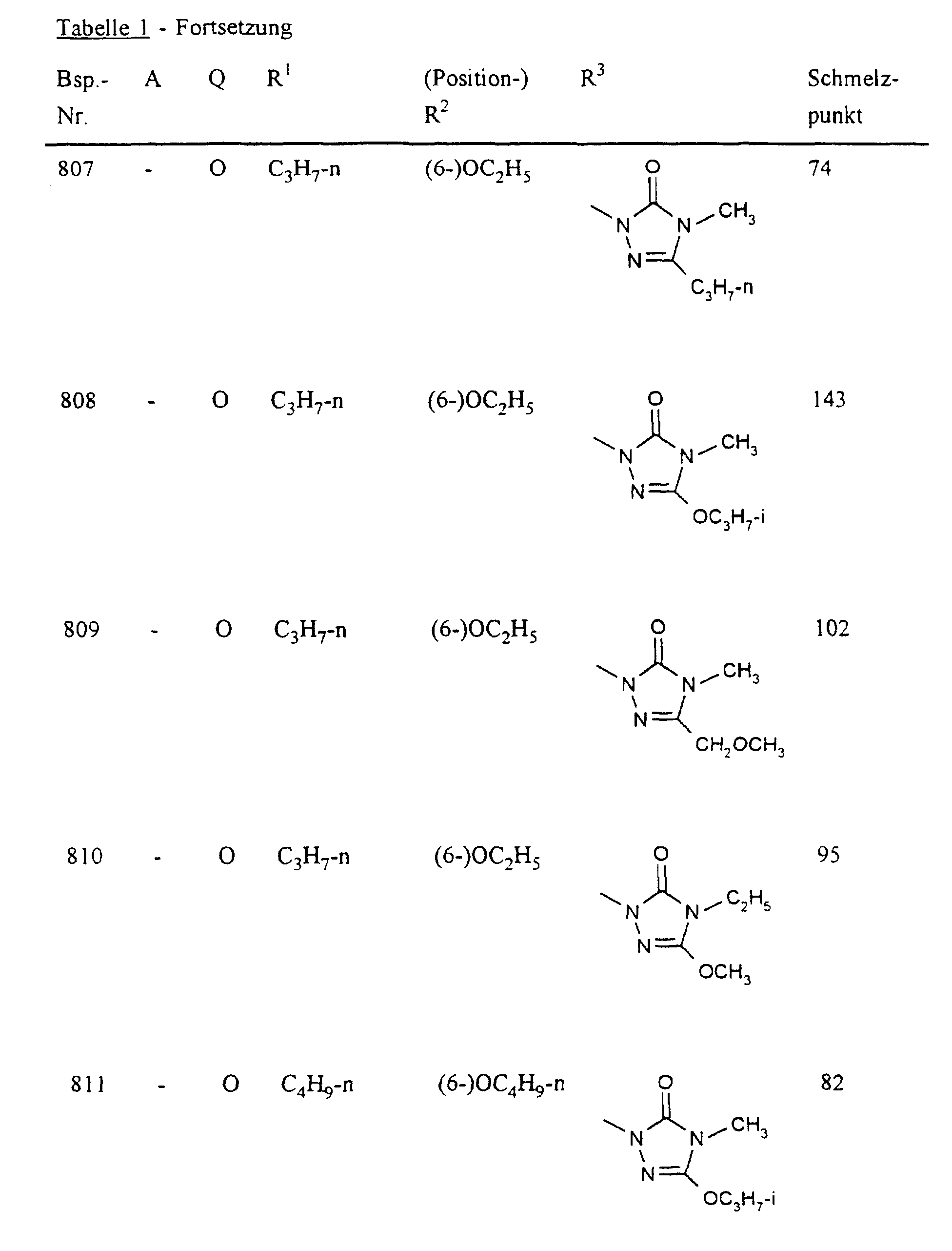

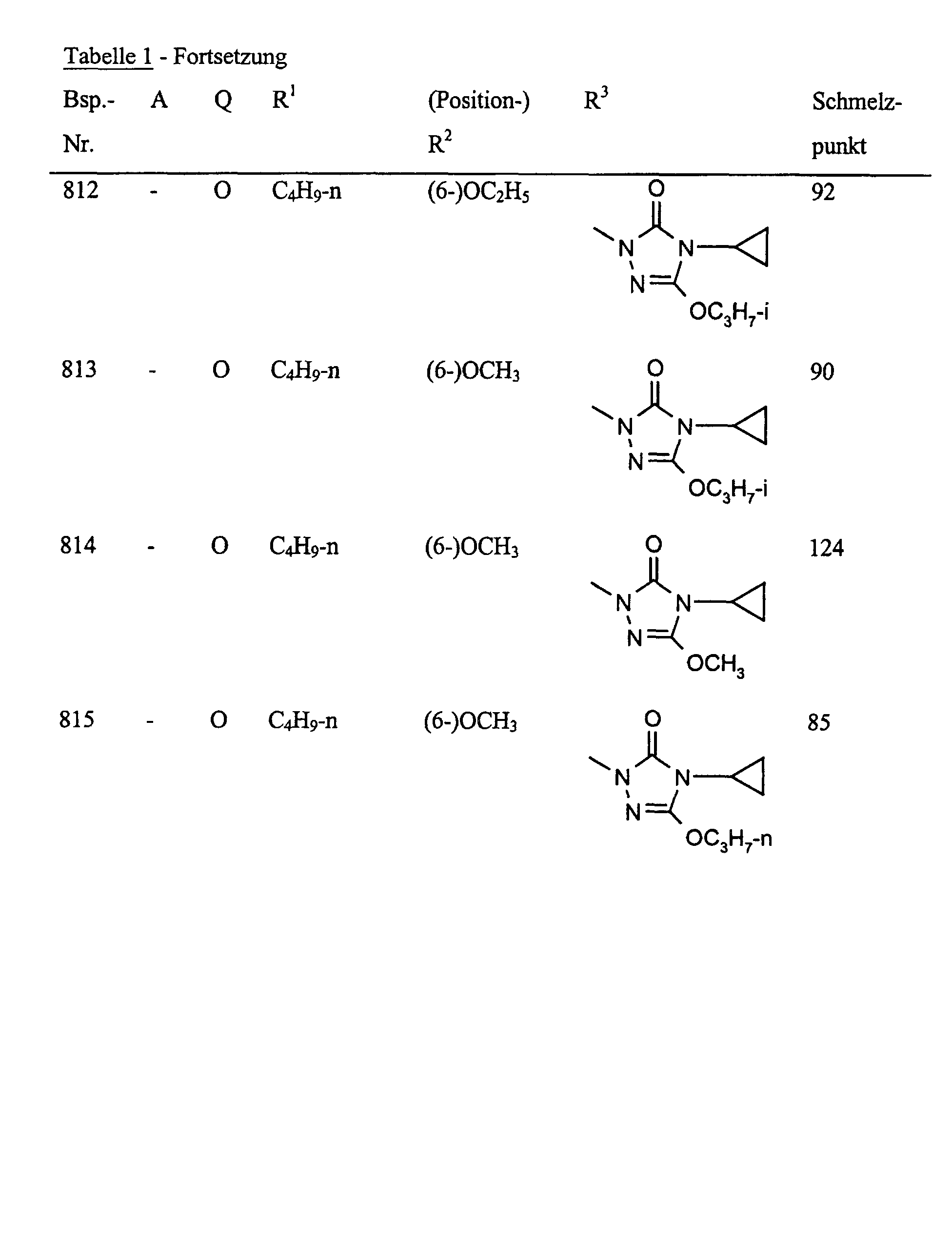

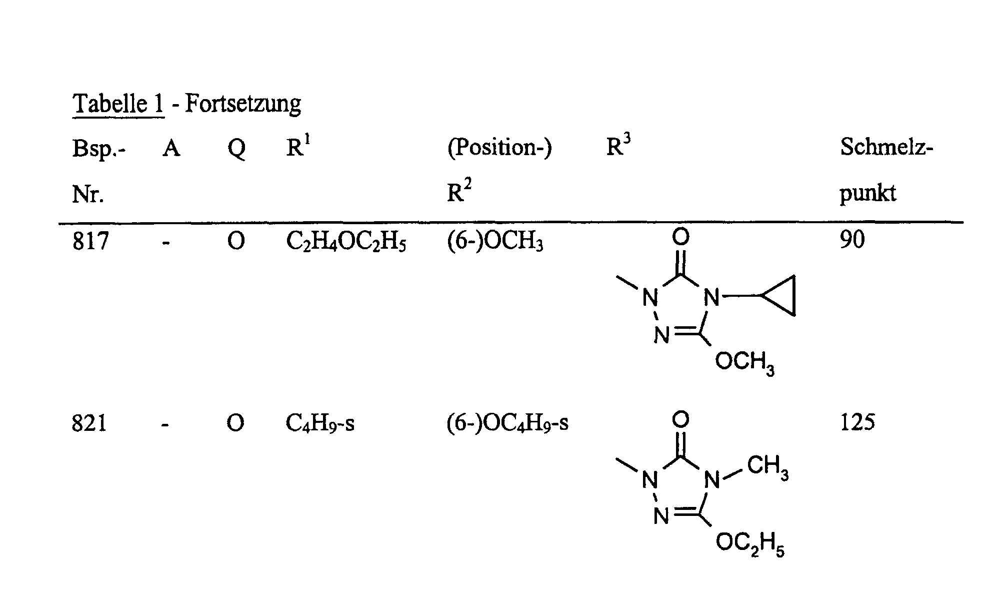

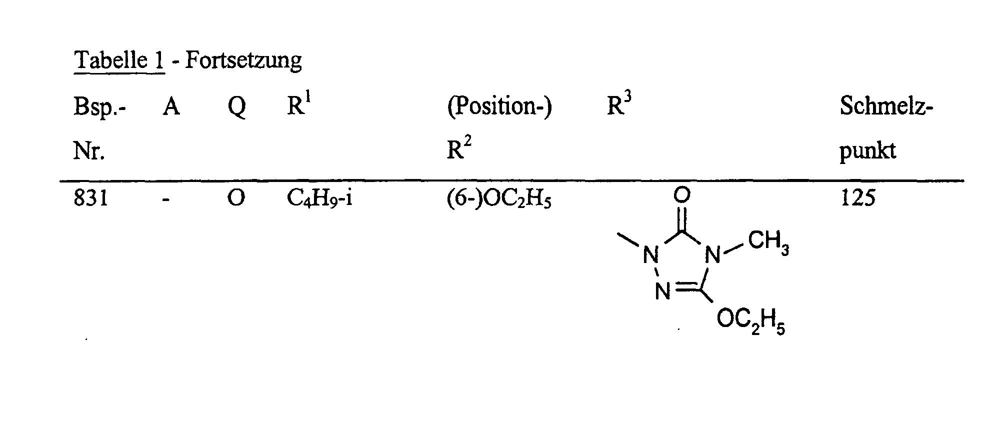

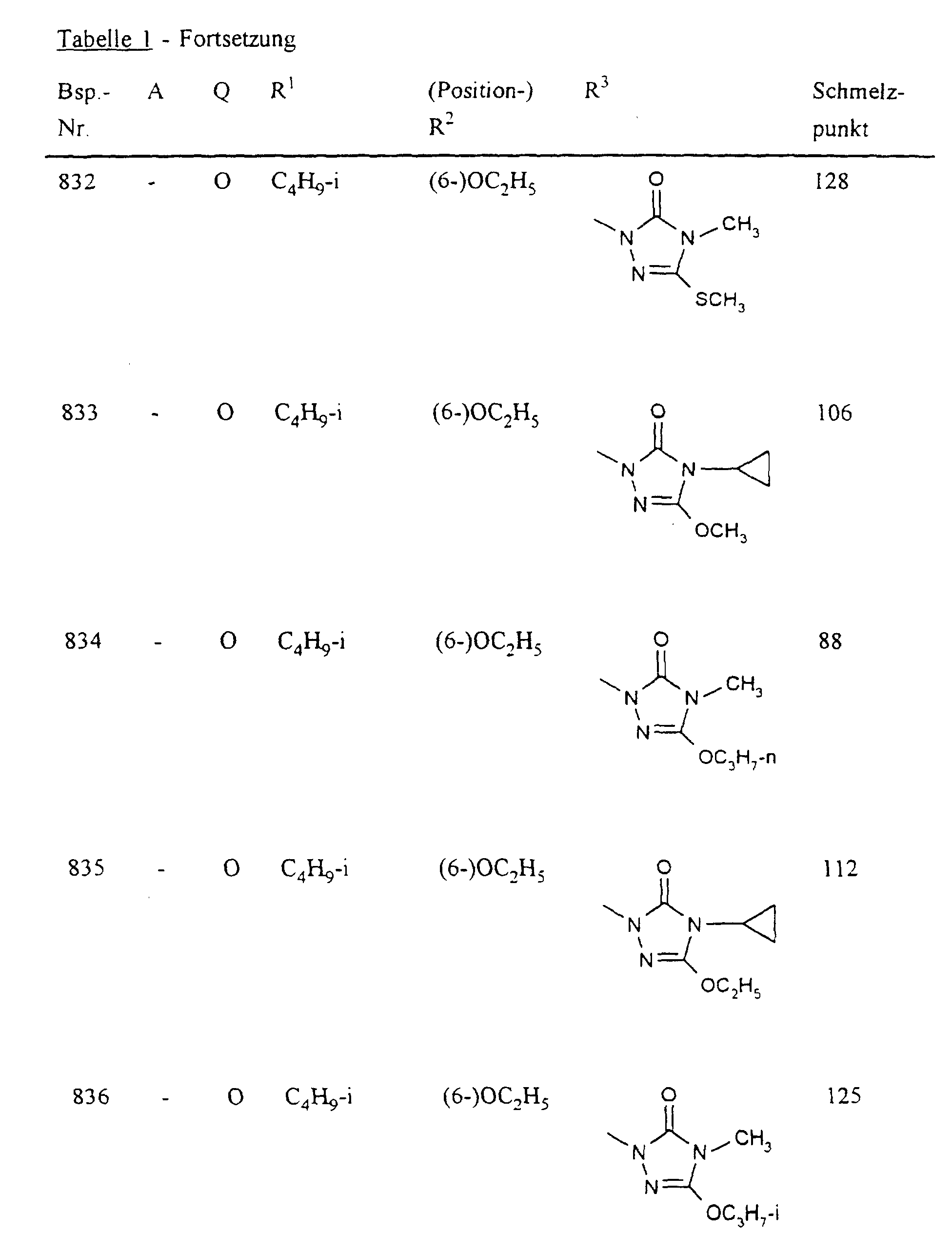

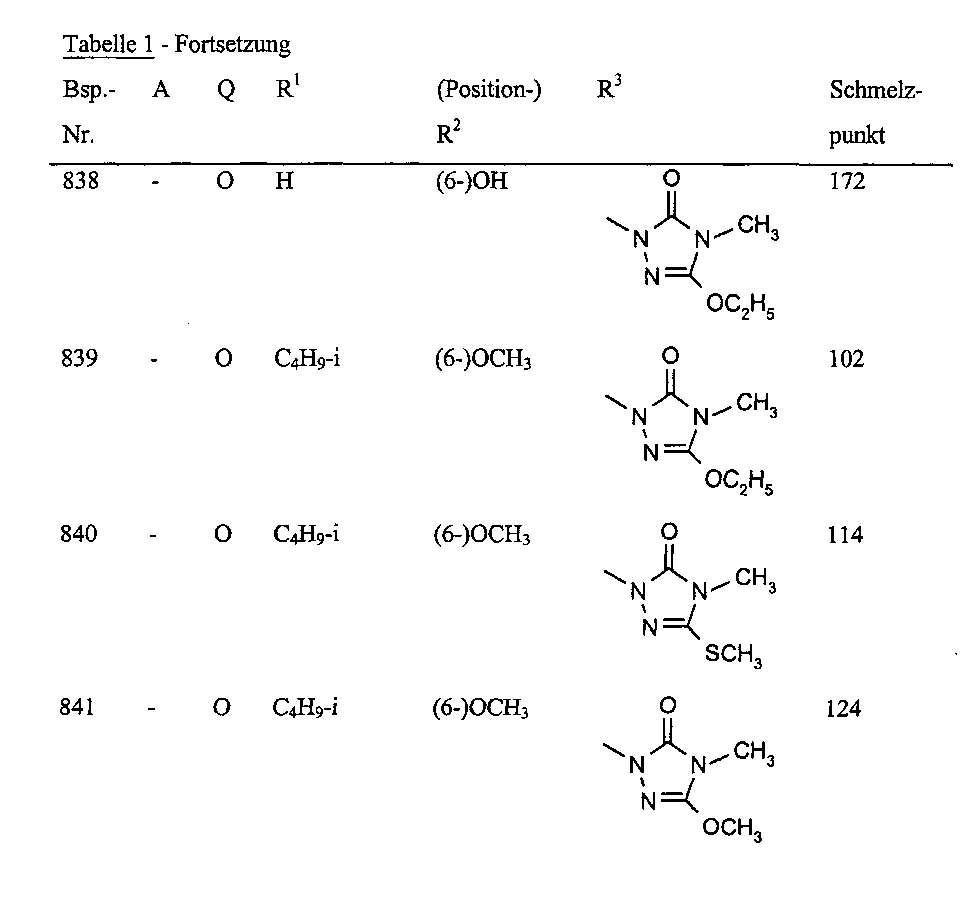

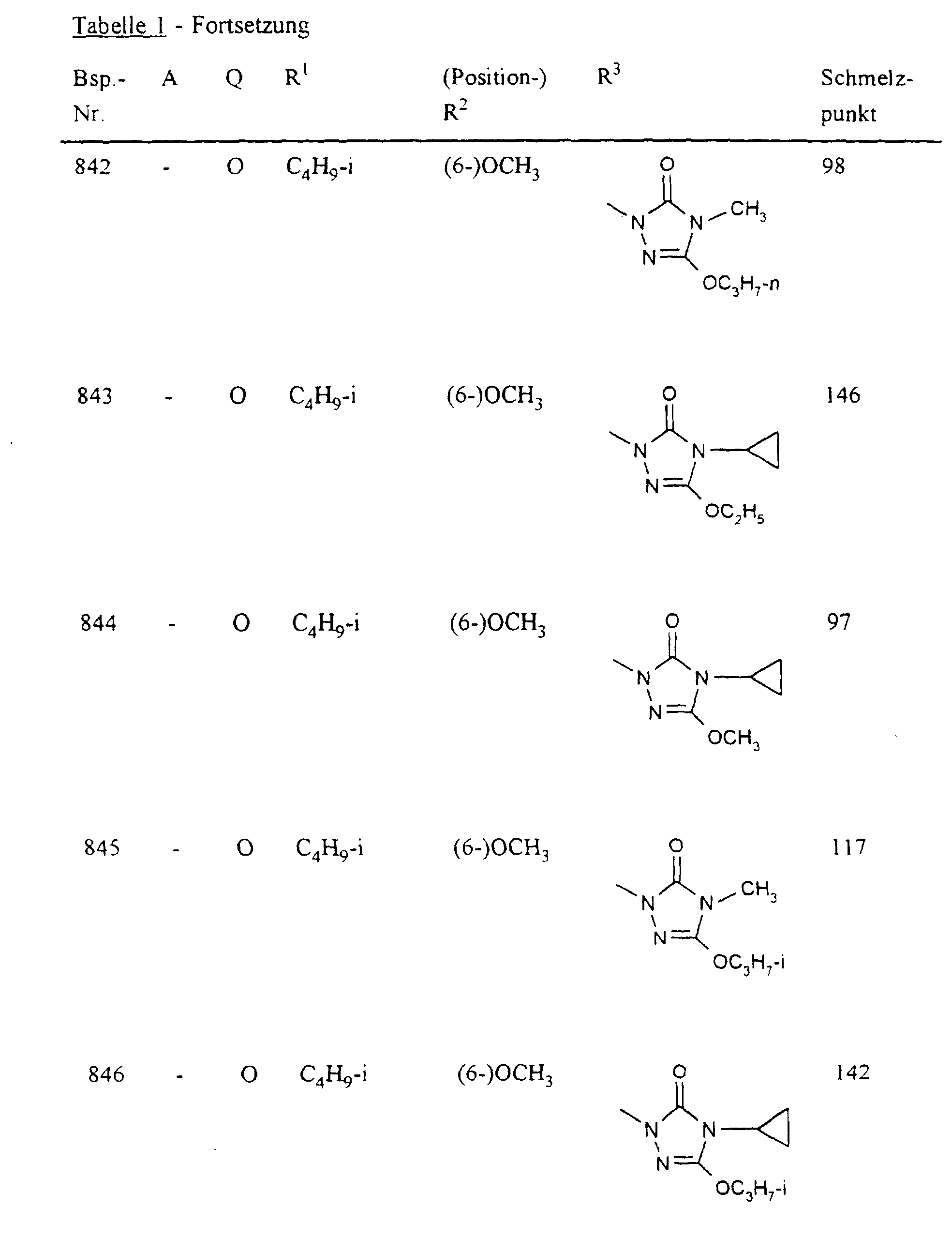

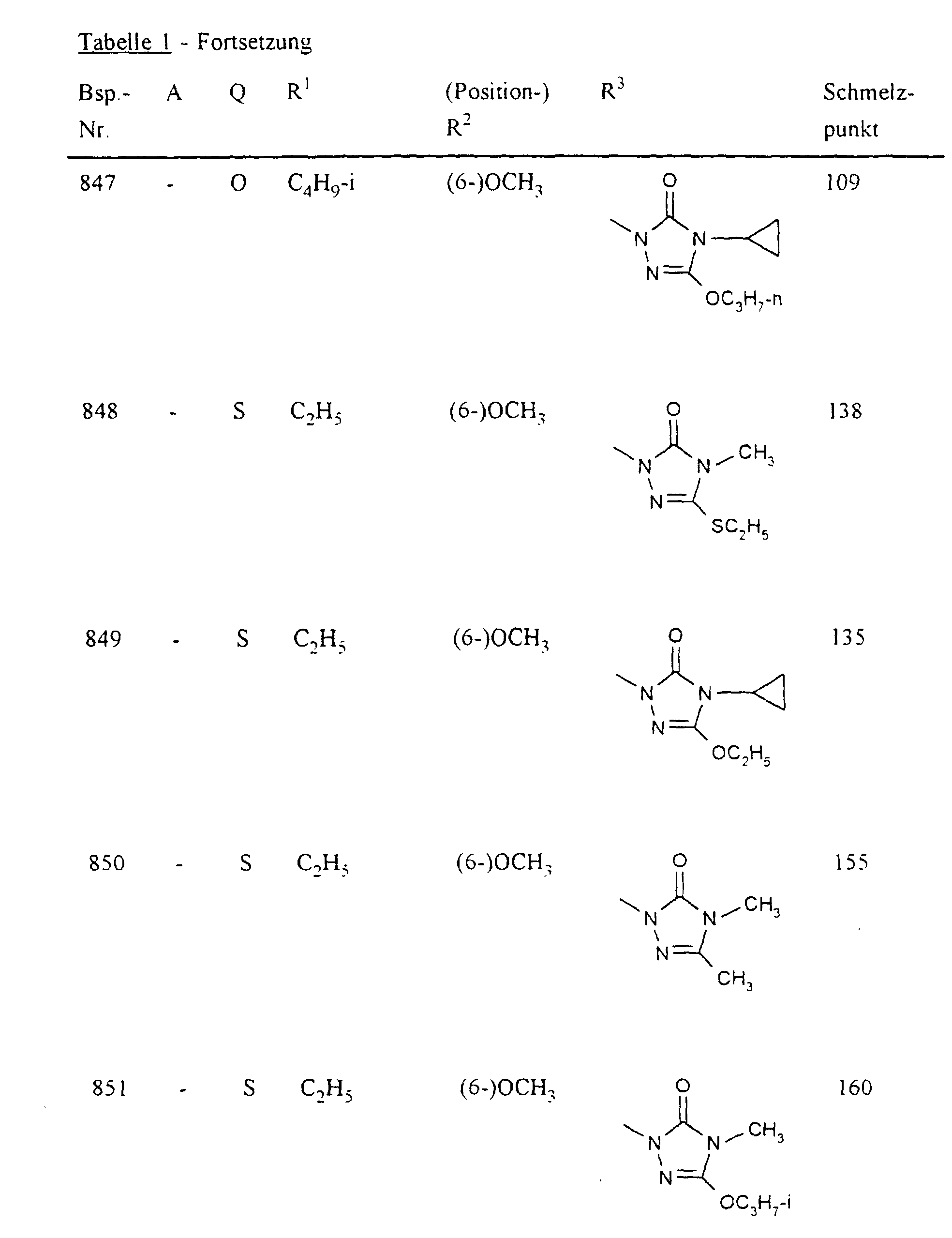

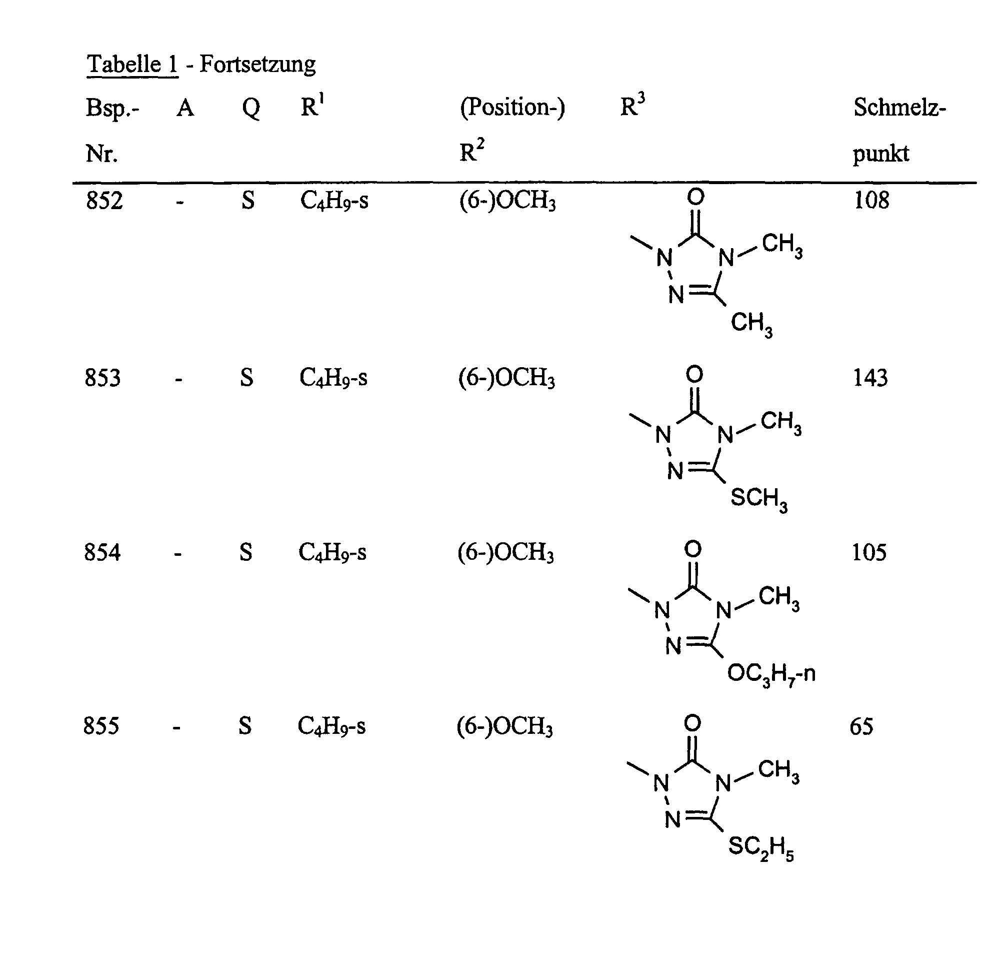

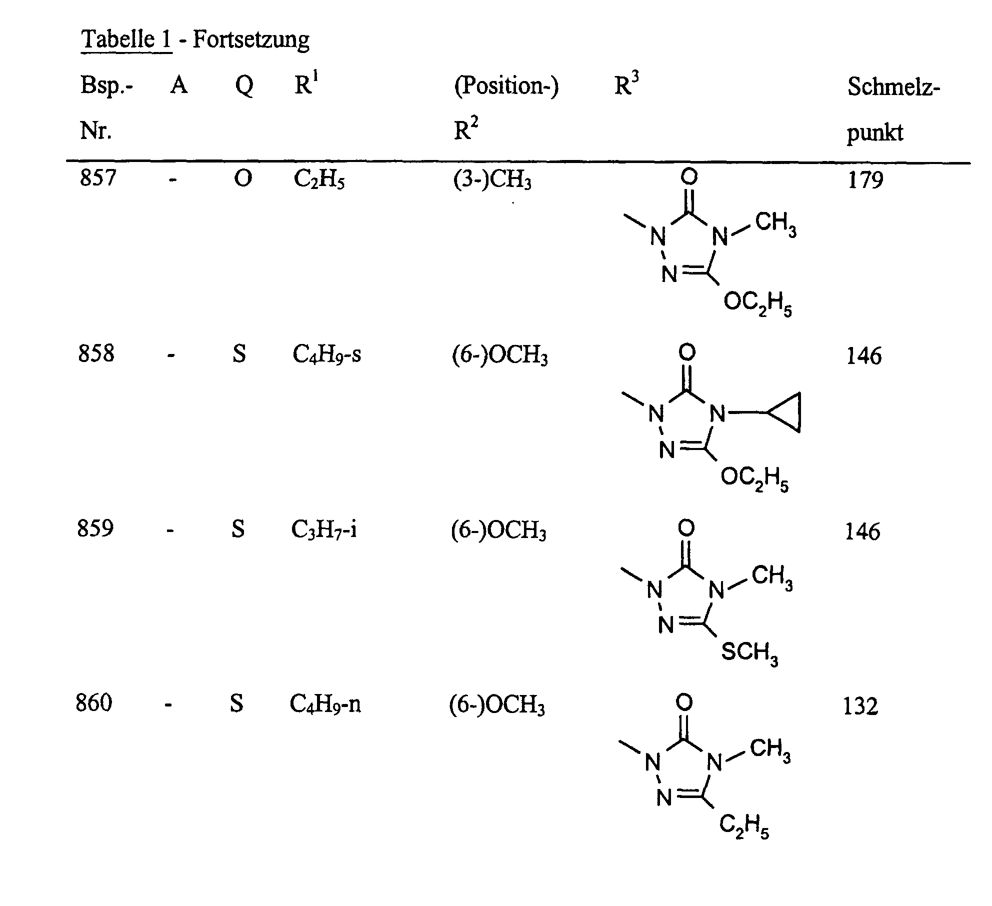

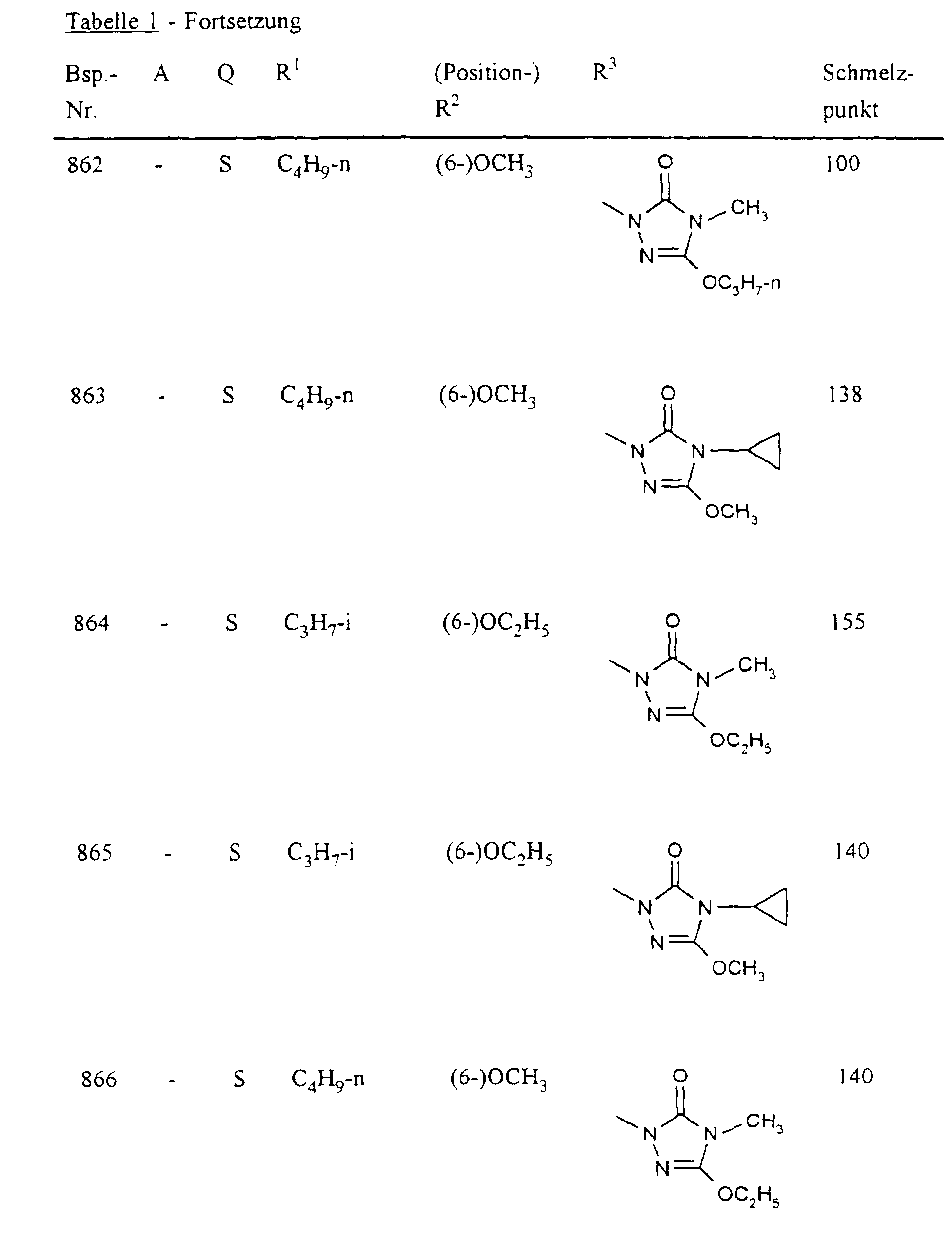

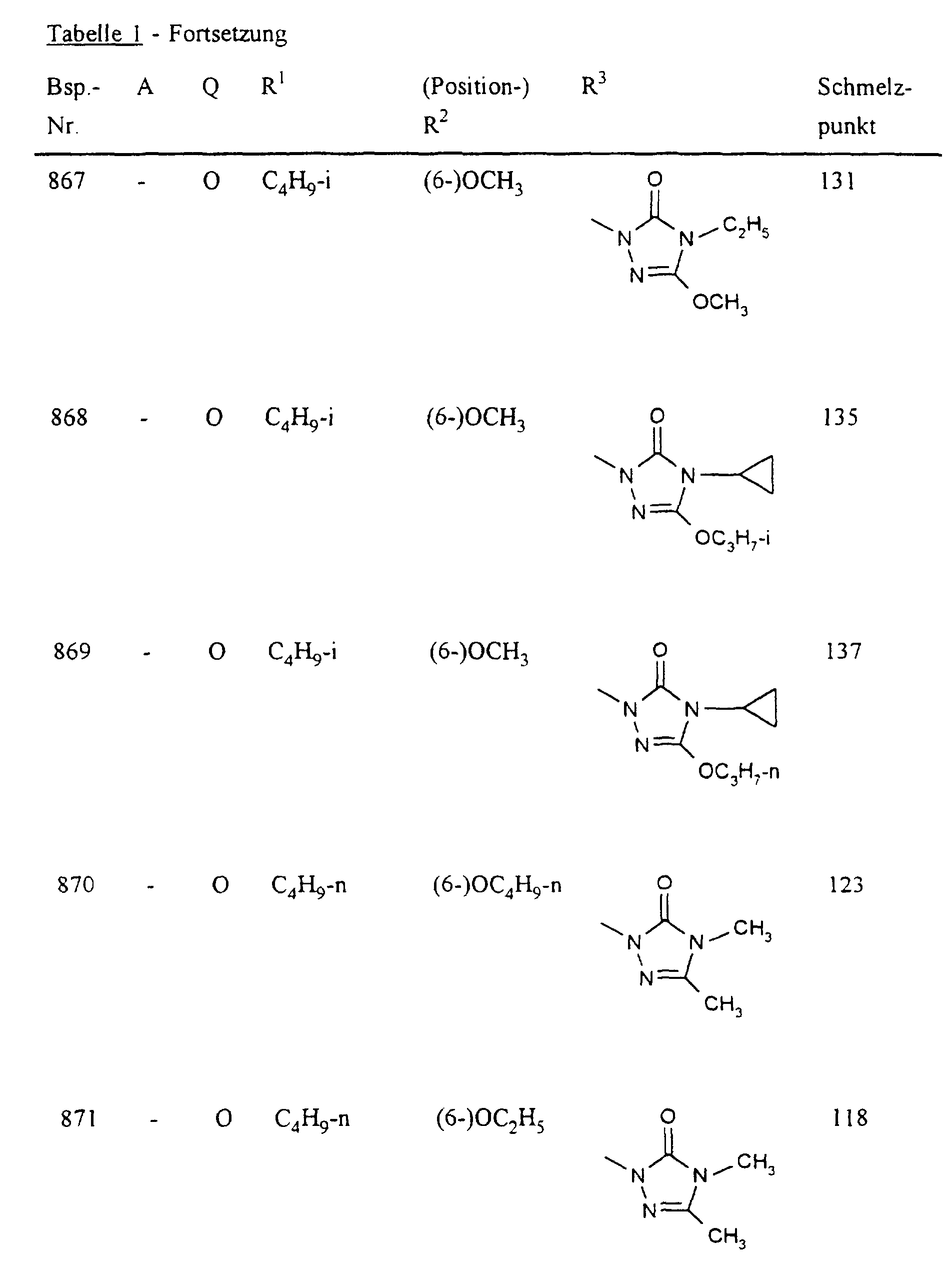

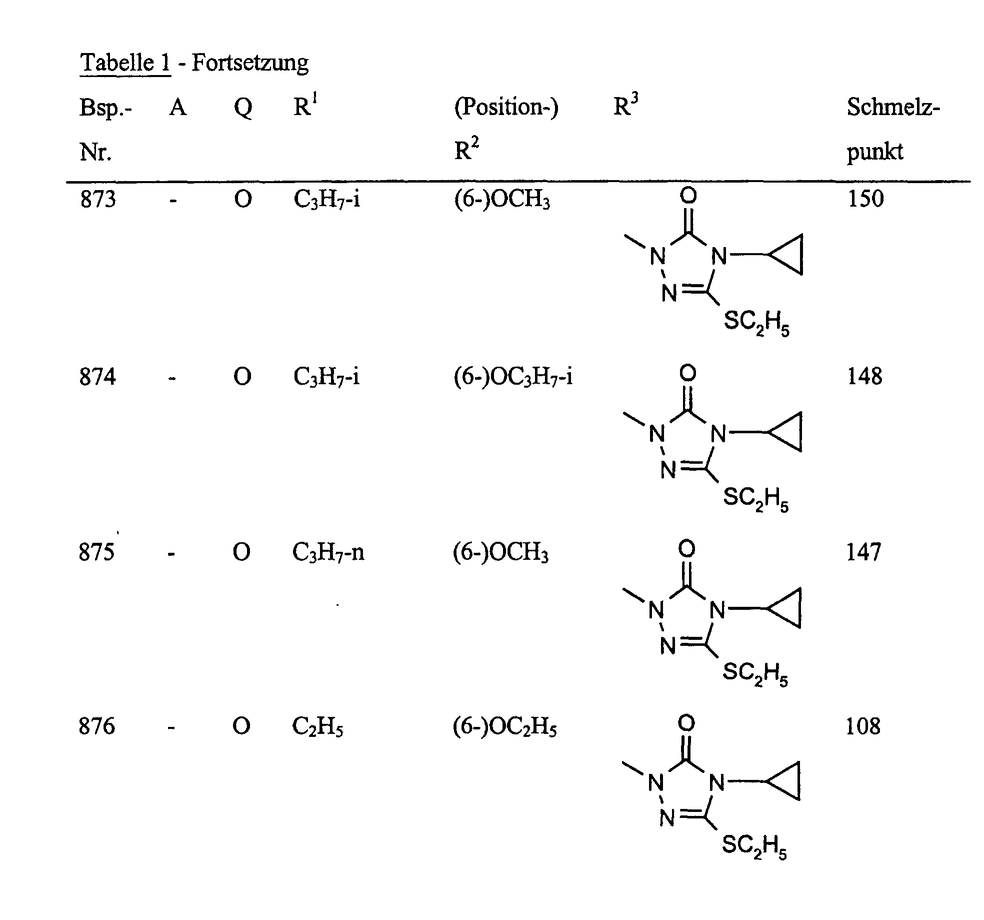

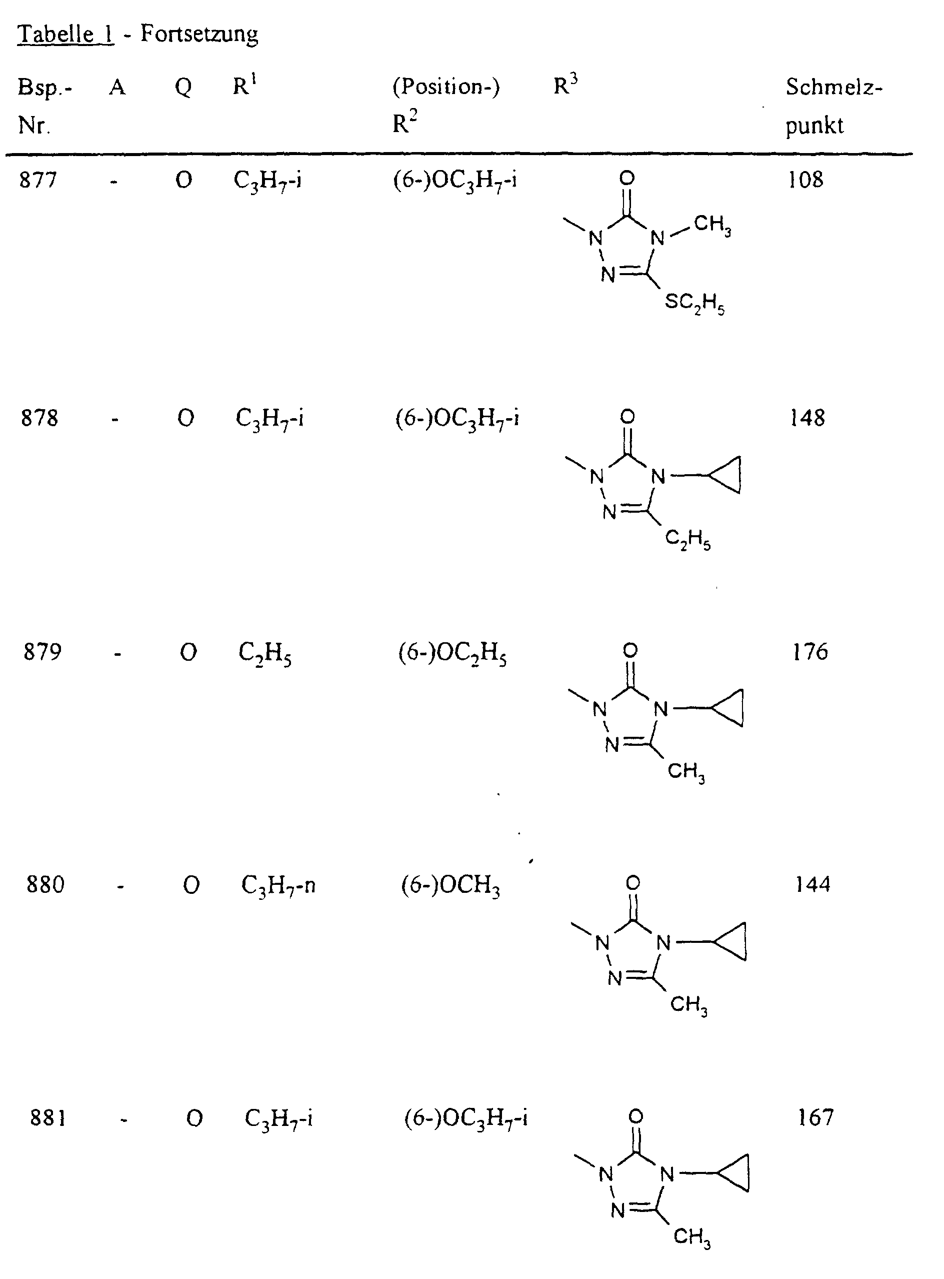

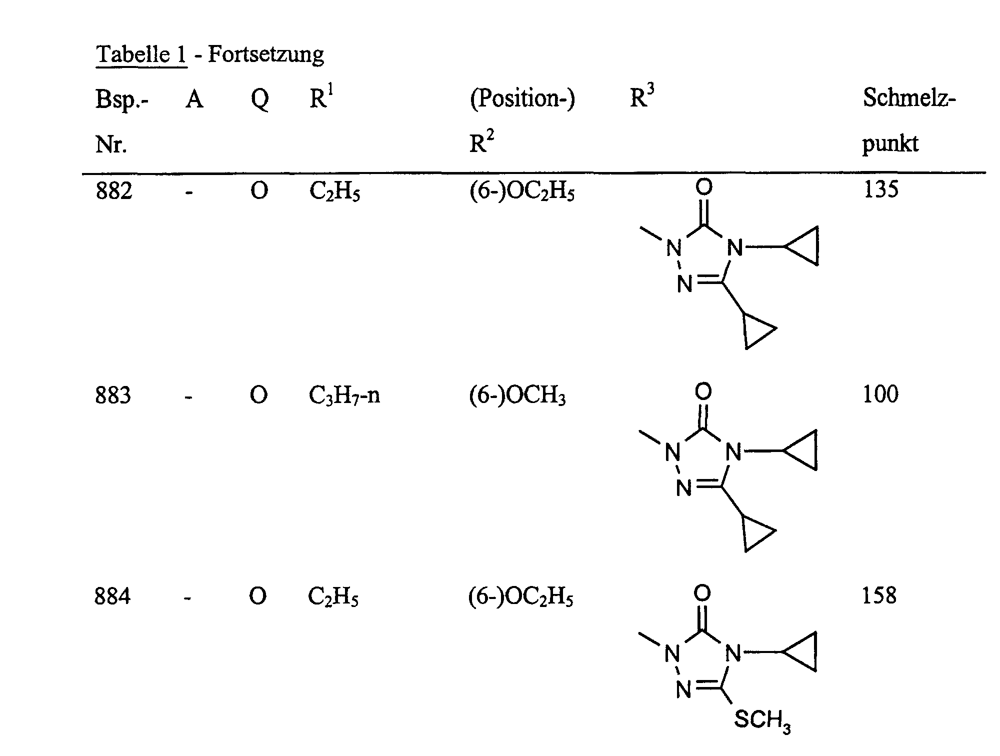

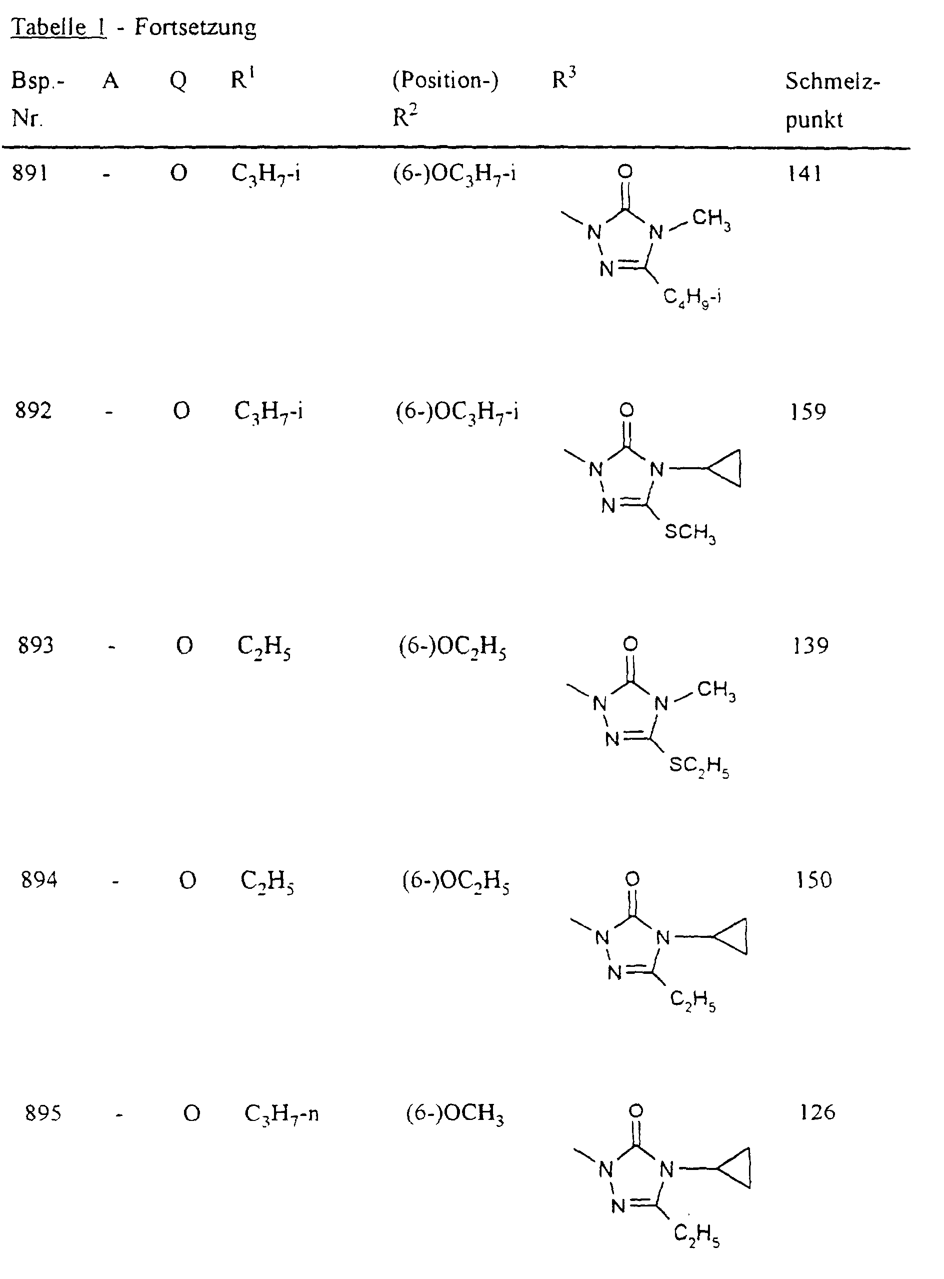

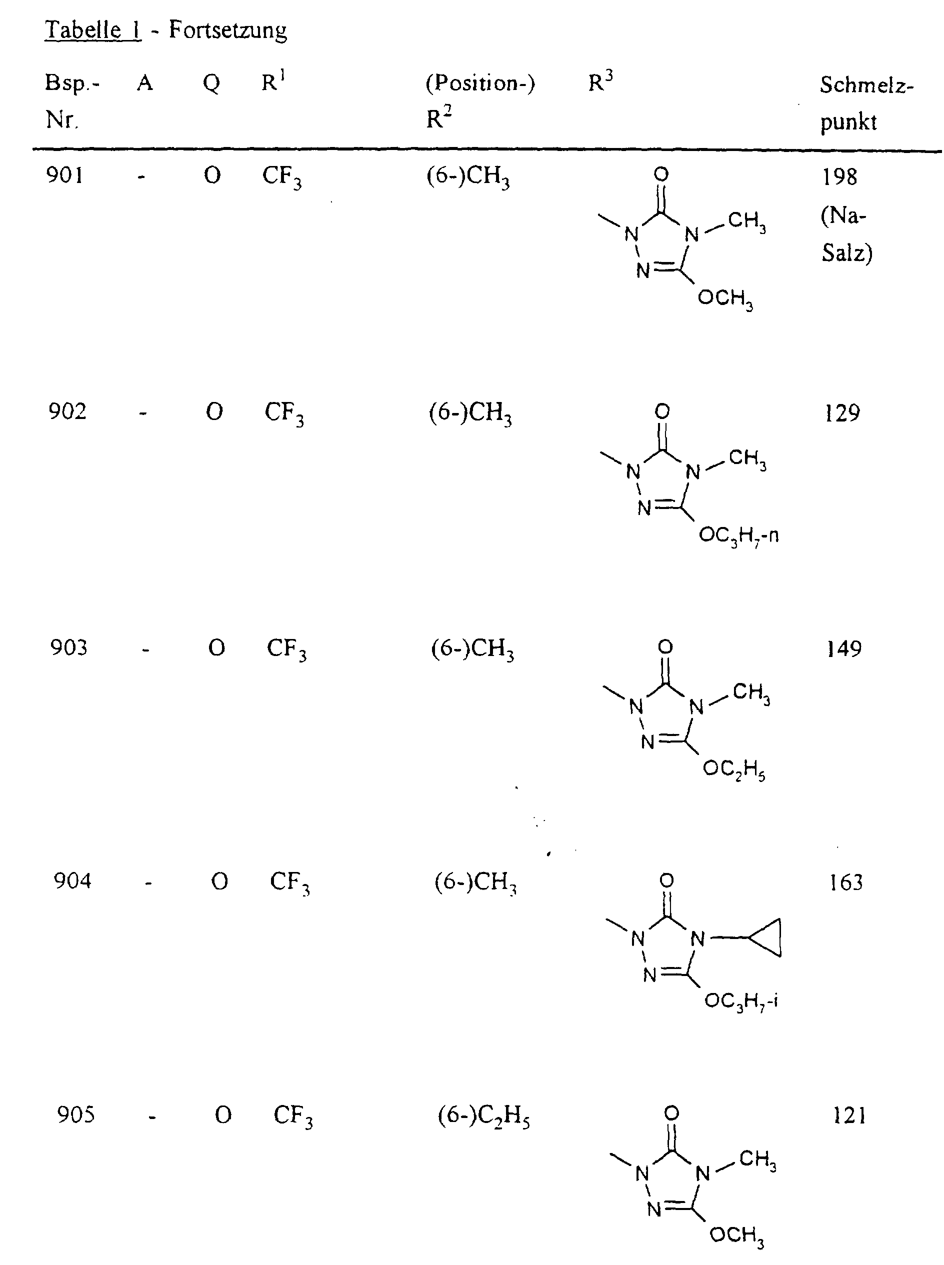

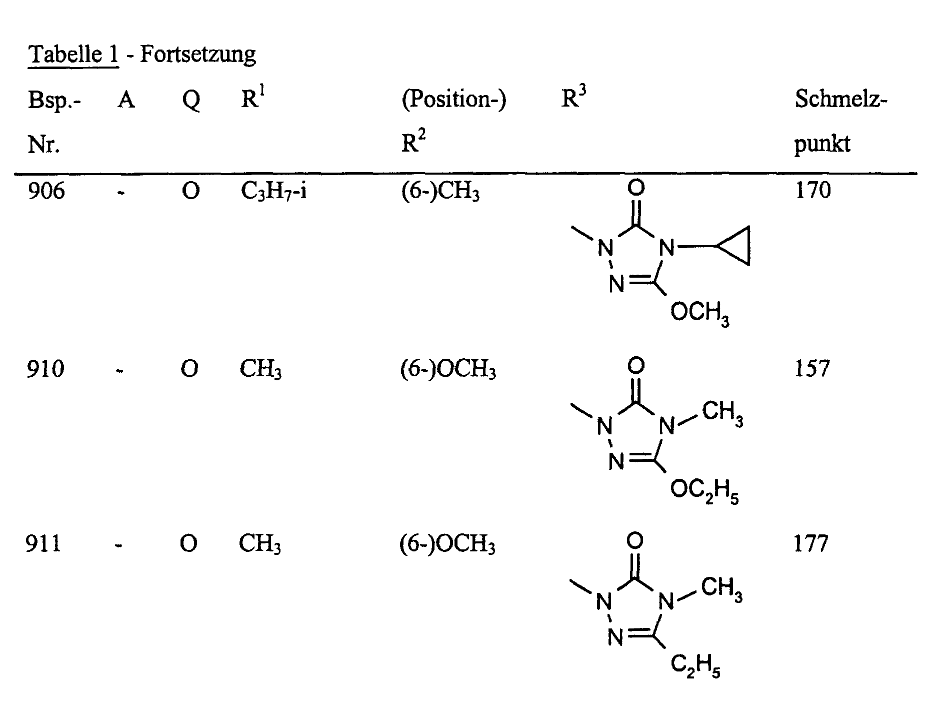

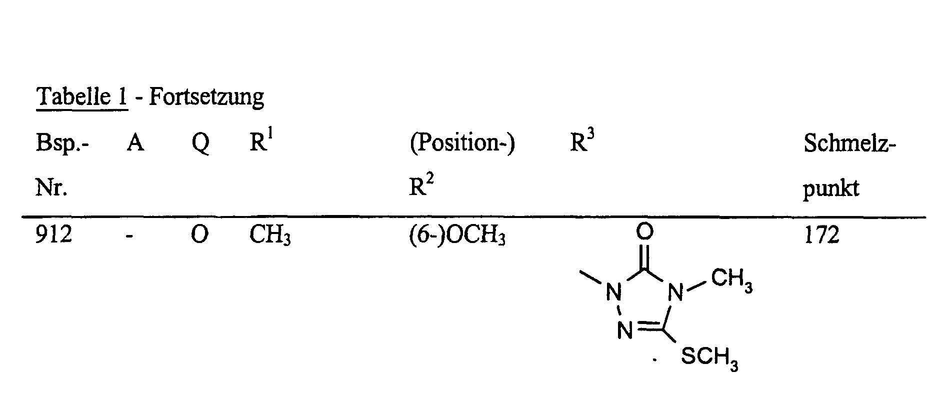

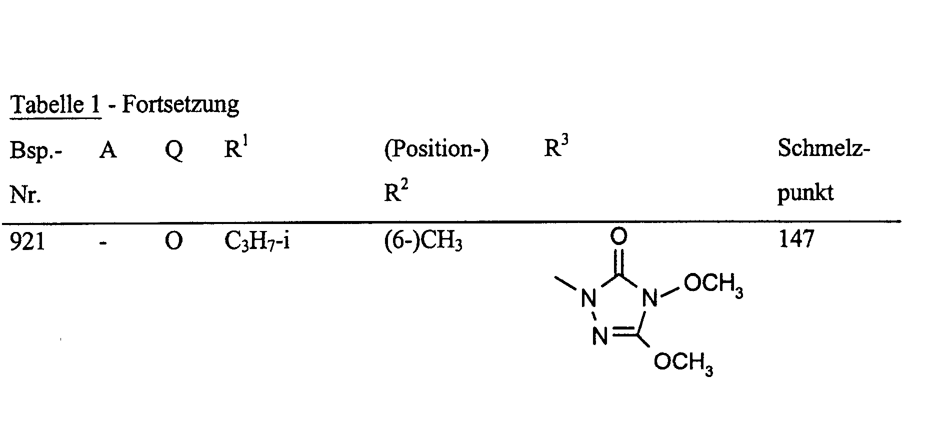

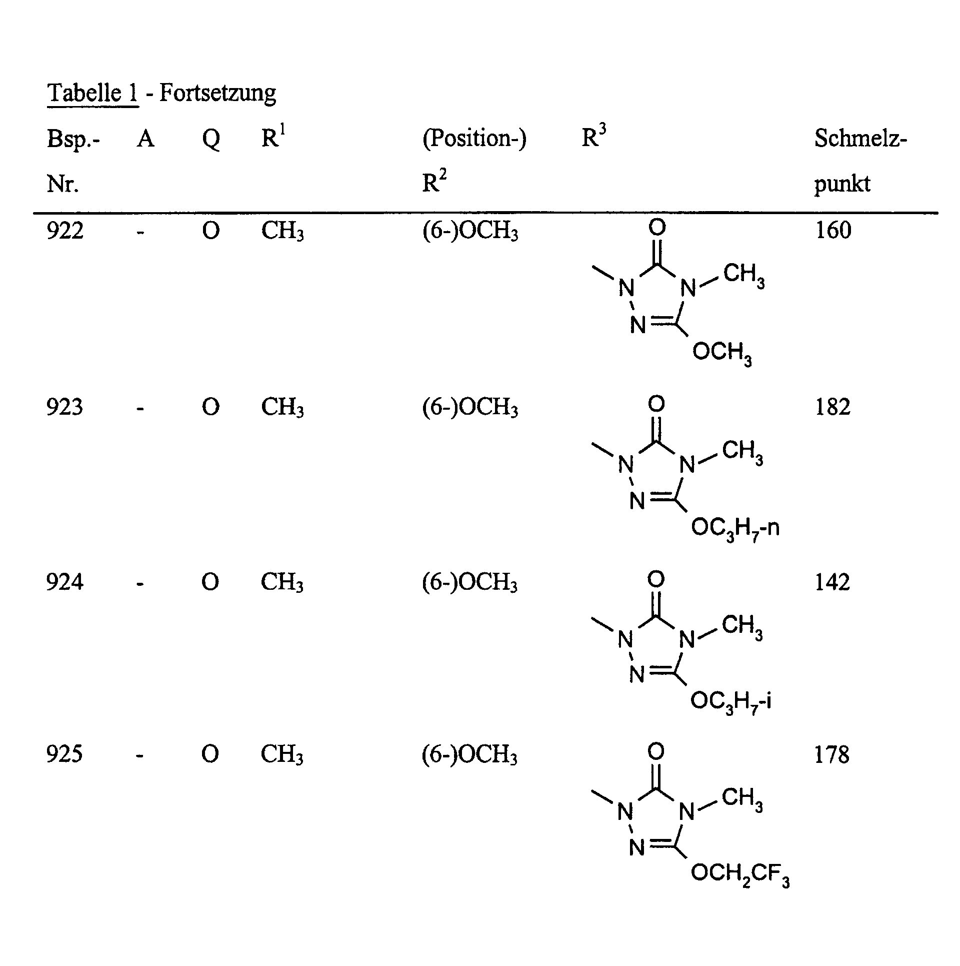

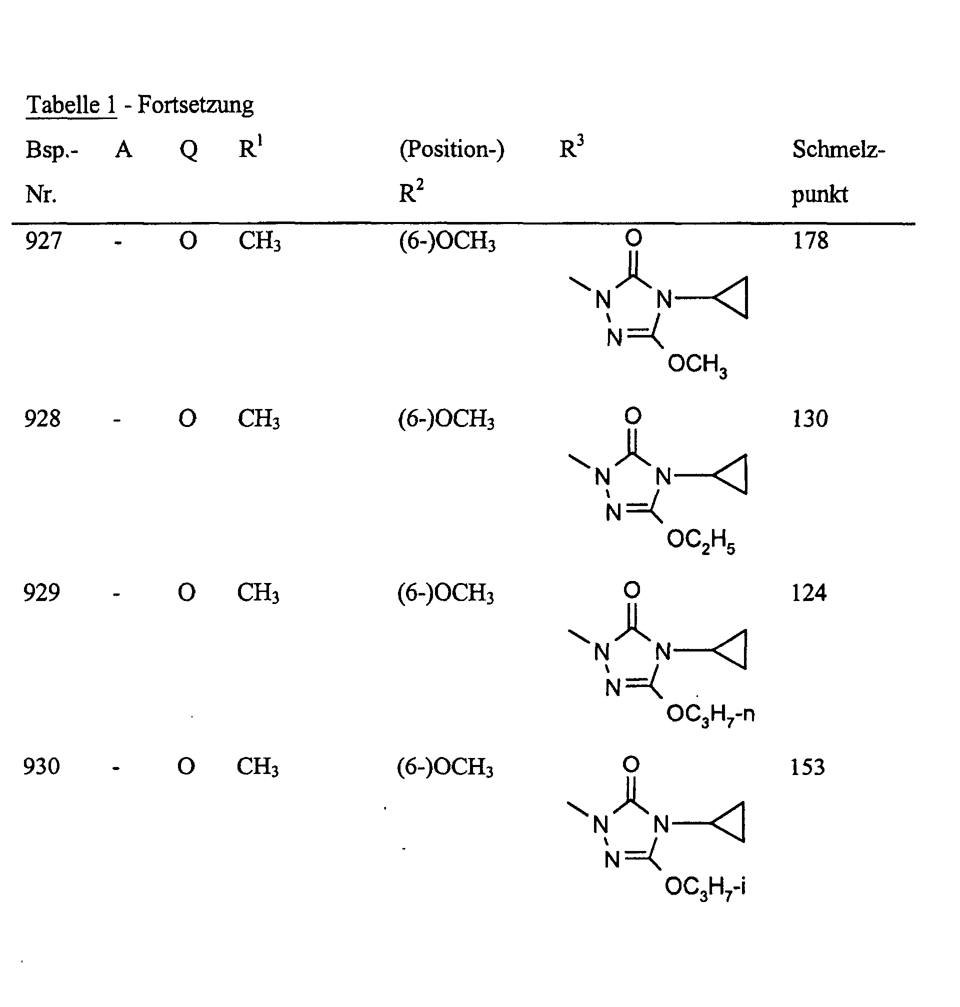

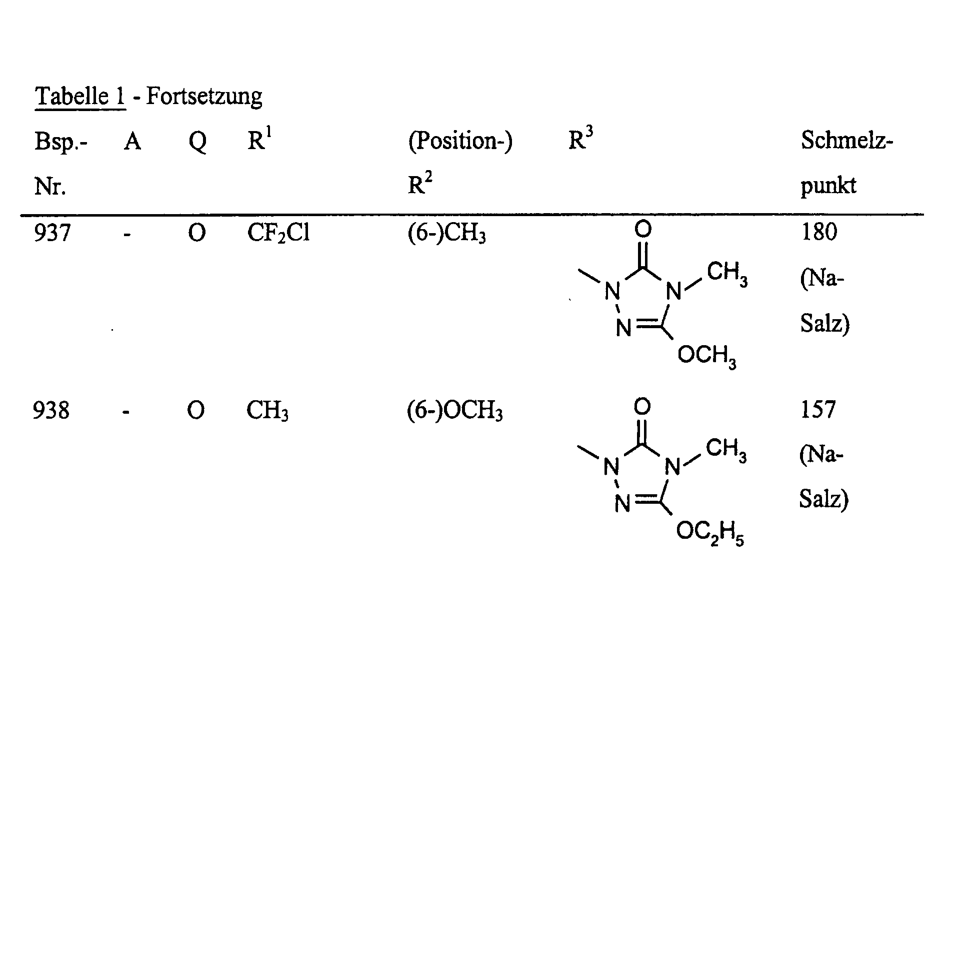

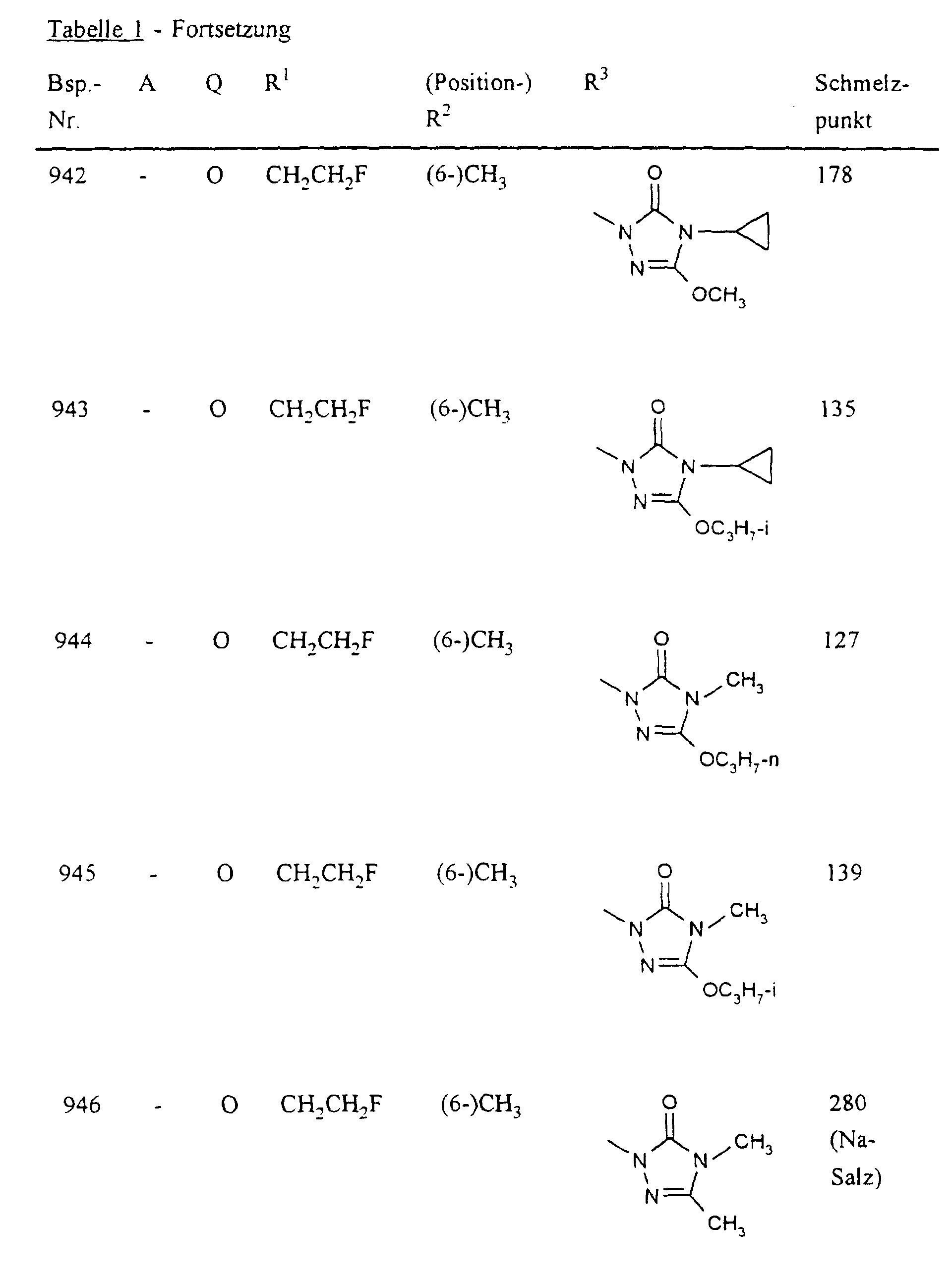

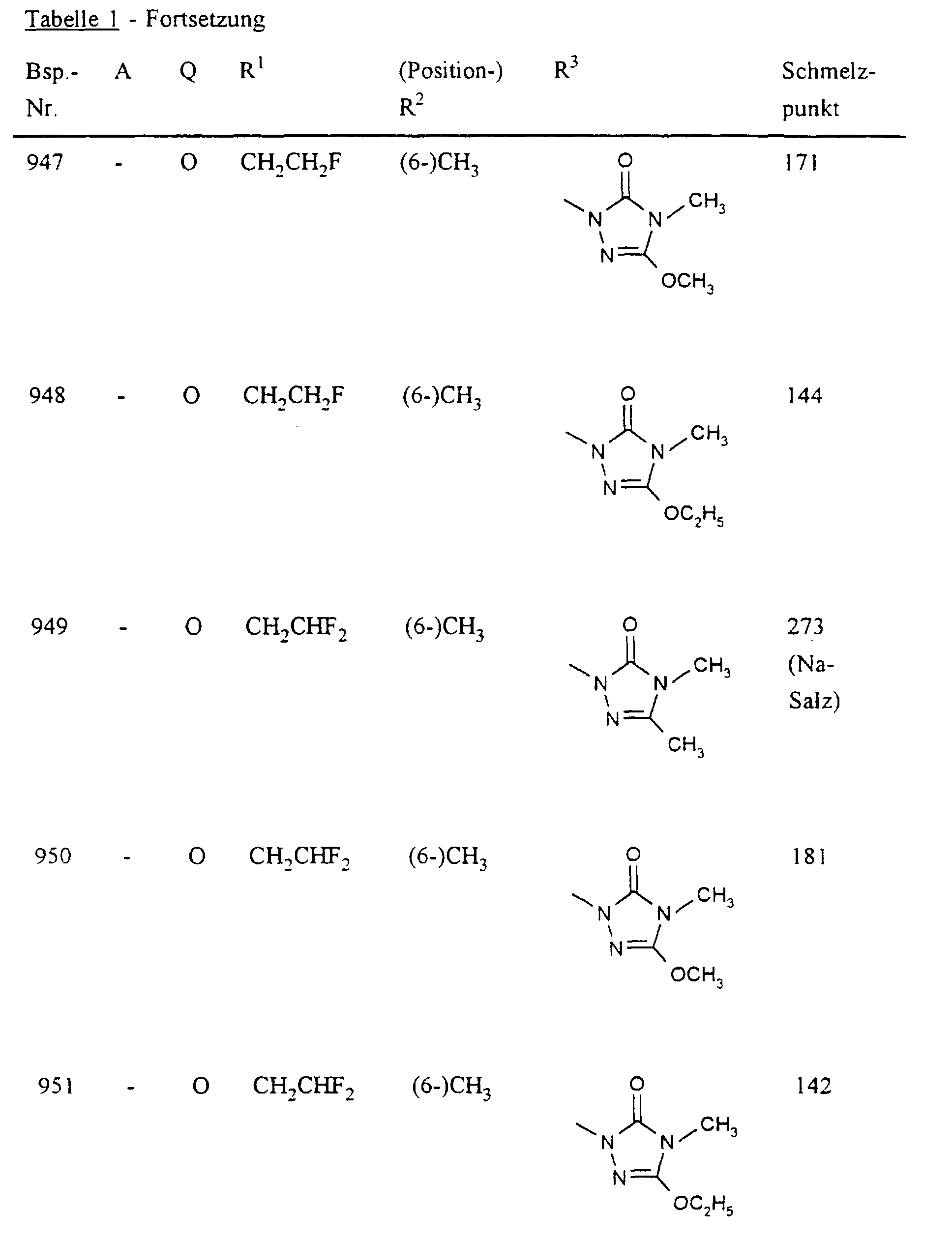

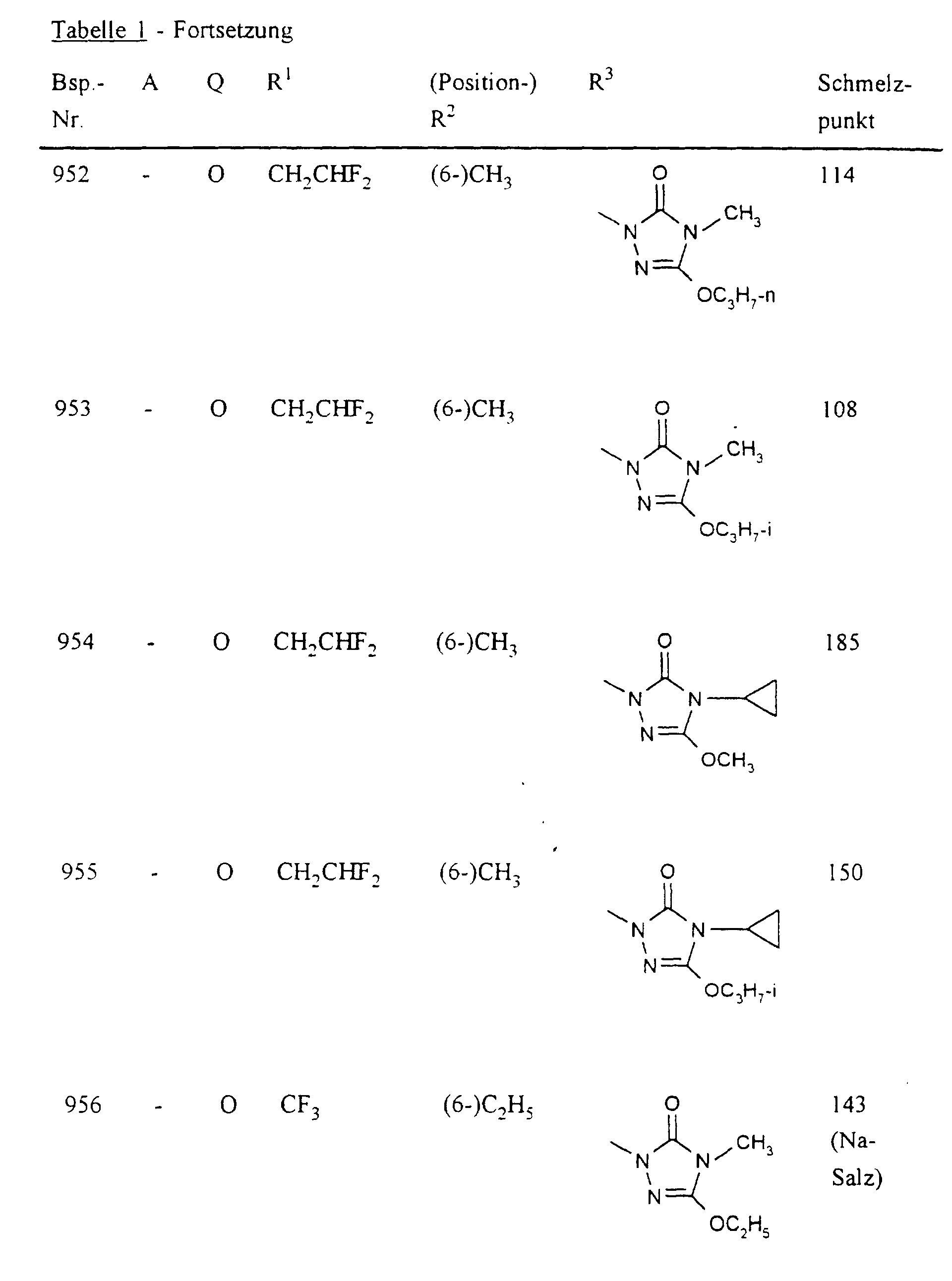

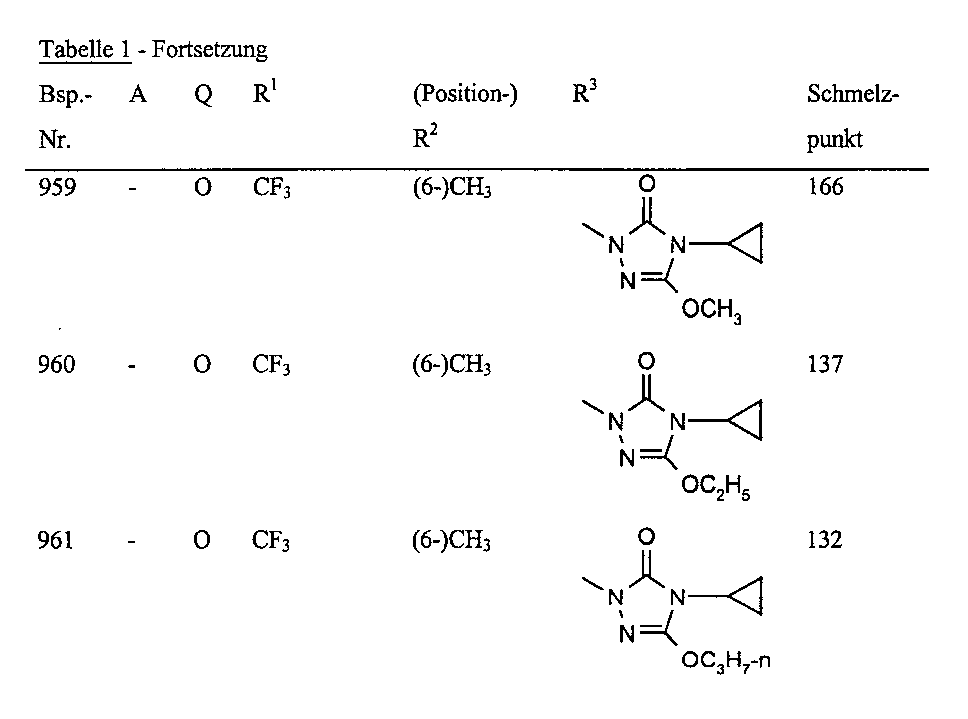

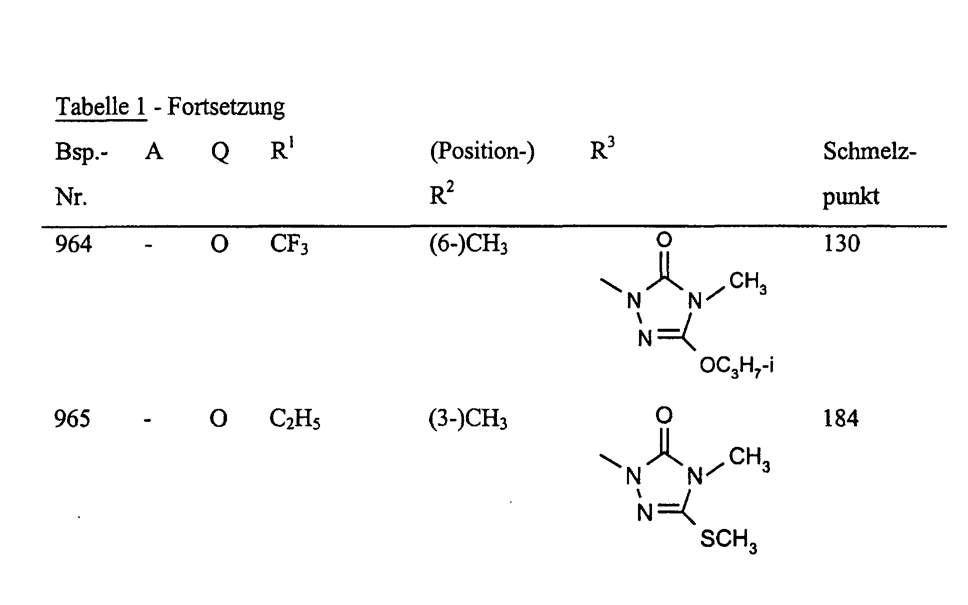

Die in Tabelle 1 als Beispiel 9 aufgeführte Verbindung kann beispielsweise wie folgt hergestellt werden:

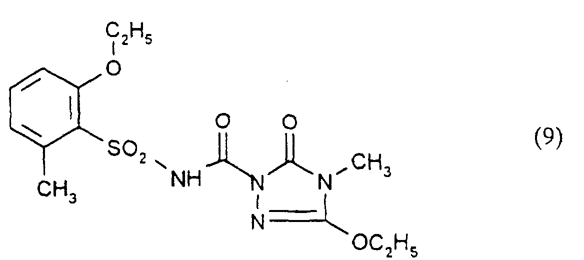

### (Verfahren (b))

1,4 g (0,01 Mol) 5-Ethoxy-4-methyl-2,4-dihydro-3H-1,2,4-triazol-3-on und 2,4 g (0,01 Mol) 2-Ethoxy-6-methyl-phenylsulfonylisocyanat werden in 50 ml Acetonitril 15 Stunden bei 20°C gerührt. Man destilliert das Lösungsmittel ab, verrührt den Rückstand mit Diethylether und saugt den Niederschlag ab.

Man erhält 3,3 g (85% der Theorie) 5-Ethoxy-4-methyl-2-(2-ethoxy-6-methyl-phenylsulfonylaminocarbonyl)-2,4-dihydro-3H-1,2,4-triazol-3-on vom Schmelzpunkt 160°C.

### Ausgangsstoffe der Formel (II) bzw. (IIa):

### Beispiel (II-1)

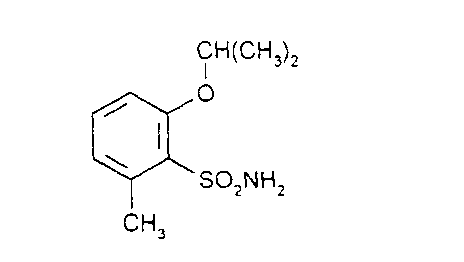

64,6 g (0,26 Mol) 2-Isopropoxy-6-methyl-benzolsulfochlorid werden in 350 ml 25%iger wässriger Ammoniaklösung 12 Stunden bei 20°C gerührt. Das kristallin angefallene Produkt wird anschließend durch Absaugen isoliert,

Man erhält 54 g (90% der Theorie) 2-Isopropoxy-6-methyl-benzolsulfonamid vom Schmelzpunkt 78°C.

### Beispiel (II-2)

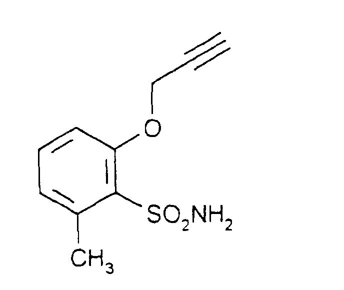

Eine Mischung aus 1,9 g (10 mMol) 2-Hydroxy-6-methyl-benzolsulfonamid, 1,2 g (10 mMol) Propargylbromid (in Form einer 80%igen Lösung in Toluol) und 1,4 g (10 mMol) Kaliumcarbonat wird 2 Stunden unter Rückfluß erhitzt. Dann wird filtriert, das Filtrat im Wasserstrahlvakuum eingeengt, der Rückstand mit Petrolether digeriert und das hierbei kristallin angefallene Produkt durch Absaugen isoliert.

Man erhält 2,1 g (93% der Theorie) 6-Methyl-2-propargyloxy-benzolsulfonamid vom Schmelzpunkt 129°C.

### Beispiel (II-3)

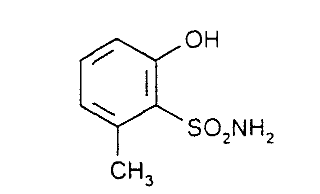

188 ml einer 1-molaren Lösung von Bor(III)-bromid in Methylenchlorid werden bei 20°C unter Rühren zu einer Lösung von 32,3 g (0,15 Mol) 2-Ethoxy-6-methylbenzolsulfonamid in 300 ml Methylenchlorid tropfenweise gegeben und die Reaktionsmischung wird 30 Minuten bei 20°C gerührt. Dann werden bei 0°C bis 5°C (Eiskühlung) 300 ml Methanol zugetropft. Nach Erwärmen auf 20°C wird im Wasserstrahlvakuum eingeengt und der Rückstand mit Essigsäureethylester verrührt. Die erhaltene Lösung wird mit Wasser gewaschen, mit Natriumsulfat getrocknet und filtriert. Das Filtrat wird im Wasserstrahlvakuum eingeengt, der Rückstand durch Verrühren mit Petrolether zur Kristallistion gebracht und das kristalline Produkt durch Absaugen isoliert.

Man erhält 20,3 g (72% der Theorie) 2-Hydroxy-6-methyl-benzolsulfonamid vom Schmelzpunkt 126°C.

Analog zu den Beispielen (II-1) bis (II-3) sowie entsprechend der allgemeinen Beschreibung des erfindungsgemäßen Herstellungsverfahrens können beispielsweise auch die in der nachstehenden Tabelle 2 aufgeführten Verbindungen der Formel (II) bzw. (IIa) hergestellt werden.

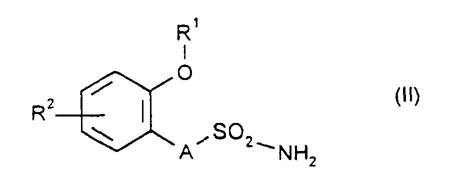

**Tabelle 2:**

| Beispiele für die Verbindungen der Formel (II) | | | | |
|---|---|---|---|---|
| Bsp.-Nr. | A | R¹ | (Position-) R² | Schmelzpunkt (°C) |
| II-4 | - | C₂H₅ | (6-) CH₃ | 104 |
| II-5 | - | n-C₃H₇ | (6-) CH₃ | 63 |
| II-6 | - | -CH₂CH₂Cl | (6-) CH₃ | 102 |
| II-7 | - | CH₃ | (6-)CH₃ | 132 |
| II-8 | - | -CH₂C₆H₅ | (6-)CH₃ | 131 |
| II-10 | - | CH₃ | (6-)C₃H₇-n | 108 |
| II-11 | - | C₂H₅ | (6-)C₃H₇-n | 80 |
| II-12 | - | C₂H₅ | (5-)CH₃ | 131 |
| II-13 | - | CH₃ | (6-)Cl | 166 |
| II-14 | - | C₂H₅ | (6-)Cl | 121 |
| II-15 | - | H | (6-)Cl | 118 |
| II-16 | - | i-C₃H₇ | (6-)Cl | 85 |
| II-17 | - | -CH₂CH=CH₂ | (6-)Cl | 106 |
| II-18 | - | -CH₂C=CH | (6-)Cl | 181 |
| II-19 | - | CF₃ | (5-)Cl | |
| II-20 | - | CHF₂ | (5-)CH₃ | 127 |
| II-21 | - | CHF₂ | (6-)CH₃ | 89 |
| II-22 | - | CH₃ | (5-)C(CH₃)₃ | 160 |
| II-23 | - | CH₃ | (5-)Cl | |
| II-24 | - | CHF₂ | (4-)CH₃ | 153 |
| II-25 | - | -CF₂CHFCl | (6-)CH₃ | 85 |
| II-26 | - | C₂H₅ | (6-)CH₂Cl | |

### Ausgangsstoffe der Formel (IV):

### Beispiel (IV-1)

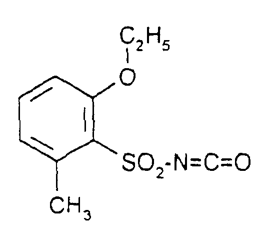

21,5 g (0,1 Mol) 2-Ethoxy-6-methyl-benzolsulfonsäureamid und 10 g (0,1 Mol) n-Butylisocyanat werden in 100 ml Chlorbenzol zum Sieden erhitzt. Bei Rückflußtemperatur wird 4 Stunden Phosgen eingeleitet. Die klare Lösung wird unter vermindertem Druck eingeengt und der Rückstand feindestilliert. Bei einem Druck von 0,8 mbar und einer Kopftemperatur von 135 - 140°C geht 2-Ethoxy-6-methyl-phenylsulfonylisocyanat über, das in der Vorlage erstarrt.

Man erhält 7,9 g 2-Ethoxy-6-methyl-phenylsulfonylisocyanat als farbloses Produkt vom Schmelzpunkt 40°C.

### Ausgangsstoffe der Formel (VI) bzw. (VIa):

### Beispiel (VI-1)

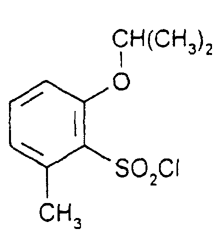

47,8 g (0,29 Mol) 2-Isopropoxy-6-methyl-anilin werden in einer Mischung aus 87 ml 1N-Salzsäure und 145 ml konz. Salzsäure gelöst und die Lösung wird auf -5°C abgekühlt. Bei -5°C bis 0°C wird dann unter Rühren eine Lösung von 22 g (0,32 Mol) Natriumnitrit in 87 ml Wasser zugetropft und die Mischung wird noch eine Stunde bei ca. 0°C gerührt. Nach Beseitigung des Nitrit-Überschusses mit Amidosulfonsäure wird die erhaltene Diazoniumsalz-Lösung bei -5°C bis 0°C zu einer gesättigten Lösung von Schwefeldioxid in 175 ml 1,2-Dichlor-ethan tropfenweise gegeben. Nach ca. 30 Minuten gibt man 1,7 g Kupfer(I)-chlorid und 1,7 g Dodecyl-trimethylammoniumbromid dazu, lässt das Reaktionsgemisch innerhalb von ca. 60 Minuten auf Raumtemperatur kommen, erwärmt es innerhalb einer weiteren Stunde auf ca. 40°C und rührt es ca. 12 Stunden bei dieser Temperatur. Bei ca 20°C werden dann 14,2 g einer 35%igen Hydrogenperoxid-Lösung dazu gegeben und die Mischung wird ca. 30 Minuten gerührt. Anschließend wird mit 300 ml Methylenchlorid verrührt, die organische Phase abgetrennt, mit Wasser gewaschen, mit Natriumsulfat getrocknet und filtriert. Vom Filtrat wird das Lösungsmittel im Wasserstrahlvakuum sorgfältig abdestilliert.

Man erhält 65,9 g (90% der Theorie) 2-Isopropoxy-6-methyl-benzolsulfochlorid als braunstichigen öligen Rückstand.

¹H-NMR (CDCl₃, TMS, δ ppm): 1,47 (d, J=6,1 Hz, 2xCH₃); 2,68 (s, CH3); 4,79 (sept., J=6,1 Hz, 1H), 6,83 (d, J=7,5 Hz, 1H); 6,95 (d, J=8,4 Hz, 1H); 7,45 (pseudo t, J=8,3 Hz, 1H).

Analog Beispiel (VI-1) können beispielsweise auch die in der nachstehenden Tabelle 3 aufgeführten Verbindungen der Formel (VI) bzw (VIa) hergestellt werden. 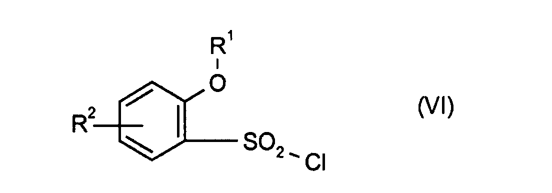 Schalterargument nicht angegeben.

**Tabelle 3:**

| Beispiele für die Verbindungen der Formel (VI) | | | |
|---|---|---|---|
| Bsp.-Nr. | R¹ | (Position-) R2 | Physikal. Daten |
| VI-2 | CH₃ | (6-) CH₃ | Fp.: 52°C |
| | | | |
| VI-3 | C₂H₅ | (6-) CH₃ | ¹H-NMR (CDCl₃, TMS, δ, ppm): 1,55 (t, J=6,97 Hz, CH₃), 2,69 (s, CH₃), 4,24 (q, J=6,97 Hz, CH₂), 6,87 (d, J=7,68 Hz, 1H), 6,95 (d, J=8,34 Hz, 1H), 7,46 (pseudo t, J=8,1 Hz, 1H) |
| | | | |
| VI-4 | n-C₃H₇ | (6-) CH₃ | ¹H-NMR (CDCl₃, TMS, δ, ppm): 1,33 (t, J=7,38 Hz, CH₃), 1,95 (m, CH₂), 2,69 (s, CH₃), 4,12 (t, J=6,3 Hz, CH₂), 6,86 (d, J=7,69 Hz, 1H), 6,94 (d, J=8,37 Hz, 1H), 7,46 (pseudo t, J=7,8 Hz, 1H) |
| VI-5 | -CH₂CH₂Cl | (6-) CH₃ | ¹H-NMR (CDCl₃, TMS, δ, ppm): 2,71 (s, CH₃), 3,94 (t, J=6,1 Hz, CH₂), 4,41 (t, J=6,1 Hz, CH₂), 6,96 (t, J=7,1 Hz, 2H), 7,5 (t, J=7,8 Hz, 1H) |
| | | | |
| VI-6 | -CH₂CH₂OC₂H₅ | (6-) CH₃ | ¹H-NMR (CDCl₃, TMS, δ, ppm): 1,23 (t, J=7 Hz, CH₃), 2,69 (s, CH₃), 3,65 (q, J=7 Hz, CH₂), 3,91 (t, J=5,16 Hz, CH₂), 4,30 (t, J=5,16 Hz, CH₂), 6,89 (d, J=7,7 Hz, 1H), 7,0 (d, J=8,3 Hz, 1H), 7,47 (pseudo t, J=8,1 Hz, 1H) |
| | | | |
| VI-7 | C₂H₅ | (5-)CH₃ | ¹H-NMR (CDCl₃, TMS, δ, ppm): 1,53 (t, J=7Hz, CH₃), 2,36 (s, CH₃), 4,25 (q, J=7 Hz, CH₂), 7,0 (d, J=8,53 Hz, 1H), 7,45 (d, J1=8,53 Hz, J2=2,15 Hz, 1H), 7,75 (d, J=2,15 Hz, 1H) |
| | | | |
| VI-8 | n-C₃H₇ | (5-)CH₃ | ¹H-NMR (CDCl₃, TMS, δ, ppm): 1,08 (t, J=7,38 Hz, CH₃), 1,85 (m, CH₂), 2,23 (s, CH₃), 3,99 (t, J=6,5 Hz), 6,74 (d, J=8,2 Hz, 1H), 6,92 (m, 1H), 7,34 (d, J=1,65 Hz, 1H) |
| | | | |
| VI-9 | i-C₃H₇ | (5-)CH₃ | ¹H-NMR (CDCl₃, TMS, δ, ppm): 1,45 (d, J=6,06, 2xCH₃), 2,35 (s, CH₃), 4,77 (sept., J=6,06 Hz, 1H), 6,99 (d, J=8,57 Hz, 1H), 7,43 (dd, J1=8,56 Hz, 1H, J2=2,1 Hz, 1H), 7,74 (d, J=2,1 Hz, 1H) |
| VI-10 | C₂H₅ | (6-)CH₂Cl | (Öl) |
| | | | |
| VI-11 | -CF₂CHFCl | (6-)CH₃ | ¹H-NMR (CDCl₃, TMS, δ, ppm): 2,78 (s, CH₃), 6,46 (td, CHFCl), 7,2-7,6 (Ar-H) |
| | | | |
| VI-12 | CHF₂ | (6-)CH₃ | ¹H-NMR (CDCl₃, TMS, δ, ppm): 2,76 (s, CH₃), 6,61 (t, CHF₂), 7,27-7,59 (Ar-H) |
| | | | |
| VI-13 | CH₃ | (5-)C(CH3)3 | Fp.: 62°C |
| | | | |
| VI-14 | CHF₂ | (4-)CH₃ | ¹H-NMR (CDCl₃, TMS, δ, ppm): 2,50 (s, CH₃), 6,68 (t, CHF₂), 7,05-7,92 (Ar-H) |
| | | | |
| VI-15 | CHF₂ | (5-)CH₃ | ¹H-NMR (CDCl₃, TMS, δ, ppm): 2,45 (s, CH₃), 6,64 (t, CHF₂), 7,35-7,86 (AR-H) |
| | | | |
| VI-16 | -CH₂CH₂Cl | (6-)CH₃ | |
| | | | |
| VI-17 | -CH₂CH=CH₂ | (6-)CH₃ | |
| | | | |
| VI-18 | -CH₂C≡CH | (6-)CH₃ | |
| | | | |
| VI-19 | -CH₂C₆H₅ | (6-)CH₃ | |

### Ausganusstoffe der Formel (X):

### Beispiel (X-1)

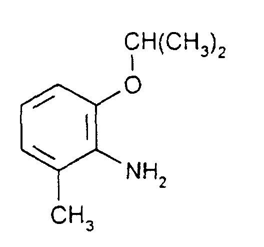

### Stufe 1: Herstellung von 2-Isopropoxy-6-methyl-nitrobenzol

Eine Mischung aus 153 g (1,0 Mol) 3-Methyl-2-nitro-phenol, 172,5 g (1,25 Mol) Kaliumcarbonat, 170 g (1,0 Mol) 2-Iod-propan und 400 ml Aceton wird 12 Stunden unter Rückfluß erhitzt. Anschließend wird im Wasserstrahlvakuum eingeengt, der Rückstand mit 400 ml MethyIenchlorid verrührt, filtriert und mit Methylenchlorid nachgewaschen. Vom Filtrat wird das Lösungsmittel im Wasserstrahlvakuum sorgfältig abdestilliert.
Man erhält 183,4 g 2-Isopropoxy-6-methyl-nitrobenzol als gelben öligen Rückstand.

¹H-NMR (CDCl₃, TMS, δ, ppm): 1,33 (d, J=6,1 Hz, 2xCH₃), 2,28 (s, CH₃), 4,6 (sept., J=6,1 Hz, 1H), 6,8 (d, J=7,7 Hz, 1H), 6,87 (d, J=8,4 Hz, 1H), 7,26 (pseudo t, J=8,1 Hz, 1H),

### Stufe 2: Herstellung von 2-Isopropoxy-6-methyl-anilin

183,3 g (0,94 Mol) 2-lsopropoxy-6-methyl-nitrobenzol werden in 1 Liter Essigsäureethylester in Gegenwart von 9,5 g Raney-Nickel unter einem WasserstoffDruck von 40 bis 60 bar 5 Stunden lang hydriert. Dann wird filtriert und vom Filtrat das Lösungsmittel im Wasserstrahlvakuum sorgfältig abdestilliert.
Man erhält 139,4 g (90% der Theorie) 2-Isopropoxy-6-methyl-anilin als orangefarbenen öligen Rückstand.

¹H-NMR (CDCl₃, TMS, δ, ppm): 1,36 (d, J=6,1 Hz, 2xCH₃), 2,16 (s, CH₃), 3,72 (s, NH₂), 4,51 (sept., J=6,1 Hz, 1H), 6,65-6,70 (m, 3H).

### Anwendungsbeispiele:

In den Anwendungsbeispielen werden die nachstehend genannten Verbindungen als Vergleichssubstanzen verwendet: 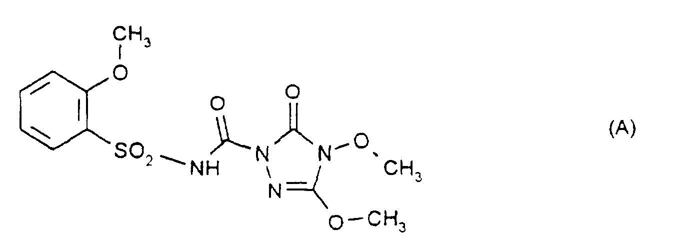 4,5-Dimethoxy-2-(2-methoxy-phenylsulfonylaminocarbonyl)-2,4-dihydro-3H-1,2,4-triazol-3-on (bekannt aus EP 534266); 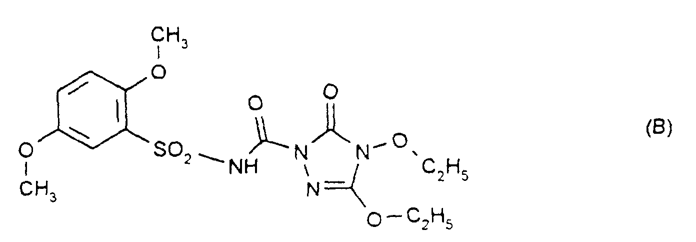 4,5-Diethoxy-2-(2,5-dimethoxy-phenylsulfonylaminocarbonyl)-2,4-dihydro-3H-1,2,4-triazol-3-on (bekannt aus EP 534266)

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät, Nach ca. 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle. Es bedeuten:
- 0 % =: keine Wirkung (wie unbehandelte Kontrolle)
- 100 % =: totale Vernichtung

In diesem Test zeigen beispielsweise die Verbindungen gemäß den Herstellungsbeispielen 1, 7-21, 23-41, 46-49, 51, 54, 55, 57, 60, 65, 68, 72-74, 78, 79, 88, 89, 199, 207, 209, 222 und 901 sehr starke Wirkung gegen Unkräuter (vgl. Tabelle A).

**Tabelle A:**

| Pre-emergence-Test/Gewächshaus | | | | | | | | |
|---|---|---|---|---|---|---|---|---|
| Wirkstoff gemäß Herstellungs-Bsp.-Nr. | Aufwandmenge (g ai./ha) | Agropyron | Lolium | Abutilon | Datura | Ipomoea | Sinapis | Viola |
| (A) | 500 | 0 | 0 | 0 | 0 | 0 | 40 | 0 |
| (B) | 250 | 0 | 60 | 0 | 30 | 0 | 60 | 40 |
| 1 | 250 | 95 | 95 | 95 | 80 | 95 | 95 | 95 |
| 7 | 250 | 95 | 95 | 100 | 80 | 95 | 95 | 90 |
| 8 | 250 | 95 | 95 | 90 | 90 | 95 | 95 | 90 |
| 9 | 250 | 95 | 95 | 95 | 95 | 95 | 95 | 95 |
| 10 | 250 | 95 | 95 | 95 | 70 | 95 | 95 | 95 |
| 11 | 250 | 90 | 90 | 90 | 70 | 80 | 95 | 90 |
| 12 | 250 | 95 | 95 | 95 | 95 | 95 | 95 | 95 |
| 13 | 250 | 95 | 95 | 95 | 80 | 80 | 95 | 90 |
| 14 | 250 | 95 | 95 | 95 | - | 90 | 95 | 95 |
| 15 | 250 | 95 | 95 | 95 | 95 | 95 | 95 | 95 |
| 16 | 125 | 95 | 95 | 95 | 80 | 95 | 95 | 95 |
| 17 | 250 | 95 | 95 | 95 | 80 | 90 | 95 | 95 |
| 18 | 125 | 95 | 95 | 70 | 70 | 90 | 90 | 70 |
| 19 | 125 | 95 | 95 | 95 | 90 | 95 | 95 | 90 |
| 20 | 125 | 95 | 95 | 95 | 70 | 90 | 95 | 95 |
| 21 | 125 | 95 | 95 | 95 | - | 80 | 95 | 95 |
| 23 | 125 | 95 | 95 | 95 | 95 | 80 | 95 | 95 |
| 24 | 125 | 95 | 95 | 100 | 95 | 70 | 95 | 95 |
| 25 | 60 | 95 | 95 | 95 | 80 | 90 | 95 | 80 |
| 26 | 125 | 80 | 90 | 80 | 60 | 90 | 95 | 80 |
| 27 | 60 | 80 | 90 | 95 | - | 80 | 90 | 70 |
| 28 | 60 | 95 | 95 | 90 | 80 | 90 | 90 | 80 |
| 29 | 125 | 95 | 95 | 100 | - | 70 | 95 | 80 |
| 30 | 60 | 95 | 95 | 95 | 90 | - | 95 | 100 |
| 31 | 60 | 95 | 95 | 95 | 95 | 95 | 95 | 95 |
| 32 | 125 | 95 | 95 | 95 | 80 | 90 | 90 | 90 |
| 33 | 60 | 90 | 95 | 95 | 80 | - | 95 | 95 |
| 34 | 60 | 95 | 95 | 100 | - | - | 95 | 90 |
| 35 | 60 | 90 | 95 | 100 | 80 | 70 | 90 | 90 |
| 36 | 125 | 95 | 95 | 80 | 80 | 80 | 80 | 80 |
| 38 | 125 | 95 | 90 | 80 | 80 | 80 | 90 | 90 |
| 39 | 125 | 95 | 95 | 95 | 70 | 90 | 90 | 80 |
| 40 | 125 | 95 | 95 | 95 | 90 | 95 | 90 | 95 |
| 41 | 125 | 90 | 95 | 95 | - | 90 | 95 | 80 |
| 46 | 60 | 90 | 95 | 90 | 80 | - | 95 | 95 |
| 47 | 60 | 95 | 95 | 95 | 95 | 95 | 95 | 95 |
| 48 | 60 | 95 | 95 | 95 | 80 | 95 | 95 | 95 |
| 49 | 60 | 90 | 95 | 95 | - | - | 95 | 95 |
| 51 | 60 | 80 | 90 | 90 | - | - | 95 | 95 |
| 54 | 60 | 80 | 90 | 95 | - | - | 100 | 95 |
| 55 | 60 | 95 | 95 | 95 | 90 | 80 | 95 | 95 |
| 57 | 60 | 95 | 95 | 95 | 70 | 95 | 95 | 95 |
| 60 | 60 | 95 | 100 | 100 | 90 | 95 | 95 | 95 |
| 65 | 125 | 95 | 100 | 95 | 95 | 95 | 95 | 95 |
| 68 | 125 | 95 | 95 | 90 | - | 90 | 95 | - |
| 72 | 125 | 95 | 95 | 95 | 95 | 95 | 95 | 95 |
| 73 | 125 | 95 | 95 | 95 | 90 | 95 | 95 | 95 |
| 74 | 125 | 95 | 95 | 95 | 100 | 95 | 95 | 95 |
| 78 | 125 | 80 | 95 | 90 | 95 | 95 | 95 | 100 |
| 79 | 125 | 95 | - | 95 | 80 | 95 | 95 | 100 |
| 88 | 125 | 95 | 90 | 95 | 70 | 95 | 95 | 95 |
| 89 | 125 | 95 | 95 | 95 | 70 | 95 | 95 | 95 |
| 222 | 60 | 95 | 95 | 100 | 80 | - | 95 | 95 |
| 199 | 60 | 95 | 100 | 100 | 95 | 90 | 95 | 95 |
| 207 | 60 | 95 | 100 | - | 95 | 95 | 95 | 95 |
| 209 | 60 | 95 | 95 | - | 95 | 95 | 95 | 100 |
| 901 | 250 | - | 100 | 100 | 100 | 95 | - | 100 |

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Hertellung einer zweckmäßigen Wirkstoffzubereitung vermischt man 1 Gewichtsteil Wirkstoff mit der angegebenen Menge Losungsmittel, gibt die angegebene Menge Emulgator zu und verdünnt das Konzentrat mit Wasser auf die gewunschte Konzentration.

Mit der Wirkstoffzubereitung spritzt man Testpflanzen, welche eine Höhe von 5 - 15 cm haben, so, daß die jeweils gewunschten Wirkstoffmengen pro Flacheneinheit ausgebracht werden. Die Konzentration der Spritzbruhe wird so gewählt, daß in 2000 l Wasser/ha die jeweils gewunschten Wirkstoffmengen ausgebracht werden. Nach drei Wochen wird der Schädigungsgrad der Pflanzen bonitiert in % Schadigung im Vergleich zur Entwicklung der unbehandelten Kontrolle

Es bedeuten.
- 0 % =: keine Wirkung (wie unbehandelte Kontrolle)
- 100 % =: totale Vernichtung

In diesem Test zeigen beispielsweise die Verbindungen gemäß den Herstellungsbeispielen 1, 7-10, 12, 13, 15, 16, 17, 25, 30, 31, 38, 40, 41, 46 und 47 sehr starke Wirkung gegen Unkrauter (vgl. Tabelle B).

**Tabelle B:**

| Post emergence-Test/Gewächshaus | | | | | | | |
|---|---|---|---|---|---|---|---|
| Wirkstoff gemäß Herstellungs-Bsp.-Nr. | Aufwandmenge (g ai./ha) | Alopecurus | Lolium | Sorghum | Abutilon | Matricaria | Stellaria |
| (A) | 250 | 20 | 30 | 40 | 0 | 0 | 0 |
| 1 | 60 | 90 | 95 | 100 | 100 | 100 | 100 |
| 7 | 125 | 95 | 80 | 100 | 100 | 95 | 100 |
| 8 | 250 | 95 | 95 | 95 | 100 | 100 | 100 |
| 9 | 125 | 100 | 90 | 100 | 100 | 100 | 100 |
| 10 | 60 | 90 | 90 | 100 | 100 | 100 | 100 |
| 12 | 250 | 95 | 95 | 100 | 100 | 100 | 100 |
| 13 | 125 | 100 | 95 | 100 | 95 | 100 | 100 |
| 15 | 125 | 100 | 95 | 100 | 100 | 100 | 100 |
| 16 | 125 | 95 | 95 | 95 | 95 | 100 | 100 |
| 17 | 250 | 95 | 90 | 95 | 100 | 100 | 100 |
| 25 | 60 | - | 95 | 95 | 95 | 100 | 100 |
| 30 | 60 | 90 | 90 | 100 | 100 | 95 | 95 |
| 31 | 15 | 90 | 80 | 100 | 95 | 95 | 95 |
| 38 | 60 | 90 | - | 90 | 95 | 100 | 95 |
| 40 | 60 | 95 | 95 | 100 | 100 | 100 | 100 |
| 41 | 60 | 95 | 95 | 100 | 100 | 95 | 100 |
| 46 | 60 | 95 | 95 | 95 | 95 | 90 | 95 |
| 47 | 15 5 | 90 | 95 | 90 | 100 | 95 | 100 |

## Patentansprüche

1. Sulfonylamino(thio)carbonylverbindungen der allgemeinen Formel (I) 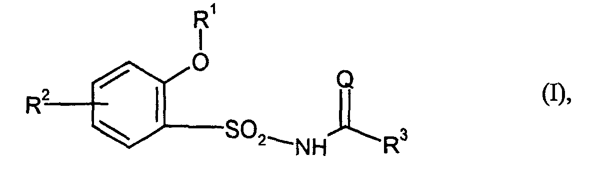 in welcher
Q für Sauerstoff oder Schwefel steht,
R¹ für Wasserstoff, Formyl, für jeweils gegebenenfalls durch Fluor, Chlor, Brom, Methoxy oder Ethoxy substituiertes Methyl, Ethyl, n- oder i-Propyl, n-, i- oder s-Butyl, Propenyl, Butenyl, Propinyl, Butinyl, Acetyl, Propionyl, Butyroyl, Methoxycarbonyl, Ethoxycarbonyl, n- oder i-Propoxycarbonyl, Methylsulfonyl, Ethylsulfonyl, n- oder i-Propylsulfonyl, n-, i-, s- oder t-Butylsulfonyl, oder für jeweils gegebenenfalls durch Fluor, Chlor oder Methyl substituiertes Cyclopropyl, Cyclopropylcarbonyl oder Cyclopropylsulfonyl steht,
R² für Cyano, Fluor, Chlor, Brom, oder für jeweils gegebenenfalls durch Fluor, Chlor, Methoxy oder Ethoxy substituiertes Methyl, Ethyl, n- oder i-Propyl, n-, i- oder s-Butyl, Propenyl, Butenyl, Propinyl, Butinyl, Methoxy, Ethoxy, n- oder i-Propoxy, n-, i- oder s-Butoxy, Propenyloxy, Butenyloxy, Propinyloxy oder Butinyloxy steht und
R³ für jeweils gegebenenfalls substituiertes Heterocyclyl der nachstehenden Formeln steht, 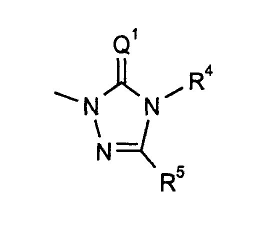 worin
Q¹ für Sauerstoff oder Schwefel steht sowie
R⁴ für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor, Methyl und/oder Ethyl substituiertes Cyclopropyl, Cyclobutyl, Cyclopentyl oder Cyclohexyl steht,
R⁵ für Wasserstoff, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Cyano, Methoxy oder Ethoxy substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl oder Cyclohexyl steht,
sowie Salze von Verbindungen der Formel (I).

2. Verbindungen der Formel (I) gemäß Anspruch 1, **dadurch gekennzeichnet, dass** darin
Q für Sauerstoff oder Schwefel steht,
R' für Methyl, Ethyl, n- oder i-Propyl steht,
R² für Chlor oder Methyl - jeweils in 5- oder 6-Position - steht und
R³ für gegebenenfalls substituiertes Triazolinyl der nachstehenden Formel steht, 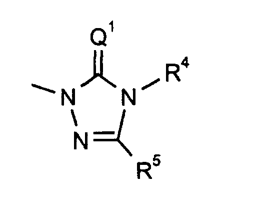
worin
Q¹ für Sauerstoff oder Schwefel steht sowie
R⁴ für jeweils gegebenenfalls durch Fluor, Chlor, Cyano, Methoxy oder Ethoxy substituiertes Methyl, Ethyl, n- oder i-Propyl oder für Cyclopropyl steht und
R⁵ für Wasserstoff, für jeweils gegebenenfalls durch Fluor, Chlor, Cyano, Methoxy oder Ethoxy substituiertes Methyl, Ethyl, n- oder i-Propyl, für jeweils gegebenenfalls durch Cyano, Methoxy oder Ethoxy substituiertes Methoxy, Ethoxy, n- oder i-Propoxy, Methylthio, Ethylthio, n- oder i-Propylthio oder Cyclopropyl steht.

3. Verfahren zur Herstellung von Verbindungen der Formel (I) gemäß Anspruch 1, **dadurch gekennzeichnet, dass** man
(a) Aminosulfonylverbindungen der allgemeinen Formel (II) 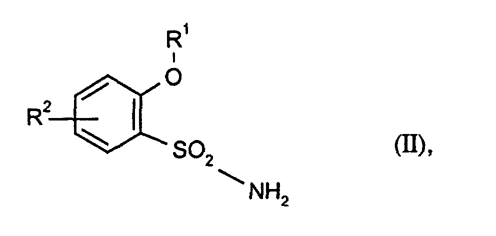 in welcher
R¹ und R² die in Anspruch 1 angegebenen Bedeutungen haben,
mit (Thio)Carbonsäurederivaten der allgemeinen Formel (III) 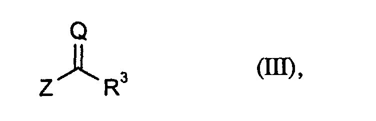 in welcher
Q und R³ die in Anspruch 1 angegebenen Bedeutungen haben und
Z für Halogen, Alkoxy, Aryloxy oder Arylalkoxy steht,
gegebenenfalls in Gegenwart eines Säureakzeptors und gegebenenfalls in Gegenwart eines Verdünnungsmittels umsetzt,
oder dass man
(b) Sulfonyliso(thio)cyanate der allgemeinen Formel (IV) 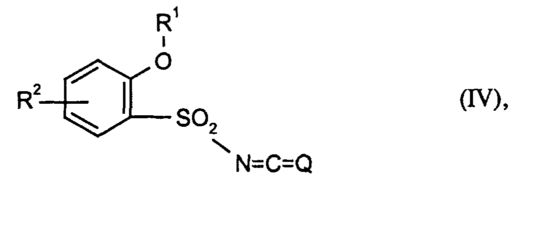 in welcher
Q, R¹ und R² die oben angegebenen Bedeutungen haben,
mit Heterocyclen der allgemeinen Formel (V)
H-R³ (V)
in welcher
R³ die oben angegebene Bedeutung hat,
gegebenenfalls in Gegenwart eines Reaktionshilfsmittels und gegebenenfalls in Gegenwart eines Verdünnungsmittels umsetzt,
oder dass man
(c) Chlorsulfonylverbindungen der allgemeinen Formel (VI) 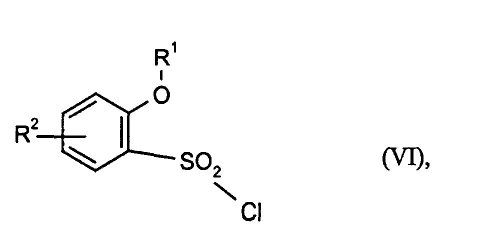 in welcher
R¹ und R² die oben angegebenen Bedeutungen haben,
mit Heterocyclen der allgemeinen Formel (V)
H-R³ (V)
in welcher
R³ die oben angegebene Bedeutung hat,
und Metall(thio)cyanaten der allgemeinen Formel (VII)
MQCN (VII)
in welcher
Q die oben angegebene Bedeutung hat und
M für ein Alkalimetall oder Erdalkalimetall-Äquivalent steht,
gegebenenfalls in Gegenwart eines Reaktionshilfsmittels und gegebenenfalls in Gegenwart eines Verdünnungsmittels umsetzt,
oder dass man
(d) Chlorsulfonylverbindungen der allgemeinen Formel (VI) 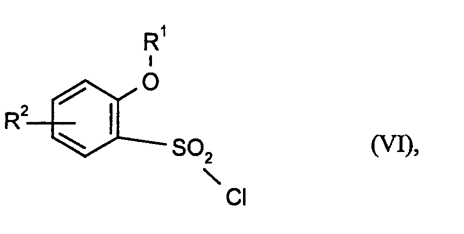 in welcher
R¹ und R² die oben angegebenen Bedeutungen haben,
mit (Thio)Carbonsäureamiden der allgemeinen Formel (VIII) 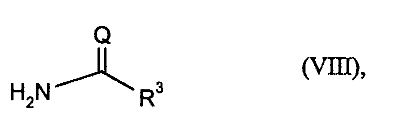 in welcher
Q und R³ die oben angegebenen Bedeutungen haben,
gegebenenfalls in Gegenwart eines Säureakzeptors und gegebenenfalls in Gegenwart eines Verdünnungsmittels umsetzt,
oder dass man
(e) Sulfonylamino(thio)carbonylverbindungen der allgemeinen Formel (IX) 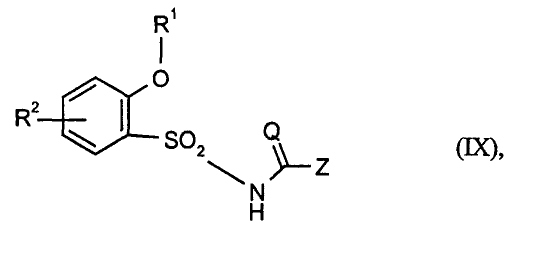
in welcher
Q, R¹ und R² die oben angegebenen Bedeutungen haben und
Z für Halogen, Alkoxy, Aryloxy oder Arylalkoxy steht,
mit Heterocyclen der allgemeinen Formel (V)
H-R³ (V)
in welcher
R³ die oben angegebene Bedeutung hat,
gegebenenfalls in Gegenwart eines Säureakzeptors und gegebenenfalls in Gegenwart eines Verdünnungsmittels umsetzt,
und gegebenenfalls die nach den Verfahren (a), (b), (c), (d) oder (e) erhaltenen Verbindungen der Formel (I) nach üblichen Methoden in Salze überführt.

4. Herbizide Mittel, **gekennzeichnet durch** einen Gehalt an mindestens einer Verbindung der Formel (I) oder einem ihrer Salze gemäß einem der Ansprüche 1 oder 2.

5. Verwendung von Verbindungen der allgemeinen Formel (I) oder deren Salzen gemäß einem der Ansprüche 1 oder 2 zur Bekämpfung von unerwünschtem Pflanzenwachstum.

6. Verfahren zur Bekämpfung von Unkräutern, **dadurch gekennzeichnet, dass** man Verbindungen der allgemeinen Formel (I) oder deren Salze gemäß einem Ansprüche 1 oder 2 auf die Unkräuter oder ihren Lebensraum einwirken lässt.

7. Verfahren zur Herstellung von herbiziden Mitteln, **dadurch gekennzeichnet, dass** man Verbindungen der allgemeinen Formel (I) oder deren Salze gemäß einem der Ansprüche 1 oder 2 mit Streckmitteln und/oder oberflächenaktiven Mitteln vermischt.

## Claims

1. Sulphonylamino(thio)carbon yl compounds of the general formula (I), 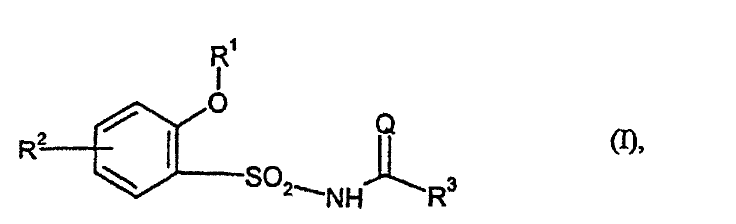 in which
Q represents oxygen or sulphur,
R¹ represents hydrogen or formyl, or represents in each case optionally fluoro-, chloro-, bromo-, methoxy- or ethoxy-substituted methyl, ethyl, n- or i-propyl, n-, i- or s-butyl, propenyl, butenyl, propinyl, butinyl, acetyl, propionyl, butyroyl, methoxycarbonyl, ethoxycarbonyl, n- or i- propoxycarbonyl, methylsulphonyl, ethylsulphonyl, n- or i- propylsulphonyl, n-, i-, s- or t-butylsulphonyl, or represents in each case optionally fluoro-, chloro- or methyl-substituted cyclopropyl, cyclopropylcarbonyl or cyclopropylsulphonyl,
R² represents cyano, fluoro, chloro or bromo, or represents in each case optionally fluoro-, chloro-, methoxy- or ethoxy-substituted methyl, ethyl, n- or i-propyl, n-, i- or s-butyl, propenyl, butenyl, propinyl, butinyl, methoxy, ethoxy, n- or i-propoxy, n-, i- or s-butoxy, propenyloxy, butenyloxy, propinyloxy or butinyloxy and
R³ represents in each case optionally substituted heterocyclyl of the formula below, 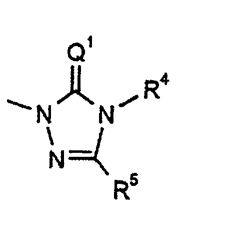
in which
Q¹ represents oxygen or sulphur, and
R⁴ represents in each case optionally fluoro-, chloro-, cyano-, methoxy- or ethoxy-substituted methyl, ethyl, n- or i-propyl, n-, i-, s- or t-butyl, or represents in each case optionally fluoro-, chloro-, methyl- and/or ethyl- substituted cyclopropyl, cyclobutyl, cyclopentyl or cyclohexyl,
R⁵ represents hydrogen, or represents in each case optionally fluoro-, chloro-, cyano-, methoxy- or ethoxy-substituted methyl, ethyl, n- or i- propyl, n-, i-, s- or t-butyl, or represents in each case optionally cyano-, methoxy- or ethoxy-substituted methoxy, ethoxy, n- or i-propoxy, n-, i-, s- or t-butoxy, methylthio, ethylthio, n- or i-propylthio, n-, i-, s- or t-butylthio, methylamino, ethylamino, n- or i-propylamino, n-, i-, s- or t-butylamino, or represents dimethylamino, diethylamino or dipropylamino, or represents in each case optionally fluoro-, chloro-, methyl- and/or ethyl-substituted cyclopropyl, cyclobutyl, cyclopentyl or cyclohexyl,
and salts of compounds of the formula (I).

2. Compounds of the formula (I) according to Claim 1, **characterized in that** in these compounds
Q represents oxygen or sulphur,
R¹ represents methyl, ethyl, n- or i-propyl,
R² represents chloro or methyl - in each case in position 5 or 6 - and
R³ represents optionally substituted triazolinyl of the formula below, 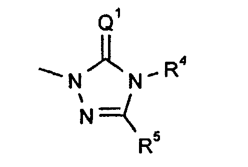
in which
Q¹ represents oxygen or sulphur, and
R⁴ represents in each case optionally fluoro-, chloro-, cyano-, methoxy- or ethoxy-substituted methyl, ethyl, n- or i-propyl, or represents cyclopropyl, and
R⁵ represents hydrogen, or represents in each case optionally fluoro-, chloro-, cyano-, methoxy- or ethoxy-substituted methyl, ethyl, n- or i-propyl, or represents in each case optionally cyano-, methoxy- or ethoxy- substituted methoxy, ethoxy, n- or i-propoxy, methylthio, ethylthio, n- or i-propylthio, or cyclopropyl.

3. Processes for the preparation of compounds of the formula (I) according to Claim 1, **characterized in that**
(a) aminosulphonyl compounds of the general formula (II) 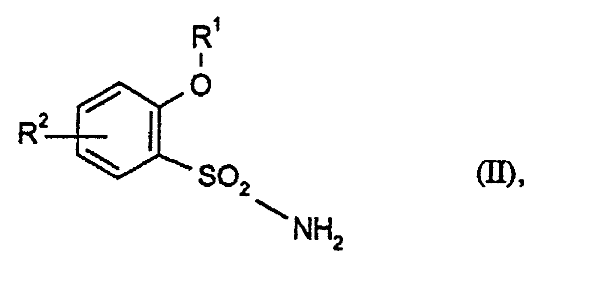 in which
R¹ and R² have the meanings given in Claim 1
are reacted with (thio)carboxylic acid derivatives of the general formula (III) 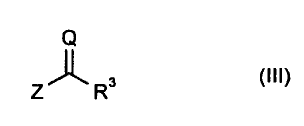 in which
Q and R³ have the meanings given in Claim 1 and
Z represents halogen, alkoxy, aryloxy or arylalkoxy,
optionally in the presence of an acid acceptor and optionally in the presence of a diluent,
or **in that**
(b) sulphonyl iso(thio)cyanates of the general formula (IV) 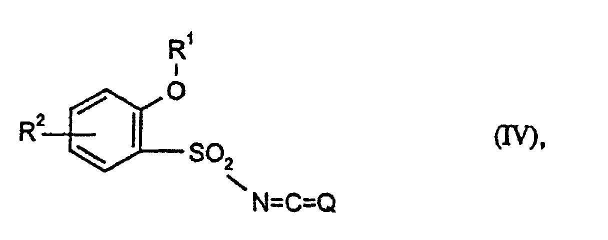 in which
Q, R¹ and R² have the meanings given above
are reacted with heterocycles of the general formula (V)
H-R³ (V)
in which
R³ has the meaning given above,
optionally in the presence of a reaction auxiliary and optionally in the presence of a diluent,
or **in that**
(c) chlorosulphonyl compounds of the general formula (VI) 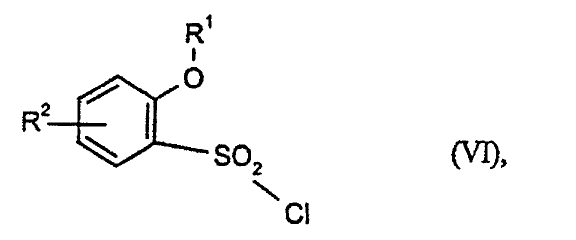 in which
R¹ and R² have the meanings given above
are reacted with heterocycles of the general formula (V)
H-R³ (V)
in which
R³ has the meaning given above
and metal (thio)cyanates of the general formula (VII)
MQCN (VII)
in which
Q has the meaning given above, and
M represents an alkali metal or alkaline earth metal equivalent,
optionally in the presence of a reaction auxiliary and optionally in the presence of a diluent,
or **in that**
(d) chlorosulphonyl compounds of the general formula (VI) 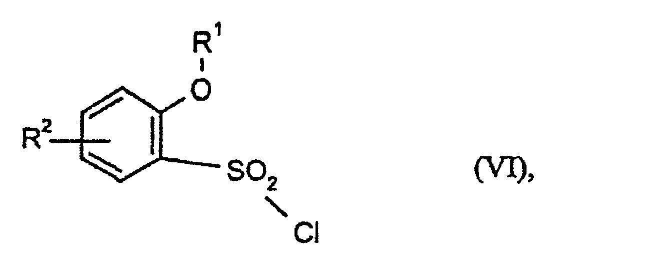 in which
R¹ and R² have the meanings given above
are reacted with (thio)carboxamides of the general formula (VIII) 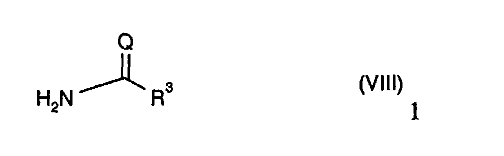 in which
Q and R³ have the meanings given above,
optionally in the presence of an acid acceptor and optionally in the presence of a diluent,
or **in that**
(e) sulphonylamino(thio)carbonyl compounds of the general formula (IX) 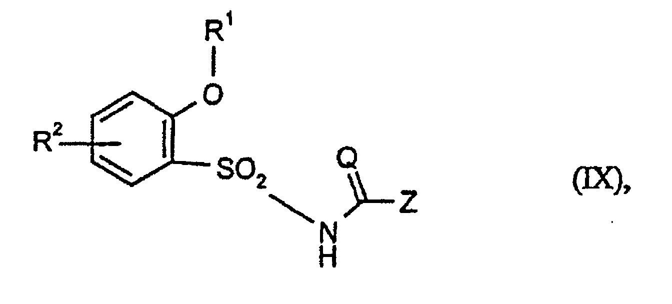 in which
Q, R¹ and R² have the meanings given above, and
Z represents halogen, alkoxy, aryloxy or arylalkoxy,
are reacted with heterocycles of the general formula (V)
H-R³ (V)
in which
R³ has the meaning given above,
optionally in the presence of an acid acceptor and optionally in the presence of a diluent,
and, if desired, the compounds of the formula (I) obtained by processes (a), (b), (c), (d) or (e) are converted into salts by customary methods.

4. Herbicidal compositions, **characterized by** a content of at least one compound of the formula (I) or one of its salts according to either of Claims 1 and 2.

5. Use of compounds of the general formula (I) or of salts thereof according to either of Claims 1 and 2 for combating unwanted plant growth.

6. Method of combating weeds, **characterized in that** compounds of the general formula (I) or salts thereof according to either of Claims 1 and 2 are caused to act on the weeds or their habitat.

7. Method of producing herbicidal compositions, **characterized in that** compounds of the general formula (I) or salts thereof according to either of Claims 1 and 2 are mixed with extenders and/or surface-active agents.

## Revendications

1. Composés sulfonylamino(thio)carbonyliques de formule générale (I) 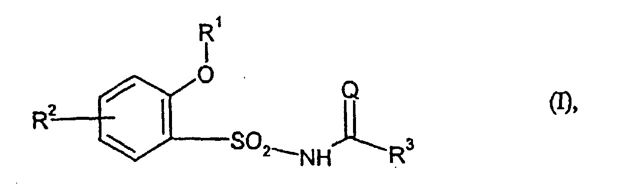 dans laquelle
Q représente l'oxygène ou le soufre,
R¹ est l'hydrogène, un reste formyle, un reste méthyle, éthyle, n-propyle, isopropyle, n-butyle, isobutyle, sec.-butyle, propényle, butényle, propynyle, butynyle, acétyle, propionyle, butyroyle, méthoxycarbonyle, éthoxycarbonyle, n-propoxycarbonyle, isopropoxycarbonyle, méthylsulfonyle, éthylsulfonyle, n-propylsulfonyle, isopropylsulfonyle, n-butylsulfonyle, isobutylsulfonyle, sec.-butylsulfonyle ou tertio-butylsulfonyle dont chacun porte éventuellement un substituant fluoro, chloro, bromo, méthoxy ou éthoxy, ou bien un reste cyclopropyle, cyclopropylcarbonyle ou cyclopropylsulfonyle dont chacun porte éventuellement un substituant fluoro, chloro ou méthyle,
R² est un groupe cyano, le fluor, le chlore, le brome ou un reste méthyle, éthyle, n-propyle, isopropyle, n-butyle, isobutyle, sec.-butyle, propényle, butényle, propynyle, butynyle, méthoxy, éthoxy, n-propoxy, isopropoxy, n-butoxy, isobutoxy, sec.-butoxy, propényloxy, butényloxy, propynyloxy ou butynyloxy dont chacun porte éventuellement un substituant fluoro, chloro, méthoxy ou éthoxy et
R³ est un reste hétérocyclyle éventuellement substitué dans chaque cas, de formule suivante 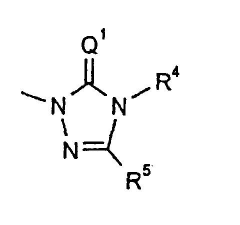
dans laquelle
Q¹ est l'oxygène ou le soufre,
R⁴ est un reste méthyle, éthyle, n-propyle, isopropyle, n-butyle, isobutyle, sec.-butyle ou tertio-butyle portant chacun, le cas échéant, un substituant fluoro, chloro, cyano, méthoxy ou éthoxy, un reste cyclopropyle, cyclobutyle, cyclopentyle ou cyclohexyle portant chacun, le cas échéant, un substituant fluoro, chloro, méthyle et/ou éthyle,
R⁵ représente l'hydrogène, un reste méthyle, éthyle, n-propyle, isopropyle, n-butyle, isobutyle, sec.-butyle ou tertio-butyle dont chacun porte éventuellement un substituant fluoro, chloro, cyano, méthoxy ou éthoxy, un reste méthoxy, éthoxy, n-propoxy, isopropoxy, n-butoxy, isobutoxy, sec.-butoxy, tertio-butoxy, méthylthio, éthylthio, n-propylthio, isopropylthio, n-butylthio, isobutylthio, sec.-butylthio, tertio-butylthio, méthylamino, éthylamino, n-propylamino, isopropylamino, n-butylamino, isobutylamino, sec.-butylamino ou tertio-butylamino dont chacun porte éventuellement un substituant cyano, méthoxy ou éthoxy, un reste diméthylamino, diéthylamino ou dipropylamino, un reste cyclopropyle, cyclobutyle, cyclopentyle ou cyclohexyle dont chacun porte éventuellement un substituant fluoro, chloro, méthyle et/ou éthyle,
ainsi que des sels de composés de formule (I).

2. Composés de formule (I) suivant la revendication 1, **caractérisés en ce que**
Q représente l'oxygène ou le soufre,
R¹ est un reste méthyle, éthyle, n-propyle ou isopropyle,
R² est le chlore ou un reste méthyle - chacun en position 5 ou 6 - et
R³ est un reste triazolinyle éventuellement substitué, de formule suivante 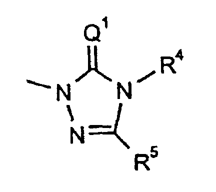
dans laquelle
Q¹ est l'oxygène ou le soufre,
R⁴ est un reste méthyle, éthyle, n-propyle ou isopropyle dont chacun porte éventuellement un substituant fluoro, chloro, cyano, méthoxy ou éthoxy, ou un reste cyclopropyle, et
R⁵ représente l'hydrogène, un reste méthyle, éthyle, n-propyle ou isopropyle dont chacun porte éventuellement un substituant fluoro, chloro, cyano, méthoxy ou éthoxy, un reste méthoxy, éthoxy, n-propoxy, isopropoxy, méthylthio, éthylthio, n-propylthio, isopropylthio ou cyclopropyle dont chacun porte éventuellement un substituant cyano, méthoxy ou éthoxy.

3. Procédé de production de composés de formule (I) suivant la revendication 1, **caractérisé en ce que** :
(a) des composés aminosulfonyliques de formule générale (II) 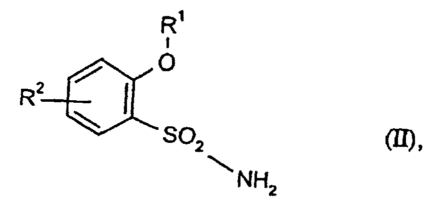 dans laquelle
R¹ et R² ont les définitions indiquées dans la revendication 1,
sont amenés à réagir avec des dérivés d'acides (thio)carbonyliques de formule générale (III) 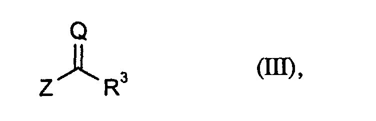 dans laquelle
Q et R³ ont les définitions indiquées dans la revendication 1, et
Z est un halogène, un reste alkoxy, aryloxy ou arylalkoxy,
le cas échéant, en présence d'un accepteur d'acide et en présence éventuelle d'un diluant,
ou **en ce que**
(b) des sulfonyliso(thio)cyanates de formule générale (IV) 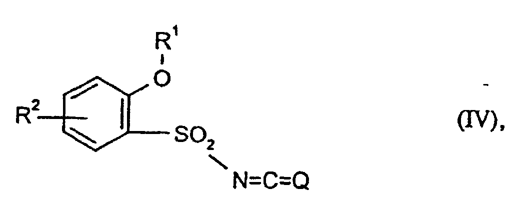 dans laquelle
Q, R¹ et R² ont les définitions indiquées ci-dessus,
sont amenés à réagir avec des hétérocycles de formule générale (V)
H-R³ (V)
dans laquelle
R³ a la définition indiquée ci-dessus,
le cas échéant, en présence d'un auxiliaire de réaction et en présence éventuelle d'un diluant,
ou **en ce que**
(c) des composés chlorosulfonyliques de formule générale (VI) 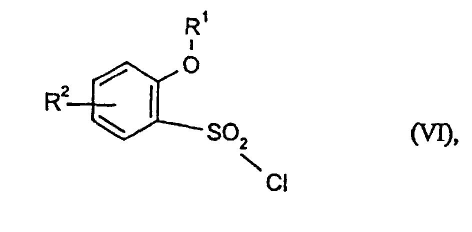 dans laquelle
R¹ et R² ont les définitions indiquées ci-dessus,
sont amenés à réagir avec des hétérocycles de formule générale (V)
H-R³ (V)
dans laquelle
R³ a la définition indiquée ci-dessus,
et des (thio)cyanates métalliques de formule générale (VII)
MQCN (VII)
dans laquelle
Q a la définition indiquée ci-dessus et
M est un métal alcalin ou un équivalent de métal alcalino-terreux,
le cas échéant, en présence d'un auxiliaire de réaction et en présence éventuelle d'un diluant,
ou **en ce que**
(d) des composés chlorosulfonyliques de formule générale (VI) 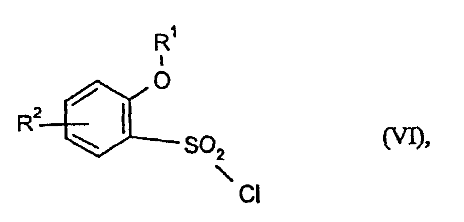 dans laquelle
R¹ et R² ont les définitions indiquées ci-dessus,
sont amenés à réagir avec des (thio)carboxamides de formule générale (VIII) 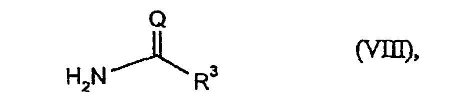 dans laquelle
Q et R³ ont les définitions indiquées ci-dessus,
ou **en ce que**
(e) des composés sulfonylamino(thio)carbonyliques de formule générale (IX) 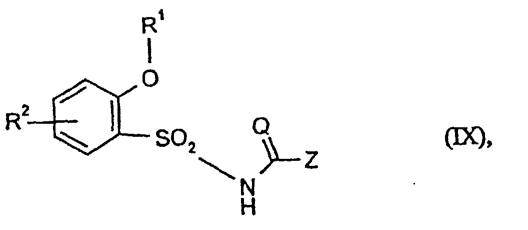
dans laquelle
Q, R¹ et R² ont les définitions indiquées ci-dessus et
Z est un halogène, un reste alkoxy, aryloxy ou arylalkoxy,
sont amenés à réagir avec des hétérocycles de formule générale (V)
H-R³ (V)
dans laquelle
R³ a la définition indiquée ci-dessus,
le cas échéant, en présence d'un accepteur d'acide et en présence éventuelle d'un diluant,
et les. composés de formule (I) obtenus selon les procédés (a), (b), (c), (d) ou (e) sont éventuellement convertis en sels selon des modes opératoires usuels.

4. Compositions herbicides, **caractérisées par** une teneur en au moins un composé de formule (I) ou en l'un de leurs sels suivant la revendication 1 ou 2.

5. Utilisation de composés de formule générale (I) ou de leurs sels suivant la revendication 1 ou 2 pour combattre une végétation indésirable.

6. Procédé pour combattre des mauvaises herbes, **caractérisé en ce qu'**on fait agir sur les mauvaises herbes ou sur leur milieu des composés de formule générale (I) ou leurs sels suivant la revendication 1 ou 2.

7. Procédé de préparation de compositions herbicides, **caractérisé en ce qu'**on mélange des composés de formule générale (I) ou leurs sels suivant la revendication 1 ou 2 avec des diluants et/ou des agents tensio-actifs.
